# EUROPEAN PATENT APPLICATION

(11) **EP 4 442 283 A1**
(43) Date of publication of application: **09.10.2024**
(21) Application number: 22900693.7
(22) Date of filing: 02.12.2022
(51) Int. Cl.: A61K 47/65, A61K 47/68, A61K 31/4745, A61K 39/395, A61P 35/00

(54) **ANTI-HUMAN TROP2 ANTIBODY-CAMPTOTHECIN DRUG CONJUGATE AND MEDICAL USE THEREOF**

(30) Priority: 03.12.2021 CN 202111469786
(71) Applicant: Systimmune, Inc., Redmond, WA 98052 (US)
(72) Inventor: ZHU, Yi, Chengdu, Sichuan 611130 (CN); ZHUO, Shi, Chengdu, Sichuan 611130 (CN); WAN, Weili, Chengdu, Sichuan 611130 (CN); ZHANG, Yong, Chengdu, Sichuan 611130 (CN); YU, Tianzi, Chengdu, Sichuan 611130 (CN); LI, Gangrui, Chengdu, Sichuan 611130 (CN)
(74) Representative: Colombo, Stefano Paolo
(86) International application number: PCT/CN2022/136278
(87) International publication number: WO 2023/098889

(57) **Abstract**

An antibody targeting human Trop2 simultaneously is coupled to a camptothecin drug to form an antibody-drug conjugate with stable treatment and excellent uniformity, and the drug-to-antibody ratio (DAR) is 6.0-8.0. The antibody-drug conjugate has a structure shown in general formula **I**, wherein Ab refers to an antibody targeting Trop simultaneously, which is coupled to a linker-camptothecin drug. In addition, also disclosed are a preparation and purification method for the antibody-drug conjugate, and an application in tumor treatment. Further, also disclosed is a linker-drug compound that can be coupled to Ab to form an antibody-drug conjugate.

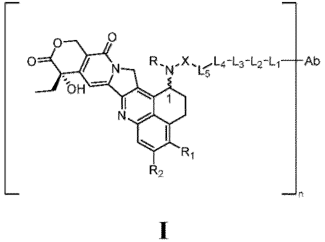

## Description

### TECHNICAL FIELD

The present invention relates to the field of biopharmaceuticals, and in particular, to an antibody-drug conjugate formed by an anti-human Trop2 antibody and a camptothecin drug, as well as a preparation method and use of the antibody-drug conjugate. The present invention also relates to a linker-drug compound that can be conjugated to Ab to form an antibody-drug conjugate.

### BACKGROUND

Human trophoblast cell surface antigen 2 (hTROP2) is a single-transmembrane type I cell membrane protein encoded by the TACSTD2 gene. It was first discovered by M. Lipinski et al. during the study on normal human and cancerous trophoblast cells. During subsequent research, the hTROP2 molecule was also known as the tumor antigen GA733-1 that was recognized by the mouse monoclonal antibody GA733 obtained by immunizing a gastric cancer cell line, as well as the epidermal glycoprotein that was recognized by the mouse monoclonal antibody RS7-3G11 obtained by immunizing a non-small cell lung cancer cell line. After the gene was cloned in 1995, it was confirmed that they were of the same molecule.

The hTROP2 protein has a full length of 323 amino acid residues, including an intracellular domain composed of 26 amino acid residues at the N terminus, an extracellular domain composed of 248 amino acid residues at the C terminus, and a transmembrane domain composed of 23 amino acid residues. The hTROP2 has four N-glycosylation sites at amino acid residues 33, 120, 168 and 208 of the extracellular domain. The TROP2 gene belongs to the TACSTD gene family and has about 50% homology with human trophoblast surface antigen 1 (hTROP1), another member of this gene family.

At present, the physiological ligands and molecular functions of the hTROP2 protein are still unclear, but it can regulate calcium signaling in tumor cells. The intracellular domain of hTROP2 has a PIP2 (phosphatidylinositol 4,5-bisphosphate) binding sequence. Protein kinase C of the Ca²⁺-dependent kinase can phosphorylate the serine residue 303. Phosphorylation of serine residue 303 causes PIP2 to be hydrolyzed by phospholipase C (PLC) into IP3 (inositol trisphosphate) and DAG (diacylglycerol), thereby regulating the process of intracellular calcium signaling.

Immunohistochemical analysis of clinical specimens shows that hTROP2 is only limitedly expressed in certain epithelial cells in normal tissues, but is overexpressed in a variety of epithelial cell cancers such as cervical cancer, breast cancer, gastric cancer, lung cancer, prostate cancer, and pancreatic cancer. Reports have shown that the expression level of hTROP2 is closely related to the invasive ability of tumors, the degree of malignancy and the poor prognosis of patients. In the meanwhile, hTROP2 can be used as a marker of prostate cancer stem cells during the initiation and development of tumors. Currently, using Trop-2 as a target for tumor therapy, anti-Trop-2 antibody-drug conjugates (ADC) are being used in the treatment of a variety of metastatic tumors, including triple-negative breast cancer (TNBC), non-small cell lung cancer, and small cell lung cancer. The American Society of Clinical Oncology (ASCO) announced relevant research on IMMU-132 in the treatment of triple-negative breast cancer in 2016. IMMU-132 is an antibody-drug conjugate that conjugates the monoclonal antibody RS7 to the drug SN-38. RS7 can specifically target hTROP2. hTROP2 has been found to be abundantly expressed in more than 80% of triple-negative breast cancer tumor cells.

Camptothecin (CPT) is a DNA topoisomerase I (TOPI) inhibitor. Camptothecin derivatives can inhibit TOPI and achieve anti-tumor effects. TOPI can catalyze the breakage and joining of single-stranded DNA during DNA translation and transcription to relax the DNA supercoil, thus promoting replication and transcription. The TOPI inhibitor SN-38 can cause DNA damage by blocking DNA chain reconnection, thereby inhibiting DNA replication and transcription and inducing cell apoptosis. In the clinical study of ADC targeting Trop-2 with humanized RS7 antibody, IMMU-132 uses a moderately stable linker. The half-life of IMMU-132 releasing SN-38 in human serum is about 24 h, and the elimination half-life in humans and mice is about 11 h. IMMU-132 has fast elimination rate and its mean residence time is about 15.4 h, which indicates that IMMU-132 will be administered more frequently in clinical treatment. In the meanwhile, during the use of topoisomerase inhibitors such as SN-38, IMMU-132 is often accompanied by serious side effects, such as diarrhea. The use of more stable linkers is expected to increase mean residence time and reduce drug use. Therefore, further research on safer and more effective Trop-2 targeting technology is urgent.

The ADC consists of three parts: an antibody, a cytotoxic small molecule drug, and a linker that connects the antibody and the drug. The small molecule drug is bound to the antibody protein through chemical coupling. The ADC guides the small molecule drug to a cancer cell target by antibody specific recognition, enters the cancer cell through endocytosis, and releases the cytotoxic drug to specifically kill the cancer cell. Clinical studies have proven that the ADC is relatively stable and highly effective in the blood, and can effectively reduce the toxicity of the cytotoxic small molecule drug to healthy tissues. ADCs are currently a hot topic in anti-cancer drug research. Trop-2, as a highly expressed protein specifically in a variety of epithelial cell cancers, is an excellent candidate target for the ADC.

### SUMMARY

Based on the comprehensive understanding of ADCs, the inventors disclose an anti-human Trop2 antibody-drug conjugate, a preparation method of the conjugate, a pharmaceutical composition comprising the conjugate, and use of the conjugate or the pharmaceutical composition. The present invention further relates to a linker-drug compound that can be coupled to an anti-human Trop2 antibody to form an antibody-drug conjugate.

A first aspect of the present invention discloses a ligand-camptothecin derivative conjugate shown in general formula **I** or a pharmaceutically acceptable salt or solvate thereof; 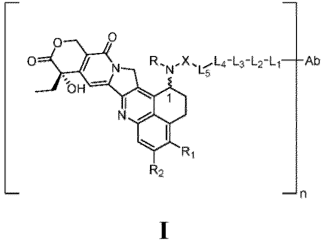 in the formula:
an Ab is an antibody targeting human Trop2 or an antigen-binding fragment thereof;
L₁ is selected, without limitation, from the group consisting of: 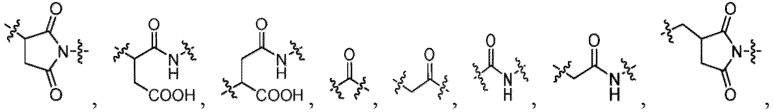 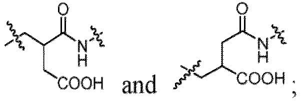
preferably, L₁ is 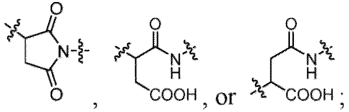
preferably, L₁ is 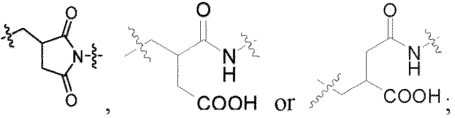
L₂ has a structure shown in formula A, 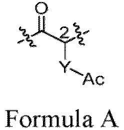
wherein Y is a scaffold, selected from the group consisting of C1-C6 alkyl, substituted C1-C6 alkyl and C3-C8 cycloalkyl; preferably Y is an C1-C6 alkyl; Ac is a hydrophilic structural unit; carbon No. 2 connected to the Y has an absolute chiral configuration R or S;
L₃ is present or absent, and when present, L₃ is selected from PEG hydrophilic units: 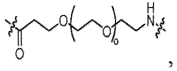 o is selected from integers between 1 and 10 (e.g., 1, 2, 3, 4, 5, 6, 7, 8, 9 or 10), preferably between 2 and 8;
L₄ is an enzyme-cleavable unit;
L₅ is a linking unit;
in formula **I**, chiral carbon atom No. 1 connected to N has an absolute chiral configuration R or S;
R is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, C6-C10 aryl, substituted C6-C10 aryl, 5- to 10-membered heteroaryl, and substituted 5- to 10-membered heteroaryl;
preferably, R is selected from the group of hydrogen atom and C1-C6 alkyl;
R₁ is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, carboxyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₁ is selected from the group of hydrogen atom or C1-C6 alkyl;
more preferably, R₁ is selected from C1-C6 alkyl;
R₂ is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, carboxyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₂ is selected from the group of hydrogen atom, halogen, and C1-C6 alkyl;
more preferably, R₂ is selected from halogen;
X is selected from the group consisting of -C(O)-CRₐR_{b}-(CR₃R₄)ₘ-O-, -C(O)-CRₐR_{b}-(CR₃R₄)ₘ-NH- and -C(O)-CRₐR_{b}-(CR₃R₄)ₘ-S-;
preferably, X is selected from -C(O)-CRₐR_{b}-(CR₃R₄)ₘ-O-;
Rₐ and R_{b} are each independently selected from the group consisting of hydrogen atom, deuterium atom, halogen, C1-C6 alkyl, deuterated C1-C6 alkyl, halogenated C1-C6 alkyl, C3-C8 cycloalkyl, C3-C8 cycloalkyl C1-C6 alkyl, C6-C10 aryl C1-C6 alkyl, C1-C6 alkoxy C1-C6 alkyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, Rₐ and R_{b} are each independently selected from the group consisting of hydrogen atom, C1-C6 alkyl, halogenated C1-C6 alkyl, C3-C8 cycloalkyl C1-C6 alkyl, and C6-C10 aryl C1-C6 alkyl;
alternatively, Rₐ, R_{b} and carbon atoms to which Rₐ and R_{b} are connected form C3-C8 cycloalkyl, C3-C8 cycloalkyl C1-C6 alkyl, 3- to 7-membered heterocyclyl, or substituted 3- to 7-membered heterocyclyl; preferably Rₐ, R_{b} and the carbon atoms to which Rₐ and R_{b} are connected form C3-C8 cycloalkyl;
R₃ and R₄ are the same or different, and are each independently hydrogen atom, deuterium atom, halogen, C1-C6 alkyl, halogenated C1-C6 alkyl, deuterated C1-C6 alkyl, C1-C6 alkoxy, hydroxyl, amino, cyano, nitro, hydroxyl C1-C6 alkyl, C3-C8 cycloalkyl, 3- to 7-membered heterocyclyl, or substituted 3- to 7-membered heterocyclyl;
preferably, R₃ and R₄ are each independently hydrogen atom or C1-C6 alkyl;
alternatively, R₃, R₄ and carbon atoms to which R₃ and R₄ are connected form C3-C8 cycloalkyl, C3-C8 cycloalkyl C1-C6 alkyl, 3- to 7-membered heterocyclyl, or substituted 3- to 7-membered heterocyclyl;
m is selected from integers between 0 and 4 (e.g., 0, 1, 2, 3 or 4), and preferably is 0 or 1; n is selected from integers between 1 and 10 (e.g., 1, 2, 3, 4, 5, 6, 7, 8, 9 or 10).

In some embodiments of the first aspect of the present invention, the ligand-camptothecin derivative conjugate shown in general formula **I** or a pharmaceutically acceptable salt or solvate thereof is disclosed, wherein the Ab is an antibody targeting TROP2 or an antigen-binding fragment thereof.

In some embodiments of the first aspect of the present invention, the ligand-camptothecin derivative conjugate shown in general formula **I** or a pharmaceutically acceptable salt or solvate thereof is disclosed, wherein the antibody of the Ab comprises: two IgG1 heavy chains; and two κ light chains.

In some embodiments of the first aspect of the present invention, the ligand-camptothecin derivative conjugate shown in general formula **I** or a pharmaceutically acceptable salt or solvate thereof is disclosed, wherein in the antibody of the Ab, the κ light chain comprises: CDRs as shown in SEQ ID NO: 7, SEQ ID NO: 8 and SEQ ID NO: 9, and the IgG1 heavy chain comprises CDRs as shown in SEQ ID NO: 1, SEQ ID NO: 2 and SEQ ID NO: 3.

In some embodiments of the first aspect of the present invention, the ligand-camptothecin derivative conjugate shown in general formula **I** or a pharmaceutically acceptable salt or solvate thereof is disclosed, wherein the antibody of the Ab comprises a heavy chain and a light chain, the light chain comprises CDRL1, CDRL2 and CDRL3 having nucleic acid coding sequences as shown in SEQ ID NO: 27, SEQ ID NO: 28, and SEQ ID NO: 29, and the heavy chain comprises CDRH1, CDRH2 and CDRH3 having nucleic acid coding sequences as shown in SEQ ID NO: 21, SEQ ID NO: 22, and SEQ ID NO: 23, respectively.

In some embodiments of the first aspect of the present invention, the ligand-camptothecin derivative conjugate shown in general formula **I** or a pharmaceutically acceptable salt or solvate thereof is disclosed, wherein the antibody of the Ab comprises a heavy chain and a light chain, the heavy chain comprises a VH having a sequence as shown in SEQ ID NO: 13 and the light chain comprises a VL having a sequence as shown in SEQ ID NO: 14.

In some embodiments of the first aspect of the present invention, the ligand-camptothecin derivative conjugate shown in general formula **I** or a pharmaceutically acceptable salt or solvate thereof is disclosed, wherein the antibody of the Ab comprises a heavy chain and a light chain, the heavy chain comprises a VH having a nucleic acid coding sequence as shown in SEQ ID NO: 33, and the light chain comprises a VL having a nucleic acid coding sequence as shown in SEQ ID NO: 34.

In some embodiments of the first aspect of the present invention, the ligand-camptothecin derivative conjugate shown in general formula **I** or a pharmaceutically acceptable salt or solvate thereof is disclosed, wherein in the antibody of the Ab, the heavy chain has an amino acid sequence as shown in SEQ ID NO: 17, and the light chain has an amino acid sequence as shown in SEQ ID NO: 18.

In some embodiments of the first aspect of the present invention, the ligand-camptothecin derivative conjugate shown in general formula **I** or a pharmaceutically acceptable salt or solvate thereof is disclosed, wherein in the antibody of the Ab, the heavy chain has a nucleic acid coding sequence as shown in SEQ ID NO: 37, and the light chain has a nucleic acid coding sequence as shown in SEQ ID NO: 38.

In some embodiments of the first aspect of the present invention, the ligand-camptothecin derivative conjugate shown in general formula **I** or a pharmaceutically acceptable salt or solvate thereof is disclosed, wherein the antibody of the Ab comprises: two IgG1 heavy chains; and two κ light chains.

In some embodiments of the first aspect of the present invention, the ligand-camptothecin derivative conjugate shown in general formula **I** or a pharmaceutically acceptable salt or solvate thereof is disclosed, wherein in the antibody of the Ab, the κ light chain comprises CDRs as shown in SEQ ID NO: 10, SEQ ID NO: 11 and SEQ ID NO: 12, and the IgG1 heavy chain comprises CDRs as shown in SEQ ID NO: 4, SEQ ID NO: 5 and SEQ ID NO: 6.

In some embodiments of the first aspect of the present invention, the ligand-camptothecin derivative conjugate shown in general formula **I** or a pharmaceutically acceptable salt or solvate thereof is disclosed, wherein the antibody of the Ab comprises a heavy chain and a light chain, the light chain comprises CDRL1, CDRL2 and CDRL3 having nucleic acid coding sequences as shown in SEQ ID NO: 30, SEQ ID NO: 31, and SEQ ID NO: 32, and the heavy chain comprises CDRH1, CDRH2 and CDRH3 having nucleic acid coding sequences as shown in SEQ ID NO: 24, SEQ ID NO: 25, and SEQ ID NO: 26, respectively.

In some embodiments of the first aspect of the present invention, the ligand-camptothecin derivative conjugate shown in general formula **I** or a pharmaceutically acceptable salt or solvate thereof is disclosed, wherein the antibody of the Ab comprises a heavy chain and a light chain, the heavy chain comprises a VH having a sequence as shown in SEQ ID NO: 15 and the light chain comprises a VL having a sequence as shown in SEQ ID NO: 16.

In some embodiments of the first aspect of the present invention, a ligand-camptothecin derivative conjugate shown in general formula **I** or a pharmaceutically acceptable salt or solvate thereof is disclosed, wherein the antibody of the Ab comprises a heavy chain and a light chain, the heavy chain comprises a VH having a nucleic acid coding sequence as shown in SEQ ID NO: 35, and the light chain comprises a VL having a nucleic acid coding sequence as shown in SEQ ID NO: 36.

In some embodiments of the first aspect of the present invention, the ligand-camptothecin derivative conjugate shown in general formula **I** or a pharmaceutically acceptable salt or solvate thereof is disclosed, wherein in the antibody of the Ab, the heavy chain has an amino acid sequence as shown in SEQ ID NO: 19, and the light chain has an amino acid sequence as shown in SEQ ID NO: 20.

In some embodiments of the first aspect of the present invention, the ligand-camptothecin derivative conjugate shown in general formula **I** or a pharmaceutically acceptable salt or solvate thereof is disclosed, wherein in the antibody of the Ab, the heavy chain has a nucleic acid coding sequence as shown in SEQ ID NO: 39, and the light chain has a nucleic acid coding sequence as shown in SEQ ID NO: 40.

In some embodiments of the first aspect of the present invention, the ligand-camptothecin derivative conjugate shown in general formula **I** or a pharmaceutically acceptable salt or solvate thereof is disclosed, wherein the X is selected, without limitation, from the following structures or isomers thereof: 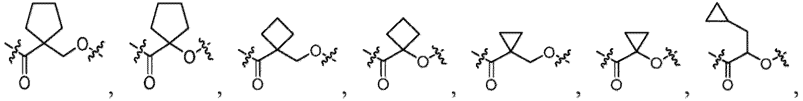 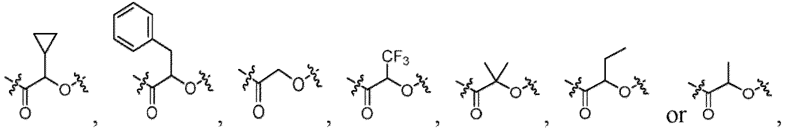 wherein a wavy line on the left is connected to a camptothecin derivative moiety, and a wavy line on the right is connected to L₅.

In some embodiments of the first aspect of the present invention, the ligand-camptothecin derivative conjugate shown in general formula **I** or a pharmaceutically acceptable salt or solvate thereof is disclosed, wherein L₄ is selected, without limitation, from peptide residues comprising an amino acid,
wherein optionally, the amino acid is further substituted with one or more substituents selected from the group consisting of deuterium atom, halogen, hydroxyl, cyano, amino, nitro, carboxyl, C1-C6 alkyl, substituted C1-C6 alkyl, C1-C6 alkoxy, and C3-C8 cycloalkyl or substituted C3-C8 cycloalkyl.
preferably, the peptide residue is a peptide residue composed of one, two or more amino acids selected from the group consisting of phenylalanine (F), glycine (G), valine (V), lysine (K), citrulline (C), serine (S), glutamic acid (E) or aspartic acid (D);
more preferably, the peptide residue is a tetrapeptide residue consisting of glycine (G)-glycine (G)-phenylalanine (F)-glycine (G).

Particularly preferably, the peptide residue is -GGFG-.

In some embodiments of the first aspect of the present invention, the ligand-camptothecin derivative conjugate shown in general formula **I** or a pharmaceutically acceptable salt or solvate thereof is disclosed, wherein
L₅ is selected, without limitation, from -NR₅(CR₆R₇)_{q}- and a chemical bond, q is selected from integers between 0 and 6 (e.g., 0, 1, 2, 3, 4, 5 or 6).

R₅, R₆ and R₇ are the same or different and each independently selected from the group consisting of hydrogen atom, deuterium atom, halogen, C1-C6 alkyl, substituted C1-C6 alkyl, deuterated C1-C6 alkyl, C3-C8 cycloalkyl, C3-C8 cycloalkyl C1-C6 alkyl, C1-C6 alkoxy C1-C6 alkyl, 3- to 7-membered heterocyclyl, substituted 3-to 7-membered heterocyclyl, C6-C10 aryl, substituted C6-C10 aryl, 5- to 10-membered heteroaryl, and substituted 5- to 10-membered heteroaryl;
preferably, R₅, R₆ and R₇ are each independently selected from hydrogen atom and C1-C6 alkyl;
more preferably, R₅, R₆ and R₇ are each independently selected from hydrogen atom.

In some embodiments, L₁ is selected, without limitation, from the group consisting of:

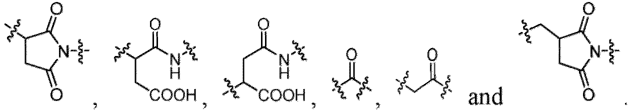

In some embodiments of the first aspect of the present invention, the ligand-camptothecin derivative conjugate shown in general formula **I** or a pharmaceutically acceptable salt or solvate thereof is disclosed, wherein the linking unit -L₁-L₂-L₃-L₄-L₅-is selected, without limitation, from the following structures: 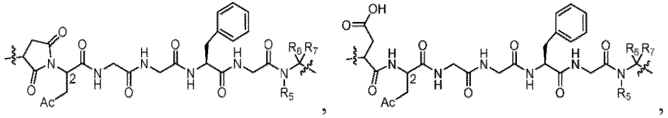 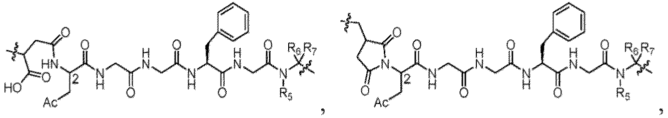 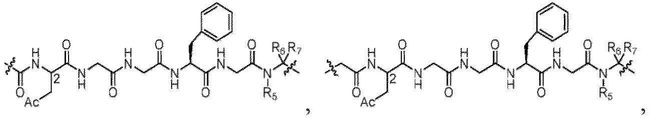 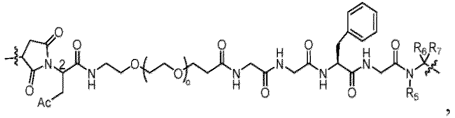 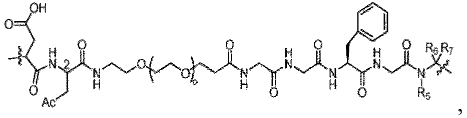 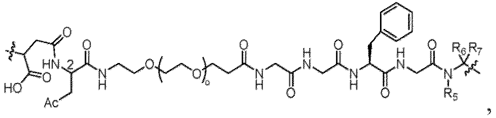 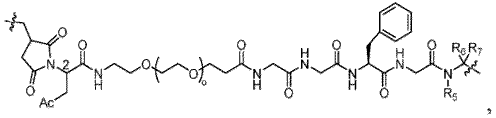 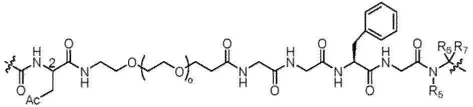 and 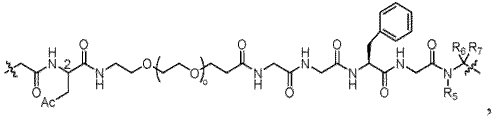 preferably, 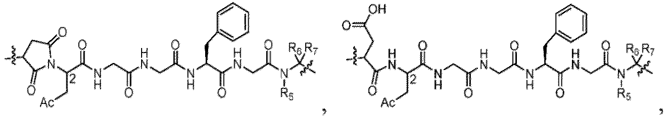 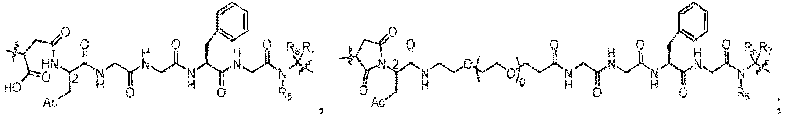 wherein
Ac is a hydrophilic structural unit;
R₅, R₆ and R₇ are the same or different and each independently selected from the group consisting of hydrogen atom, deuterium atom, halogen, C1-C6 alkyl, substituted C1-C6 alkyl, deuterated C1-C6 alkyl, C3-C8 cycloalkyl, C3-C8 cycloalkyl C1-C6 alkyl, C1-C6 alkoxy C1-C6 alkyl, 3- to 7-membered heterocyclyl, substituted 3-to 7-membered heterocyclyl, C6-C10 aryl, substituted C6-C10 aryl, 5- to 10-membered heteroaryl, and substituted 5- to 10-membered heteroaryl;
preferably, R₅, R₆ and R₇ are each independently selected from hydrogen atom or C1-C6 alkyl;
more preferably, R₅, R₆ and R₇ are each independently selected from hydrogen atom;
carbon atom No. 2 connected to N has an absolute chiral configuration R or S;
a wavy line on the left is connected to an antibody or an antigen-binding fragment of the antibody, and a wavy line on the right is connected to X;
o is selected from integers between 1 and 10 (e.g., 1, 2, 3, 4, 5, 6, 7, 8, 9 or 10).

A second aspect of the present invention discloses a ligand-camptothecin derivative conjugate shown in general formula **II** or a pharmaceutically acceptable salt or solvate thereof; 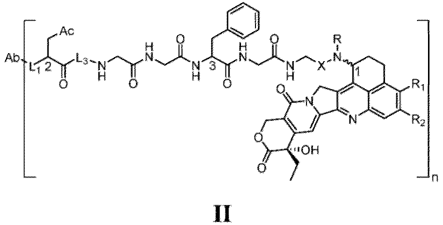 wherein
an Ab is an antibody targeting human Trop2 or an antigen-binding fragment thereof;
L₁ is a linking unit connected to the Ab and is selected, without limitation, from: 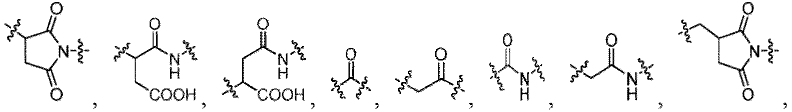 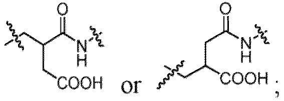
L₁ is preferably 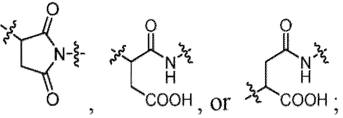
L₁ is preferably 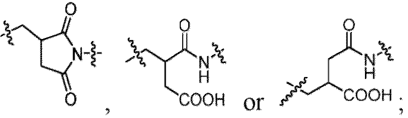
L₃ is present or absent, and when present, L₃ is selected from 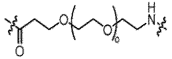 , and o is selected from integers between 1 and 10 (e.g., 1, 2, 3, 4, 5, 6, 7, 8, 9 or 10), preferably between 2 and 8;
the Ac is a hydrophilic structural unit;
chiral carbon atoms at positions 1, 2 and 3 have an absolute chiral configuration R or S;
R is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, C6-C10 aryl, substituted C6-C10 aryl, 5- to 10-membered heteroaryl, and substituted 5- to 10-membered heteroaryl;
preferably, R is selected from the group of hydrogen atom and C1-C6 alkyl;
R₁ is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, carboxyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₁ is selected from the group of hydrogen atom and C1-C6 alkyl;
more preferably, R₁ is selected from C1-C6 alkyl;
R₂ is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, carboxyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₂ is selected from the group of hydrogen atom, halogen, and C1-C6 alkyl;
more preferably, R₂ is selected from halogen;
X is selected from the group consisting of -C(O)-CRₐR_{b}-(CR₃R₄)ₘ-O-, -C(O)-CRₐR_{b}-(CR₃R₄)ₘ-NH- or -C(O)-CRₐR_{b}-(CR₃R₄)ₘ-S-;
preferably, X is selected from -C(O)-CRₐR_{b}-(CR₃R₄)ₘ-O-;
Rₐ and R_{b} are each independently selected from the group consisting of hydrogen atom, deuterium atom, halogen, C1-C6 alkyl, deuterated C1-C6 alkyl, halogenated C1-C6 alkyl, C3-C8 cycloalkyl, C3-C8 cycloalkyl C1-C6 alkyl, C1-C6 alkoxy C1-C6 alkyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, Rₐ and R_{b} are each independently selected from the group consisting of hydrogen atom, C1-C6 alkyl, halogenated C1-C6 alkyl, C3-C8 cycloalkyl C1-C6 alkyl, or C6-C10 aryl C1-C6 alkyl;
alternatively, Rₐ, R_{b} and carbon atoms to which Rₐ and R_{b} are connected form C3-C8 cycloalkyl, C3-C8 cycloalkyl C1-C6 alkyl, 3- to 7-membered heterocyclyl, or substituted 3- to 7-membered heterocyclyl; preferably Rₐ, R_{b} and the carbon atoms to which Rₐ and R_{b} are connected form C3-C8 cycloalkyl;
R₃ and R₄ are the same or different, and are each independently hydrogen atom, deuterium atom, halogen, C1-C6 alkyl, halogenated C1-C6 alkyl, deuterated C1-C6 alkyl, C1-C6 alkoxy, hydroxyl, amino, cyano, nitro, hydroxyl C1-C6 alkyl, C3-C8 cycloalkyl, 3- to 7-membered heterocyclyl, or substituted 3- to 7-membered heterocyclyl;
preferably, R₃ and R₄ are each independently hydrogen atom or C1-C6 alkyl;
alternatively, R₃, R₄ and carbon atoms to which R₃ and R₄ are connected form C3-C8 cycloalkyl, C3-C8 cycloalkyl C1-C6 alkyl, 3- to 7-membered heterocyclyl, or substituted 3- to 7-membered heterocyclyl;
m is selected from integers between 0 and 4 (e.g., 0, 1, 2, 3, or 4) and preferably is 0 or 1;
n is selected from integers between 1 and 10 (e.g., 1, 2, 3, 4, 5, 6, 7, 8, 9 or 10).

In some embodiments of the first and second aspects of the present invention, the ligand-camptothecin derivative conjugate or a pharmaceutically acceptable salt or solvate thereof is disclosed, wherein the Ac has a structure shown in formula B, 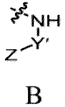 wherein
Z is selected, without limitation, from the group consisting of one or more of hydrophilic structural carboxyl, phosphoric acid, polyphosphoric acid, phosphorous acid, sulfonic acid, sulfinic acid and polyethylene glycol (PEG);
preferably, Z is selected from the group consisting of hydrophilic structure carboxyl, phosphoric acid and PEG;
Y' is an optional scaffold connecting -NH- and Z; preferably, Y' is a C1-C6 alkylene (e.g., methylene);
the Ac is connected to the labeled 2-position carbon in structural formula **I** through the scaffold Y.

In some embodiments of the first and second aspects of the present invention, the ligand-camptothecin derivative conjugate or a pharmaceutically acceptable salt or solvate thereof is disclosed, wherein the Ac is selected, without limitation, from the group consisting of glycine, (*D*/*L*) alanine, (*D*/*L*) leucine, (*D*/*L*) isoleucine, (*D*/*L*) valine, (*D*/*L*) phenylalanine, (*D*/*L*) proline, (*D*/*L*) tryptophan, (*D*/*L*) serine, (*D*/*L*) tyrosine, (*D*/*L*) cysteine, (*D*/*L*) cystine, (*D*/*L*) arginine, (*D*/*L*) histidine, (*D*/*L*) methionine, (*D*/*L*) asparagine, (*D*/*L*) glutamine, (*D*/*L*) threonine, (*D*/*L*) aspartic acid, (*D*/*L*) glutamic acid, natural or unnatural amino acid derivatives or the following structures or isomers thereof, 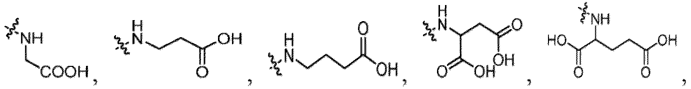 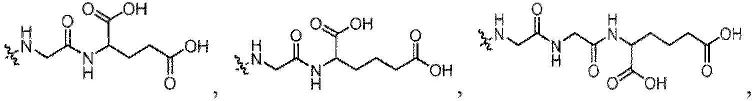 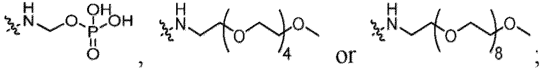 preferably

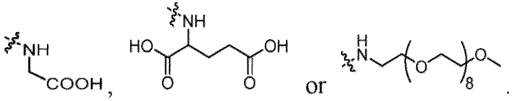

In some embodiments of the first and second aspects of the present invention, the ligand-camptothecin derivative conjugate or a pharmaceutically acceptable salt or solvate thereof is disclosed, wherein the Ac is selected, without limitation, from the group consisting of glycine, phosphoric acid, (D/L) glutamic acid and polyethylene glycol hydrophilic structure.

In some embodiments of the first and second aspects of the present invention, the ligand-camptothecin derivative conjugate or a pharmaceutically acceptable salt or solvate thereof is disclosed, wherein the 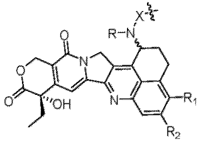 has a structure shown in formula d; 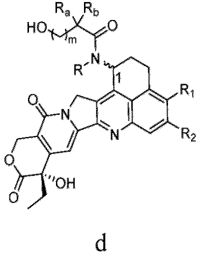 wherein
R is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, C6-C10 aryl, substituted C6-C10 aryl, 5- to 10-membered heteroaryl, and substituted 5- to 10-membered heteroaryl;
preferably, R is selected from the group of hydrogen atom and C1-C6 alkyl;
R₁ is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, carboxyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₁ is selected from the group of hydrogen atom and C1-C6 alkyl;
more preferably, R₁ is selected from C1-C6 alkyl;
R₂ is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, carboxyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₂ is selected from the group of hydrogen atom, halogen, and C1-C6 alkyl;
more preferably, R₂ is selected from halogen;
Rₐ and R_{b} are each independently selected from the group consisting of hydrogen atom, deuterium atom, halogen, C1-C6 alkyl, deuterated C1-C6 alkyl, halogenated C1-C6 alkyl, C3-C8 cycloalkyl, C3-C8 cycloalkyl C1-C6 alkyl, C1-C6 alkoxy C1-C6 alkyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, Rₐ and R_{b} are each independently selected from the group consisting of hydrogen atom, C1-C6 alkyl, halogenated C1-C6 alkyl, C3-C8 cycloalkyl C1-C6 alkyl, and C6-C10 aryl C1-C6 alkyl;
preferably, Rₐ and R_{b} are each independently selected from the group consisting of hydrogen atom, C1-C6 alkyl, halogenated C1-C6 alkyl, C3-C8 cycloalkyl C1-C6 alkyl, and C6-C10 aryl; preferably, Rₐ and R_{b} are each independently selected from the group consisting of hydrogen atom, methyl, ethyl, trifluoromethyl, cyclopropylmethyl, and phenyl;
alternatively, Rₐ, R_{b} and carbon atoms to which Rₐ and R_{b} are connected form C3-C8 cycloalkyl, C3-C8 cycloalkyl C1-C6 alkyl, 3- to 7-membered heterocyclyl, or substituted 3- to 7-membered heterocyclyl; preferably Rₐ, R_{b} and the carbon atoms to which Rₐ and R_{b} are connected form C3-C8 cycloalkyl (e.g., C3-C5 cycloalkyl);
1-position chiral carbon atom has an absolute chiral configuration R or S;
m is 0 or 1.

In some embodiments of the first and second aspects of the present invention, the ligand-camptothecin derivative conjugate or a pharmaceutically acceptable salt or solvate thereof is disclosed, wherein the structural formula d is selected, without limitation, from the following compounds:

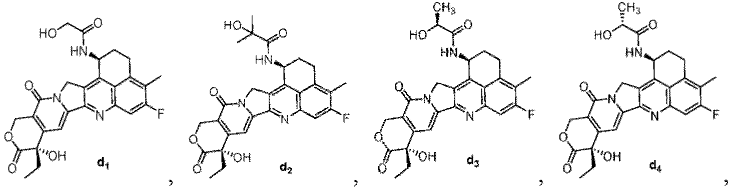

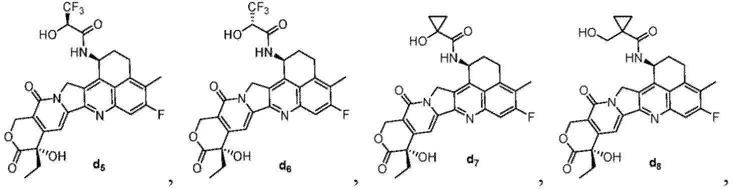

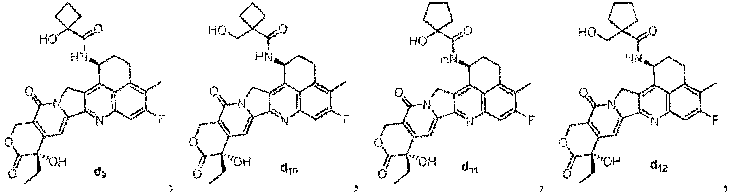

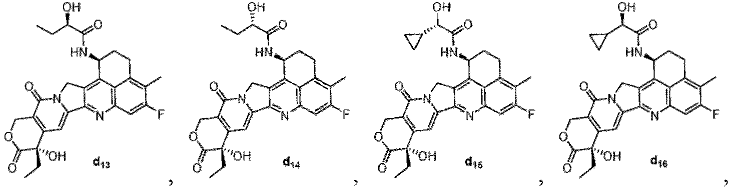

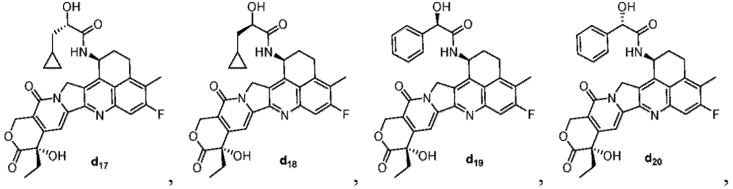

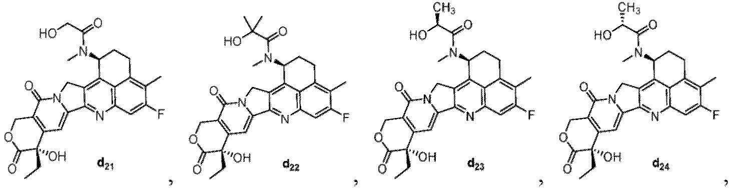

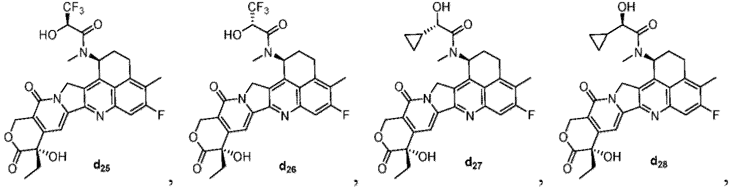

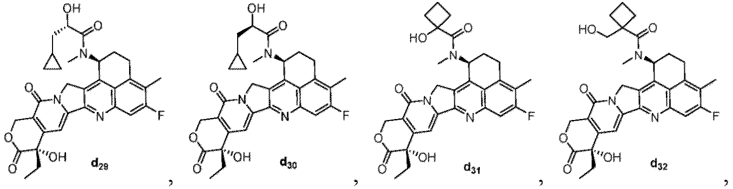

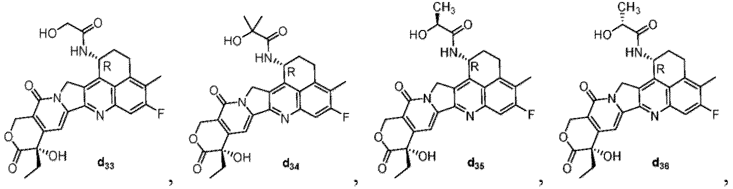

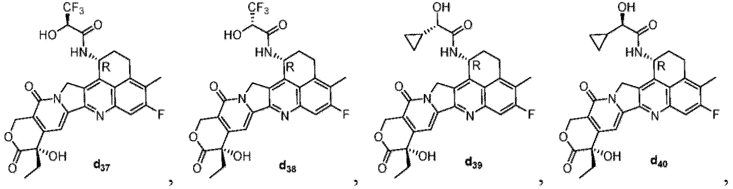

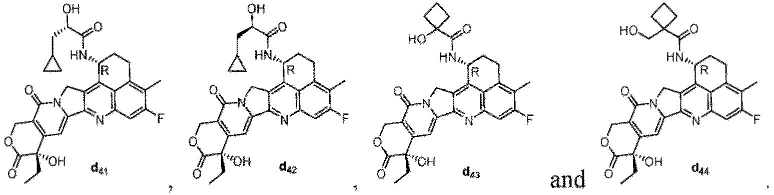

In certain embodiments of the present invention, L₁ may comprise a succinimide group. In these embodiments, the ligand-drug conjugate can be hydrolyzed under easily hydrolyzable conditions, and hydrolysis occurs at the succinimide group of the linking unit. When the ligand comprises multiple linker-drug units, the following cases may occur depending on the degree of hydrolysis:
succinimide groups are completely non-hydrolyzed, that is, the succinimide groups are all in a closed-ring form 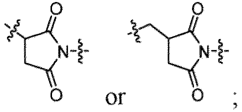
succinimide groups are not completely hydrolyzed, that is, some of the succinimide groups are in a closed-ring form 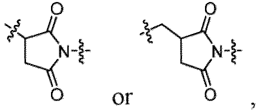 and other succinimide groups are in an open-ring form 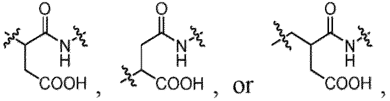 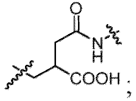
succinimide groups are completely hydrolyzed, that is, the succinimide groups are all in open-ring forms

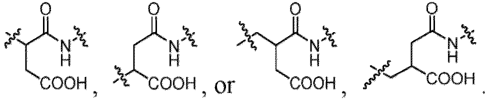

Therefore, when there are multiple Li's containing succinimide groups in the ADC (i.e., the Ab is connected to multiple drug-linker units containing succinimide groups), these succinimide groups may be all in closed-ring forms, partially in open-ring forms or all in open-ring forms.

It should be understood that although the succinimide group appearing in the chemical structural formula of ADC in the present application is in a closed ring form, it actually covers three cases: fully closed-ring, partially open ring and fully open ring.

A third aspect of the present invention discloses a linker-drug compound or a pharmaceutically acceptable salt or solvate thereof, having a structure shown in formula III, 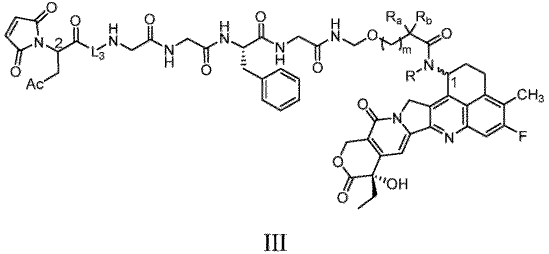 wherein
R is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, C6-C10 aryl, substituted C6-C10 aryl, 5- to 10-membered heteroaryl, and substituted 5- to 10-membered heteroaryl;
Rₐ is selected from the group consisting of hydrogen atom, deuterium atom, halogen, C1-C6 alkyl, deuterated C1-C6 alkyl, halogenated C1-C6 alkyl, C3-C8 cycloalkyl, C3-C8 cycloalkyl C1-C6 alkyl, C1-C6 alkoxy C1-C6 alkyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
R_{b} is selected from the group consisting of hydrogen atom, deuterium atom, halogen, C1-C6 alkyl, deuterated C1-C6 alkyl, halogenated C1-C6 alkyl, C3-C8 cycloalkyl, C3-C8 cycloalkyl C1-C6 alkyl, C1-C6 alkoxy C1-C6 alkyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
alternatively, Rₐ, R_{b} and carbon atoms to which Rₐ and R_{b} are connected form C3-C8 cycloalkyl, C3-C8 cycloalkyl C1-C6 alkyl, 3- to 7-membered heterocyclyl, and substituted 3- to 7-membered heterocyclyl;
preferably, Rₐ and R_{b} are each independently selected from the group consisting of hydrogen atom, C1-C6 alkyl, halogenated C1-C6 alkyl, C3-C8 cycloalkyl C1-C6 alkyl, and C6-C10 aryl; preferably, Rₐ and R_{b} are each independently selected from the group consisting of hydrogen atom, methyl, ethyl, trifluoromethyl, cyclopropylmethyl, and phenyl;
alternatively, Rₐ, R_{b} and carbon atoms to which Rₐ and R_{b} are connected form C3-C8 cycloalkyl, C3-C8 cycloalkyl C1-C6 alkyl, 3- to 7-membered heterocyclyl, or substituted 3- to 7-membered heterocyclyl; preferably Rₐ, R_{b} and the carbon atoms to which Rₐ and R_{b} are connected form C3-C8 cycloalkyl (e.g., C3-C5 cycloalkyl);
L₃ is present or absent, and when present, L₃ is selected from 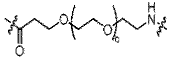 , and o is selected from integers between 1 and 10 (e.g., 1, 2, 3, 4, 5, 6, 7, 8, 9 or 10);
1-position or 2-position chiral carbon atom has an absolute chiral configuration R or S;
the Ac is a hydrophilic structural unit;
m is 0 or 1.

In some embodiments of the third aspect of the present invention, the linker-drug compound or a pharmaceutically acceptable salt or solvate thereof is disclosed, wherein the Ac has a structure shown in formula B, 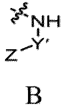 wherein
Z is composed of one or more selected from the group consisting of of hydrophilic structural carboxyl, phosphoric acid, polyphosphoric acid, phosphorous acid, sulfonic acid, sulfinic acid, and polyethylene glycol (PEG);
Y' is an optional scaffold connecting -NH- and Z; preferably, Y' is C1-C6 alkylene (e.g., methylene);
the Ac is connected to the labeled 2-position carbon in structural formula **I** through the scaffold Y.

In some embodiments of the third aspect of the present invention, the linker-drug compound or a pharmaceutically acceptable salt or solvate thereof is disclosed, wherein the Ac is selected, without limitation, from glycine, (D/L) alanine, (D/L) leucine, (D/L) isoleucine, (D/L) valine, (D/L) phenylalanine, (D/L) proline, (D/L) tryptophan, (D/L) serine, (D/L) tyrosine, (D/L) cysteine, (D/L) cystine, (D/L) arginine, (D/L) histidine, (D/L) methionine, (D/L) asparagine, (D/L) glutamine, (D/L) threonine, (D/L) aspartic acid, (D/L) glutamic acid, natural or unnatural amino acid derivatives or the following structures, 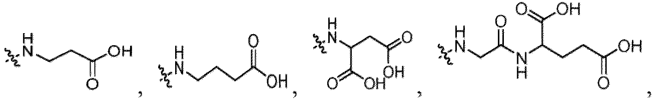 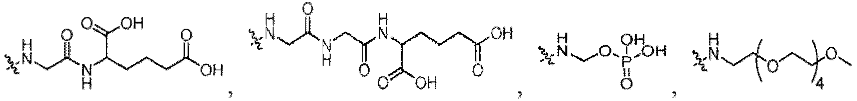 and

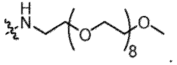

In some embodiments of the third aspect of the present invention, the linker-drug compound or a pharmaceutically acceptable salt or solvate thereof is disclosed, wherein the Ac is selected, without limitation, from the group consisting of glycine, phosphoric acid, (D/L) glutamic acid and polyethylene glycol hydrophilic structure.

In some embodiments of the third aspect of the present invention, the linker-drug compound or a pharmaceutically acceptable salt or solvate thereof is disclosed, wherein the linker-drug compound is selected, without limitation, from the following structures or isomers thereof, 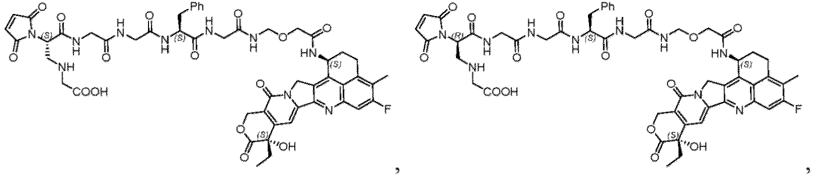 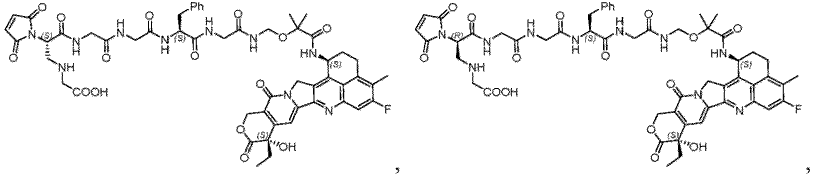 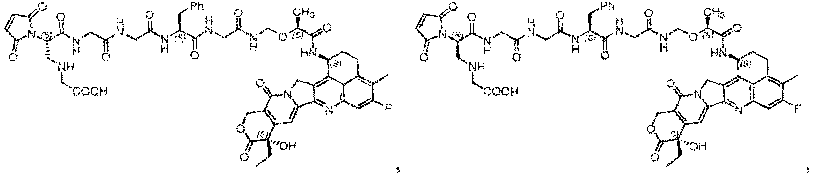 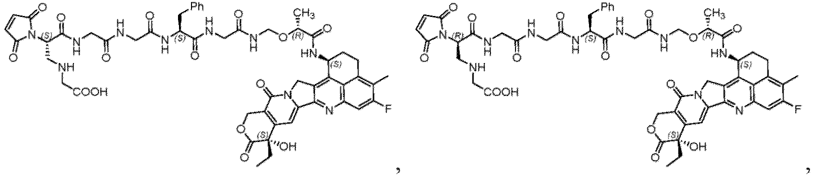 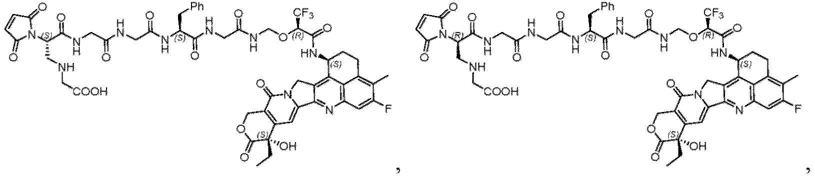 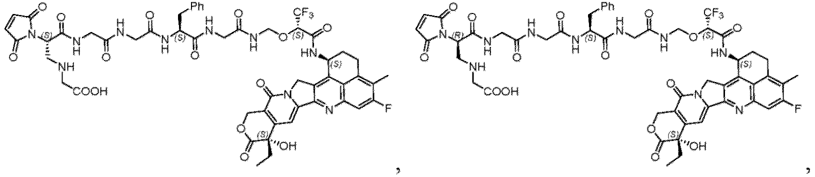 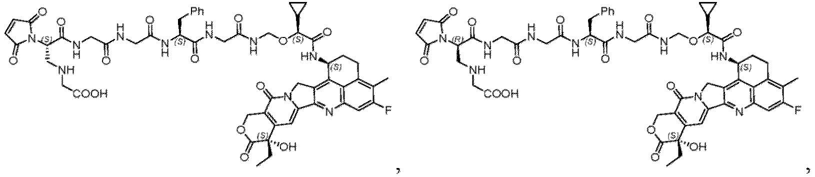 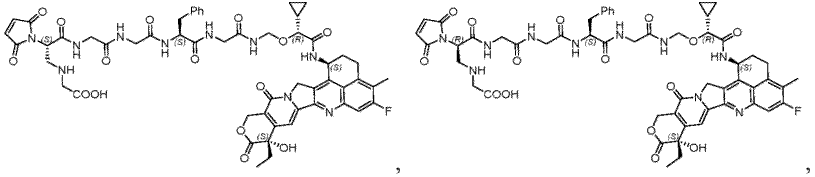 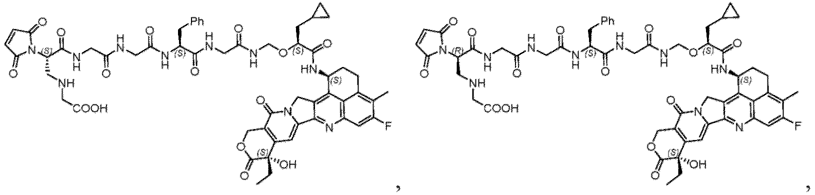 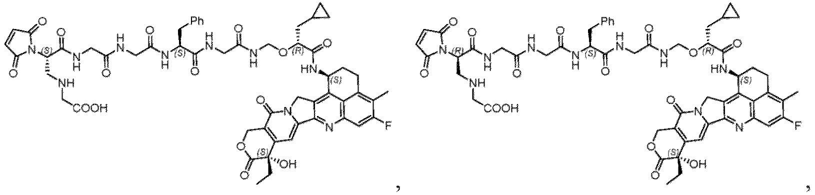 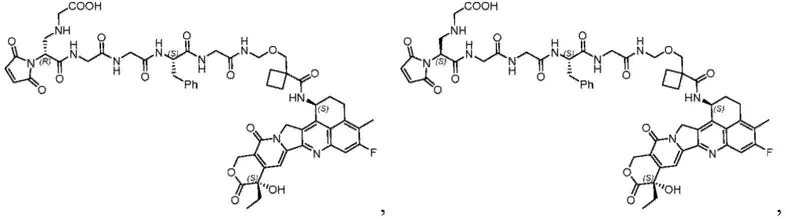 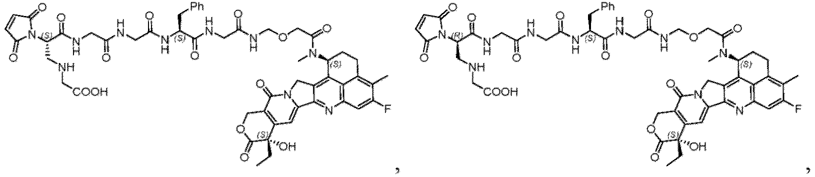 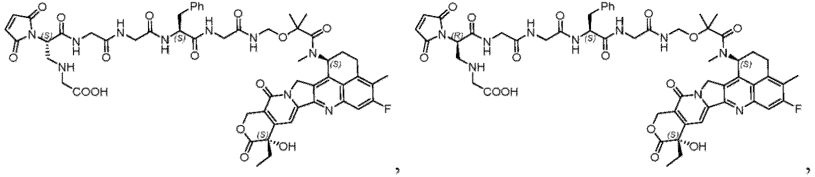 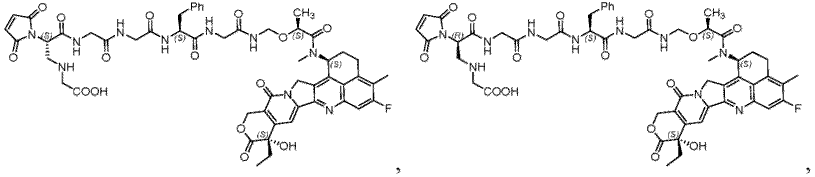 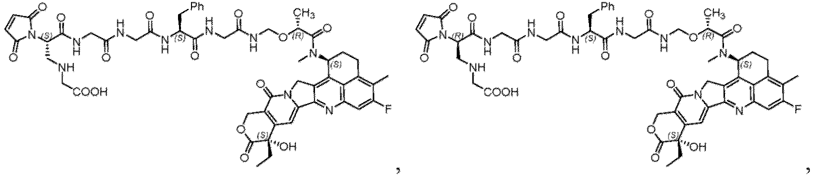 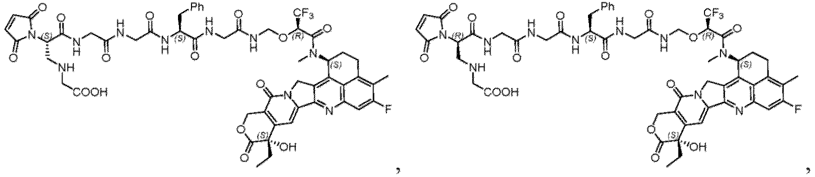 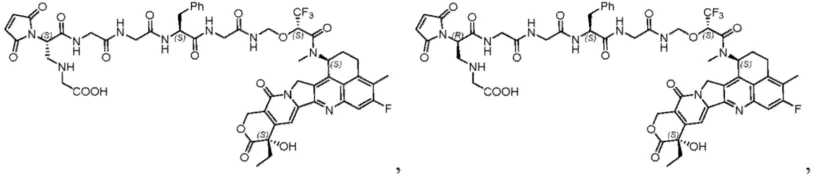 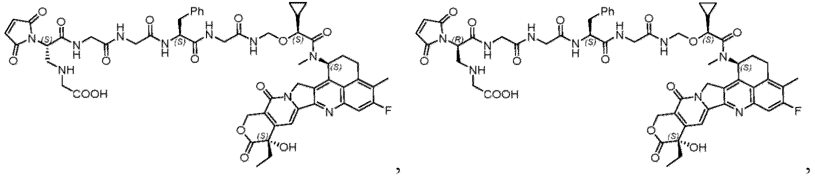 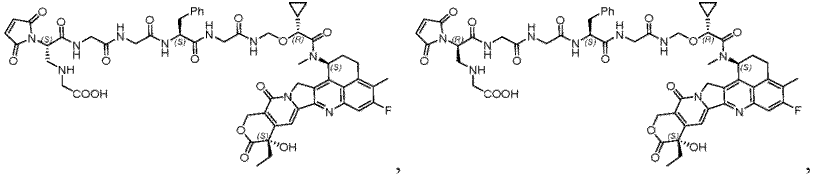 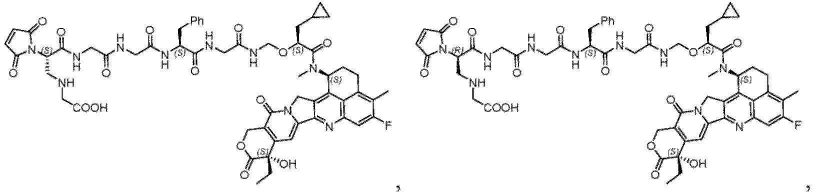 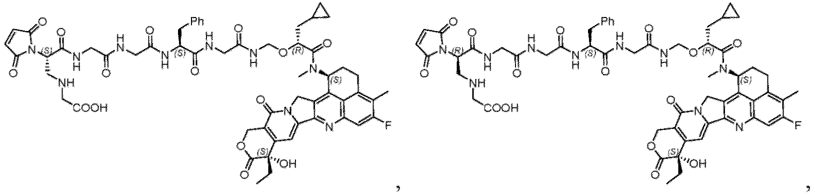 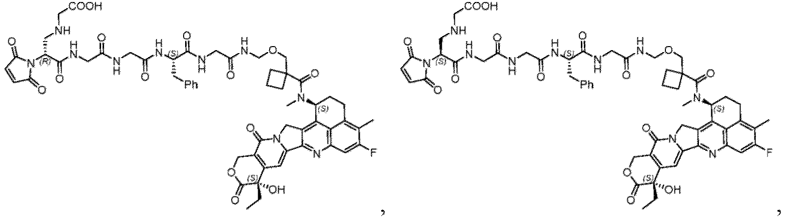 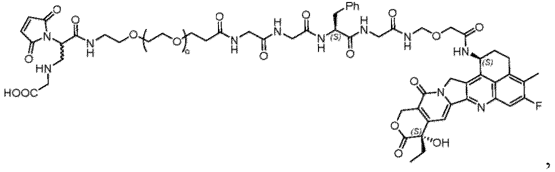 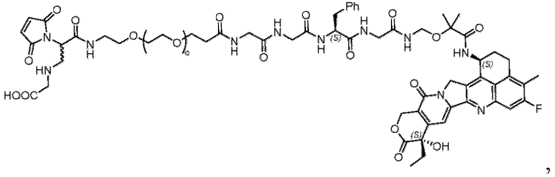 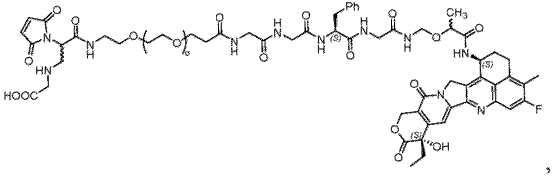 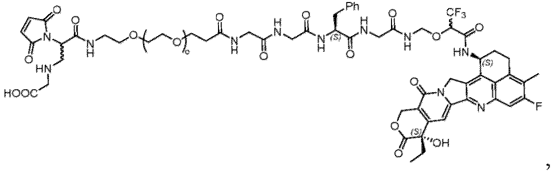 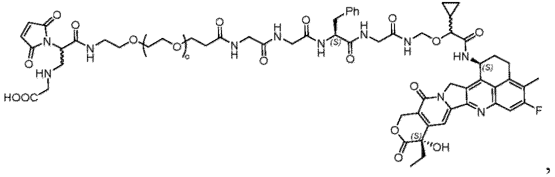 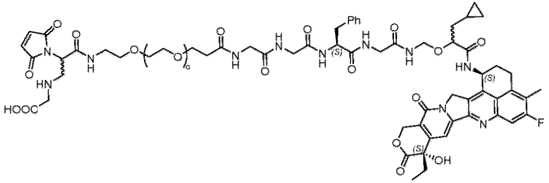 and 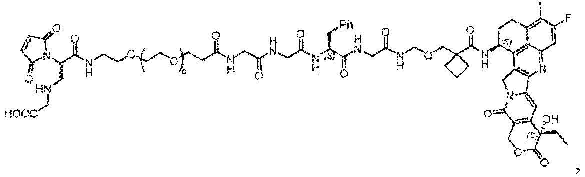 wherein o is selected from integers between 1 and 10 (e.g., 1, 2, 3, 4, 5, 6, 7, 8, 9 or 10).

The linker-drug compound disclosed in the third aspect of the present invention or a pharmaceutically acceptable salt or solvate thereof can be used as an intermediate for coupling with the ligand Ab to form the ligand-camptothecin derivative conjugate shown in the formula **I** as described in the first aspect or in the formula **II** as described in the second aspect.

A fourth aspect of the present invention discloses a method for preparing the ligand-camptothecin derivative conjugate shown in the general formula **I** as described in the first aspect or in the general formula **II** as shown in the second aspect, or a pharmaceutically acceptable salt or solvate, comprising the following steps, 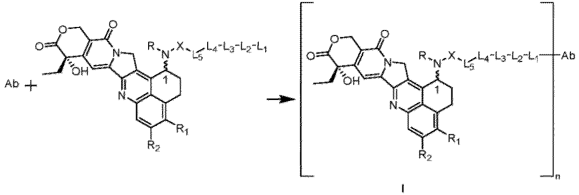 or 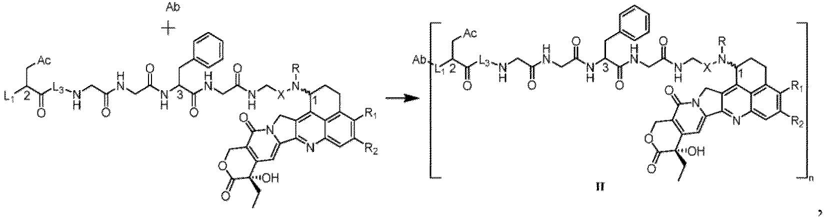
allowing a reduced antibody or an antigen-binding fragment thereof to have a coupling reaction with a linker-drug compound to obtain the ligand-camptothecin derivative conjugate shown in the general formula **I** or general formula **II**;
wherein 1-positionm 2-position or 3-position chiral carbon atom has an absolute chiral configuration R or S;
the Ab, L₁, L₂, L₃, L₄, L₅, X, R, R₁, R₂ and n are as defined above.

The present application further relates to use of the linker-drug compound as disclosed in the third aspect or a pharmaceutically acceptable salt or solvate thereof as an intermediate in the preparation of a ligand-camptothecin derivative conjugate or a pharmaceutically acceptable salt or solvate thereof. In certain embodiments, the ligand-camptothecin derivative conjugate or a pharmaceutically acceptable salt or solvate thereof is the ligand-camptothecin derivative conjugate as described in the first, second and fourth aspects of the present invention or a pharmaceutically acceptable salt or solvate thereof. In certain embodiments, the preparation is carried out according to the preparation method as disclosed in the fourth aspect.

In some embodiments of the first, second and fourth aspects of the present invention, the ligand-camptothecin derivative conjugate or a pharmaceutically acceptable salt or solvate thereof is disclosed, wherein the ligand-camptothecin derivative conjugate or a pharmaceutically acceptable salt or solvate thereof is selected, without limitation, from the following structures, their derived structures in which rings in succinimide groups are in open form, and their isomers, 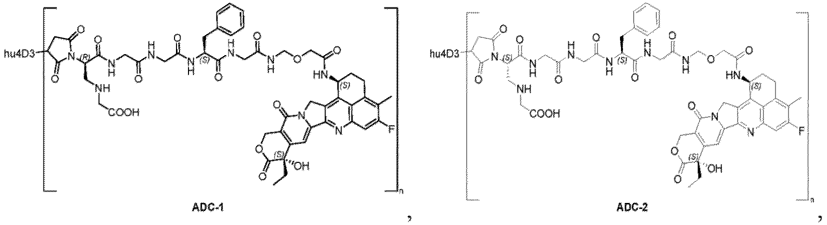 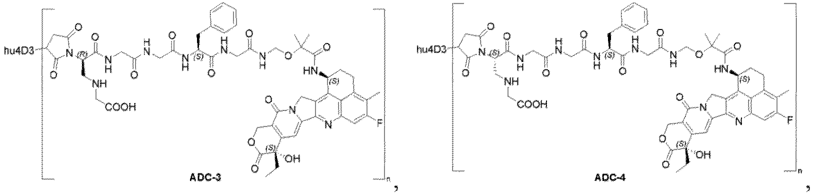 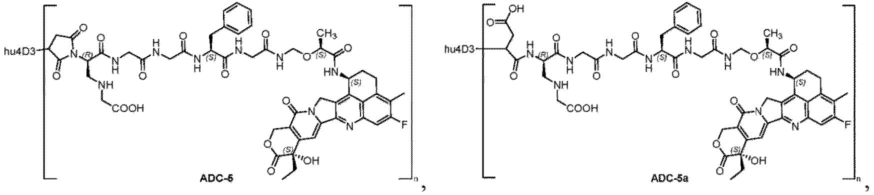 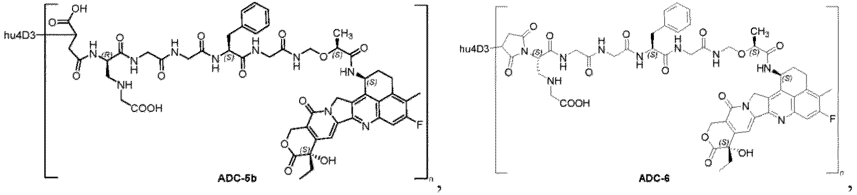 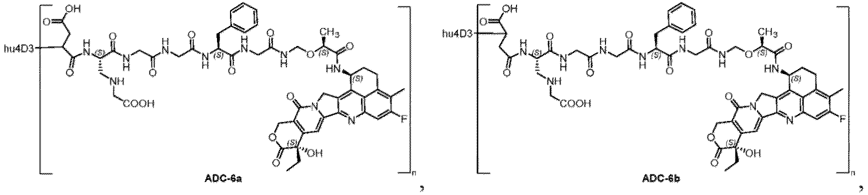 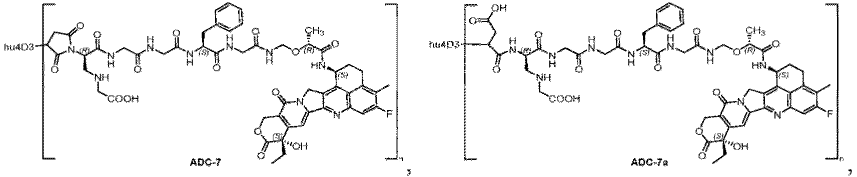 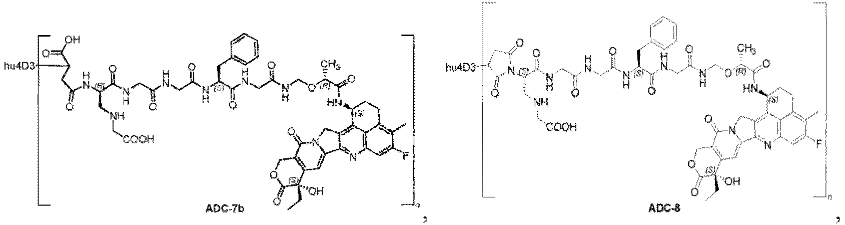 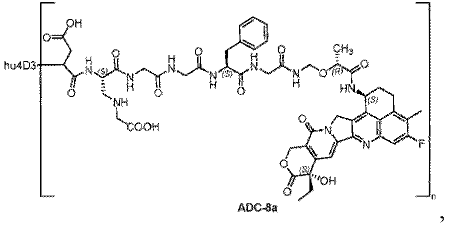 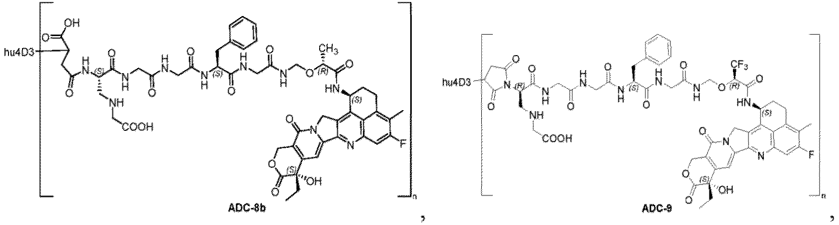 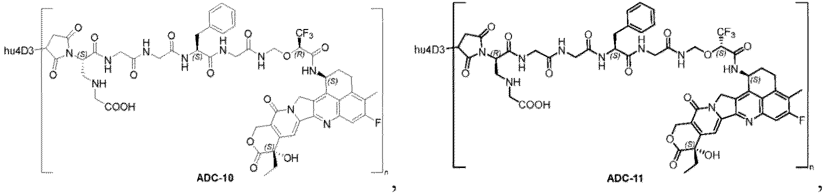 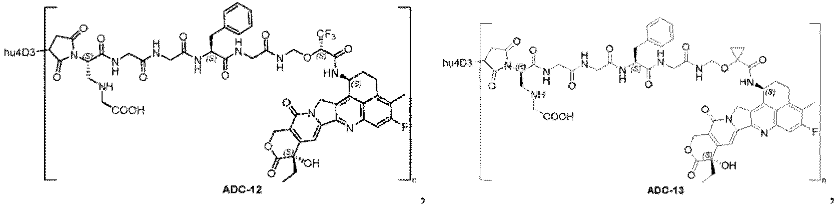 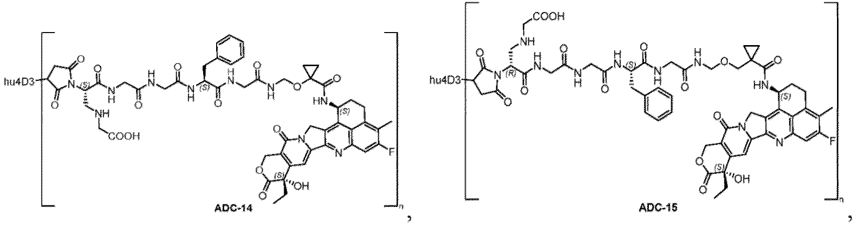 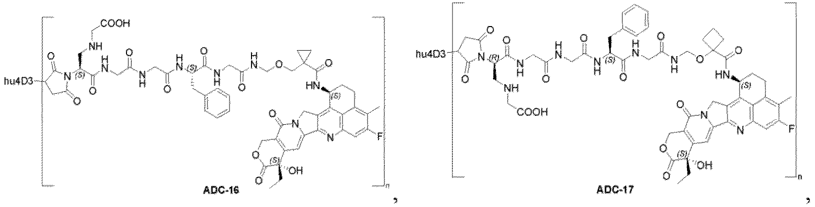 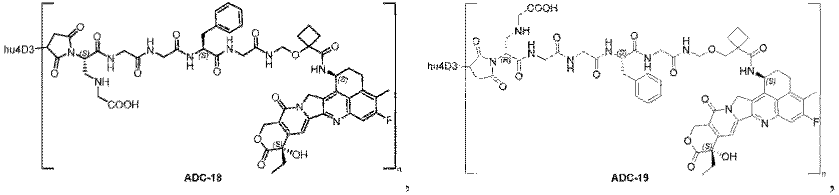 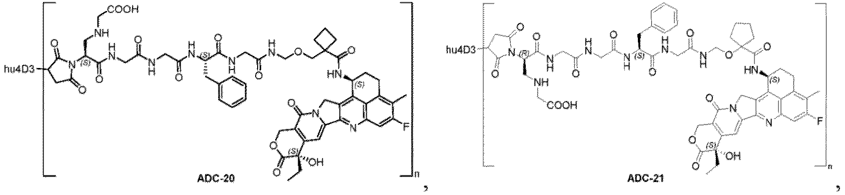 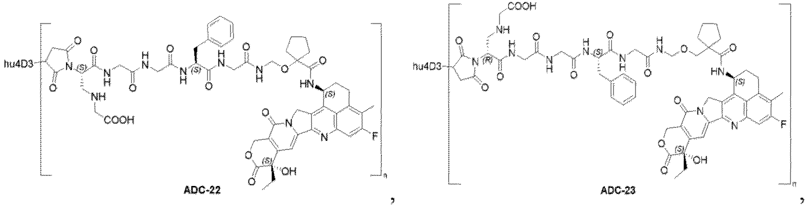 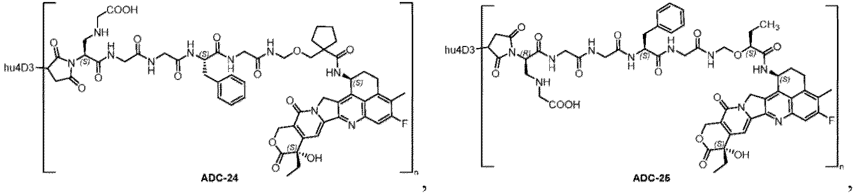 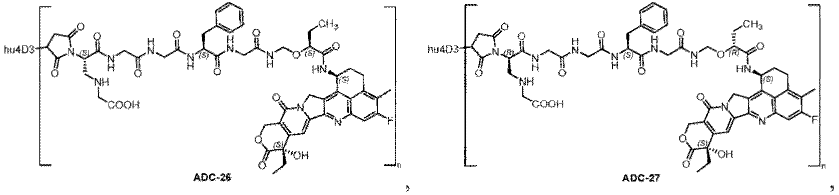 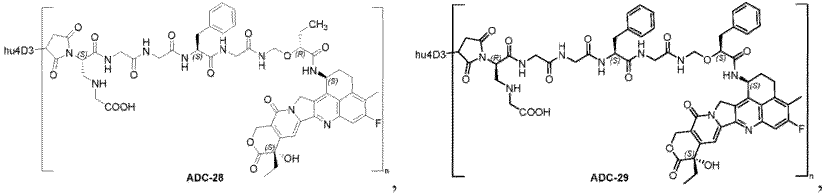 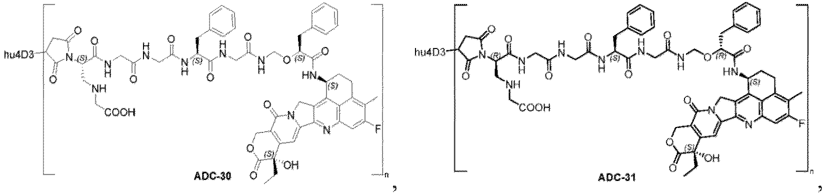 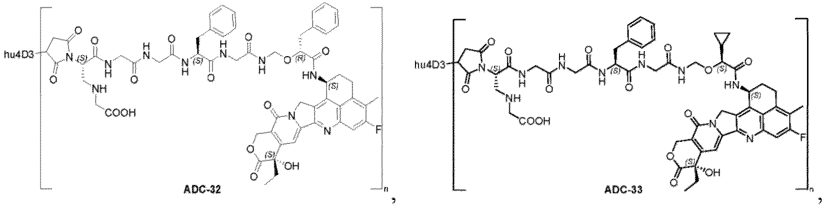 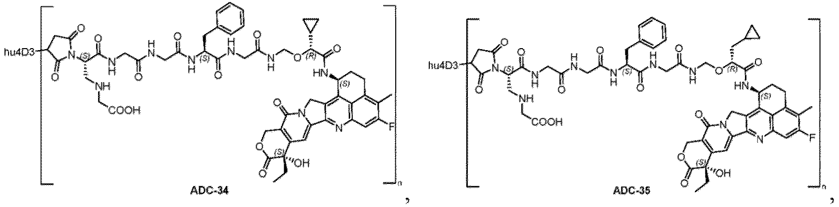 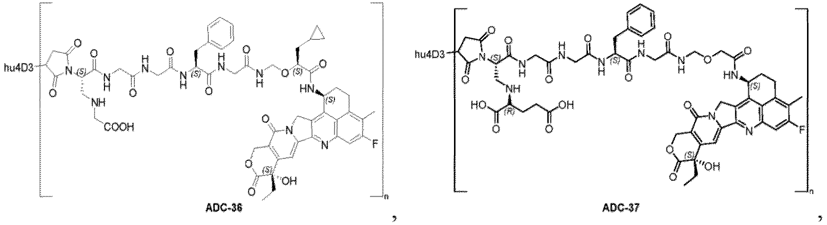 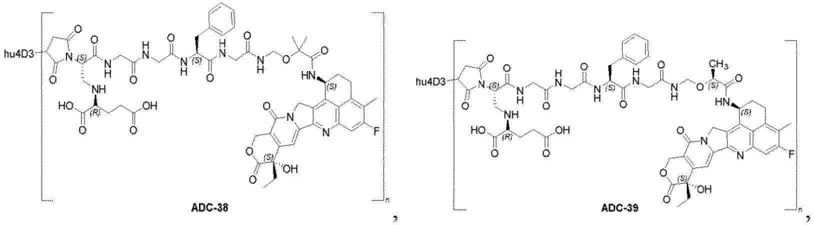 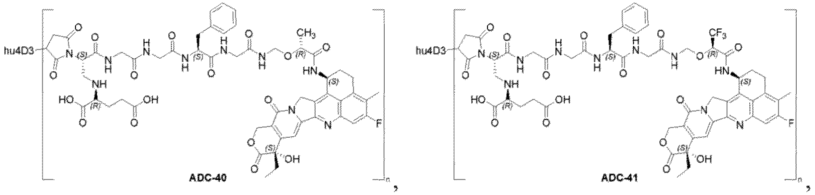 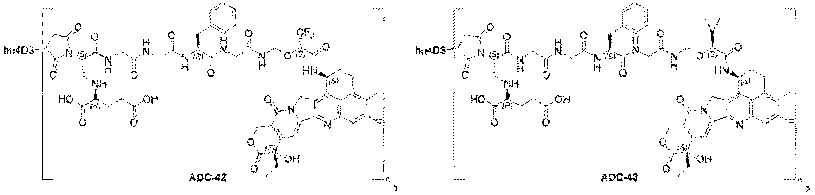 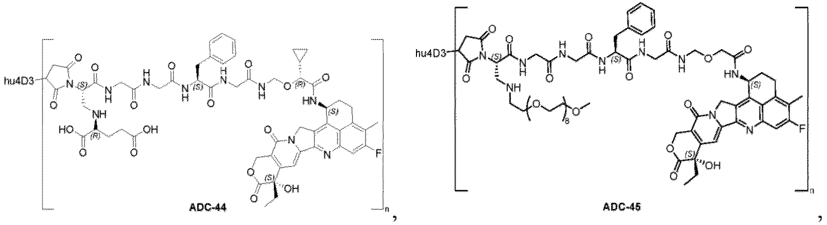 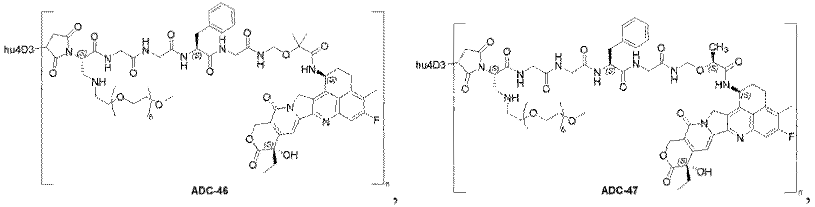 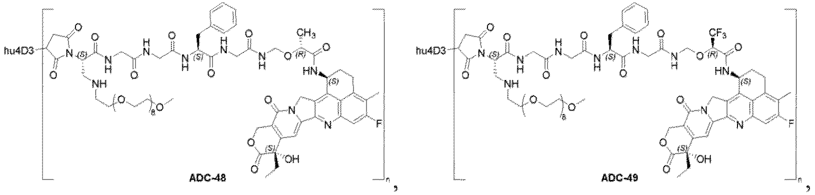 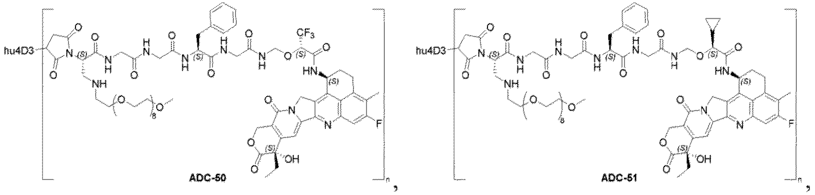 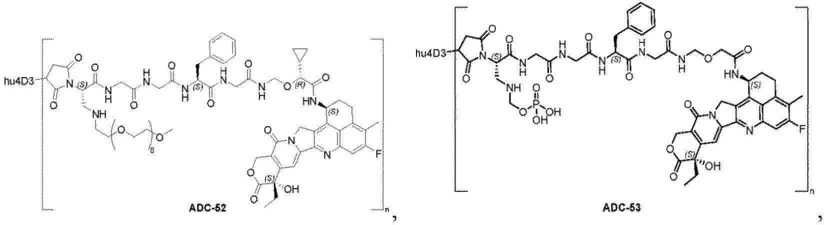 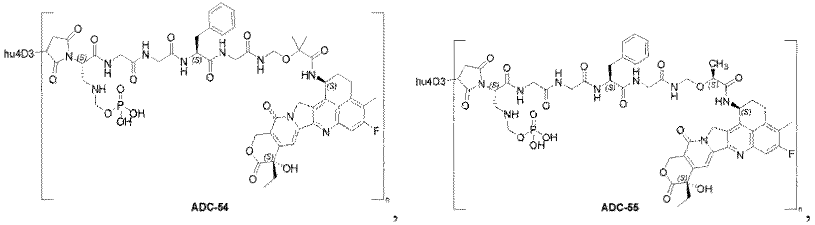 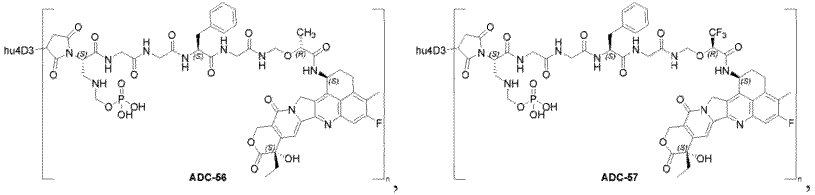 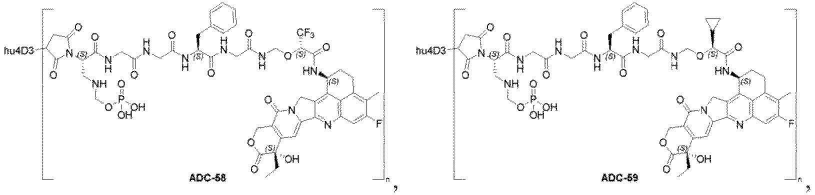 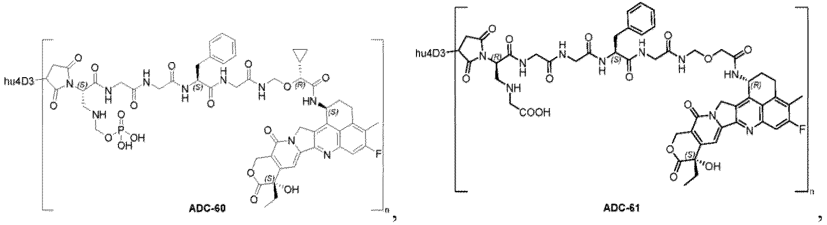 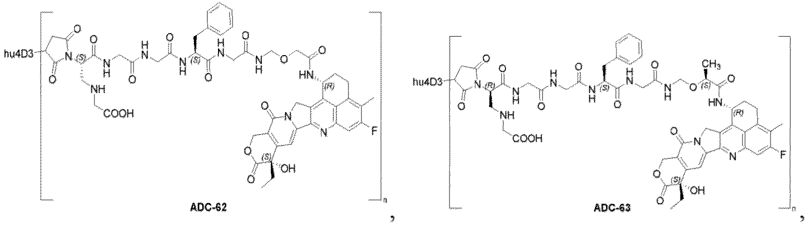 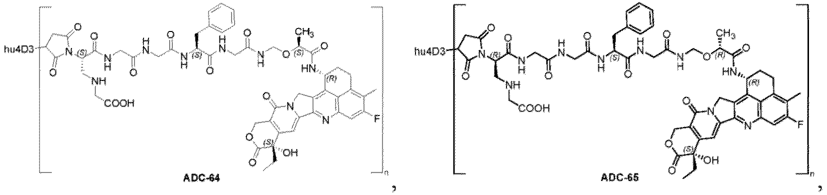 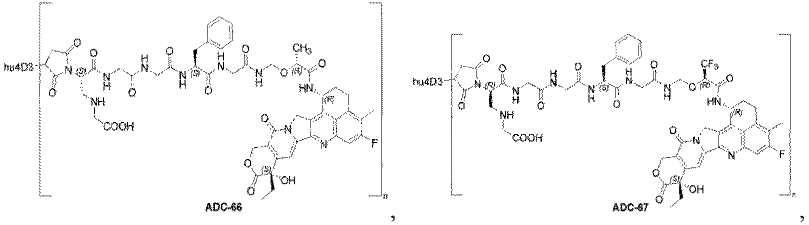 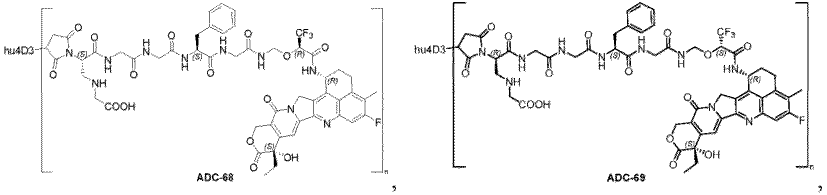 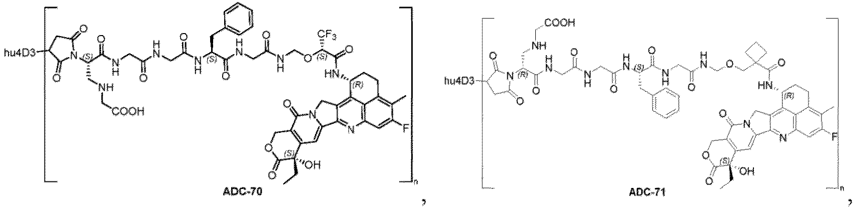 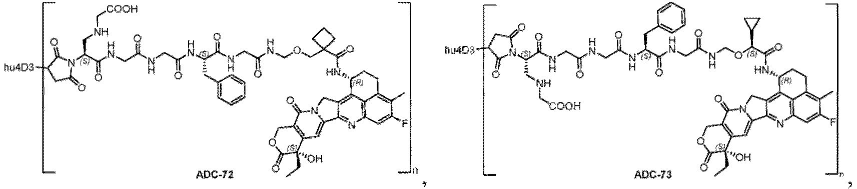 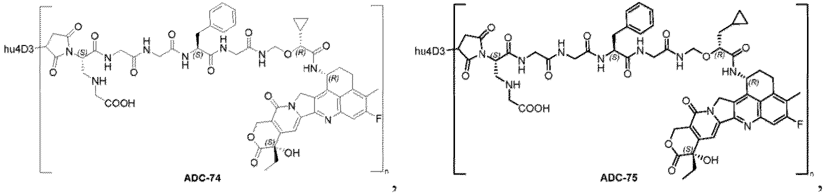 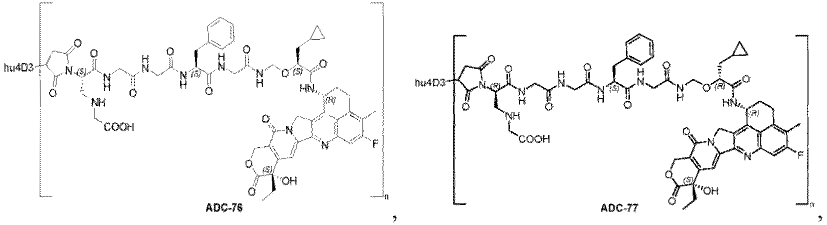 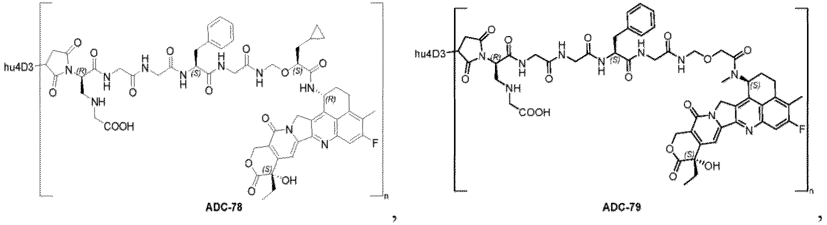 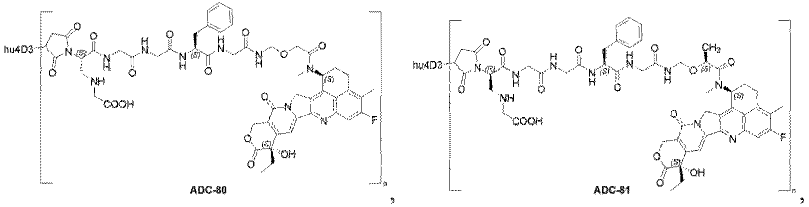 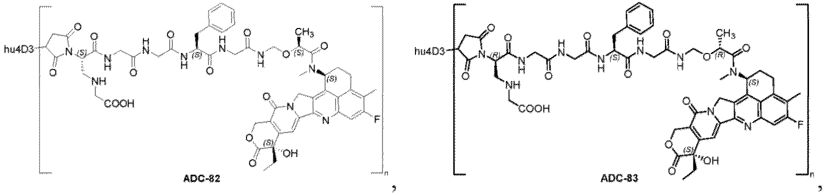 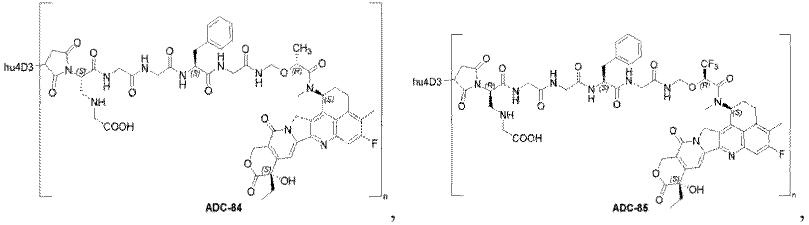 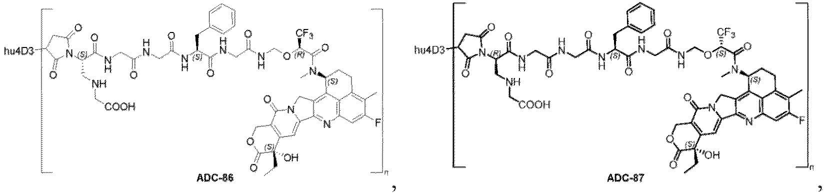 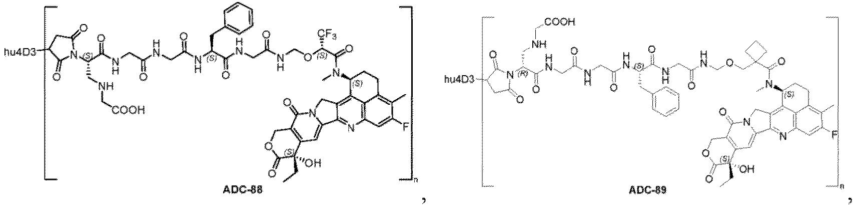 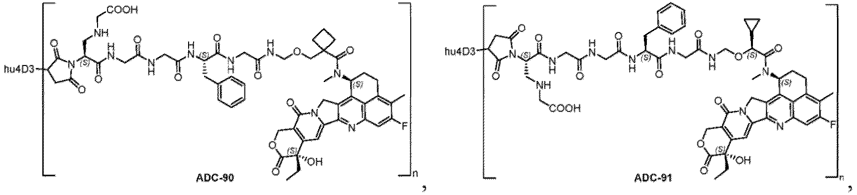 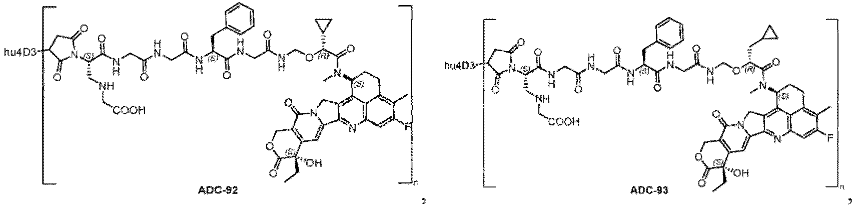 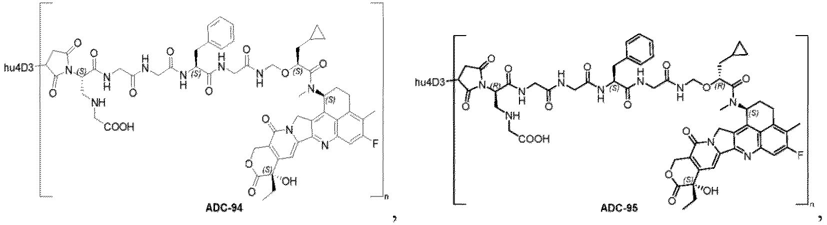 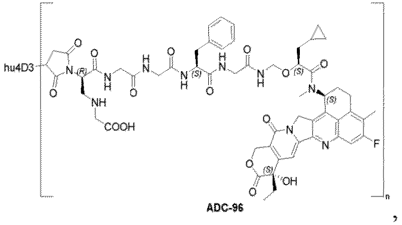 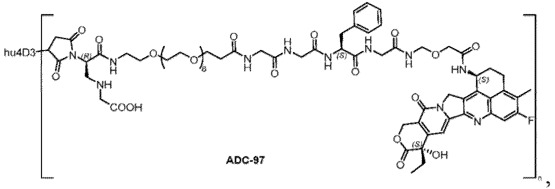 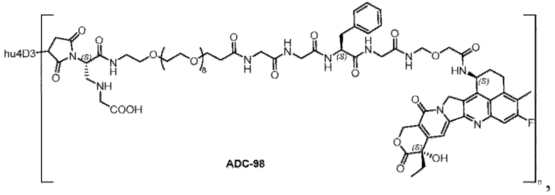 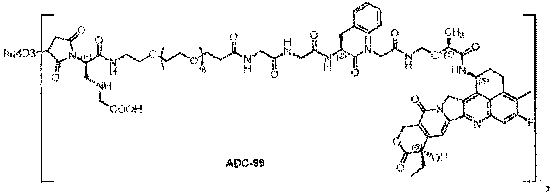 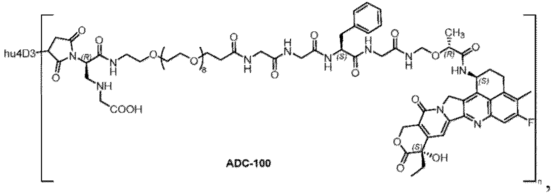 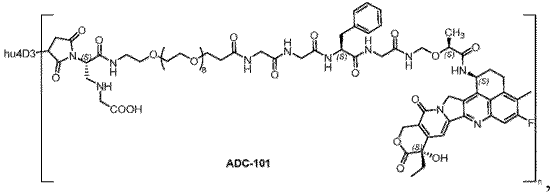 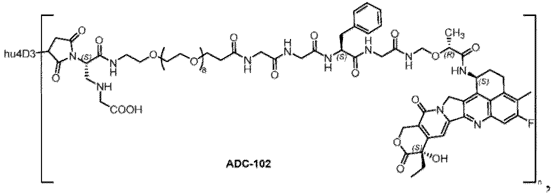 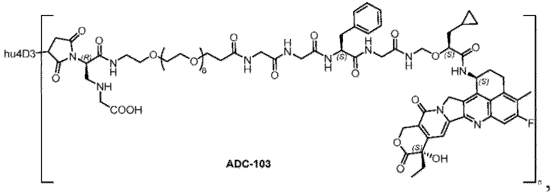 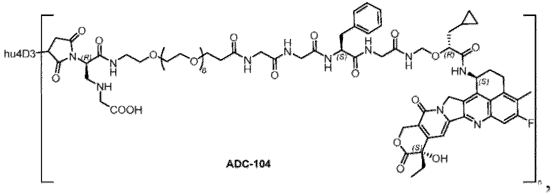 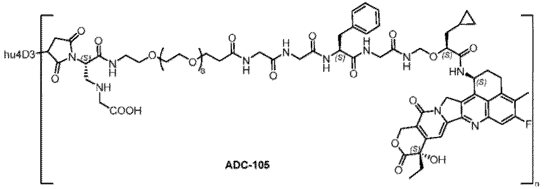 and 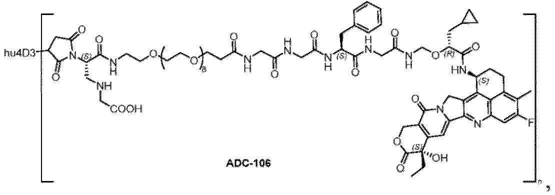 wherein
hu4D3 is an antibody targeting human Trop2 or an antigen-binding fragment thereof;
n is selected from integers between 1 and 10 (e.g., 1, 2, 3, 4, 5, 6, 7, 8, 9 or 10).

In some embodiments of the first, second and fourth aspects of the present invention, the ligand-camptothecin derivative conjugate or a pharmaceutically acceptable salt or solvate thereof is disclosed, wherein the ligand-camptothecin derivative conjugate or a pharmaceutically acceptable salt or solvate thereof is selected, without limitation, from the following structures, their derived structures in which rings in succinimide groups are in open form, and their isomers, 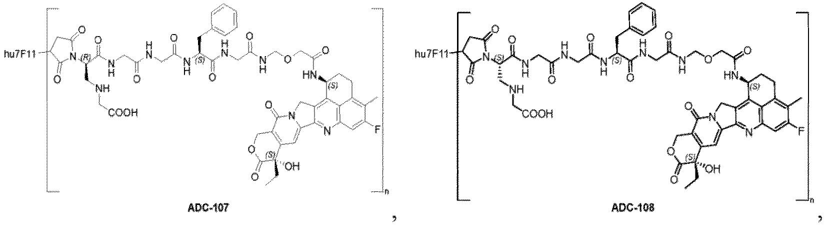 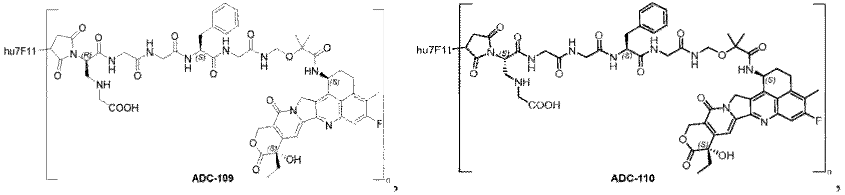 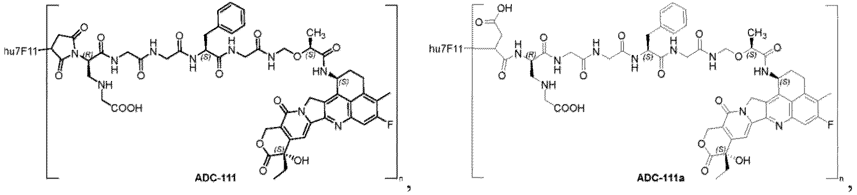 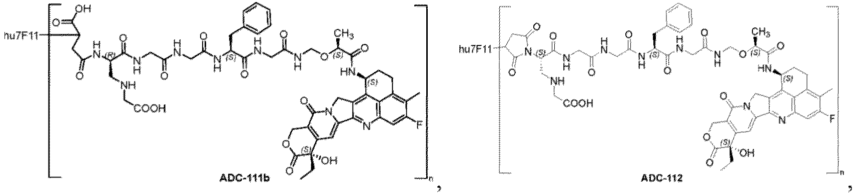 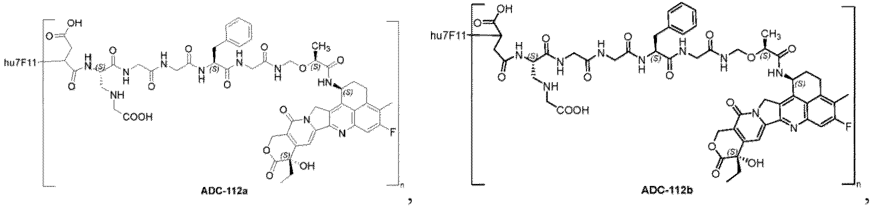 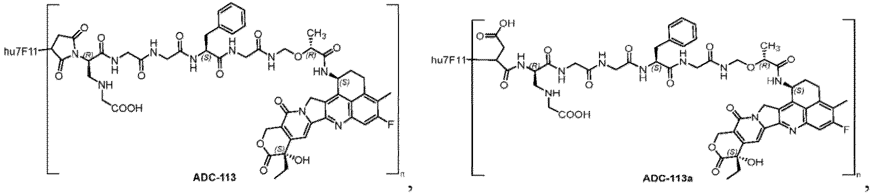 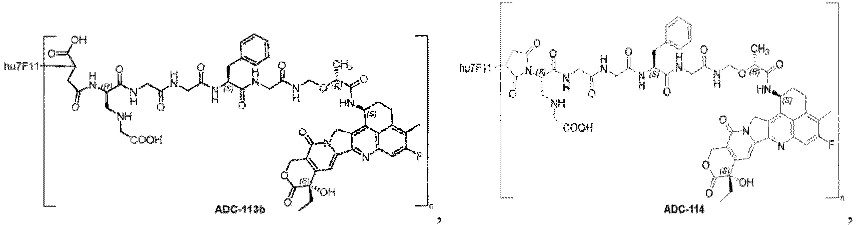 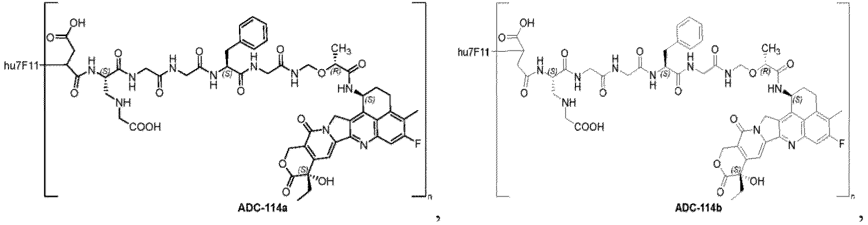 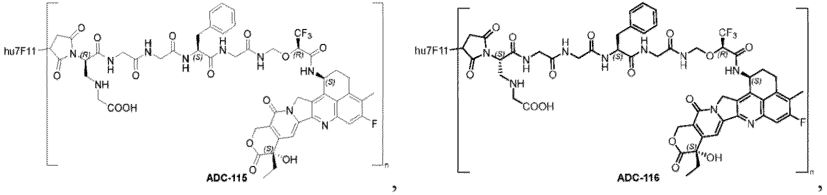 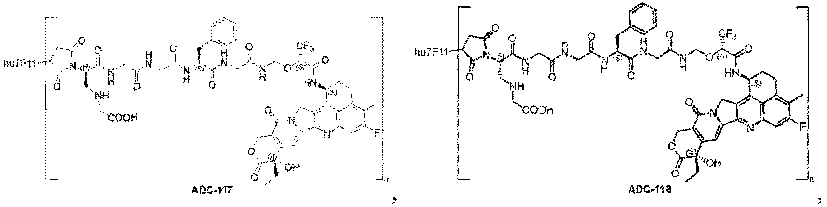 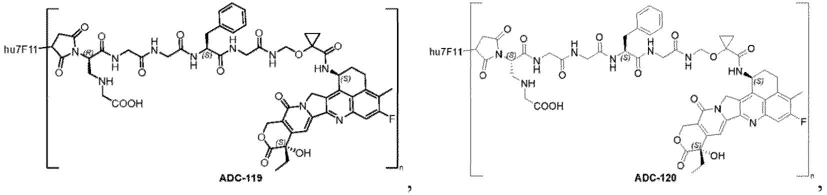 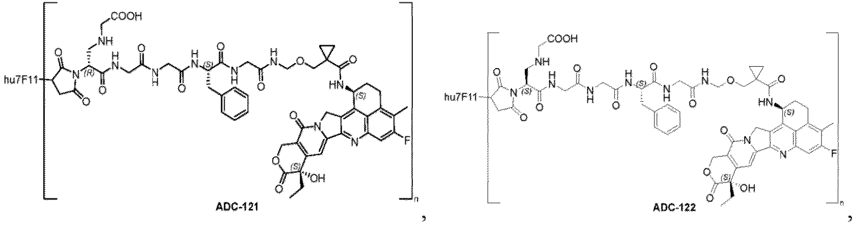 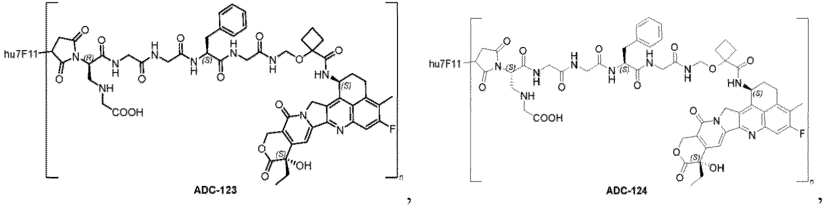 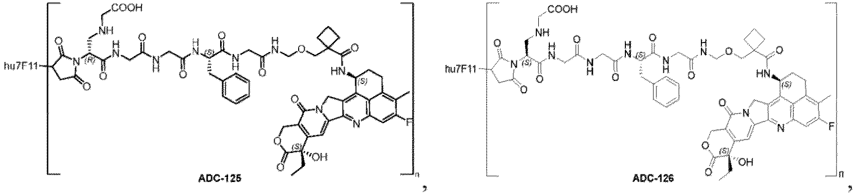 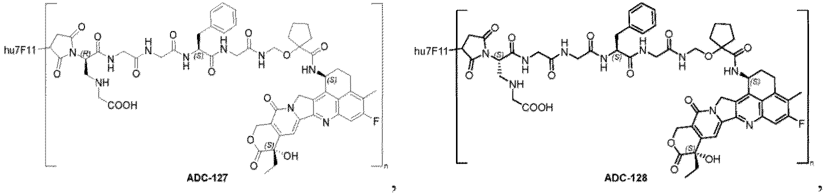 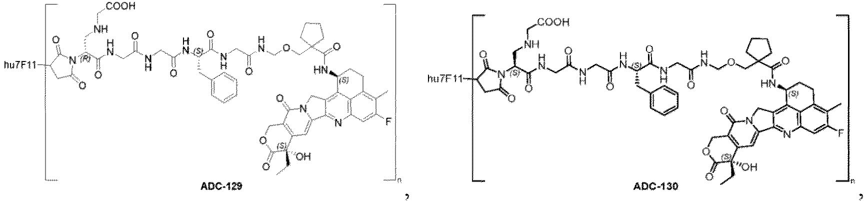 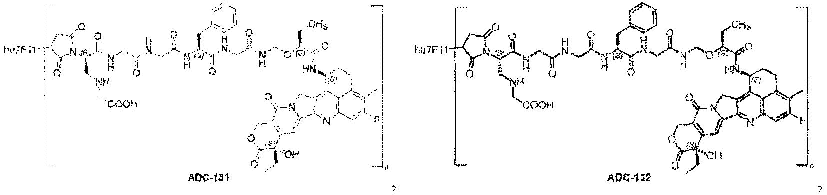 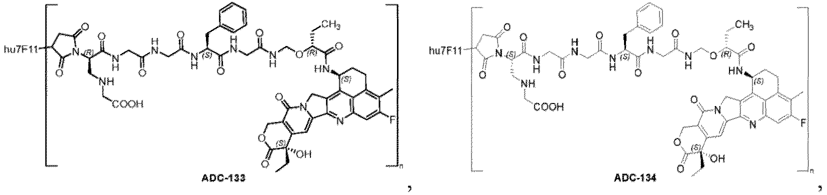 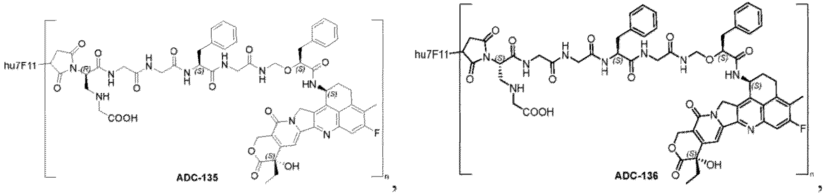 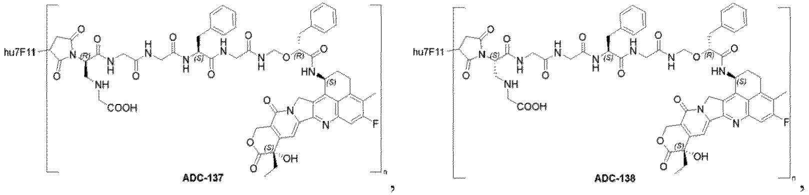 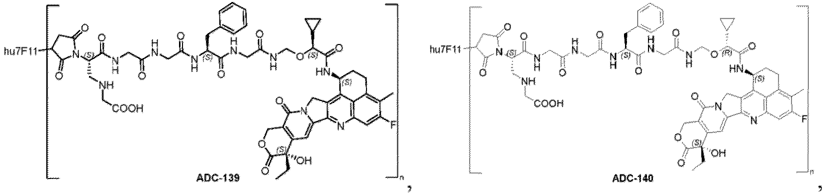 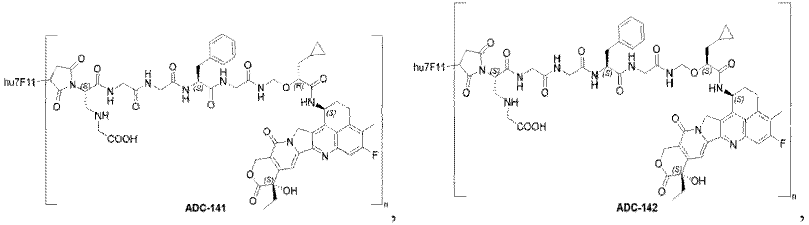 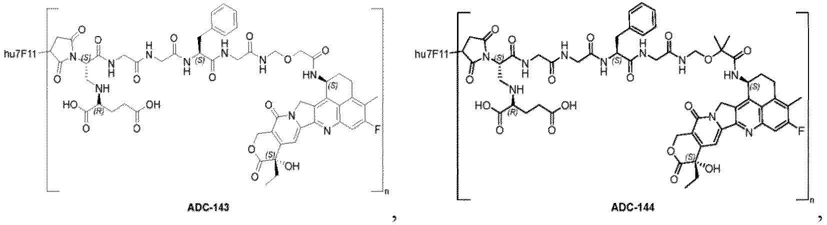 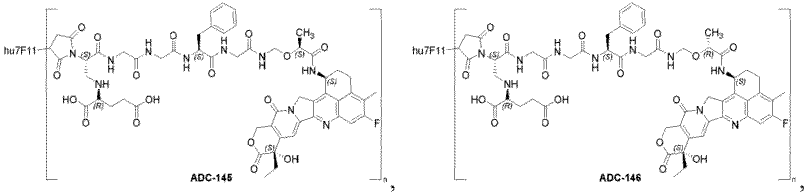 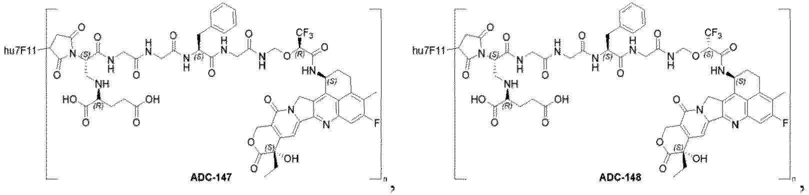 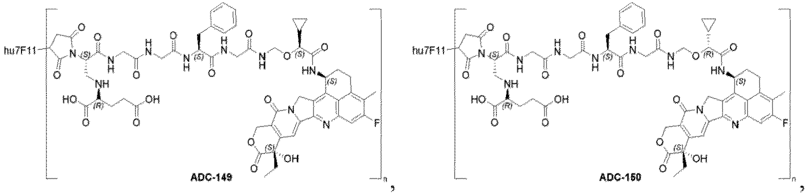 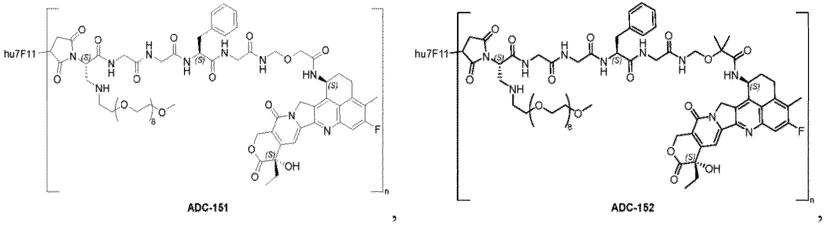 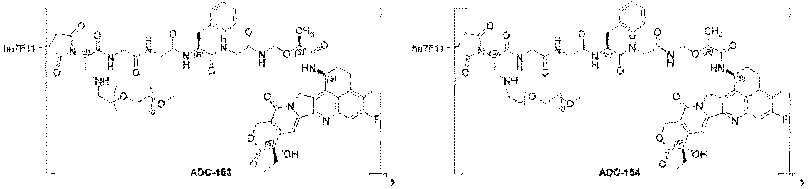 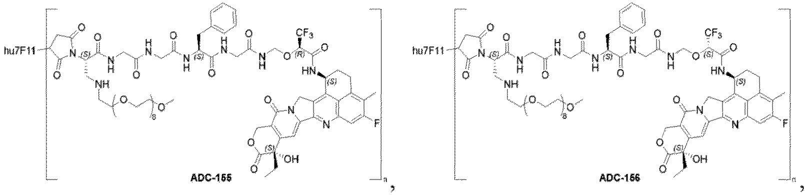 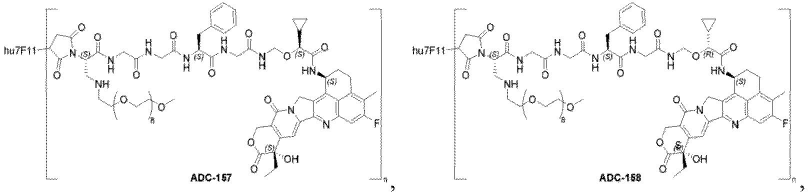 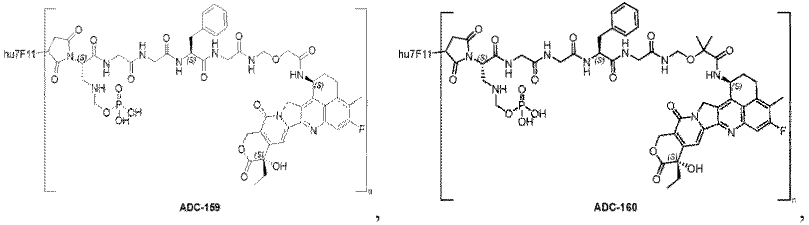 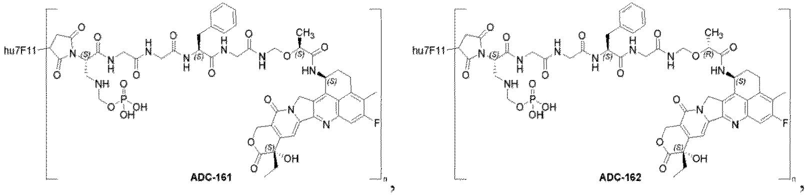 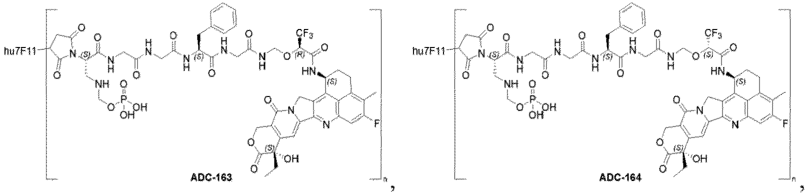 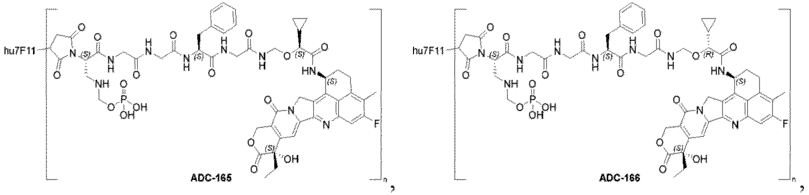 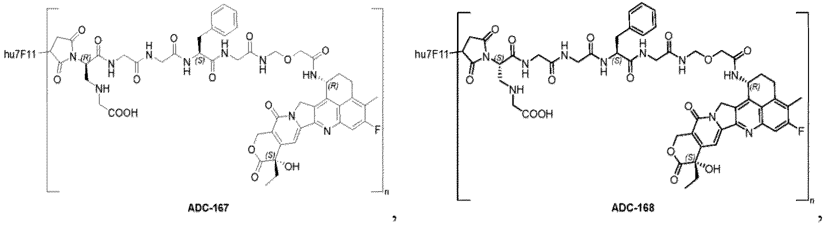 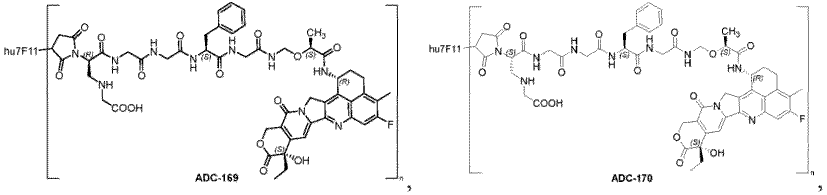 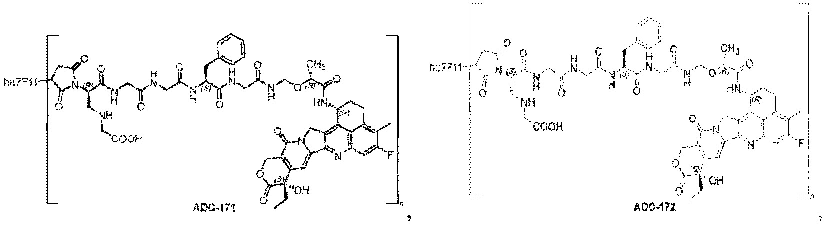 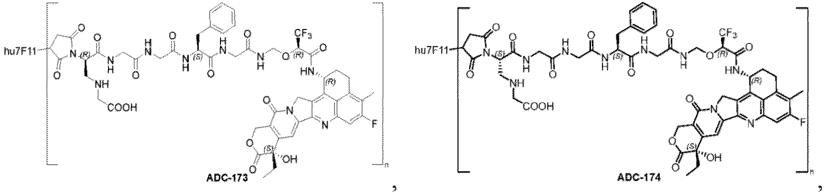 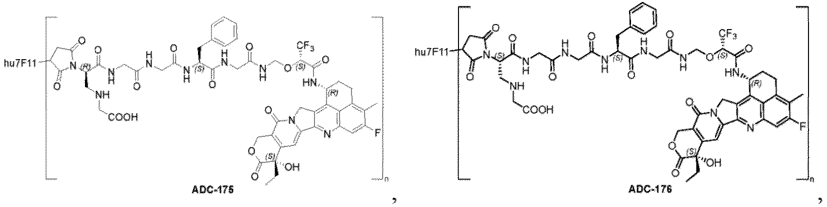 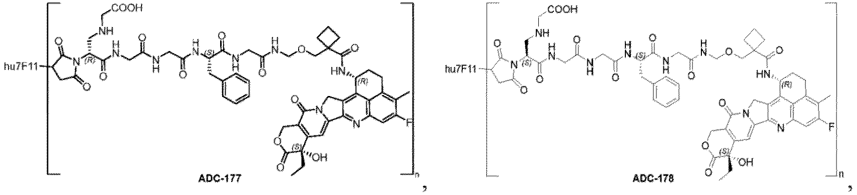 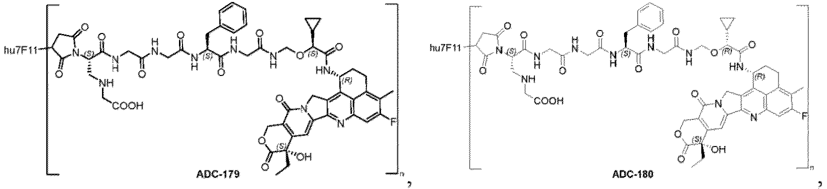 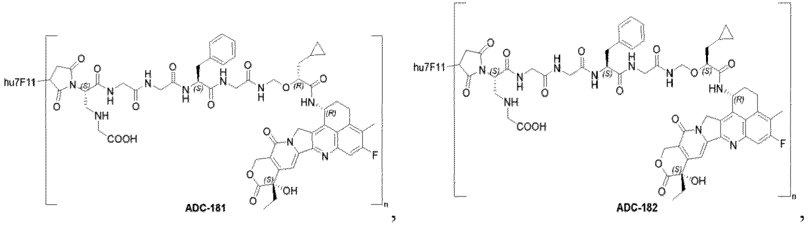 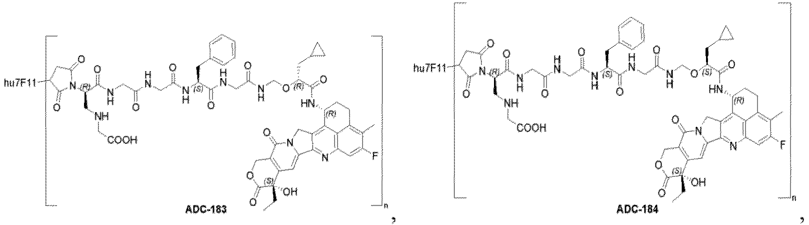 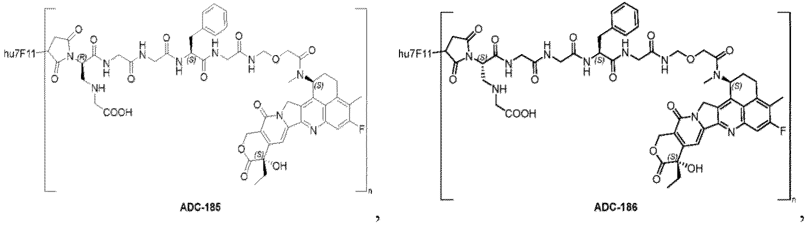 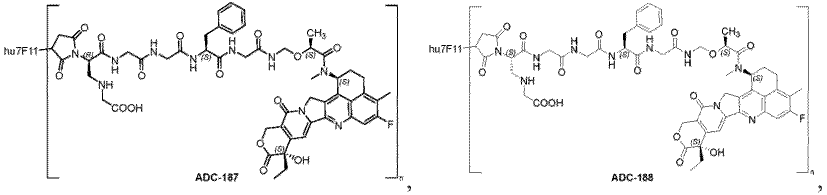 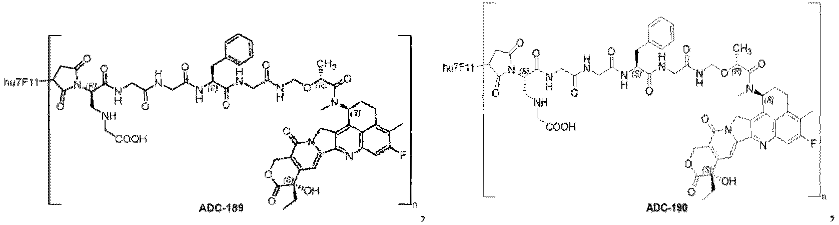 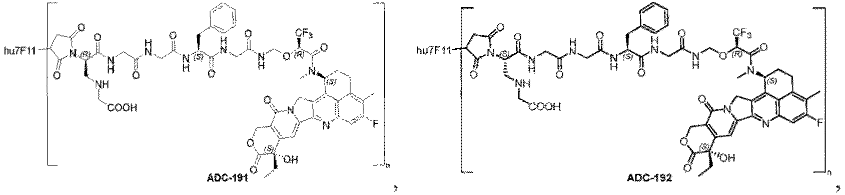 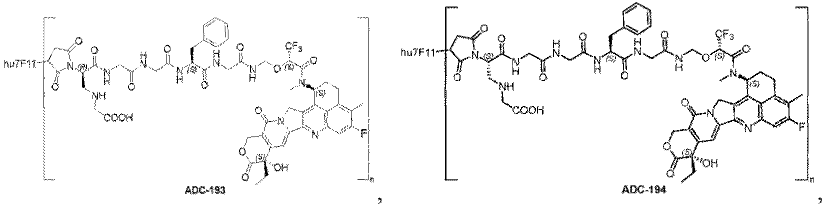 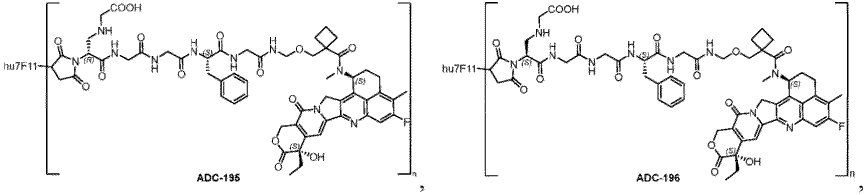 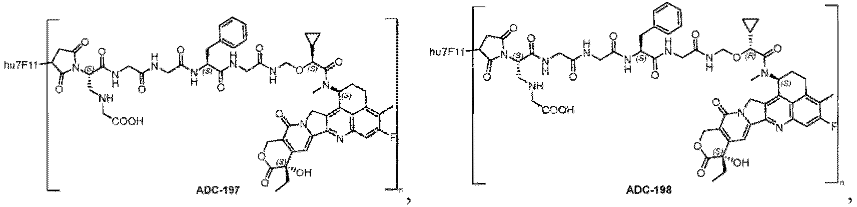 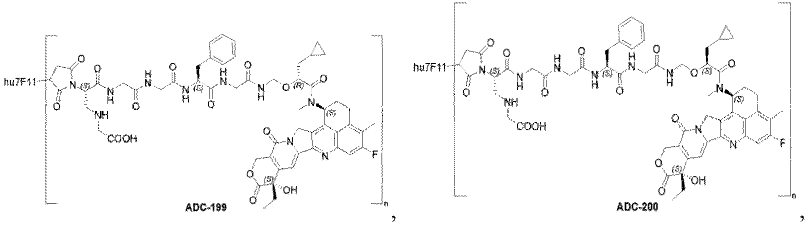 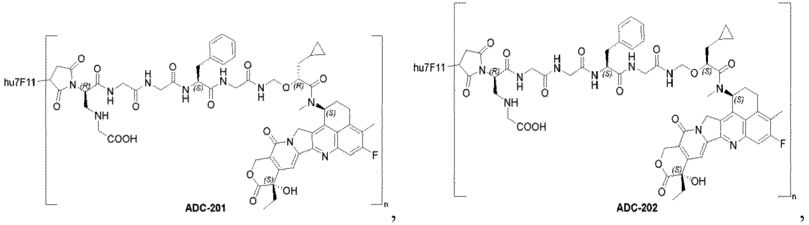 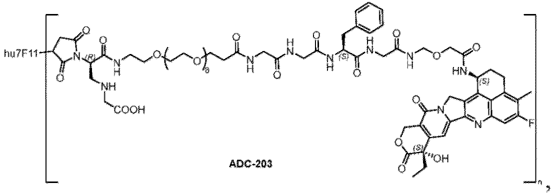 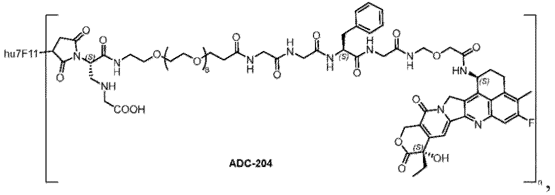 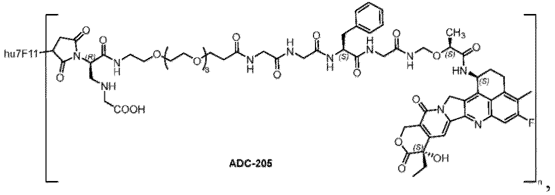 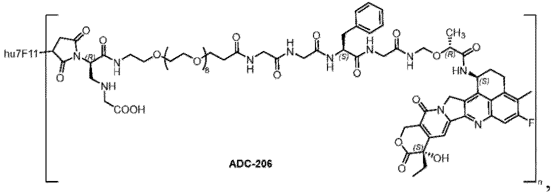 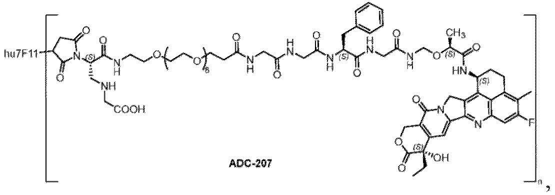 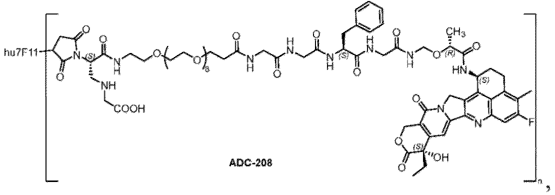 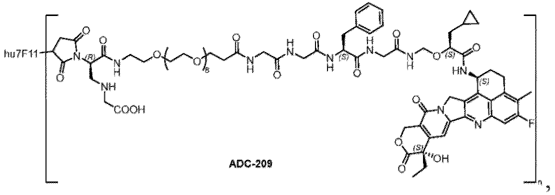 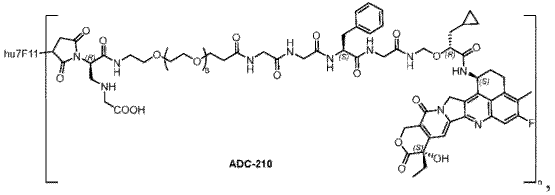 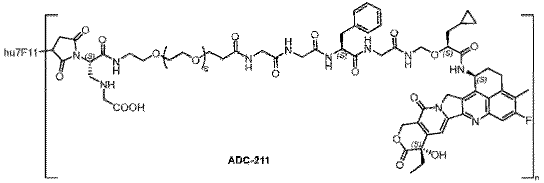 and 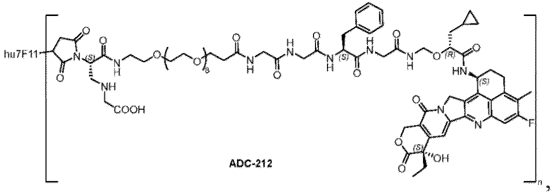 wherein
hu7F 11 is an antibody targeting human Trop2 or an antigen-binding fragment thereof;
n is selected from integers between 1 and 10 (e.g., 1, 2, 3, 4, 5, 6, 7, 8, 9 or 10).

In some embodiments of the first, second and fourth aspects of the present invention, the ligand-camptothecin derivative conjugate or a pharmaceutically acceptable salt or solvate thereof or the linker-drug compound or a pharmaceutically acceptable salt or solvate thereof is disclosed, wherein the pharmaceutically acceptable salt includes sodium salts, potassium salts, calcium salts or magnesium salts formed with acidic functional groups in the structural formula, and acetates, trifluoroacetates, citrates, oxalates, tartrates, malates, nitrates, chlorides, bromides, iodides, sulfates, bisulfates, phosphates, lactates, oleates, ascorbates, salicylates, formates, glutamates, methanesulfonates, ethanesulfonates, benzenesulfonates or p-toluenesulfonates formed with basic functional groups in the structure.

A fifth aspect of the present invention discloses a pharmaceutical composition comprising the ligand-camptothecin derivative conjugate as described in the first aspect and the second aspect or a pharmaceutically acceptable salt or solvate thereof or the linker-drug compound as described in the third aspect or a pharmaceutically acceptable salt or solvate thereof, and optionally a pharmaceutically acceptable carrier.

A sixth aspect of the present invention discloses a pharmaceutical formulation comprising the ligand-camptothecin derivative conjugate as described in the first aspect and the second aspect or a pharmaceutically acceptable salt or solvate thereof or the linker-drug compound as described in the third aspect or a pharmaceutically acceptable salt or solvate thereof.

A seventh aspect of the present invention discloses use of the ligand-camptothecin derivative conjugate as described in the first aspect and the second aspect or a pharmaceutically acceptable salt or solvate thereof, or the linker-drug compound as described in the third aspect or a pharmaceutically acceptable salt or solvate thereof, the pharmaceutical composition as described in the fifth aspect and/or the pharmaceutical formulation as described in the sixth aspect in preparation of a medicament for treating or preventing a cancer or tumor;
alternatively, the ligand-camptothecin derivative conjugate as described in the first aspect and the second aspect or a pharmaceutically acceptable salt or solvate thereof, or the linker-drug compound as described in the third aspect or a pharmaceutically acceptable salt or solvate thereof, or the pharmaceutical composition as described in the fifth aspect and/or the pharmaceutical formulation as described in the sixth aspect for treating or preventing a cancer or tumor;
preferably, the cancer or tumor expresses TROP2;
more preferably, the cancer or tumor is selected from solid tumors or blood tumors, such as adenocarcinoma, ovarian cancer, cervical cancer, uterine cancer, prostate cancer, kidney cancer, urethra cancer, bladder cancer, liver cancer, gastric cancer, endometrial cancer, salivary gland cancer, esophageal cancer, lung cancer, colon cancer, triple negative breast cancer, rectal cancer, colorectal cancer, bone cancer, skin cancer, thyroid cancer, pancreatic cancer, melanoma, glioma, neuroblastoma, glioblastoma multiforme, sarcoma, lymphoma and leukemia.

An eighth aspect of the present invention discloses a method for treating or preventing a cancer or tumor, including: administrating to a subject in need thereof a prophylactically or therapeutically effective amount of the ligand-camptothecin derivative conjugate as described in the first aspect and the second aspect or a pharmaceutically acceptable salt or solvate thereof, or the linker-drug compound as described in the third aspect or a pharmaceutically acceptable salt or solvate thereof, or the pharmaceutical composition as described in the fifth aspect and/or the pharmaceutical formulation as described in the sixth aspect;
preferably, the cancer or tumor expresses TROP2;
more preferably, the cancer or tumor is selected from solid tumors or blood tumors, such as adenocarcinoma, ovarian cancer, cervical cancer, uterine cancer, prostate cancer, kidney cancer, urethra cancer, bladder cancer, liver cancer, gastric cancer, endometrial cancer, salivary gland cancer, esophageal cancer, lung cancer, colon cancer, triple negative breast cancer, rectal cancer, colorectal cancer, bone cancer, skin cancer, thyroid cancer, pancreatic cancer, melanoma, glioma, neuroblastoma, glioblastoma multiforme, sarcoma, lymphoma and leukemia.

In the above aspects of the present invention and their embodiments,
the "C1-C6 alkyl" and the "C1-C6 alkyl" in various composite groups involving the "C1-C6 alkyl" (for example, "substituted C1-C6 alkyl", "deuterated C1-C6 alkyl") can be replaced by "C1-C20 alkyl", "C1-C12 alkyl" or "C1-C10 alkyl";
the "C3-C8 cycloalkyl" and the "C3-C8 cycloalkyl" in various composite groups involving the "C3-C8 cycloalkyl" can be replaced by "C3-C20 cycloalkyl" or "C3-C10 cycloalkyl";
the "C1-C6 alkoxy" and the "C1-C6 alkoxy" in various composite groups involving the "C1-C6 alkoxy" can be replaced by "C1-C20 alkoxy", "C1-C12 alkoxy" or "C1-C10 alkoxy";
the "C6-C10 aryl" and the "C6-C10 aryl" in various composite groups involving the "C6-C10 aryl" can be replaced by "C6-C12 aryl";
the "3- to 7-membered heterocyclyl" and the "3- to 7-membered heterocyclyl" in various composite groups involving the "3- to 7-membered heterocyclyl" can be replaced by "3- to 20-membered heterocyclyl", "3- to 12-membered heterocyclyl" or "3- to 10-membered heterocyclyl".

### Beneficial Effect

The anti-human Trop2 antibody-drug conjugate provided by the present invention is an antibody-drug conjugate obtained by coupling a humanized anti-human Trop2 antibody and a camptothecin drug. Compared with existing drugs of the same type, the anti-human Trop2 antibody-drug conjugate has good molecular stability and good preclinical efficacy, and is expected to have excellent clinical therapeutic effects.

### Brief Description of Figures

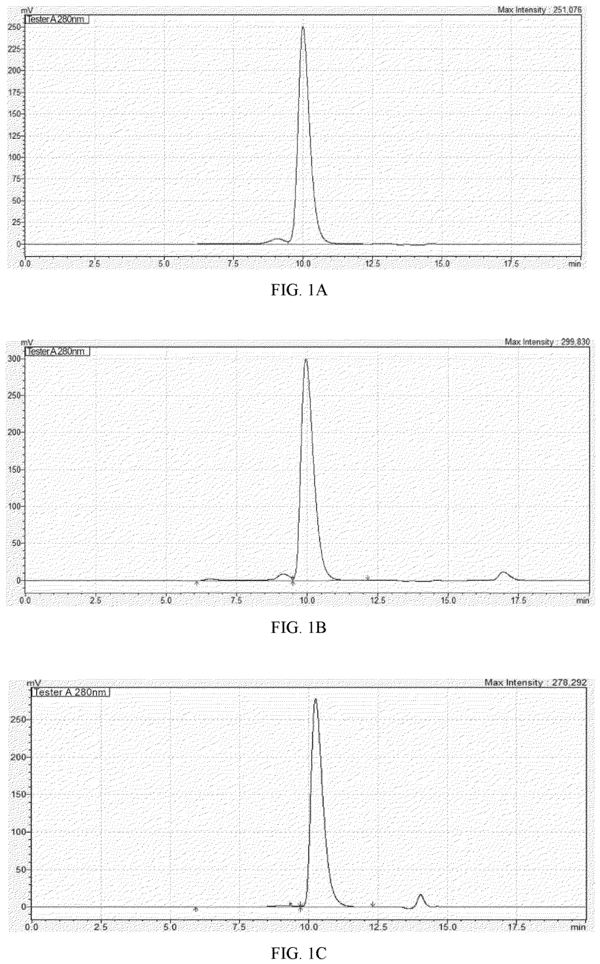

FIG. 1A illustrates the aggregation of DS1062a (DAR=4) detected by SEC-HPLC.
FIG. 1B illustrates the aggregation of ADC-1 detected by SEC-HPLC.
FIG. 1C illustrates the aggregation of ADC-2 detected by SEC-HPLC.

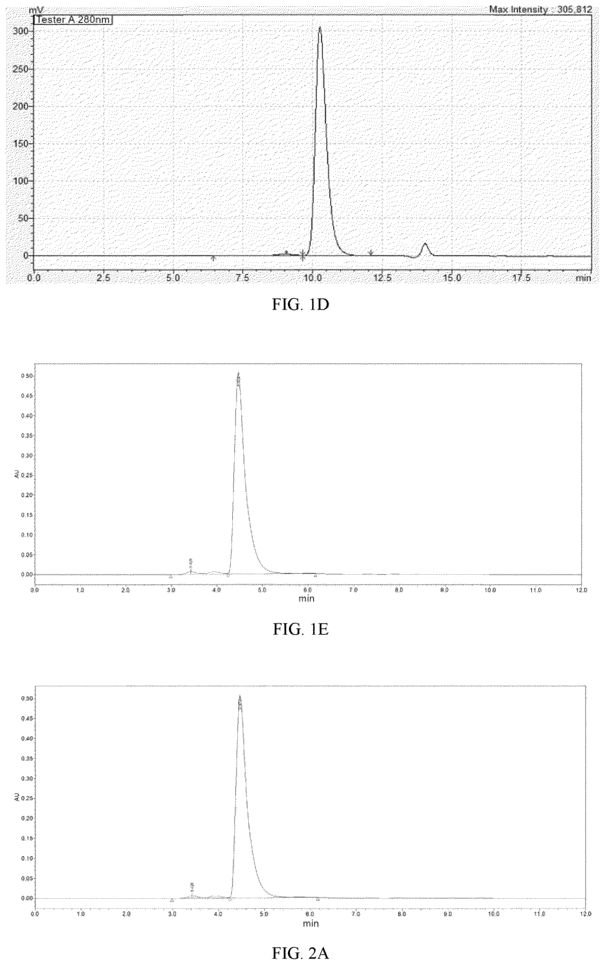

FIG. 1D illustrates the aggregation of ADC-6 detected by SEC-HPLC.
FIG. 1E illustrates the aggregation of ADC-10 detected by SEC-HPLC.
FIG. 2A illustrates the DAR of DS1062a (DAR=4) determined by RP-HPLC.

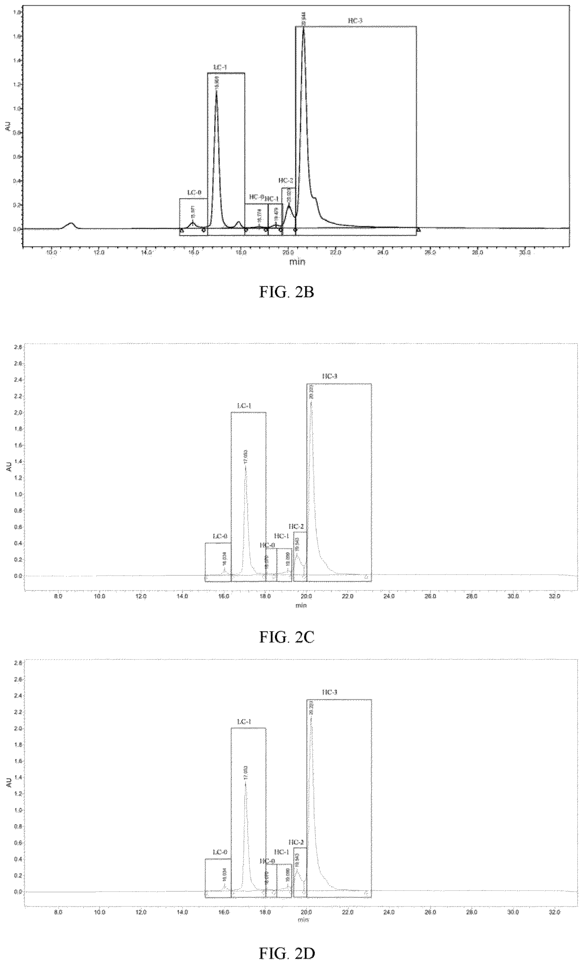

FIG. 2B illustrates the DAR of ADC-1 determined by RP-HPLC.
FIG. 2C illustrates the DAR of ADC-2 determined by RP-HPLC.
FIG. 2D illustrates the DAR of ADC-12 determined by RP-HPLC.

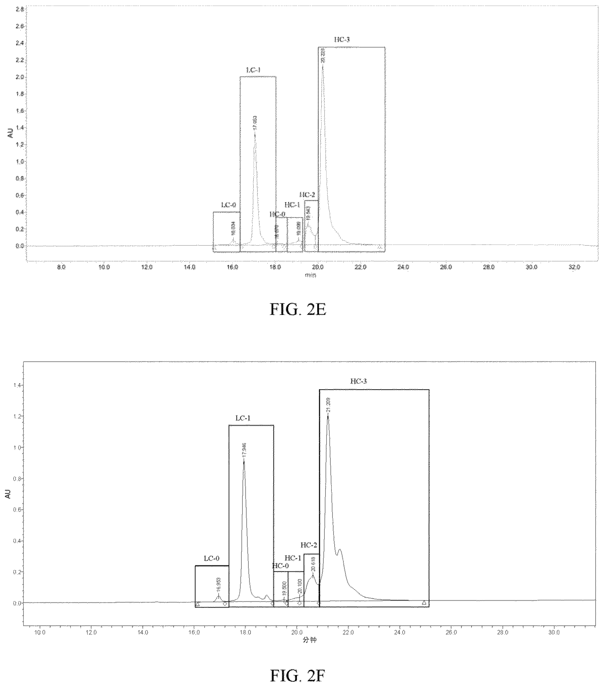

FIG. 2E illustrates the DAR of ADC-6 determined by RP-HPLC.
FIG. 2F illustrates the DAR of ADC-10 determined by RP-HPLC.
FIG. 3A illustrates that DS1062a maintains the same affinity for antigen TROP2 as antibody TINA1.
FIG. 3B illustrates that ADC-1 maintains the same affinity for antigen TROP2 as antibody TINA1.
FIG. 3C illustrates that ADC-2 maintains the same affinity for antigen TROP2 as antibody hu4D3.
FIG. 3D illustrates that ADC-12 maintains the same affinity for antigen TROP2 as antibody hu4D3.
FIG. 3E illustrates that ADC-6 maintains the same affinity for antigen TROP2 as antibody hu4D3.
FIG. 3F illustrates that ADC-10 maintains the same affinity for antigen TROP2 as antibody hu4D3.
FIG. 4 illustrates the efficacy evaluation of ADC and corresponding antibodies on BXPC-3 cell.
FIG. 5A illustrates that the in vivo efficacy of ADC-6, ADC-10 and ADC-12 in a BXPC3 single tumor model is superior to that of DS1062a and ADC-1.
FIG. 5B illustrates the results of in vivo efficacy experiments of ADC-1, ADC-6, ADC-12 and DS1062a in A431+SW620 heterogeneous tumor.
FIG. 6A illustrates the in vitro tumor inhibitory activity of naked antibodies hu4D3 and hu7F11 and compounds **d₃**, **d₃₈**, **d₃₉**, **d₄₄** in an experimental model of human tumor cell line N87.
FIG. 6B illustrates the in vitro tumor inhibitory activity of naked antibodies hu4D3 and hu7F11 and compounds **d₃**, **d₃₈**, **d₃₉**, **d₄₄** in an experimental model of human tumor cell line SW620.
FIG. 6C illustrates the in vitro tumor inhibitory activity of naked antibodies hu4D3 and hu7F11 and compounds **d₃, d₃₈, d₃₉, d₄₄** in an experimental model of human tumor cell line HCC827.
FIG. 6D illustrates the in vitro tumor inhibitory activity of naked antibodies hu4D3 and hu7F11 and compounds **d₃, d₃₈, d₃₉, d₄₄** in an experimental model of human tumor cell line FaDu.
FIG. 6E illustrates the in vitro tumor inhibitory activity of naked antibodies hu4D3 and hu7F11 and compounds **d₃, d₃₈, d₃₉, d₄₄** in an experimental model of human tumor cell line A431+SW620.
FIG. 7A illustrates the in vitro tumor inhibitory activity of ADC-47, ADC-70, ADC-72, ADC-73, ADC-112, ADC-176, ADC-178, and ADC-179 in an experimental model of human tumor cell line N87.
FIG. 7B illustrates the in vitro tumor inhibitory activity of ADC-47, ADC-70, ADC-72, ADC-73, ADC-112, ADC-176, ADC-178, and ADC-179 in an experimental model of human tumor cell line SW620.
FIG. 7C illustrates the in vitro tumor inhibitory activity of ADC-47, ADC-70, ADC-72, ADC-73, ADC-112, ADC-176, ADC-178, and ADC-179 in an experimental model of human tumor cell line HCC827.
FIG. 7D illustrates the in vitro tumor inhibitory activity of ADC-47, ADC-70, ADC-72, ADC-73, ADC-112, ADC-176, ADC-178, and ADC-179 in an experimental model of human tumor cell line FaDu.
FIG. 7E illustrates the in vitro tumor inhibitory activity of ADC-47, ADC-70, ADC-72, ADC-73, ADC-112, ADC-176, ADC-178, and ADC-179 in an experimental model of human tumor cell line A431+SW620.

### DETAILED DESCRIPTION OF EMBODIMENTS

### Abbreviations and Definitions

Unless otherwise stated, the following terms and phrases, as used herein, are intended to have the following meanings. When a trade name is used herein, the trade name includes the product formulation, generics, and active ingredients of the product with the trade name, unless the context indicates otherwise.

Unless stated to the contrary, the terms used in the claims and description herein have the following meanings.

The term "ligand" generally refers to a macromolecular compound capable of recognizing and binding to an antigen or receptor related to a target cell. The role of ligands is to deliver drugs to the target cell populationbound to the ligands. These ligands include but are not limited to protein hormones, lectins, growth factors, antibodies or other molecules capable of binding to cells. In the embodiments of the present invention, the ligand is represented as Ab. The ligand can form a linking bond with a linking unit through a heteroatom on the ligand, and is preferably an antibody or an antigen-binding fragment thereof. The antibody is selected from chimeric antibodies, humanized antibodies, fully human antibodies or murine antibodies. Preferably, the antibody is a monoclonal antibody.

A ligand unit is a targeting agent that specifically binds to a target moiety. The ligand is capable of specifically binding to a cellular component or to a cellular component or to other target molecules of interest. The targeting moiety or target is usually on the cell surface. In some aspects, the ligand unit functions to deliver the drug unit to a specific target cell population with which the ligand unit interacts. The ligands include, but are not limited to, proteins, polypeptides and peptides, as well as non-proteins such as sugars. Suitable ligand units include, for example, antibodies, such as full-length (intact) antibodies and antigen-binding fragments thereof. In embodiments where the ligand unit is a non-antibody targeting agent, it may be a peptide or polypeptide, or a non-protein molecule. Examples of such targeting agents include interferons, lymphokines, hormones, growth factors and colony-stimulating factors, vitamins, nutrient transport molecules, or any other cell-binding molecules or substances. In some embodiments, the linker is covalently attached to the sulfur atom of the ligand. In some aspects, the sulfur atom is that of a cysteine residue that forms an interchain disulfide bond of the antibody. In another aspect, the sulfur atom is a sulfur atom that has been introduced into a cysteine residue of the ligand unit, which forms an interchain disulfide bond of the antibody. In another aspect, the sulfur atom is a sulfur atom that has been introduced into a cysteine residue of the ligand unit (e.g., by site-directed mutagenesis or chemical reaction). In other aspects, the sulfur atom bound by the linker is selected from cysteine residues that form interchain disulfide bonds of an antibody or cysteine residues that have been introduced into a ligand unit (e.g., by site-directed mutagenesis or chemical reaction). In some embodiments, the EU index numbering system is in accordance with Kabat {[Kabat E.A et al., (1991)] Sequences of proteins of Immunological Interest, Fifth Edition, NIH Publication 91-3242"}.

As used herein, "antibody" or "antibody unit" includes, within its scope, any part of the antibody structure. This unit can bind, reactively associate, or complex a receptor, antigen, or other receptor unit present in the target cell population. The antibody may be any protein or proteinaceous molecule that binds, complexes, or reacts with a portion of the cell population to be treated or biomodified. The antibody constituting the antibody-drug conjugate of the present invention retains its original antigen-binding ability in the wild state. Therefore, the antibody of the present invention can specifically bind to an antigen. Antigens involved include, for example, tumor-associated antigens (TAA), cell surface receptor proteins and other cell surface molecules, regulators of cell survival, regulators of cell proliferation, molecules associated with tissue growth and differentiation (such as known or predicted functional molecules), lymphokines, cytokines, molecules involved in cell cycle regulation, molecules involved in angiogenesis, and molecules related to angiogenesis (such as known or predicted functional molecules). Tumor-associated factors may be cluster differentiation factors (such as CD proteins).

Antibodies for use in antibody-drug conjugates include, but are not limited to, antibodies directed against cell surface receptors and tumor-associated antigens. Such tumor-associated antigens are well known in the art, and can be prepared by well-known antibody production methods and information in the art. In order to develop effective cellular-level targets for cancer diagnosis and treatment, researchers strive to find transmembrane peptides or other tumor-associated peptides. These targets can be specifically expressed on the surface of one or more cancer cells, with little or no expression on the surface of one or more non-cancer cells. Typically, such tumor-associated polypeptides may be more overexpressed on the surface of cancer cells relative to the surface of non-cancer cells. Identifying such tumor-associated factors can greatly improve the specific targeting properties of antibody-based cancer treatment. For convenience, antigen-related information well known in the industry is marked as follows, including name, other names, and Genbank accession numbers. Nucleic acid and protein sequences corresponding to tumor-associated antigens can be found in public databases, such as Genbank. The tumor-associated antigens targeted by the antibody include all amino acid sequence variants and homologs, and have at least 70%, 80%, 85%, 90% or 95% homology with the sequence confirmed in the reference, or have completely consistent biological properties and characteristics with the tumor-associated antigen sequences in the reference cited.

The term "inhibit" or "inhibition of" means reduction in a detectable amount or complete prevention.

The term "cancer" refers to a physiological condition or disease characterized by unregulated cell growth. The "tumor" includes cancer cells.

The term "autoimmune disease" is a disease or disorder arising from and directed against an individual's own tissues or proteins.

The term "drug" refers to cytotoxic drug, represented by d, which is a chemical molecule that has strong ability to disrupt the normal growth of tumor cells. In principle, the cytotoxic drug can kill tumor cells at high enough concentrations. However, due to lack of specificity, while killing tumor cells, the cytotoxic drug can also cause apoptosis of normal cells, leading to serious side effects. The term includes toxins such as small molecule toxins or enzymatically active toxins of bacterial, fungal, plant or animal origin, radioactive isotopes (such as radioactive isotopes of At²¹¹, I¹³¹, I¹²⁵, Y⁹⁰, Re¹⁸⁶, Re¹⁸⁸, Sm¹⁵³, Bi²¹², P³² and Lu¹⁷⁶), toxic drugs, chemotherapeutic drugs, antibiotics and ribozymes, preferably toxic drugs.

The term "linker" or "linking fragment" or "linking unit" refers to a chemical structural fragment or bond that is connected to a ligand at one end and a drug at the other end. It can also be connected to other linkers and then connected to the drug.

The linker, including extenders, spacers and amino acid units, can be synthesized by methods known in the art, such as those described in US2005-0238649A1. The linker can be a "cleavable linker" that facilitates release of the drug in cells. For example, acid-labile linkers (e.g., hydrazone), protease-sensitive (e.g., peptidase-sensitive) linkers, photolabile linkers, dimethyl linkers, or disulfide-containing linkers can be used (Chari et al. Cancer Research 52:127-131, 1992); U.S. Patent No. 5,208,020.

According to the mechanism of intracellular drug release, "linker" or "linker of the antibody drug conjugate" as used herein can be divided into two categories: non-cleavable linkers and cleavable linkers. For antibody-drug conjugates containing non-cleavable linkers, the drug release mechanism is as follows: after the conjugate binds to the antigen and is endocytosed by cells, the antibody is enzymatically hydrolyzed in lysosomes, releasing active molecules composed of small molecule drugs, linkers, and antibody amino acid residues. The resulting change in the structure of the drug molecule does not weaken its cytotoxicity, but because the active molecule is charged (amino acid residue), it cannot penetrate into neighboring cells. Therefore, such active drugs cannot kill adjacent tumor cells (bystander effect) that do not express the target antigen (antigen-negative cells) (Ducry et al., 2010, Bioconjugate Chem. 21: 5-13). For antibody-drug conjugates containing cleavable linkers, the drug release mechanism is as follows: after the conjugate binds to the antigen and is endocytosed by cells, it is broken in the target cell and releases the active ingredient (the small molecule drug itself). The cleavable linkers are mainly divided into: chemosensitive linkers and enzyme-sensitive linkers. The chemosensitive linkers can be selectively cleaved due to differences in the properties of plasma and cytoplasm or tumor microenvironments. Such properties include pH, glutathione concentration, etc. Linkers that are sensitive to pH are relatively stable in the neutral or weakly alkaline environment of blood (pH 7.3-7.5), but will be hydrolyzed in the weakly acidic tumor microenvironment (pH 5.0-6.5) and lysosomes (pH 4 .5-5.0), such as hydrazones, carbonates, acetals, and ketals. Due to the limited plasma stability of acid-cleaved linkers, antibody-drug conjugates based on this type of linker usually have a short half-life (2-3 days). This short half-life limits the application of pH-sensitive linkers in new generation antibody-drug conjugates to a certain extent. Glutathione-sensitive linkers are also called disulfide linkers. Drug release is caused by the difference between high intracellular glutathione concentration (millimolar range) and relatively low glutathione concentration in the blood (micromolar range). This is especially true for tumor cells, where low oxygen content leads to increased activity of reductase enzymes and thus higher glutathione concentration. Disulfide bonds are thermodynamically stable and therefore have good stability in plasma. Enzyme-labile linkers, such as peptide linkers, enable better control of drug release. Peptide linkers can be effectively cleaved by lysosomal proteases, such as cathepsin B. Such as peptide linkers are believed to be very stable in the plasma circulation because proteases are usually inactive outside cells due to inappropriate extracellular pH and serum protease inhibitors. Enzyme-labile linkers are widely used as cleavable linkers for antibody-drug conjugates due to their high plasma stability and good intracellular cleavage selectivity and effectiveness.

The term "antibody-drug conjugate" refers to connecting an antibody to a biologically active drug by a stable linking unit. In the present invention, "ligand-drug conjugate" is preferably an antibody-drug conjugate (ADC), which refers to connecting a monoclonal antibody or antibody fragment to a biologically active toxic drug by a stable linking unit.

The three-letter and single-letter codes for amino acids used in the present disclosure are as described in J. boil. Chem. 1968, 243, 3558.

The term "alkyl" refers to a saturated aliphatic hydrocarbon group, which is a linear or branched chain group containing 1 to 20 carbon atoms (i.e., "C1-C20 alkyl"), preferably an alkyl containing 1 to 12 carbon atoms (i.e., "C1-C12 alkyl"), more preferably an alkyl containing 1 to 10 carbon atoms (i.e., "C1-C10 alkyl"), most preferably an alkyl containing 1 to 6 carbon atoms (i.e., "C1-C6 alkyl"). Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl,n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio, heterocycloalkylthio, and oxo.

The term "substituted alkyl" refers to that hydrogen in an alkyl group is replaced by a substituent group. Unless otherwise stated in the context, the substituent of the alkyl group may be a variety of groups selected from the group consisting of: - halogen, -OR', -NR'R", -SR', -SiR'R"R‴, -OC(O)R', -C(O)R', -CO₂R', -CONR'R", -OC(O)NR'R", -NR"C (O) R', -NR'-C(O)NR"R‴, -NR"C(O)₂R', -NH-C(NH₂)=NH, -NR'C(NH₂)=NH, -NH-C(NH₂)=NR', -S(O)R', -S(O)₂R', - S(O)₂NR'R", -NR'S(O)₂R", -CN and -NO₂. The number of substituents ranges from 0 to (2m'+1), where m' is the total number of carbon atoms in the group. R', R" and R‴ each independently refer to hydrogen, unsubstituted C₁₋₈ alkyl, unsubstituted C6-C12 aryl (or C6-C10 aryl), C6-C12 aryl (or C6-C10 aryl) substituted by one to three halogens, unsubstituted C₁₋₈ alkyl, C₁₋₈ alkoxy or C₁₋₈ thioalkoxy, or unsubstituted C6-C12 aryl (or C6-C10 aryl)-C₁₋₄ alkyl. When R' and R" are connected to the same nitrogen atom, they can form a 3-, 4-, 5-, 6- or 7-membered ring together with the nitrogen atom. For example, -NR'R" includes 1-pyrrolidinyl and 4-morpholinyl.

The term "alkylene" refers to a saturated linear or branched aliphatic hydrocarbon group having two residues derived from the removal of two hydrogen atoms from the same carbon atom or two different carbon atoms of the parent alkane. The alkylene is a linear or branched group having 1 to 20 carbon atoms, preferably 1 to 12 carbon atoms, and more preferably 1 to 6 carbon atoms. Non-limiting examples of alkylene include, but are not limited to, methylene (-CH₂-, 1,1-ethylene (-CH(CH₃)-), 1,2-ethylene (-CH₂CH₂)-, 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), 1,5-pentylene (-CH₂CH₂CH₂CH₂CH₂-), and the like. The alkylene can be substituted or unsubstituted. When substituted, the substituent group(s) can be substituted at any available connection point. The substituent group(s) is preferably one or more groups independently optionally selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocycloalkylthio and oxo.

The term "alkoxy" refers to an -O-(alkyl) and an -O- (cycloalkyl) group, wherein the alkyl and cycloalkyl are as defined above. Non-limiting examples of C1-C6 alkoxy include: methoxy, ethoxy, propoxy, butoxy, cyclopropyloxy, cyclobutyloxy, cyclopentyloxy, and cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio, and heterocycloalkylthio.

The term "cycloalkyl" refers to a saturated or partially unsaturated monocyclic or polycyclic hydrocarbon substituent group having 3 to 20 carbon atoms (i.e., "C3-C20 cycloalkyl"), preferably 3 to 12 carbon atoms (i.e. "C3-C12 cycloalkyl"), more preferably 3 to 10 carbon atoms (i.e. "C3-C10 cycloalkyl"), and most preferably 3 to 8 carbon atoms (i.e. "C3-C8 cycloalkyl"). Non-limiting examples of monocyclic cycloalkyl (e.g., "C3-C8 cycloalkyl") include cyclopropyl, cyclobutyl, cyclopentyl, cyclopentenyl, cyclohexyl, cyclohexenyl, cyclohexenyl Dialkenyl, cycloheptyl, cycloheptatrienyl, cyclooctyl, etc. Polycyclic cycloalkyl includes a cycloalkyl having a spiro ring, fused ring or bridged ring.

The term "heterocyclyl" refers to a saturated or partially unsaturated monocyclic or polycyclic hydrocarbon group containing 3 to 20 ring atoms (i.e.,"3- to 20-membered heterocyclyl"), in which one or more ring atoms are heteroatoms selected from the group consisting of N, O and S(O)m (where m is an integer of 0 to 2), but excluding -O-O-, -O-S- or -S-S- in the ring, with the remaining ring atoms being carbon atoms. Preferably, the heterocyclyl contains 3 to 12 ring atoms (i.e. "3- to 12-membered heterocyclyl"), of which 1 to 4 atoms are heteroatoms; and more preferably the cycloalkyl ring contains 3 to 10 ring atoms (i.e. "3- to 10-membered heterocyclyl"). Non-limiting examples of monocyclic heterocyclyl (e.g., 3- to 7-membered heterocyclyl) include pyrrolidinyl, piperidinyl, piperazinyl, morpholinyl, thiomorpholinyl, homopiperazinyl, and the like. Polycyclic heterocyclyl includes a heterocyclyl having a spiro ring, fused ring or bridged ring.

The term "cycloalkylalkyl" refers to an alkyl group substituted by one cycloalkyl or more, preferably one cycloalkyl, wherein the alkyl and the cycloalkyl are as defined above. Examples of cycloalkylalkyl include C3-C8 cycloalkyl C1-C6 alkyl.

The term "haloalkyl" refers to an alkyl group substituted by one or more halogen(s), where the alkyl is as defined above. Examples of haloalkyl include halogenated C1-C6 alkyl.

The term "deuterated alkyl" refers to an alkyl group substituted by one or more deuterium atom(s), where the alkyl is as defined above. Examples of deuterated alkyl include deuterated C1-C6 alkyl.

The term "C6-C12 aryl" refers to a group of a carbocyclic aromatic system having 6 to 12 carbon atoms.

The term "C6-C10 aryl" refers to a group of a carbocyclic aromatic system having 6 to 10 carbon atoms. Examples of C6-C10 aryl are phenyl, naphthyl, etc.

The term "5- to 10-membered heteroaryl" refers to an aromatic heterocyclic ring, usually a 5-, 6-, 7-, 8-, 9-, or 10-membered heterocyclic ring with 1 to 3 heteroatoms selected from N, O or S. A heteroaryl ring may optionally be further fused or attached to aromatic and nonaromatic carbocyclic and heterocyclic rings. Non-limiting examples of the 5- to 10-membered heteroaryl are, for example, pyridyl, pyrazinyl, pyrimidinyl, pyridazinyl, indolyl, imidazolyl, thiazolyl, isothiazolyl, thioxazolyl, pyrrolyl, phenyl-pyrrolyl, furyl, phenyl-furyl, oxazolyl, isoxazolyl, pyrazolyl, thienyl, benzofuranyl, benzothienyl, benzo-1,3-dioxolane (benzodioxin), isoindolyl, benzimidazolyl, indazolyl, quinolyl, isoquinolyl, 1,2,3-triazolyl, 1-phenyl-1,2,3-triazolyl, 2,3-indolinyl, 2,3-dihydrobenzofuranyl, 2,3-dihydrobenzothienyl, benzopyrin pyryl, 2,3-dihydrobenzoxazinyl, 2,3-dihydroquinoxalinyl, etc.

The term "substituted C6-C10 aryl" or "substituted 5- to 10-membered heteroaryl" or "substituted 3- to 7-membered heterocyclyl" refers to that hydrogen in aryl or heteroaryl or heterocyclyl is substituted by a substituent group, unless otherwise stated in the context, the substituent of aryl or heteroaryl or heterocyclyl may be a variety of groups selected from the group consisting of: -halogen, -OR', -NR'R", -SR', -SiR'R"R‴, -OC(O)R', -C(O)R', -CO₂R', -CONR'R", -OC(O)NR'R", -NR"C (O) R', -NR'-C(O)NR"R‴, -NR"C(O)₂R', -NH-C(NH₂)=NH, -NR'C(NH₂)=NH, -NH-C(NH₂)=NR', -S(O)R', -S(O)₂R', -S(O)₂NR'R", -NR'S(O)₂R", -CN and -NO₂. The number of substituents ranges from 0 to (2m'+1), where m' is the total number of carbon atoms in the group. R', R" and R‴ each independently refer to hydrogen, unsubstituted C₁₋₈ alkyl, unsubstituted C6-C12 aryl (or C6-C10 aryl), C6-C12 aryl (or C6-C10 aryl) substituted by one to three halogens, unsubstituted C₁₋₈ alkyl, C₁₋₈ alkoxy or C₁₋₈ thioalkoxy, or unsubstituted C6-C12 aryl (or C6-C10 aryl)-C₁₋₄ alkyl. when R' and R" are connected to the same nitrogen atom, they can form a 3-, 4-, 5-, 6- or 7-membered ring together with the nitrogen atom. For example, -NR'R" includes 1-pyrrolidinyl and 4-morpholinyl.

The term "hydroxy" refers to an -OH group.

The term "halogen" refers to fluorine, chlorine, bromine or iodine.

The term "amino" refers to a -NH₂ group. The term "nitro" refers to a -NO₂ group.

The term "amide" refers to a -C(O)N(alkyl) or -C(O)N(cycloalkyl) group, where the alkyl and cycloalkyl are as defined above.

The term "carboxylate group" refers to a -C(O)O(alkyl) or -C(O)O(cycloalkyl) group, where the alkyl and cycloalkyl are as defined above.

The present invention also comprises formula **I** in various deuterated forms. Each of the available hydrogen atoms attached to the carbon atom can be independently replaced by a deuterium atom. Those skilled in the art can synthesize formula **I** in a deuterated form with reference to the relevant literatures. The formula **I** in deuterated form can be prepared by employing commercially available deuterated raw materials, or they can be synthesized by conventional techniques with deuterated reagents. Non-limiting examples of deuterated reagents include: deuterated borane, trideuterated borane in tetrahydrofuran, deuterated lithium aluminum hydride, deuterated iodoethane, deuterated iodomethane and the like.

The term "antibody" refers to immunoglobulin, a four-peptide chain structure connected together by interchain disulfide bond between two identical heavy chains and two identical light chains. Different immunoglobulin heavy chain constant regions exhibit different amino acid compositions and sequences, hence presenting different antigenicity. Accordingly, immunoglobulins can be divided into five types, or called immunoglobulin isotypes, namely IgM, IgD, IgG, IgA and IgE, with corresponding heavy chains *µ*, *δ, γ, α* and ε, respectively. According to the amino acid composition of hinge region and the number and location of heavy chain disulfide bonds, the same type of Ig can further be divided into different sub-types, for example, IgG can be divided into IgG1, IgG2, IgG3 and IgG4. Light chain can be divided into *κ* or λ chain based on different constant region. Each of the five types of Ig can have a *κ* or λ chain. The antibodies described in the present invention are preferably specific antibodies against the cell surface antigens on the target cells, and non-limiting examples are one or more of the following antibodies: anti-EGFRvIII antibody, anti-DLL-3 antibody, anti-PSMA antibody, anti-CD70 antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-cladin 18.2 antibody, anti-Mesothelin antibody, anti-HER2(ErbB2) antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3(ErbB3) antibody, anti-MUC1/CD227 antibody, anti-AXL antibody, anti-CD166 antibody, anti-B7-H3(CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody, anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody, anti-CD138 antibody, anti-CD352 antibody, anti-CD25 antibody, and anti-CD123 antibody.

The term "solvate" or "solvent compound" refers to a pharmaceutically acceptable solvate formed by a ligand-drug conjugate of the present invention with one or more solvent molecule(s). Non-limiting examples of solvent molecules include water, ethanol, acetonitrile, isopropanol, DMSO, and ethyl acetate.

The term "drug loading" refers to the average number of cytotoxic drugs loaded on each ligand in formula **I**, and can also be expressed as a drug to antibody ratio (DAR). The drug loading can range from 0 to 12, preferably from 1 to 10 cytotoxic drugs (d) per antibody (Ab). In an embodiment of the present invention, the drug loading is expressed as n, and exemplary values can be an average of 1, 2, 3, 4, 5, 6, 7, 8, 9, 10. The average number of drugs per ADC molecule after coupling reaction can be determined by conventional methods such as UV/visible spectroscopy, mass spectrometry, ELISA test and HPLC characterization.

In an embodiment of the present invention, the cytotoxic drug is conjugated to an opened cysteine thiol-SH between antibody chains and/or the thiol-SH of site-directed mutated cysteine residue by a linking unit. Typically, the number of drug molecules capable of being conjugated to the antibody in a coupling reaction will be less than the theoretical maximum.

The following non-limiting methods can be used to control the loading of the ligand-cytotoxic drug conjugates:
(1) controlling the molar ratio of the linking reagent to the monoclonal antibody,
(2) controlling the reaction time and temperature, and
(3) selecting different reaction reagents.

The preparation of conventional pharmaceutical compositions can be found in the Chinese Pharmacopoeia.

The term "pharmaceutically acceptable salt" or "medicinal salt" generally refers to the salts of the ligand-drug conjugate of the present invention or salts of the compound of the present invention. Such salts may have safety and/or effectiveness when used in mammals, and may have corresponding biological activity. The ligand-drug conjugate compound of the present invention comprises at least one carboxyl, so it can form a salt with abase. Non-limiting examples of the pharmaceutically acceptable salt include: sodium salt, potassium salt, calcium salt, and magnesium salt.

The term "pharmaceutically acceptable salt" or "medicinal salt" generally refers to the salts of the antibody-drug conjugate of the present invention or salts of the compound of the present invention. Such salts may have safety and/or effectiveness when used in mammals, and may have corresponding biological activity. The ligand-drug conjugate compound of the present invention comprises at least one amino, so it can form a salt with an acid. Non-limiting examples of the pharmaceutically acceptable salt include: hydrochloride, hydrobromide, hydroiodide, sulfate, hydrogen sulfate, citrate, acetate, succinate, ascorbate, oxalate, nitrate, pearate, hydrogen phosphate, dihydrogen phosphate, salicylate, hydrogen citrate, tartrate, maleate, fumarate, formate, benzoate, mesylate, ethanesulfonate, benzenesulfonate, and p-toluenesulfonate.

"Acidic amino acid" refers to an amino acid with an isoelectric point less than 7. An acidic amino acid molecule often has one or more acidic groups such as carboxyl, which can be effectively ionized into negative ions in the structure to increase hydrophilicity. Acidic amino acids can be natural or unnatural amino acids.

"Natural amino acids" refer to amino acids synthesized by organisms. Natural amino acids are generally in the L-form, but there are a few exceptions, such as glycine. The natural amino acids include natural and biologically synthesized amino acids.

"Unnatural amino acids" refer to amino acids obtained by synthetic means.

The present invention will be further described below with reference to specific examples. It should be understood that these examples are only used to illustrate the present invention and are not intended to limit the scope of the present invention. The experimental methods without specifying specific conditions in the following examples are generally conducted under conventional conditions or conditions recommended by the manufacturers. Unless otherwise stated, all percentages, proportions, ratios, or parts are calculated by weight.

### Example 1

### Synthesis of Compound M1:

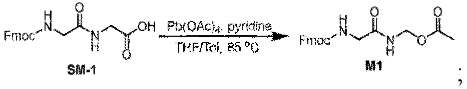

In a 5000 mL single-neck flask, N-fluorenylmethoxycarbonyl-glycine-glycine (100 g, 282 mmol, 1.0 eq), lead tetraacetate (175 g, 395 mmol, 1.4 eq), 2000 mL of dry tetrahydrofuran and 670 mL of toluene were mixed and stirred adequately, and then heated to 85 °C in the presence of N₂ to react for 2.5 h. After the reaction was found to be completed from the monitoring of the TLC, the reaction system was cooled to room temperature and filtered, the filtrate was concentrated under reduced pressure, and the residue was purified by column chromatography to obtain Compound **M1** (87 g); LC-MS: [M+NH₄]⁺=386.0.

### Example 2

### Synthesis of Compound M3:

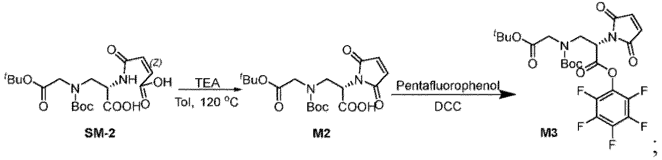

In a 1000 mL single-neck flask, Compound **SM-2** (prepared according to the synthesis route disclosed by patent application CN108452321A) (40 g, 96 mmol, 1.0 eq), triethylamine (26.7 mL, 2.0 eq), and toluene (400 mL) were mixed and then heated to 120 °C to reflux for 2 h. After the reaction was found to be completed from the monitoring of the TLC, the reaction system was cooled to 50 °C and rotary evaporation under reduced pressure was performed to remove the solvent. The reaction product was then dissolved in ethyl acetate (150 mL) and water (40 mL), and 1M of HCl was then added to adjust the pH to 2-3 with stirring in an ice bath. The resulting solution was allowed for liquid separation, the aqueous layer was extracted once more with ethyl acetate, the organic layers were combined, and dried with anhydrous Na₂SO₄. After filtration, the filtrate was concentrated to obtain a light yellow oily rude product. The rude product was purified by column chromatography (DCM:MeOH=40:1) to obtain Compound **M2** (26.6 g); LC-MS: [M+H]⁺=399.3.

In a 1000 mL single-neck flask, Compound **M2** (26.5 g, 60.5 mmol, 1.0 eq), pentafluorophenol (12.2 g, 66.5 mmol, 1.1 eq), DCC (13.7 g, 66.5 mmol, 1.1 eq) and THF (300 mL) were mixed and the reaction was allowed at room temperature for 30 min and monitored by TLC. The insoluble matter was filtered out. The reaction solution was directly purified by preparation. The preparation solution was concentrated in a reduced-pressure (water pump) water bath at 35 °C to remove acetonitrile, and then lyophilized to obtain Compound **M3** (31.5 g) with a yield of 64%; LC-MS: [M+H]⁺=565.1.

### Example 3

### Synthesis of Compound ent-M3:

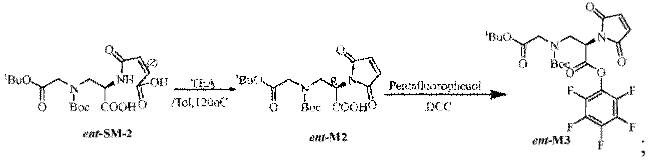

Compound ***ent*-M3** (27.8 g) was obtained by referring to the synthesis route of example 2; LC-MS: [M+H]⁺=565.2.

### Example 4

### Synthesis of Compound 1:

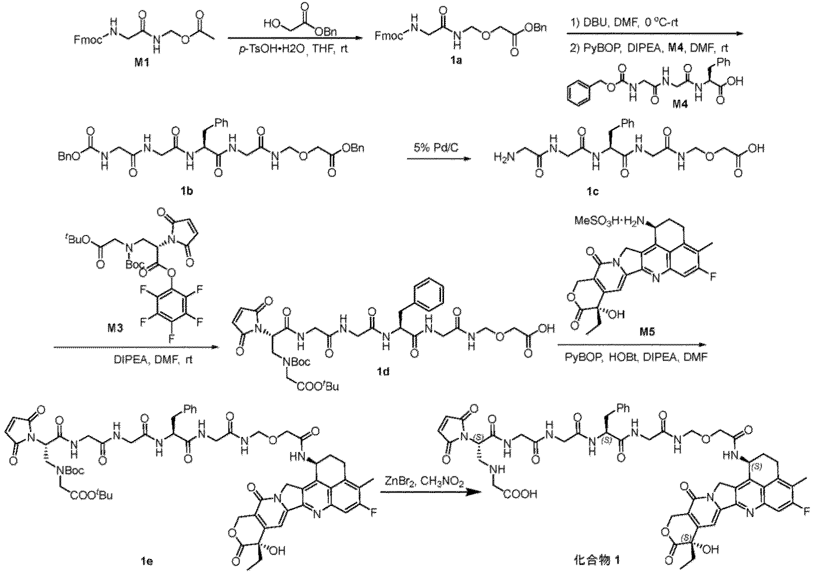

### First step: Compound 1a

In a 250 mL single-neck flask, **M1** (6 g, 16.3 mmol), 100 mL of THF, and p-toluenesulfonic acid monohydrate (0.31 g, 1.63 mmol) were mixed and stirred and then cooled to 0 °C, benzyl glycolate (5.4 g, 32.6 mmol) was then added dropwise. The resulting solution was then brought naturally to room temperature and stirred for about 2-4 h. After the reaction was found to be completed from the monitoring of the TLC, the reaction system was quenched with a saturated NaHCO₃ solution, and then extracted with ethyl acetate, the product was washed with saturated NaCl solution, then dried with anhydrous Na₂SO₄, filtered and concentrated, and the residue is purified on a silica gel column (PE:EA=10:1-5:1-1:1) to obtain **1a** (4 g), with a yield of 52%; LC-MS: [M+H]⁺=475.18.

### Second step: Compound 1b

In a 25 mL single-neck flask, Compound **1a** (2 g, 4.2 mmol) and 10 mL of DMF were mixed and stirred at 0 °C, and DBU (766 mg, 5.04 mmol) was then added to have a reaction for 1 h. After Fmoc deprotection was found to be completed from the monitoring of the TLC, the resulting product was ready for later use.

In another 25 mL single-neck flask, **M4** (prepared according to the synthesis route disclosed in CN111051330 A) (1.73 g, 4.2 mmol), PyBOP (2.61 g, 5.04 mmol), HOBt (680 mg, 5.04 mmol) and 10 mL of DMF were mixed, DIPEA (830 uL, 5.04 mmol) was then added in an ice water bath, the resulting reaction solution was stirred for 30 min and then placed in a reaction flask to have a reaction at room temperature. After the reaction was found to be completed from the monitoring of the HPLC, the reaction solution was purified through preparative liquid chromatography to obtain a product preparation solution. The preparation solution was extracted with dichloromethane, then washed with saturated NaCl solution, dried with anhydrous Na₂SO₄ and filtered. The filtrate was concentrated under reduced pressure to obtain a solid product **1b** (1.7 g), with a yield of 63%; LCMS: [M+H]⁺=648.26.

### Third step: Compound 1c

In a 25 mL single-neck flask, Compound **1b** (900 mg, 1.39 mmol) was completely dissolved in 15 mL of DMF and 900 mg of 5% Pd/C was then added to have a hydrogenation reaction for 2 h. After the reaction was found to be completed, the reaction solution was filtered and the filtrate was directly put into the next reaction without purification.

### Fourth step: Compound 1d

The crude product 1c was placed in a flask in an ice-water bath, followed by addition of DIPEA (235 uL, 1.39 mmol), and then Compound **M3** (784 mg, 1.39 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain **1d** (504 mg); LC-MS: [M+H]⁺=804.4.

### Fifth step: Compound 1e

In a 50 mL single-neck flask, **1d** (500 mg, 0.62 mmol), **M5** (310 mg, 0.62 mmol), PyBOP (448 mg, 0.86 mmol), HOBt (116 mg, 0.86 mmol) and 15 mL of DMF were mixed and then DIPEA (378 uL, 2.29 mmol) was added in condition of an ice-water bath. The reaction was allowed at room temperature for 2 h. After the reaction was found to be completed from the monitoring of the HPLC, the reaction solution was purified by high-performance liquid chromatography to obtain a preparation solution of Compound **1e.** The preparation solution was lyophilized to obtain **1e** (210 mg); LC-MS: [M+H]⁺=1221.6.

### Sixth step: Compound 1

In a 25 mL single-neck flask, **1e** (100 mg, 0.081 mmol), zinc bromide (368 mg, 1.63 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound 1 (60 mg); LC-MS: [M+H]⁺=1065.3.

### Example 5

### Synthesis of Compound 2:

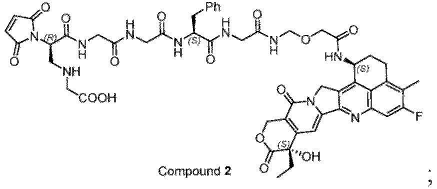

Compound **2** (51 mg) was obtained by referring to the synthesis route of example 4; LC-MS: [M+H]⁺=1065.3.

### Example 6

### Synthesis of Compound 3:

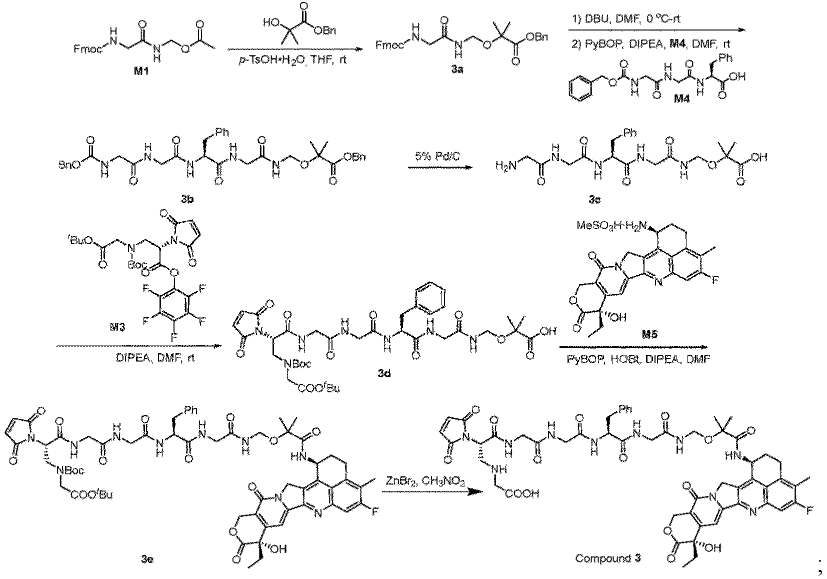

### First step: Compound 3a

In a 250 mL single-neck flask, **M1** (6 g, 16.3 mmol), 100 mL of THF, and p-toluenesulfonic acid monohydrate (0.31 g, 1.63 mmol) were mixed and stirred and then cooled to 0 °C, 2-hydroxy-2-methylpropionic acid benzyl ester (6.3 g, 32.6 mmol) was then added dropwise. The resulting solution was then brought naturally to room temperature to have a reaction for about 2-4 h. After the reaction was found to be completed from the monitoring of the TLC, the reaction system was quenched with a saturated NaHCO₃ solution, and then extracted with ethyl acetate, the product was washed with saturated NaCl solution, then dried with anhydrous Na₂SO₄, filtered and concentrated, and the residue is purified on a silica gel column (PE:EA=10:1-5:1-2:1) to obtain **3a** (4.2 g), with a yield of 52%; LC-MS: [M+H]⁺=503.3.

### Second step: Compound 3b

In a 25 mL single-neck flask, Compound **3a** (2 g, 4.0 mmol) and 10 mL of DMF were mixed and stirred at 0 °C, and DBU (760 mg, 5.0 mmol) was then added to have a reaction for 1 h. After Fmoc deprotection was found to be completed from the monitoring of the TLC, the resulting product was ready for later use.

In another 25 mL single-neck flask, **M4** (1.65 g, 4.0 mmol), PyBOP (2.59 g, 5.0 mmol), HOBt (675 mg, 5.0 mmol) and 10 mL of DMF were mixed, DIPEA (823 uL, 5.04 mmol) was then added in an ice water bath, the resulting reaction solution was stirred for 30 min and then placed in a reaction flask to have a reaction at room temperature. After the reaction was found to be completed from the monitoring of the HPLC, the reaction solution was purified through liquid chromatography to obtain a product preparation solution. The preparation solution was extracted with dichloromethane, then washed with saturated NaCl solution, dried with anhydrous Na₂SO₄ and filtered. The filtrate was concentrated under reduced pressure to obtain a solid product **3b** (1.4 g), with a yield of 53%; LC-MS: [M+H]⁺=676.2.

### Third step: Compound 3c

In a 25 mL single-neck flask, Compound **3b** (700 mg, 1.04 mmol) was completely dissolved in 10 mL of DMF and 700 mg of 5% Pd/C was then added to have a hydrogenation reaction for 1.5 h. After the reaction was found to be completed, the reaction solution was filtered and the filtrate was directly put into the next reaction without purification.

### Fourth step: Compound 3d

The crude product **3c** was placed in a flask in an ice-water bath, followed by addition of DIPEA (210 uL, 1.25 mmol), and then Compound **M3** (704 mg, 1.25 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain **3d** (486 mg); LC-MS: [M+H]⁺=830.5.

### Fifth step: Compound 3e

In a 50 mL single-neck flask, **3d** (300 mg, 0.36 mmol), **M5** (180 mg, 0.36 mmol), PyBOP (260 mg, 0.5 mmol), HOBt (67 mg, 0.5 mmol) and 10 mL of DMF were mixed and then DIPEA (219.5 uL, 1.33 mmol) was added in condition of an ice-water bath. The reaction was allowed at room temperature for 3 h. After the reaction was found to be completed from the monitoring of the HPLC, the reaction solution was purified by high-performance liquid chromatography to obtain a preparation solution of Compound **3e.** The preparation solution was lyophilized to obtain **3e** (157 mg); LC-MS: [M+H]⁺=1249.6.

### Sixth step: Compound 3

In a 25 mL single-neck flask, **3e** (100 mg, 0.08 mmol), zinc bromide (360 mg, 1.6 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **3** (64 mg); LC-MS: [M+H]⁺=1093.1.

### Example 7

### Synthesis of Compound 4:

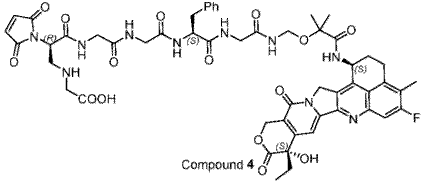

Compound **4** (60 mg) was obtained by referring to the synthesis route of example 6; LC-MS: [M+H]⁺=1093.2.

### Example 8

### Synthesis of Compound 5A:

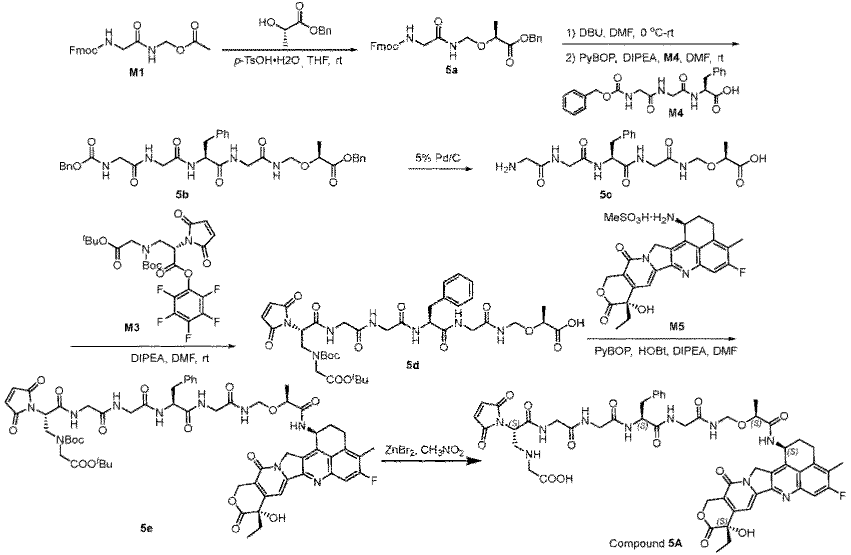

### First step: Compound 5a

In a 25 mL single-neck flask, **M1** (500 mg, 1.4 mmol, 1.0 eq), p-toluenesulfonic acid monohydrate (26 mg, 0.1 mmol, 0.1 eq) and 10 mL of THF were mixed and stirred and then cooled to 0 °C, *L-lactic acid benzyl ester* (1.2 g, 7.0 mmol, 5 eq) was then added slowly. The resulting solution was stirred and brought to room temperature. After the reaction was found to be completed from the monitoring of the TLC, the reaction system was quenched with a saturated NaHCO₃ solution, and then extracted with ethyl acetate, the product was dried with anhydrous Na₂SO₄, filtered and concentrated, and the residue is purified on a reversed phase column to obtain **5a** (400 mg);
LC-MS: [M+NH₄]⁺=506.2.

¹H NMR (400 Mz, CDCl₃/CD₃OD):1.39 (3H, d, *J* = 6.8 Hz), 3.78 (2H, t, *J* = 4.0 Hz), 4.17-4.27 (2H, m), 4.42 (2H, d, *J* = 4.0 Hz), 4.72-4.85 (2H, m), 5.11-5.58 (2H, m), 5.43 (1H, s), 7.06 (1H, t, *J* = 8.0 Hz), 7.25-7.33 (6H, m), 7.38 (2H, t, J=8.0 Hz), 7.57 (2H, d, *J* = 8.0 Hz), 7.75 (2H, d, *J* = 8.0 Hz).

### Second step: Compound 5b

In a 25 mL single-neck flask, Compound **5a** (400 mg, 0.8 mmol, 1.0 eq) and 4 mL of DMF were mixed and stirred and then cooled to 0 °C, DBU (137 mg, 0.9 mmol, 1.1 eq) was then added slowly. The resulting solution was then brought to room temperature to have a reaction. Marking as reaction solution 1 after the reaction was found to be completed from the monitoring of the TLC;
In another 25 mL single-neck flask, **M4** (372 mg, 0.9 mmol, 1.1 eq), PyBOP (852 mg, 1.6 mmol, 2.0 eq) and 3 mL of DMF were mixed and stirred at room temperature for 5 min, reaction solution 1 was then added, the reaction system was allowed at room temperature. After the reaction was found to be completed from the monitoring of the TLC, the reaction solution was purified by high-performance liquid chromatography to obtain Compound **5b** (326 mg); LC-MS: [M+NH₄]⁺=679.2.

### Third step: Compound 5c

In a 100 mL single-neck flask, 5b (4.0 g, 6.05 mmol, 1.0 eq) was dissolved in DMF (60 mL) and 5% Pd/C (4 g) was then added to have a hydrogenation reaction for 4 h (reaction progress was monitored by HPLC). Pd/C was filtered, and the filtrate without concentration was directly put in an ice water bath (about 0 °C) for further use.

### Fourth step: Compound 5d

The crude product **5c** was placed in a flask in an ice-water bath, followed by addition of DIPEA (1.1 mL, 1.1 eq), and then Compound **M3** (3.4 g, 6.05 mmol). The reaction was allowed at room temperature for 2 h. After the reaction was found to be completed from the monitoring of the HPLC, the reaction solution was purified by high-performance liquid chromatography to obtain a preparation solution. The preparation solution was lyophilized to obtain **5d** (3.15 g); LC-MS: [M-H]⁻=816.3.

### Fifth step: Compound 5e

In a 100 mL single-neck flask, **5d** (2.07 g, 2.53 mmol, 1.0 eq), **M5** (1.35 g, 2.53 mmol, 1.0 eq), PyBOP (1.98 g, 3.79 mmol, 1.5 eq), HOBt (0.51 g, 3.79 mmol, 1.5 eq) and DMF (40 mL) were added, and DIPEA (1.05 mL, 1.5 eq) was added in condition of an ice-water bath, the resulting solution was brought to room temperature to have a reaction for 2 h (monitored by HPLC). the reaction solution was purified by high-performance liquid chromatography to obtain a preparation solution. The reaction solution was directly purified by preparation. The preparation solution was concentrated in a reduced-pressure (water pump) water bath at 35 °C to remove acetonitrile, and then lyophilized to obtain Compound **5e** (1.92 g) with a yield of 61%; LC-MS: [M+H]⁺=1235.4.

### Sixth step: Compound 5A

In a 100 mL single-neck flask, Compound **5e** (1.0 g, 0.8 mmol, 1.0 eq) and 35 mL of nitromethane were mixed and dissolved, followed by addition of zinc bromide (3.64 g, 16 mmol, 20.0 eq). Reaction was allowed in an oil bath at 40 °C (the reaction system was preheated and stabilized in advance) for 30 min. The reaction solution was concentrated in a reduced-pressure (water pump) water bath at 45 °C to remove nitromethane to obtain a yellow solid residue (under the monitoring of HPLC). The preparation solution of Compound **5A** was obtained by acid preparation. The preparation solution was concentrated in a reduced-pressure (water pump) water bath at 35 °C to remove acetonitrile, and then lyophilized to obtain Compound **5A** (786 mg) with a yield of 90%.

LC-MS: [M+H]⁺=1079.4;
¹H NMR (400 MHz, DMSO-d6) δ 9.39 - 9.02 (m, 1H), 8.70 (t, *J* = 6.5 Hz, 1H), 8.64 (t, *J* = 5.7 Hz, 1H), 8.56 (d, *J* = 8.8 Hz, 1H), 8.34 (t, *J* = 5.7 Hz, 1H), 8.16 (d, *J* = 8.2 Hz, 1H), 8.01 (t, *J* = 5.5 Hz, 1H), 7.71 (d, *J* = 10.9 Hz, 1H), 7.30 (s, 1H), 7.28 - 7.15 (m, 4H), 7.14 (s, 2H), 5.53 (dd, J= 14.5, 6.4 Hz, 1H), 5.49 - 5.34 (m, 2H), 5.22 (d, *J* = 18.8 Hz, 1H), 5.09 (d, *J* = 18.7 Hz, 1H), 5.03 (dd, *J* = 9.6, 3.9 Hz, 1H), 4.73 (dd, *J* = 9.9, 6.9 Hz, 1H), 4.59 (dd, *J* = 10.1, 6.5 Hz, 1H), 4.49 (ddd, *J* = 13.2, 8.6, 4.4 Hz, 1H), 4.14 (dd, *J* = 13.3, 6.6 Hz, 2H), 3.93 (s, 2H), 3.84 (dd, *J* = 16.5, 6.3 Hz, 1H), 3.76 (dd, *J* = 16.9, 5.7 Hz, 2H), 3.70 (d, *J* = 5.2 Hz, 2H), 3.60 (dd, *J* = 16.7, 5.4 Hz, 1H), 3.52 (dd, *J* = 16.4, 5.1 Hz, 1H), 3.45 (dd, *J* = 12.8, 10.1 Hz, 1H), 3.25 - 3.15 (m, 1H), 3.14 - 3.05 (m, 1H), 3.01 (dd, *J* = 13.7, 4.1 Hz, 1H), 2.73 (dd, *J* = 13.5, 9.8 Hz, 1H), 2.54 - 2.47 (m, 1H), 2.33 (s, 2H), 2.17 (d, *J* = 5.5 Hz, 2H), 1.91 - 1.79 (m, 2H), 1.33 (d, *J* = 6.6 Hz, 2H), 0.87 (t, *J* = 7.3 Hz, 2H).

### Example 9

### Synthesis of Compound 5B:

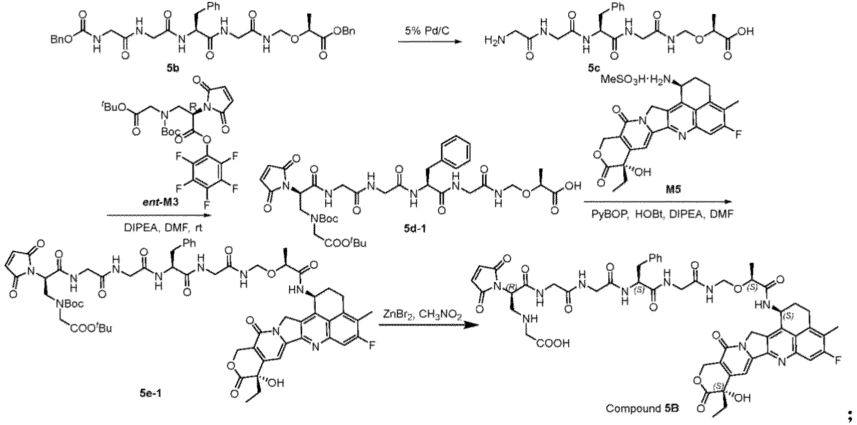

### First step: Compound 5d-1

In a 25 mL single-neck flask, Compound **5b** (300 mg, 0.45 mmol, 1.0 eq) was stirred and completely dissolved in DMF (3 mL), 5% Pd/C (300 mg) was then added, hydrogen was replaced three times, and hydrogenation reaction was carried out for 2 h. After the reaction was found to be completed from the monitoring of the HPLC, the reaction solution was filtered to remove Pd/C, the filtrate was cooled to 0-5 °C, DIPEA (65 mg, 0.5 mmol, 1.1 eq) was then added, followed by addition of ***ent-*M3** (255 mg, 0.45 mmol), and the resulting solution was brought to 20±5 °C to have a reaction for 1 h. After the reaction was found to be completed from the monitoring of the HPLC, the reaction solution was purified by HPLC, and the preparation solution of the product was collected and lyophilized to obtain Compound **5d-1** (200 mg) with a yield of 54%; LC-MS: [M-H]=816.3.

### Second step: Compound 5e-1

In a 25 mL single-neck flask, Compound **5d-1** (200 mg, 0.24 mmol, 1.0 eq), **M5** (127 mg, 0.24 mmol, 1.0 eq), PyBOP (187 mg, 0.36 mmol, 1.2eq), HOBt (48 mg, 0.36 mmol, 1.2 eq) and DMF (6 mL) were mixed and then cooled to 0-5°C in an ice water bath, followed by addition of DIPEA (62 mg, 0.48 mmol, 2.0 eq). The reaction was allowed at 20±5 °C for 2 h. After the reaction was found to be completed from the monitoring of the HPLC. The reaction solution was directly purified by HPLC preparation. The product preparation solution was collected and then lyophilized to obtain Compound **5e-1** (162.8 mg); LC-MS: [M+H]⁺=1235.4.

### Third step: Compound 5B

In a 25 mL single-neck flask, Compound **5e-1** (110 mg, 0.089 mmol, 1.0 eq), ZnBr₂ (400 mg, 1.78 mmol, 20.0 eq) and CH₃NO₂ (10 mL) were added in sequence. The reaction was allowed at 40 °C for 0.5 h and then terminated. Reduced-pressure rotary evaporation was performed at 45 °C to obtain a yellow solid, then sampled, and monitored by HPLC. The solid subjected to rotary evaporation was directly purified by HPLC preparation. The product preparation solution was collected and then lyophilized to obtain Compound **5B** (73.4 mg), with a yield of 76.5%; LC-MS: [M+H]⁺=1079.4.

### Example 10

### Synthesis of Compound 6A:

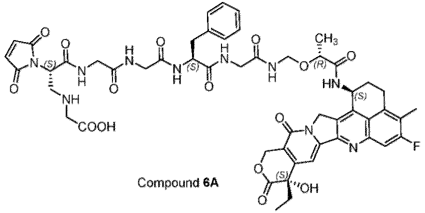

Compound **6A** (71 mg) was obtained by referring to the synthesis route of example 8; LC-MS:[M+H]⁺=1079.4.

### Example 11

### Synthesis of Compound 6B:

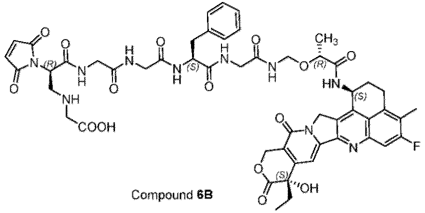

Compound **6B** (59 mg) was obtained by referring to the synthesis route of example 9; LC-MS: [M+H]⁺=1079.4.

### Example 12

### Synthesis of Compounds 7A and 7B:

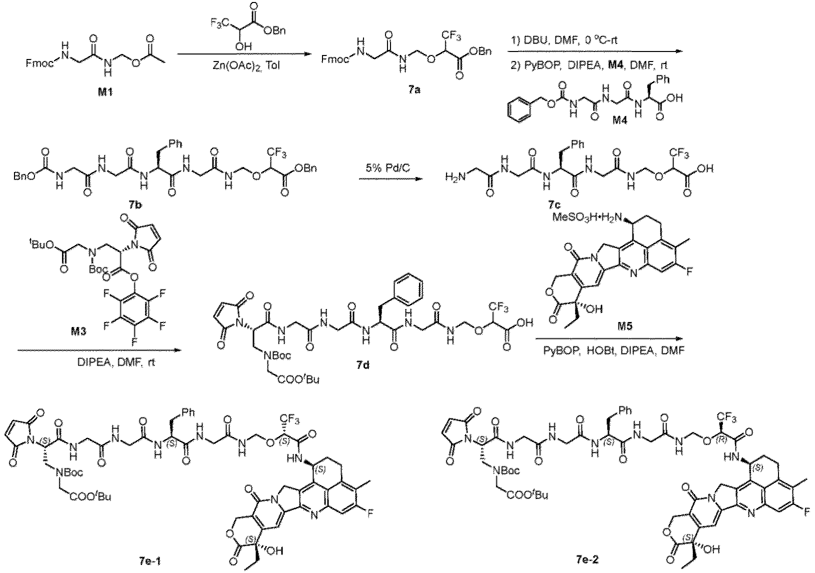

### First step: Compound 7a

In a 250 mL single-neck flask, **M1** (10 g, 27.1 mmol), 3, 3, 3-trifluorolactic acid benzyl ester (prepared according to the synthesis route disclosed in patent application WO2020063673A1) (12.7 g, 54.3 mmol), zinc acetate (9.96 g, 54.3 mmol) and 100 mL of toluene were mixed and heated to 100 °C to react for 4 h. After the reaction was found to be completed, the reaction solution was cooled to room temperature, then filtered to remove the insoluble matter, the filtrate was concentrated to obtain the crude product. The crude product was purified by silica gel column chromatography (PE:EA=10:1-5:1-2:1) to obtain 5.15 g of the target, with a yield of 35.1%; LC-MS: [M+H]⁺=543.17.

### Second step: Compound 7b

In a 50 mL single-neck flask, Compound **7a** (5 g, 9.2 mmol) was completely dissolved in 15 mL of DMF and DBU (1.68 g, 11 mmol) was then added in an ice water bath to react for 1 h, marking as reaction solution 1;

In another 50 mL single-neck flask, **M4** (3.8 g, 9.2 mmol), PyBOP (5.75 g, 11 mmol), HOBt (1.49 g, 11 mmol) and 10 mL DMF were mixed and dissolved completely. DIPEA (1.82 mL, 11 mmol) was then added in an ice water bath to further react for 30 min, reaction solution 1 was added, and the reaction was allowed at room temperature for 2 h and monitored by HPLC. After the reaction was found to be completed, the reaction solution was purified by high-performance liquid chromatography to obtain a preparation solution. The preparation solution was extracted with dichloromethane, then washed with saturated NaCl solution, dried with anhydrous Na₂SO₄ and filtered. The filtrate was concentrated to obtain 4.1 g of a solid product with a yield of 62.3%; LC-MS: [M+H]⁺=716.25.

### Third step: Compound 7d

In a 25 mL single-neck flask, **7b** (900 mg,1.26 mmol) was completely dissolved in 15 mL of DMF and 900 mg of 5% Pd/C was then added to have a hydrogenation reaction for 2 h. After the reaction was found to be completed, the reaction solution was filtered and the filtrate was put into a flask in an ice-water bath, followed by addition of DIPEA (228 uL,1.38 mmol), and then **M3** (712 mg, 1.26 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain 525 mg of the product, with a yield of 47.9%; LC-MS: [M-H]⁻ =870.33.

### Fourth step: Compound 7e

In a 50 mL single-neck flask, **7d** (500 mg, 0.57 mmol), **M5** (305 mg, 0.57 mmol), PyBOP (448 mg, 0.86mmol), HOBt (116 mg, 0.86 mmol) and 15 mL of DMF were mixed and then DIPEA (378 uL, 2.29 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compound **7e-1** and Compound **7e-2.** The preparation solutions were lyophilized separately to obtain 150 mg of Compound **7e-1,** LC-MS: [M+H]⁺=1289.46; 220 mg of Compound **7e-2,** LC-MS: [M+H]⁺=1289.46.

### Fifth step: Compound 7A

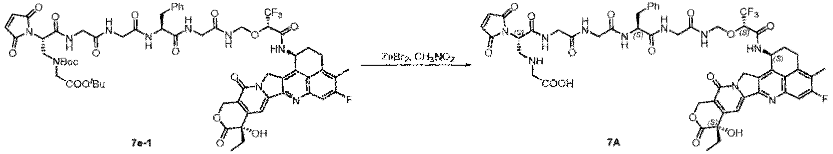

In a 25 mL single-neck flask, **7e-1** (100 mg, 0.077 mmol), zinc bromide (349 mg, 1.55 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 52 mg of a solid; TOF result: 1133.3613.

### Sixth step: Compound 7B

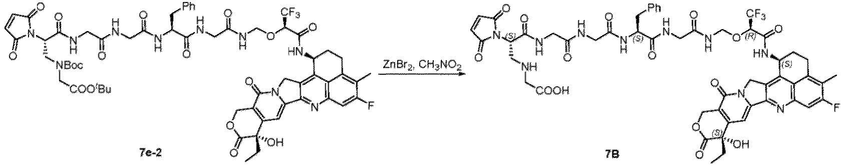

In a 25 mL single-neck flask, **7e-2** (100 mg, 0.077 mmol), zinc bromide (349 mg, 1.55 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 63 mg of a solid; TOF result: 1133.3668.

### Example 13

### Synthesis of Compounds 8A and 8B:

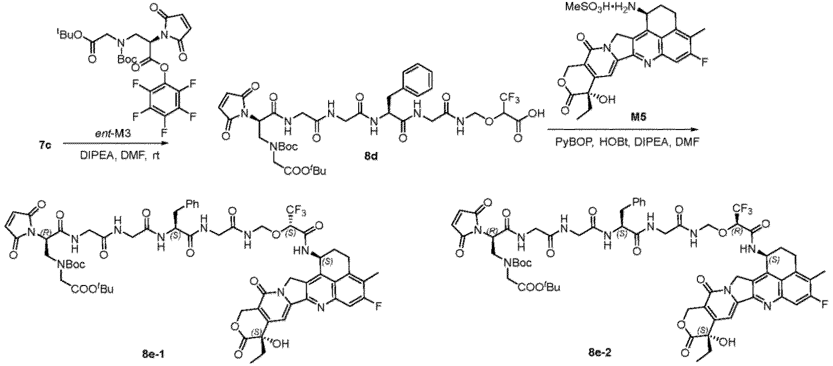

### First step: Compound 8d

In a 25 mL single-neck flask, **7c** (900 mg, 1.83 mmol) was completely dissolved in 20 mL of DMF, followed by addition of DIPEA (303 uL, 1.83 mmol), and then ***ent-*M3** (1034 mg, 1.83 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain 613 mg of the product, with a yield of 38.5%; LC-MS: [M-H]⁻=870.32.

### Second step: Compounds 8e-1 and Compound 8e-2

In a 50 mL single-neck flask, **8d** (500 mg, 0.57 mmol), **M5** (305 mg, 0.57 mmol), PyBOP (448 mg, 0.86 mmol), HOBt (116 mg, 0.86 mmol) and 15 mL of DMF were mixed and then DIPEA (378 uL, 2.29 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compound **8e-1** and Compound **8e-2.** The preparation solutions were lyophilized separately to obtain 140 mg of Compound **8e-1** and 210 mg of Compound **8e-2.** LC-MS of Compound **8e-1:** [M+H]⁺=1289.47; LC-MS of Compound **8e-2:** [M+H]⁺=1289.47.

### Third step: Compound 8A

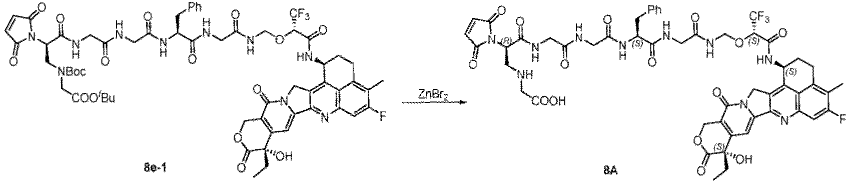

In a 25 mL single-neck flask, **8e-1** (100 mg, 0.077 mmol), zinc bromide (349 mg, 1.55 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 50 mg of a solid; TOF result: 1133.3623.

### Fourth step: Compound 8B

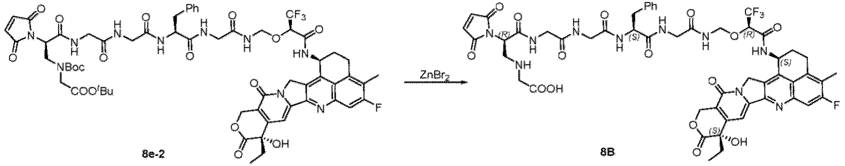

In a 25 mL single-neck flask, Compound **8e-2** (100 mg, 0.077 mmol), zinc bromide (349 mg, 1.55 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 58 mg of a solid; TOF result: 1133.3653.

### Example 14

### Synthesis of Compound 9A:

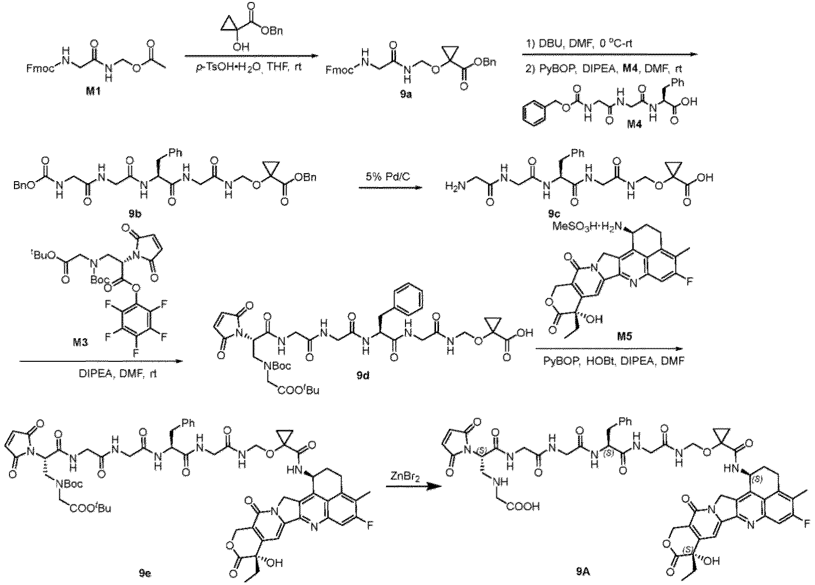

### First step: Compound 9a

In a 250 mL single-neck flask, **M1** (6 g, 16.3 mmol), 100 mL of THF, and p-toluenesulfonic acid monohydrate (0.31 g, 1.63 mmol) were mixed and stirred and then cooled to 0 °C, 2-hydroxy-2-cyclopropylacetic acid benzyl ester (prepared according to the synthesis route disclosed in patent application US20050020645 A1) (6.3 g, 32.6 mmol) was then added dropwise. The resulting solution was then brought naturally to room temperature to have a reaction for about 2-4 h. After the reaction was found to be completed from the monitoring of the TLC, the reaction system was quenched with a saturated NaHCO₃ solution, and then extracted with ethyl acetate, the product was washed with saturated NaCl solution, then dried with anhydrous Na₂SO₄, filtered and concentrated, and the residue is purified on a silica gel column (PE:EA=10:1-5:1-2:1) to obtain **9a** (3.7 g), with a yield of 45%; LC-MS: [M+H]⁺=501.5.

### Second step: Compound 9b

In a 25 mL single-neck flask, Compound **9a** (2 g, 4.0 mmol) and 10 mL of DMF were mixed and stirred at 0 °C, and DBU (760 mg, 5.0 mmol) was then added to have a reaction for 1 h. After Fmoc deprotection was found to be completed from the monitoring of the TLC, the resulting product was ready for later use.

In another 25 mL single-neck flask, **M4** (1.65 g, 4.0 mmol), PyBOP (2.59 g, 5.0 mmol), HOBt (675 mg, 5.0 mmol) and 10 mL of DMF were mixed, DIPEA (823 uL, 5.04 mmol) was then added in an ice water bath, the resulting reaction solution was stirred for 30 min and then placed in a reaction flask to have a reaction at room temperature. After the reaction was found to be completed from the monitoring of the HPLC, the reaction solution was purified through liquid chromatography to obtain a product preparation solution. The preparation solution was extracted with dichloromethane, then washed with saturated NaCl solution, dried with anhydrous Na₂SO₄ and filtered. The filtrate was concentrated under reduced pressure to obtain 1.5 g of a solid product, with a yield of 56%; LC-MS: [M+H]⁺=674.7.

### Third step: Compound 9c

In a 25 mL single-neck flask, Compound **9b** (900 mg, 1.3 mmol) was completely dissolved in 10 mL of DMF and 900 mg of 5% Pd/C was then added to have a hydrogenation reaction for 1.5 h. After the reaction was found to be completed, the reaction solution was filtered and the filtrate was directly put into the next reaction without purification.

### Fourth step: Compound 9d

The crude product **9c** was placed in a flask in an ice-water bath, followed by addition of DIPEA (223 uL, 1.3 mmol), and then Compound **M3** (750 mg, 1.3 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain **9d** (529 mg); LC-MS: [M+H]⁺=828.4.

### Fifth step: Compound 9e

In a 50 mL single-neck flask, **9d** (500 mg, 0.6 mmol), **M5** (300 mg, 0.6 mmol), PyBOP (416 mg, 0.8 mmol), HOBt (108 mg, 0.5 mmol) and 15 mL of DMF were mixed and then DIPEA (351 uL, 2.13 mmol) was added in condition of an ice-water bath. The reaction was allowed at room temperature for 3 h. After the reaction was found to be completed from the monitoring of the HPLC, the reaction solution was purified by high-performance liquid chromatography to obtain a preparation solution of Compound **9e.** The preparation solution was lyophilized to obtain **9e** (257 mg); LC-MS: [M+H]⁺=1247.5.

### Sixth step: Compound 9A

In a 25 mL single-neck flask, **9e** (100 mg, 0.08 mmol), zinc bromide (360 mg, 1.6 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **9A** (55 mg); LC-MS: [M+H]⁺=1091.3.

### Example 15

### Synthesis of Compound 9B:

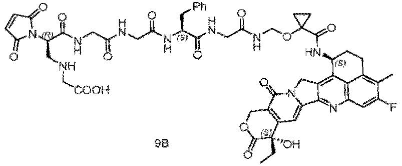

Compound **9B** (44 mg) was obtained by referring to the synthesis route of example 14; LC-MS: [M+H]⁺=1091.3.

### Example 16

### Synthesis of Compound 10A:

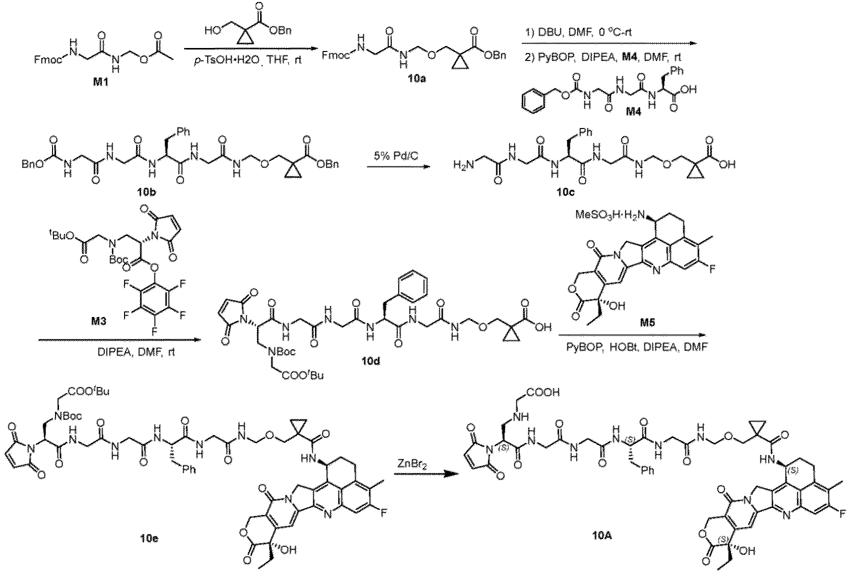

### First step: Compound 10a

In a 250 mL single-neck flask, **M1** (6 g, 16.3 mmol), 100 mL of THF, and p-toluenesulfonic acid monohydrate (0.31 g, 1.63 mmol) were mixed and stirred and then cooled to 0 °C, 3-hydroxy-2-cyclopropylpropionic acid benzyl ester (prepared according to the synthesis route disclosed in patent application WO2013187496A1) (6.7 g, 32.6 mmol) was then added dropwise. The resulting solution was then brought to room temperature naturally to have a reaction for about 2-4 h. After the reaction was found to be completed from the monitoring of the TLC, saturated NaHCO₃ solution was added to the reaction system, and then extracted with ethyl acetate, the product was washed with saturated NaCl solution, then dried with anhydrous Na₂SO₄, filtered and concentrated, and the residue was purified on a silica gel column (PE:EA=10:1-5:1-2:1) to obtain **10a** (4.9 g), with a yield of 58%; LC-MS: [M+H]⁺=515.4.

### Second step: Compound 10b

In a 25 mL single-neck flask, Compound **10a** (4 g, 7.8 mmol) and 10 mL of DMF were mixed and stirred at 0 °C, and DBU (1.2 g, 8.0 mmol) was then added. The reaction was carried out for 1 h. After Fmoc deprotection was found to be completed from the monitoring of the TLC, the resulting product was ready for later use.

In another 25 mL single-neck flask, **M4** (3.3 g, 8.0 mmol), PyBOP (5.2 g, 10.0 mmol), HOBt (1.35 g, 10.0 mmol) and 10 mL of DMF were mixed, DIPEA (1.65 mL, 10.1 mmol) was then added in an ice water bath, the resulting reaction solution was stirred for 50 min and then placed in a reaction flask to have a reaction at room temperature. After the completion of the reaction monitored by HPLC, the reaction solution was purified through liquid chromatography to obtain a product preparation solution. The preparation solution was extracted with dichloromethane, then washed with saturated NaCl solution, the organic layers was dried with anhydrous Na₂SO₄ and filtered. The filtrate was concentrated under reduced pressure to obtain 2.3 g of a solid product, with a yield of 42%; LCMS: [M+H]⁺=688.8.

### Third step: Compound 10c

In a 25 mL single-neck flask, Compound **10b** (1.0 g, 1.45 mmol) was completely dissolved in 15 mL of DMF and 1.0 g of 5% Pd/C was then added to have a hydrogenation reaction for 1.5 h. After the reaction was found to be completed, the reaction solution was filtered and the filtrate was directly put into the next reaction without purification.

### Fourth step: Compound 10d

The crude product **10c** was placed in a flask in an ice-water bath, followed by addition of DIPEA (258 uL, 1.5 mmol) and Compound **M3** (837 mg, 1.45 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain **10d** (499 mg); LC-MS: [M-H]⁻=842.4.

### Fifth step: Compound 10e

In a 50 mL single-neck flask, **10d** (400 mg, 0.48 mmol), **M5** (240 mg, 0.48 mmol), PyBOP (250 mg, 0.48 mmol), HOBt (104 mg, 0.48 mmol) and 15 mL of DMF were mixed and then DIPEA (330 uL, 2.0 mmol) was added in condition of an ice-water bath. The reaction was allowed at room temperature for 3 h. After the reaction was found to be completed from the monitoring of the HPLC, the reaction solution was purified by high-performance liquid chromatography to obtain a preparation solution of Compound **10e.** The preparation solution was lyophilized to obtain **10e** (188 mg); LC-MS: [M+H]⁺=1261.5.

### Sixth step: Compound 10A

In a 25 mL single-neck flask, **10e** (100 mg, 0.08 mmol), zinc bromide (360 mg, 1.6 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **10A** (61 mg); LC-MS: [M+H]⁺=1105.4.

### Example 17

### Synthesis of Compound 10B:

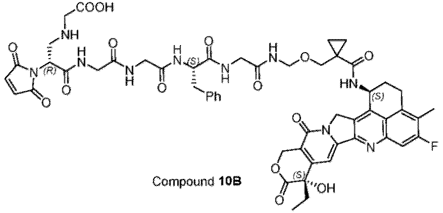

Compound **10B** (75 mg) was obtained by referring to the synthesis route of example 16; LC-MS: [M+H]⁺=1105.4.

### Example 18

### Synthesis of Compound 11A:

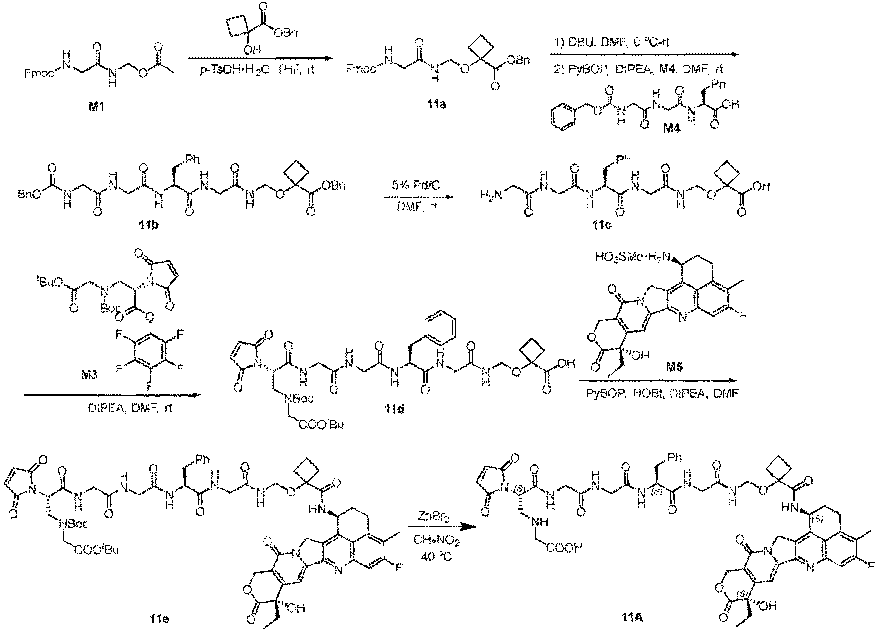

### First step: Compound 11a

In a 250 mL single-neck flask, **M1** (6 g, 16.3 mmol), 100 mL of THF, and p-toluenesulfonic acid monohydrate (0.31 g, 1.63 mmol) were mixed and stirred and then cooled to 0 °C, 2-hydroxy-2-cyclobutyl acetic acid benzyl ester (prepared according to the synthesis route disclosed by the literature: Journal of Medicinal Chemistry, 2013, 56 (13), 5541-5552.) (6.7 g, 32.6 mmol) was then added dropwise. The resulting solution was then brought to room temperature to have a reaction for about 2-4 h. After the reaction was found to be completed from the monitoring of the TLC, a saturated NaHCO₃ solution was added , and organic layers was extracted with ethyl acetate, the product was washed with saturated NaCl solution,then dried with anhydrous Na₂SO₄, filtered and concentrated, and the residue is purified on a silica gel column (PE:EA=10:1-5:1-2:1) to obtain **11a** (5.1 g), with a yield of 62%; LC-MS: [M+H]⁺=515.7.

### Second step: Compound 11b

In a 25 mL single-neck flask, Compound **11a** (4 g, 7.8 mmol) and 10 mL of DMF were mixed and stirred at 0 °C, and DBU (1.2 g, 8.0 mmol) was then added. And the reaction was carried out for 1 h. After Fmoc deprotection was found to be completed from the monitoring of the TLC, the resulting product was ready for later use.

In another 25 mL single-neck flask, **M4** (3.3 g, 8.0 mmol), PyBOP (5.2 g, 10.0 mmol), HOBt (1.35 g, 10.0 mmol) and 10 mL of DMF were mixed, DIPEA (1.63 mL, 10.0 mmol) was then added in an ice water bath, the resulting reaction solution was stirred for 40 min and then placed in a reaction flask to have a reaction at room temperature. After the reaction was found to be completed from the monitoring of the HPLC, the reaction solution was purified through liquid chromatography to obtain a product preparation solution. The preparation solution was extracted with dichloromethane, then washed with saturated NaCl solution, dried with anhydrous Na₂SO₄ and filtered. The filtrate was concentrated under reduced pressure to obtain 2.3 g of a solid product, with a yield of 42%; LCMS: [M+H]⁺=688.3.

### Third step: Compound 11c

In a 25 mL single-neck flask, Compound **11b** (2.0 g, 2.9 mmol) was completely dissolved in 25 mL of DMF and 2.0 g of 5% Pd/C was then added to have a hydrogenation reaction for 3 h. After the reaction was found to be completed, the reaction solution was filtered and the filtrate was directly put into the next reaction without purification.

### Fourth step: Compound 11d

The crude product **11c** was placed in a flask in an ice-water bath, followed by addition of DIPEA (516 uL, 3.0 mmol), and then Compound **M3** (1.7 g, 2.9 mmol). The reaction was allowed at room temperature for 2 h. After the reaction was found to be completed from the monitoring of the HPLC, the reaction solution was purified by high-performance liquid chromatography to obtain a preparation solution. The preparation solution was lyophilized to obtain **11d** (934 mg); LC-MS: [M-H]⁻=842.4.

### Fifth step: Compound 11e

In a 50 mL single-neck flask, **11d** (800 mg, 0.96 mmol), **M5** (480 mg, 0.96 mmol), PyBOP (500 mg, 0.96 mmol), HOBt (208 mg, 0.96 mmol) and 30 mL of DMF were mixed and then DIPEA (660 uL, 4.0 mmol) was added in condition of an ice-water bath. The reaction was allowed at room temperature for 4 h. After the reaction was found to be completed from the monitoring of the HPLC, the reaction solution was purified by high-performance liquid chromatography to obtain a preparation solution of Compound **11e.** The preparation solution was lyophilized to obtain **11e** (401 mg); LC-MS: [M+H]⁺=1261.4.

### Sixth step: Compound 11A

In a 25 mL single-neck flask, **11e** (150 mg, 0.12 mmol), zinc bromide (532 mg, 2.4 mmol) and 10 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **11A** (86 mg); LC-MS: [M+H]⁺=1105.4.

### Example 19

### Synthesis of Compound 11B:

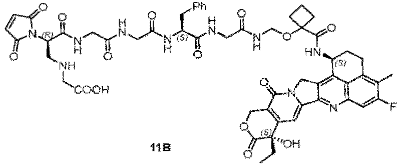

Compound **11B** (50 mg) was obtained by referring to the synthesis route of example 18. LC-MS: [M+H]⁺ 1105.4.

### Example 20

### Synthesis of Compound 12A:

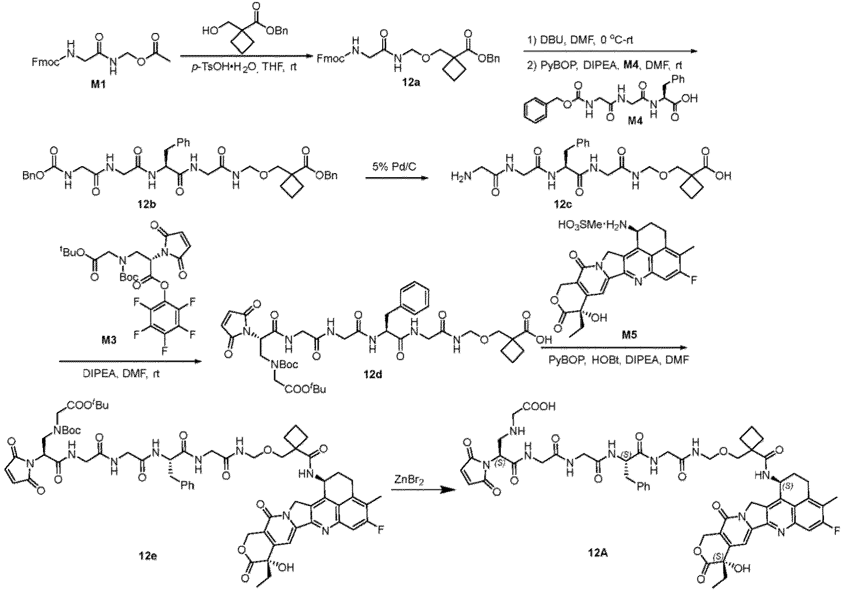

### First step: Compound 12a

In a 250 mL single-neck flask, **M1** (6 g, 16.3 mmol), 100 mL of THF, and p-toluenesulfonic acid monohydrate (0.31 g, 1.63 mmol) were mixed and stirred and then cooled to 0 °C, 3-hydroxy-2-cyclobutylpropionic acid benzyl ester (prepared according to the synthesis route disclosed in patent application WO2009011285A1) (7.2 g, 32.6 mmol) was then added dropwise. The resulting solution was then brought to room temperature to have a reaction for about 2-4 h. After the reaction was found to be completed from the monitoring of the TLC, a saturated NaHCO₃ solution was added, then organic layers were washed with saturated NaCl solution, and extracted with ethyl acetate, dried with anhydrous Na₂SO₄, filtered and concentrated, and the residue is purified on a silica gel column (PE:EA=10:1-5:1-2:1) to obtain **12a** (4.5 g), with a yield of 52%; LC-MS: [M+H]⁺=529.4.

### Second step: Compound 12b

In a 25 mL single-neck flask, Compound **12a** (4 g, 7.6 mmol) and 10 mL of DMF were mixed and stirred at 0 °C, and DBU (1.2 g, 8.0 mmol) was then added to have a reaction for 1 h. After Fmoc deprotection was found to be completed from the monitoring of the TLC, the resulting product was ready for later use.

In another 25 mL single-neck flask, **M4** (3.2 g, 7.6 mmol), PyBOP (4.7 g, 9.0 mmol), HOBt (1.22 g, 9.0 mmol) and 10 mL of DMF were mixed, DIPEA (1.49 mL, 0.9 mmol) was then added in an ice water bath, the resulting reaction solution was stirred for 30 min and then placed in a reaction flask to have a reaction at room temperature. After the reaction was found to be completed from the monitoring of the HPLC, the reaction solution was purified through liquid chromatography to obtain a product preparation solution. The preparation solution was extracted with dichloromethane, then washed with saturated NaCl solution, dried with anhydrous Na₂SO₄ and filtered. The filtrate was concentrated under reduced pressure to obtain 2.0 g of a solid product, with a yield of 37%; LC-MS: [M+H]⁺=702.8.

### Third step: Compound 12c

In a 25 mL single-neck flask, Compound **12b** (1.0 g, 1.43 mmol) was completely dissolved in 15 mL of DMF and 1.0 g of 5% Pd/C was then added to have a hydrogenation reaction for 1.5 h. After the reaction was found to be completed, the reaction solution was filtered and the filtrate was directly put into the next reaction without purification.

### Fourth step: Compound 12d

The crude product **12c** was placed in a flask in an ice-water bath, followed by addition of DIPEA (258 uL, 1.5 mmol), and then Compound M3 (825 mg, 1.43 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain **12d** (522 mg); LC-MS: [M-H]⁻=856.4.

### Fifth step: Compound 12e

In a 50 mL single-neck flask, **12d** (400 mg, 0.47 mmol), **M5** (240 mg, 0.47 mmol), PyBOP (250 mg, 0.47 mmol), HOBt (101 mg, 0.47 mmol) and 15 mL of DMF were mixed and then DIPEA (330 uL, 2.0 mmol) was added in condition of an ice-water bath. The reaction was allowed at room temperature for 3 h. After the reaction was found to be completed from the monitoring of the HPLC, the reaction solution was purified by high-performance liquid chromatography to obtain a preparation solution of Compound **12e.** The preparation solution was lyophilized to obtain **12e** (198 mg); LC-MS: [M+H]⁺=1275.4.

### Sixth step: Compound 12A

In a 25 mL single-neck flask, **12e** (100 mg, 0.08 mmol), zinc bromide (360 mg, 1.6 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **12A** (55 mg); LC-MS: [M+H]⁺=1119.4.

### Example 21

### Synthesis of Compound 12B:

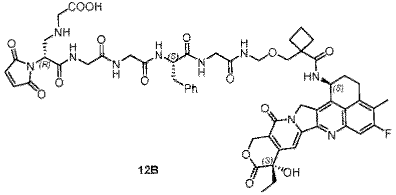

Compound **12B** (50 mg) was obtained by referring to the synthesis route of example 20; LC-MS: [M+H]⁺=1119.4.

### Example 22

### Synthesis of Compound 13A:

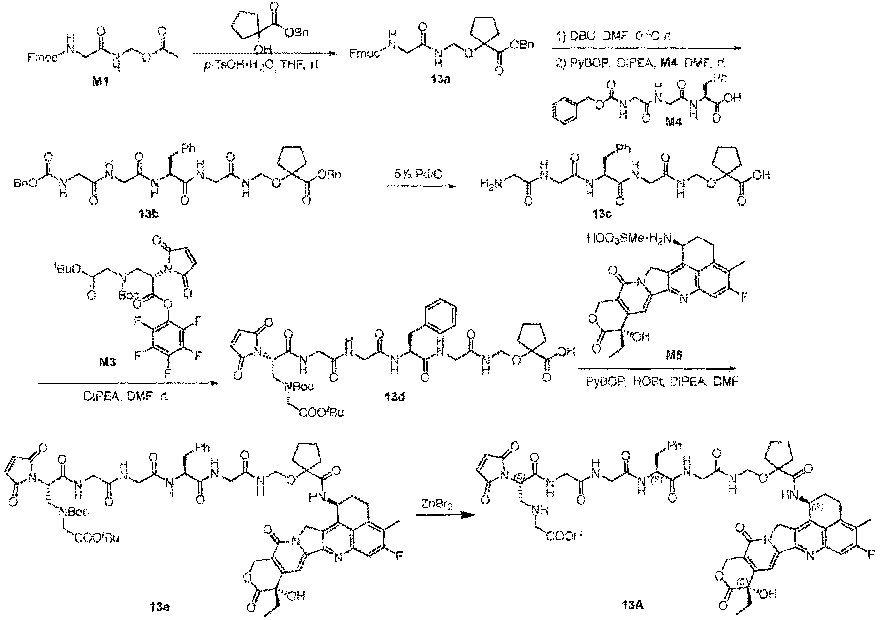

### First step: Compound 13a

In a 250 mL single-neck flask, **M1** (6 g, 16.3 mmol), 100 mL of THF, and p-toluenesulfonic acid monohydrate (0.31 g, 1.63 mmol) were mixed and stirred and then cooled to 0 °C, 2-hydroxy-2-cyclopentylacetic acid benzyl ester (prepared according to the synthesis route disclosed by the literature: Journal of Medicinal Chemistry, 2013, 56 (13), 5541-5552.) (7.2 g, 32.6 mmol) was then added dropwise. The resulting solution was brought naturally to room temperature to have a reaction for about 2-4 h. After the reaction was found to be completed from the monitoring of the TLC, the reaction system was quenched with a saturated NaHCO₃ solution, and then extracted with ethyl acetate, the product was washed with saturated NaCl solution, then dried with anhydrous Na₂SO₄, filtered and concentrated, and the residue is purified on a silica gel column (PE:EA=10:1-5:1-2:1) to obtain **13a** (4.6 g), with a yield of 53%; LC-MS: [M+H]⁺=529.5.

### Second step: Compound 13b

In a 25 mL single-neck flask, Compound **13a** (4 g, 7.6 mmol) and 10 mL of DMF were mixed and stirred at 0 °C, and DBU (1.17 g, 7.8 mmol) was then added to have a reaction for 1 h. After Fmoc deprotection was found to be completed from the monitoring of the TLC, the resulting product was ready for later use.

In another 25 mL single-neck flask, **M4** (3.14 g, 7.6 mmol), PyBOP (4.42 g, 8.5 mmol), HOBt (1.15 g, 8.5 mmol) and 10 mL of DMF were mixed, DIPEA (1.39 mL, 0.85 mmol) was then added in an ice water bath, the resulting reaction solution was stirred for 30 min and then placed in a reaction flask to have a reaction at room temperature. After the reaction was found to be completed from the monitoring of the HPLC, the reaction solution was purified through liquid chromatography to obtain a product preparation solution. The preparation solution was extracted with dichloromethane, then washed with saturated NaCl solution, dried with anhydrous Na₂SO₄ and filtered. The filtrate was concentrated under reduced pressure to obtain 2.1 g of a solid product, with a yield of 39%; LC-MS: [M+H]⁺=702.8.

### Third step: Compound 13c

In a 25 mL single-neck flask, Compound **13b** (1.5 g, 1.87 mmol) was completely dissolved in 25 mL of DMF and 1.5 g of 5% Pd/C was then added to have a hydrogenation reaction for 3 h. After the reaction was found to be completed, the reaction solution was filtered and the filtrate was directly put into the next reaction without purification.

### Fourth step: Compound 13d

The crude product **13c** was placed in a flask in an ice-water bath, followed by addition of DIPEA (333 uL, 1.93 mmol), and then Compound **M3** (1.1 g, 1.87 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain **13d** (519 mg); LC-MS: [M-H]⁻=856.6.

### Fifth step: Compound 13e

In a 50 mL single-neck flask, **13d** (400 mg, 0.47 mmol), **M5** (240 mg, 0.48 mmol), PyBOP (250 mg, 0.48 mmol), HOBt (103 mg, 48 mmol) and 15 mL of DMF were mixed and then DIPEA (330 uL, 2.0 mmol) was added in condition of an ice-water bath. The reaction was allowed at room temperature for 4 h. After the reaction was found to be completed from the monitoring of the HPLC, the reaction solution was purified by high-performance liquid chromatography to obtain a preparation solution of Compound **13e.** The preparation solution was lyophilized to obtain **13e** (187 mg); LC-MS: [M+H]⁺=1275.5.

### Sixth step: Compound 13A

In a 25 mL single-neck flask, **13e** (100 mg, 0.08 mmol), zinc bromide (355 mg, 0.16 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **13A** (60 mg); LC-MS: [M+H]⁺=1119.6.

### Example 23

### Synthesis of Compound 13B:

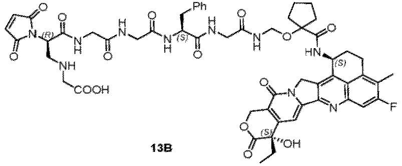

Compound **13B** (51 mg) was obtained by referring to the synthesis route of example 22; LC-MS: [M+H]⁺=1119.6.

### Example 24

### Synthesis of Compound 14A:

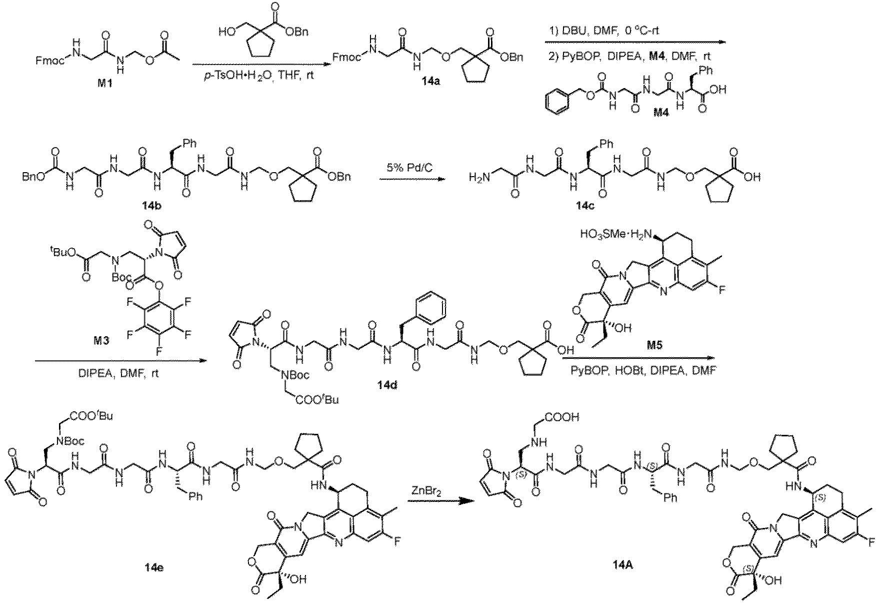

### First step: Compound 14a

In a 250 mL single-neck flask, **M1** (6 g, 16.3 mmol), 100 mL of THF, and p-toluenesulfonic acid monohydrate (0.31 g, 1.63 mmol) were mixed and stirred and then cooled to 0 °C, 3-hydroxy-2-cyclopentylpropanoic acid benzyl ester (prepared according to the synthesis route disclosed by patent application WO2009011285A1) (7.6 g, 32.6 mmol) was then added dropwise. The resulting solution was then brought to room temperature to have a reaction for about 2-4 h. After the reaction was found to be completed from the monitoring of the TLC, the reaction system was quenched with a saturated NaHCO₃ solution, and then extracted with ethyl acetate, the product was washed with saturated NaCl solution, then dried with anhydrous Na₂SO₄, filtered and concentrated, and the residue is purified on a silica gel column (PE:EA=10:1-5:1-2:1) to obtain **14a** (4.4 g), with a yield of 49%; LC-MS: [M+H]⁺=543.6.

### Second step: Compound 14b

In a 25 mL single-neck flask, Compound **14a** (4 g, 7.4 mmol) and 10 mL of DMF were mixed and stirred at 0 °C, and DBU (1.2 g, 8.0 mmol) was then added to have a reaction for 1 h. After Fmoc deprotection was found to be completed from the monitoring of the TLC, the resulting product was ready for later use.

In another 25 mL single-neck flask, **M4** (3.1 g, 7.4 mmol), PyBOP (4.6 g, 8.8 mmol), HOBt (1.19 g, 8.8 mmol) and 10 mL of DMF were mixed, DIPEA (1.49 mL, 9.0 mmol) was then added in an ice water bath, the resulting reaction solution was stirred for 30 min and then placed in a reaction flask to have a reaction at room temperature. After the reaction was found to be completed from the monitoring of the HPLC, the reaction solution was purified through liquid chromatography to obtain a product preparation solution. The preparation solution was extracted with dichloromethane, then washed with saturated NaCl solution, dried with anhydrous Na₂SO₄ and filtered. The filtrate was concentrated under reduced pressure to obtain 2.6 g of a solid product, with a yield of 49%; LC-MS: [M+H]⁺=716.4.

### Third step: Compound 14c

In a 25 mL single-neck flask, Compound **14b** (1.0 g, 1.4 mmol) was completely dissolved in 15 mL of DMF and 1.0 of 5% Pd/C was then added to have a hydrogenation reaction for 1.5 h. After the reaction was found to be completed, the reaction solution was filtered and the filtrate was directly put into the next reaction without purification.

### Fourth step: Compound 14d

The crude product **14c** was placed in a flask in an ice-water bath, followed by addition of DIPEA (248 uL, 1.5 mmol), and then Compound **M3** (808 mg, 1.4 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain **14d** (500 mg); LC-MS: [M-H]⁻=870.5.

### Fifth step: Compound 14e

In a 50 mL single-neck flask, **14d** (400 mg, 0.46 mmol), **M5** (235 mg, 0.46 mmol), PyBOP (245 mg, 0.46 mmol), HOBt (99 mg, 0.46 mmol) and 15 mL of DMF were mixed and then DIPEA (331 uL, 2.0 mmol) was added in condition of an ice-water bath. The reaction was allowed at room temperature for 3 h. After the reaction was found to be completed from the monitoring of the HPLC, the reaction solution was purified by high-performance liquid chromatography to obtain a preparation solution of Compound **14e.** The preparation solution was lyophilized to obtain **14e** (146 mg); LC-MS: [M+H]⁺=1289.5.

### Sixth step: Compound 14A

In a 25 mL single-neck flask, **14e** (100 mg, 0.08 mmol), zinc bromide (360 mg, 1.6 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **14A** (52 mg); LC-MS: [M+H]⁺=1133.4.

### Example 25

### Synthesis of Compound 14B:

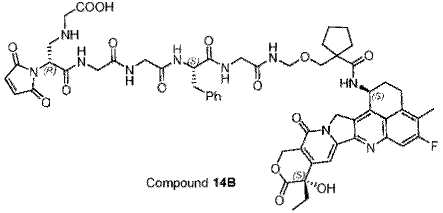

Compound **14B** (48 mg) was obtained by referring to the synthesis route of example 24; LC-MS: [M+H]⁺=1133.4.

### Example 26

### Synthesis of Compounds 15A and 15B:

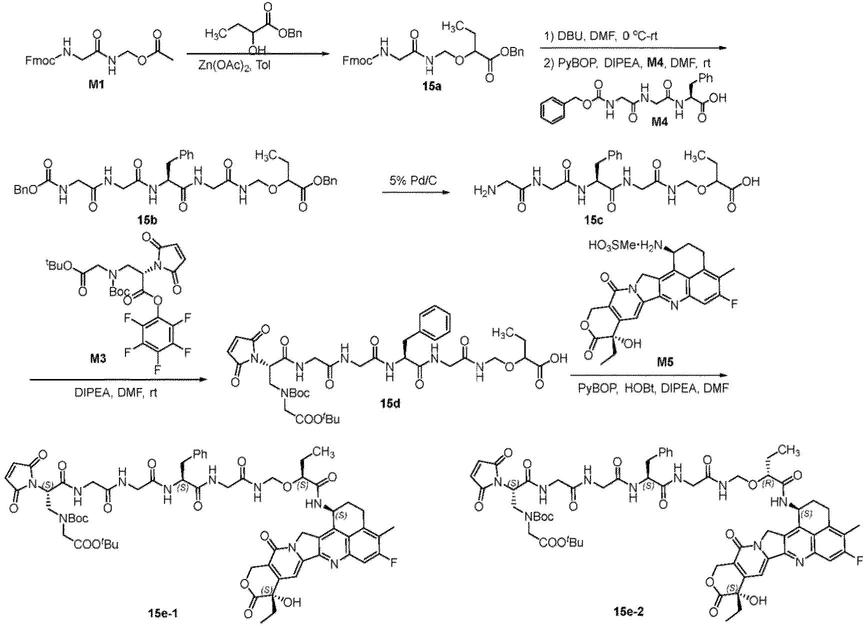

### First step: Compound 15a

In a 250 mL single-neck flask, **M1** (10 g, 27.1 mmol), 2-hydroxy-butyric acid benzyl ester (prepared according to the synthesis route disclosed in the literature Chemical Communications, 2019, 55 (53), 7699-7702.) (10.5 g, 54.3 mmol), zinc acetate (9.96 g, 54.3 mmol) and 100 mL of toluene were mixed and heated to 100 °C to react for 4 h. After the reaction was found to be completed, the reaction solution was cooled to room temperature, then filtered to remove the insoluble matter, and the filtrate was concentrated to obtain a crude product. The crude product was purified by silica gel column chromatography (PE:EA=10:1-5:1-2:1) to obtain 5.67 g of the target, with a yield of 42%; LC-MS: [M+H]⁺=503.5.

### Second step: Compound 15b

In a 50 mL single-neck flask, Compound **15a** (5 g, 9.95 mmol) was completely dissolved in 15 mL of DMF and DBU (1.68 g, 11 mmol) was then added in an ice water bath to react for 1 h, marking as reaction solution 1;
In another 50 mL single-neck flask, **M4** (4.1 g, 10.0 mmol), PyBOP (5.75 g, 11 mmol), HOBt (1.49 g, 11 mmol) and 10 mL DMF were mixed and dissolved completely. DIPEA (1.82 mL, 11 mmol) was then added in an ice water bath to further react for 40 min, reaction solution 1 was added, and the reaction was allowed at room temperature for 2 h and monitored by HPLC. After the reaction was found to be completed, the reaction solution was purified by high-performance liquid chromatography to obtain a preparation solution. The preparation solution was extracted with dichloromethane, then washed with saturated NaCl solution, dried with anhydrous Na₂SO₄ and filtered. The filtrate was concentrated to obtain 4.6 g of a solid product with a yield of 68%; LC-MS: [M+H]⁺=676.7.

### Third step: Compound 15d

In a 25 mL single-neck flask, **15b** (2.0 g, 2.96 mmol) was completely dissolved in 15 mL of DMF and 2.0 g of 5% Pd/C was then added to have a hydrogenation reaction for 2 h. After the reaction was found to be completed, the reaction solution was filtered and the filtrate was put into a flask in an ice-water bath, followed by addition of DIPEA (496 uL, 3.0 mmol), and then **M3** (1.7 g, 2.96 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain 1120.0 mg of the product, with a yield of 45%; LC-MS: [M-H]⁻ =830.3.

### Fourth step: Compound 15e

In a 50 mL single-neck flask, **15d** (500 mg, 0.60 mmol), **M5** (321 mg, 0.60 mmol), PyBOP (469 mg, 0.90 mmol), HOBt (121 mg, 0.90 mmol) and 15 mL of DMF were mixed and then DIPEA (446 uL, 2.7 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compound **15e-1** and Compound **15e-2.** The preparation solutions were lyophilized separately to obtain 138 mg of Compound **15e-1,** LC-MS: [M+H]⁺=1249.5; 140 mg of Compound **15e-2,** LC-MS: [M+H]⁺=1249.5.

### Fifth step: Compound 15A

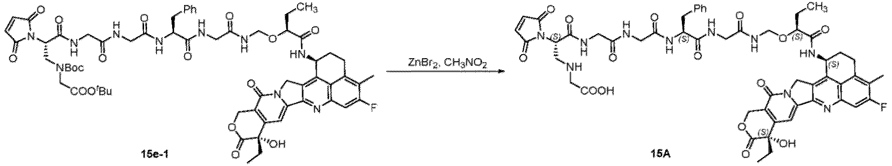

In a 25 mL single-neck flask, **15e-1** (100 mg, 0.08 mmol), zinc bromide (360 mg, 1.6 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 59 mg of a solid; LC-MS: [M+H]⁺=1093.4.

### Sixth step: Compound 15B

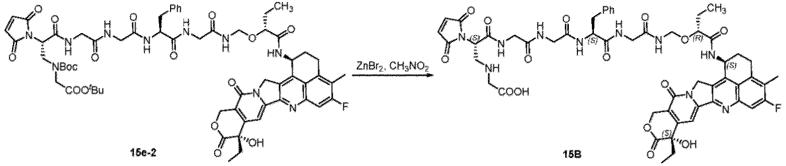

In a 25 mL single-neck flask, **15e-2** (100 mg, 0.08 mmol), zinc bromide (360 mg, 1.6 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 60 mg of a solid; LC-MS: [M+H]⁺=1093.4.

### Example 27

### Synthesis of Compounds 16A and 16B:

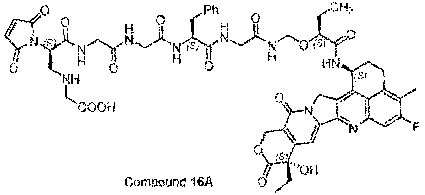

Compound **16A** (55 mg) was obtained by referring to the synthesis route of example 26; LC-MS: [M+H]⁺=1093.4.

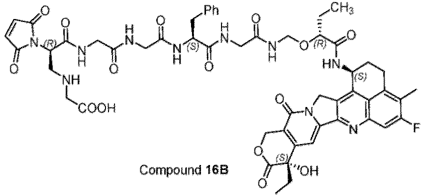

Compound **16B** (54 mg) was obtained by referring to the synthesis route of example 26; LC-MS: [M+H]⁺=1093.4.

### Example 28

### Synthesis of Compounds 17A and 17B:

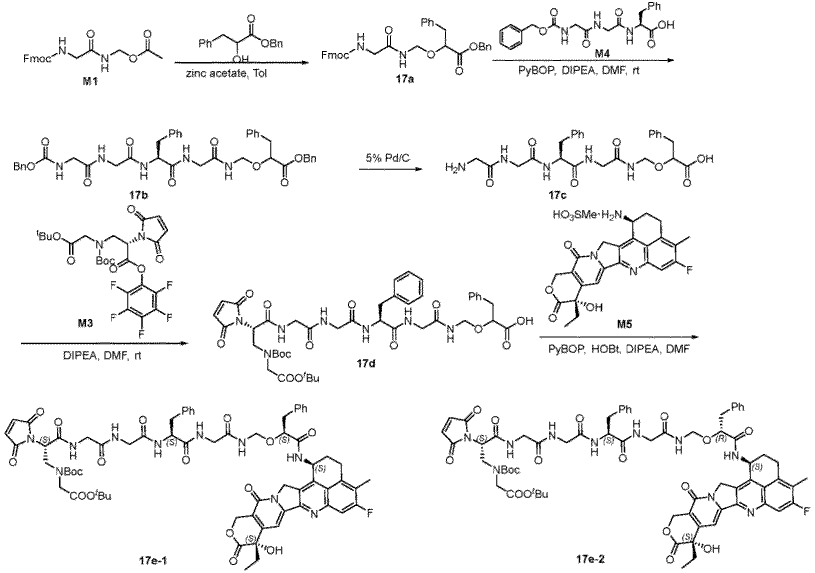

### First step: Compound 17a

In a 250 mL single-neck flask, **M1** (10 g, 27.1 mmol), 2-hydroxybenzenepropanoic acid benzyl ester (prepared according to the synthesis route disclosed in literature Nature Communications, 2020. 11 (1), 56.) (14.7 g, 54.3 mmol), zinc acetate (9.96 g, 54.3 mmol) and 100 mL of toluene were mixed and heated to 100 °C to react for 4 h. After the reaction was found to be completed, the reaction solution was cooled to room temperature, then filtered to remove the insoluble matter, and the filtrate was concentrated to obtain a crude product. The crude product was purified by silica gel column chromatography (PE:EA=10:1-5:1-2:1) to obtain 6.13 g of the target, with a yield of 40%; LC-MS: [M+H]⁺=565.6.

### Second step: Compound 17b

In a 50 mL single-neck flask, Compound **17a** (5 g, 8.86 mmol) was completely dissolved in 15 mL of DMF and DBU (1.53 g, 10 mmol) was then added in an ice water bath to react for 1 h, marking as reaction solution 1;
In another 50 mL single-neck flask, **M4** (3.6 g, 8.86 mmol), PyBOP (5.23 g, 10 mmol), HOBt (1.36 g, 10 mmol) and 10 mL of DMF were mixed and dissolved completely. DIPEA (1.65 mL, 10 mmol) was then added in an ice water bath to further react for 30 min, reaction solution 1was added, and the reaction was allowed at room temperature for 2 h and monitored by HPLC. After the reaction was found to be completed, the reaction solution was purified by high-performance liquid chromatography to obtain a preparation solution. The preparation solution was extracted with dichloromethane, then washed with saturated NaCl solution, dried with anhydrous Na₂SO₄ and filtered. The filtrate was concentrated to obtain 5.0 g of a solid product with a yield of 77%; LC-MS: [M+H]⁺=738.3.

### Third step: Compound 17d

In a 25 mL single-neck flask, **17b** (3.0 g, 4.07 mmol) was completely dissolved in 15 mL of DMF, and 3.0 g of 5% Pd/C was then added to have a hydrogenation reaction for 2 h. After the reaction was found to be completed, the reaction solution was filtered and the filtrate was put into a flask in an ice-water bath, followed by addition of DIPEA (744 uL, 4.5 mmol), and then **M3** (2.34 g, 4.07 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain 1.2 g of the product, with a yield of 33%; LC-MS: [M-H]⁻=892.4.

### Fourth step: Compound 17e

In a 50 mL single-neck flask, **17d** (500 mg, 0.56 mmol), **M5** (300 mg, 0.56 mmol), PyBOP (438 mg, 0.84 mmol), HOBt (113 mg, 0.84 mmol) and 15 mL of DMF were mixed and then DIPEA (330 uL, 2.0 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compound **17e-1** and Compound **17e-2.** The preparation solutions were lyophilized separately to obtain 156 mg of Compound **17e-1,** LC-MS: [M+H]⁺=1311.4; 150 mg of Compound **17e-2,** LC-MS: [M+H]⁺=1311.7.

### Fifth step: Compound 17A

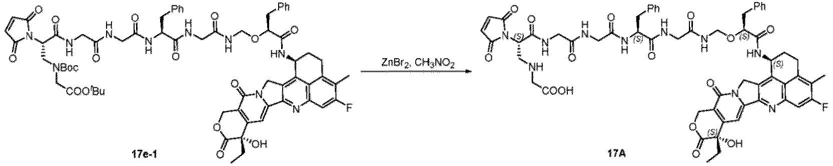

In a 25 mL single-neck flask, **17e-1** (100 mg, 0.08 mmol), zinc bromide (360 mg, 1.6 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 43 mg of a solid; LC-MS: [M+H]⁺=1155.4.

### Sixth step: Compound 17B

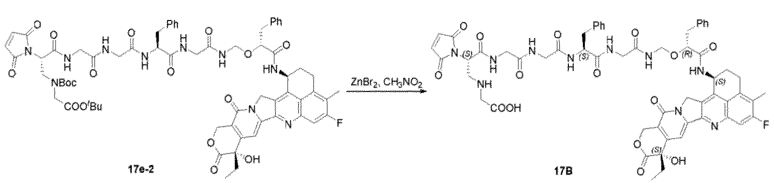

In a 25 mL single-neck flask, **17e-2** (100 mg, 0.08 mmol), zinc bromide (360 mg, 1.6 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 40 mg of a solid; LC-MS: [M+H]⁺=1155.4.

### Example 29

### Synthesis of Compounds 18A and 18B:

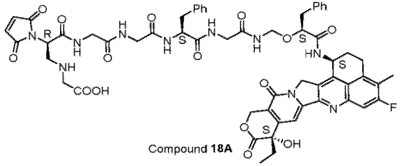

Compound **18A** (54 mg) was obtained by referring to the synthesis route of example 28; LC-MS: [M+H]⁺=1155.4.

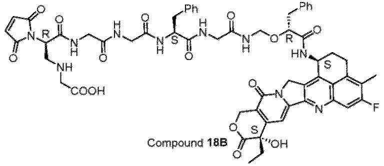

Compound **18B** (55 mg) was obtained by referring to the synthesis route of example 28; LC-MS: [M+H]⁺=1155.4.

### Example 30

### Synthesis of Compounds 19A and 19B:

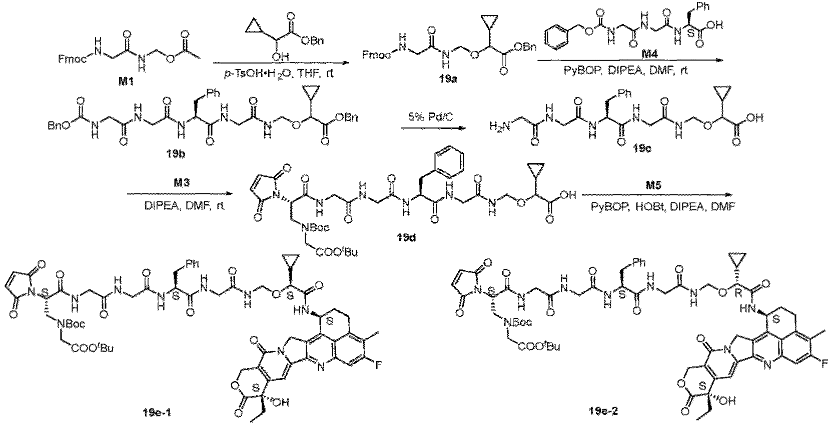

### First step: Compound 19a

In a 250 mL single-neck flask, **M1** (10 g, 27.1 mmol), 2-cyclopropyl-2-hydroxyacetic acid benzyl ester (prepared according to the synthesis route disclosed in atent application WO2020244657A1) (11.2 g, 54.3 mmol), zinc acetate (9.96 g, 54.3 mmol) and 100 mL of toluene were mixed and heated to 100 °C to react for 4 h. After the reaction was found to be completed, the reaction solution was cooled to room temperature, then filtered to remove the insoluble matter, and the filtrate was concentrated to obtain a crude product. The crude product was purified by silica gel column chromatography (PE:EA=10:1-5:1-2:1) to obtain 4.97 g of the target, with a yield of 36%; LC-MS: [M+H]⁺=515.2.

### Second step: Compound 19b

In a 50 mL single-neck flask, Compound **19a** (4 g, 7.8 mmol) was completely dissolved in 10 mL of DMF andDBU (1.42 g, 9.3 mmol) was then added in an ice water bath to react for 1 h, marking as reaction solution 1;
In another 50 mL single-neck flask, **M4** (3.2 g, 7.8 mmol), PyBOP (4.5 g, 8.6 mmol), HOBt (1.16 g, 8.6 mmol) and 10 mL of DMF were mixed and dissolved completely. DIPEA (1.65 mL, 10 mmol) was then added in an ice water bath to further react for 30 min, reaction solution 1 was added, and the reaction was allowed at room temperature for 2 h and monitored by HPLC. After the reaction was found to be completed, the reaction solution was purified by high-performance liquid chromatography to obtain a preparation solution. The preparation solution was extracted with dichloromethane, then washed with saturated NaCl solution, dried with anhydrous Na₂SO₄ and filtered. The filtrate was concentrated to obtain 4.2 g of a solid product with a yield of 78%; LC-MS: [M+H]⁺=688.3.

### Third step: Compound 19d

In a 25 mL single-neck flask, **19b** (1000 mg, 1.45 mmol) was completely dissolved in 15 mL of DMF and 1000 mg of 5% Pd/C was then added to have a hydrogenation reaction for 2 h. After the reaction was found to be completed, the reaction solution was filtered and the filtrate was put into a flask in an ice-water bath, followed by addition of DIPEA (248 uL, 1.5 mmol), and then **M3** (720 mg, 1.45 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain 503 mg of the product, with a yield of 41%; LC-MS: [M-H]⁻ =842.3.

### Fourth step: Compounds 19e-1 and 19e-2

In a 50 mL single-neck flask, **19d** (500 mg, 0.59 mmol), **M5** (317 mg, 0.59 mmol), PyBOP (339 mg, 0.65 mmol), HOBt (88 mg, 0.86 mmol) and 10 mL of DMF were mixed and then DIPEA (292 uL, 1.77 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compound **19e-1** and Compound **19e-2.** The preparation solutions were lyophilized separately to obtain 112 mg of Compound **19e-1,** LC-MS: [M+H]⁺=1261.5; 131 mg of Compound **19e-2,** LC-MS: [M+H]⁺=1261.5.

### Fifth step: Compound 19A

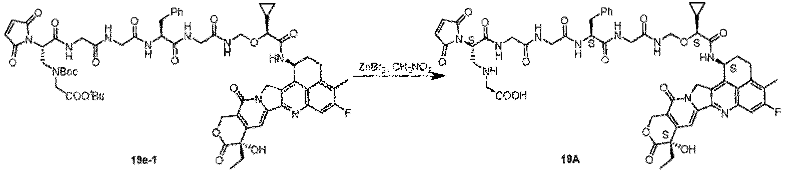

In a 25 mL single-neck flask, **19e-1** (100 mg, 0.079 mmol), zinc bromide (357 mg, 1.59 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 55 mg of a solid; LC-MS: [M+H]⁺=1105.4.

### Sixth step: Compound 19B

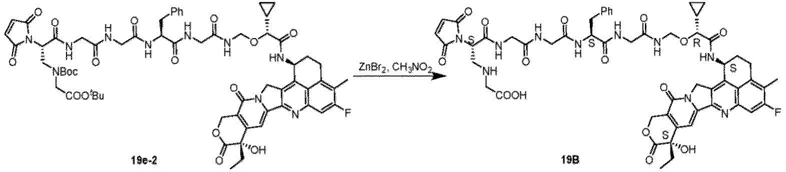

In a 25 mL single-neck flask, **19e-2** (100 mg, 0.079 mmol), zinc bromide (357 mg, 1.59 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 58 mg of a solid; LC-MS: [M+H]⁺=1105.4.

### Example 31

### Synthesis of Compounds 20A and 20B:

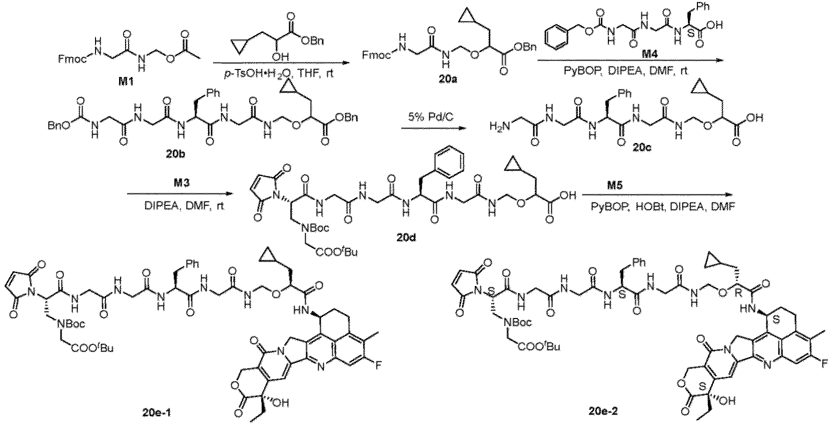

### First step: Compound 20a

In a 250 mL single-neck flask, **M1** (10 g, 27.1 mmol), 2-hydroxycyclopropylpropionic acid benzyl ester (prepared according to the synthesis route disclosed by the patent application WO2020063676A) (12.0 g, 54.3 mmol), zinc acetate (9.96 g, 54.3 mmol) and 100 mL of toluene were mixed and heated to 100 °C to react for 4 h. After the reaction was found to be completed, the reaction solution was cooled to room temperature, then filtered to remove the insoluble matter, and the filtrate was concentrated to obtain a crude product. The crude product was purified by silica gel column chromatography (PE:EA=10:1-5:1-2:1) to obtain 5.09 g of the target; LC-MS: [M+H]⁺=529.2.

### Second step: Compound 20b

In a 50 mL single-neck flask, Compound **20a** (4 g, 7.6 mmol) was completely dissolved in 10 mL of DMF and DBU (1.39 g, 9.1 mmol) was then added in an ice water bath to react for1 h, marking as reaction solution 1;

In another 50 mL single-neck flask, **M4** (3.12 g, 7.6 mmol), PyBOP (4.5 g, 8.6 mmol), HOBt (1.16 g, 8.6 mmol) and 10 mL of DMF were mixed and dissolved completely. DIPEA (1.65 mL, 10 mmol) was then added in an ice water bath to further react for 30 min, reaction solution 1 was added, and the reaction was allowed at room temperature for 2 h and monitored by HPLC. After the reaction was found to be completed, the reaction solution was purified by high-performance liquid chromatography to obtain a preparation solution. The preparation solution was extracted with dichloromethane, then washed with saturated NaCl solution, dried with anhydrous Na₂SO₄ and filtered. The filtrate was concentrated to obtain 4.5 g of a solid product with a yield of 84%; LC-MS: [M+H]⁺=702.3.

### Third step: Compound 20d

In a 25 mL single-neck flask, **20b** (1000 mg, 1.42 mmol) was completely dissolved in 15 mL of DMF and 1000 mg of 5% Pd/C was then added to have a hydrogenation reaction for 2 h. After the reaction was found to be completed, the reaction solution was filtered and the filtrate was put into a flask in an ice-water bath, followed by addition of DIPEA (248 uL, 1.5 mmol), and then **M5** (708 mg, 1.42 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain 443 mg of the product, with a yield of 36%; LC-MS: [M-H]⁻ =856.4.

### Fourth step: Compounds 20e-1 and 20e-2

In a 50 mL single-neck flask, **20d** (400 mg, 0.47 mmol), ixotecan mesylate (250 mg, 0.47 mmol), PyBOP (223 mg, 0.56 mmol), HOBt (83 mg, 0.56 mmol) and 10 mL of DMF were mixed and then DIPEA (248 uL, 1.5 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compound **20e-1** and Compound **20e-2.** The preparation solutions were lyophilized separately to obtain 103 mg of Compound **20e-1,** LC-MS: [M+H]⁺=1275.5; 103 mg of Compound **20e-2,** LC-MS: [M+H]⁺=1275.5.

### Fifth step: Compound 20A

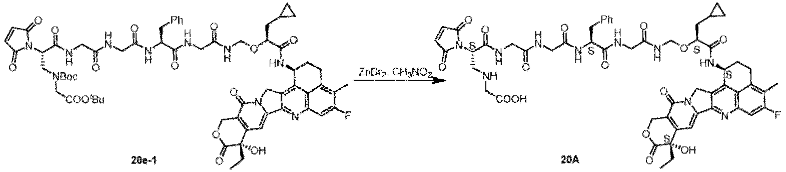

In a 25 mL single-neck flask, **8A** (100 mg, 0.078 mmol), zinc bromide (352 mg, 1.57 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 51 mg of a solid; LC-MS: [M+H]⁺=1119.4.

### Sixth step: Compound 20B

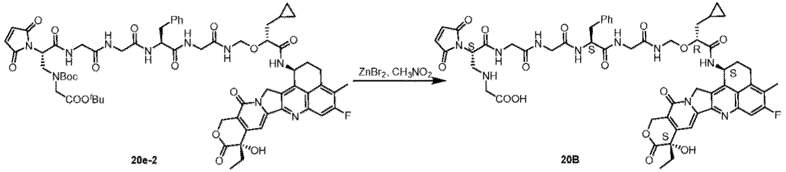

In a 25 mL single-neck flask, **20e-2** (100 mg, 0.079 mmol), zinc bromide (357 mg, 1.59 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 47 mg of a solid; LC-MS: [M+H]⁺=1119.4.

### Example 32

### Synthesis of Compound 21:

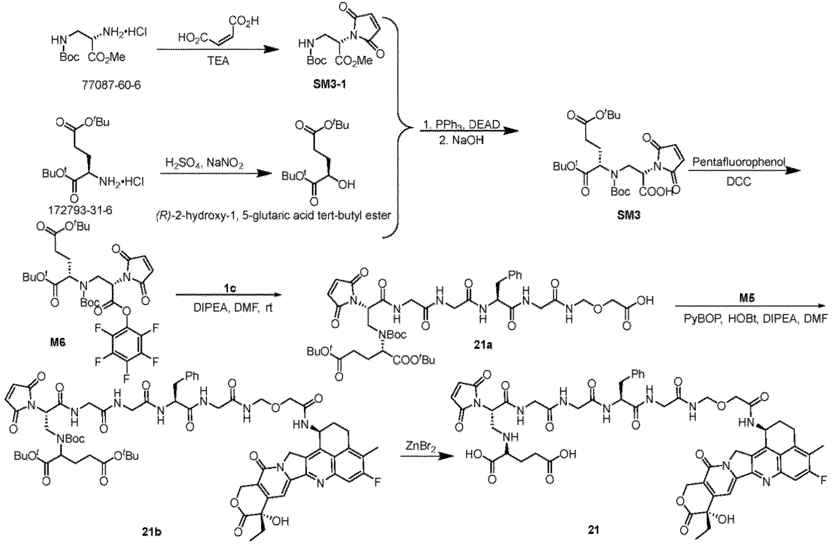

### First step: Compound SM3-1

In a 2000 mL single-neck flask, 77087-60-6 (100 g, 458 mmol), maleic acid (53.4 g, 460 mmol), TEA (64 mL, 460 mmol) and 1000 mL of toluene were mixed and heated to 100 °C to react for 5 h. After the reaction was found to be completed, the reaction solution was cooled to room temperature, then filtered to remove the insoluble matter, and the filtrate was concentrated to obtain a crude product. The crude product was purified by silica gel column chromatography (PE:EA=100:1-50:1-20:1) to obtain 75.6 g of the target; LC-MS: [M+H]⁺=299.1.

### Second step: Compound (R)-2-hydroxy-1, 5-glutaric acid tert-butyl ester

In a 2000 mL single-neck flask, 172793-31-6 (100 g, 338 mmol) and 1000 mL water were added, followed by addition of sodium nitrite (35 g, 507 mmol) and concentrated sulfuric acid (32 mL, 35 mmol), and the resulting solution was brought to room temperature to react for 24 h. After the reaction was found to be completed, the reaction solution was extracted three times with 500 mL of ethyl acetate. The organic phase was dried with anhydrous Na₂SO₄ and filtered, concentrated under reduced pressure to remove the solvent to obtain a crude product. The crude product was purified by silica gel column chromatography (PE:EA=50:1-30:1-2:1) to obtain 91.2 g of the target; LC-MS: [M+H]⁺=261.4.

### Third step: Compound SM3

In a 2000 mL single-neck flask,(*R*)-2-hydroxy-1, 5-glutaric acid tert-butyl ester (50 g, 192 mmol) and 1000 mL of anhydrous tetrahydrofuran were mixed and cooled in an ice water bath to 0 °C, then followed by addition of PPh₃ (87.7 g, 288 mmol), DEAD (50.2 g, 288 mmol) and **SM3-1** (57.3, 192 mmol), and the resulting solution was brought to room temperature to react for 13 h. After the reaction was found to be completed, the reaction solution was filtered to remove the insoluble matter. The filtrate was concentrated to obtain a crude product. The crude product was purified by silica gel column chromatography (PE:EA=50:1-30:1-1:1) to obtain 68.6 g of the product;
The above product was dissolved in 500 mL of methanol, then cooled in an ice water bath to 0 °C, NaOH (64 mL, 190 mmol, 3 M/L) was added dropwise at this temperature to react for 12 h, HCl (6M/L) was added to adjust pH to 3. The reaction solution was extracted five times with 500 mL of dichloromethane, dried with anhydrous Na₂SO₄ and filtered. The filtrate was concentrated under reduced pressure. The crude product was purified by column chromatography (DCM/MeOH=50/1-20/1-2/1) to obtain 50.4 g of **SM3**; LC-MS: [M-H]⁻=525.5.

### Fourth step: Compound M6

In a 2000 mL single-neck flask, **SM3** (50 g, 95 mmol, 1.0 eq), pentafluorophenol (19.2 g, 104.5 mmol, 1.1 eq), DCC (21.5 g, 104.5 mmol, 1.1 eq) and THF (600 mL) were mixed and the reaction was allowed at room temperature for 1 h and monitored by TLC. The insoluble matter was filtered out. The reaction solution was directly purified by preparation. The preparation solution was concentrated in a reduced-pressure (water pump) water bath at 35 °C to remove acetonitrile, and then lyophilized to obtain Compound **M6** (51.9 g) with a yield of 79%; LC-MS: [M+H]+=693.3.

### Fifth step: Compound 21a

In a 25 mL single-neck flask, **1c** (1 g, 2.36 mmol) was completely dissolved in 25 mL of DMF, followed by addition of DIPEA (430 uL, 2.6 mmol) and **M6** (1177 mg, 2.36 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain 555 mg of the product; LC-MS: [M-H]⁻=931.0.

### Sixth step: Compound 21b

In a 100 mL single-neck flask, **21a** (500 mg, 0.54 mmol), ixotecan mesylate **M5** (285 mg, 0.54 mmol), PyBOP (239 mg, 0.6 mmol), HOBt (239 mg, 0.6 mmol) and 10 mL of DMF were mixed and then DIPEA (248 uL, 1.5 mmol) was added in condition of an ice-water bath. The reaction was allowed at room temperature for 2 h. After the reaction was found to be completed from the monitoring of the HPLC, the reaction solution was purified by high-performance liquid chromatography to obtain a preparation solution of Compound **21b.** The preparation solution was lyophilized to obtain 231 mg of the compound; LC-MS: [M+H]⁺=1349.5.

### Seventh step: Compound 21

In a 25 mL single-neck flask, Compound **21b** (200 mg, 0.1488 mmol), zinc bromide (665 mg, 2.96 mmol) and 10 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 103 mg of a solid; LC-MS: [M+H]⁺=1137.5.

### Example 33

### Synthesis of Compound 22:

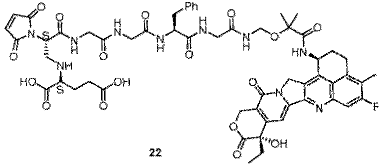

Taking Compounds **M6** and **3c** as starting raw materials, Compound **22** (91 mg) was obtained by referring to the synthesis route of example 32; LC-MS: [M+H]⁺=1165.5.

### Example 34

### Synthesis of Compounds 23 and 24:

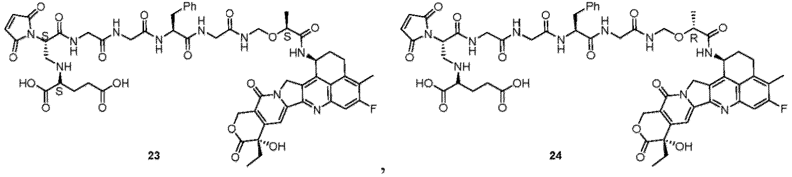

Taking Compounds **M6** and **5c** as starting raw materials, 102 mg of Compound **23** was obtained by referring to the synthesis route of example 32, LC-MS:
[M+H]⁺=1151.4; 99 mg of Compound **24** was obtained, LC-MS: [M+H]⁺=1151.4.

### Example 35

### Synthesis of Compounds 25 and 26:

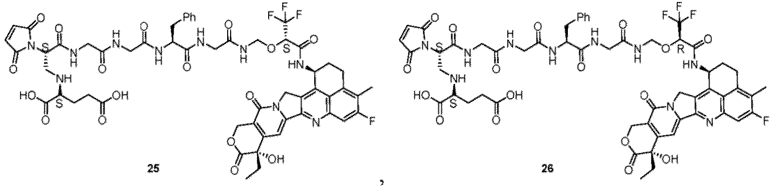

Taking Compounds **M6** and **7c** as starting raw materials, 83 mg of Compound **25** was obtained by referring to the synthesis route of example 32, LC-MS:
[M+H]⁺=1205.7; 80 mg of Compound **26** was obtained, LC-MS: [M+H]⁺=1205.7.

### Example 36

### Synthesis of Compounds 27 and 28:

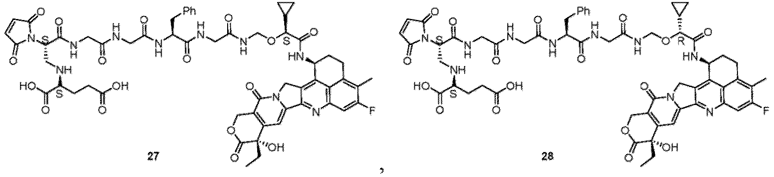

Taking Compounds **M6** and **19c** as starting raw materials, 100 mg of Compound **27** was obtained by referring to the synthesis route of example 32, LC-MS:
[M+H]⁺=1177.5; 101 mg of Compound **28** was obtained, LC-MS: [M+H]⁺=1177.5.

### Example 37

### Synthesis of Compound 29:

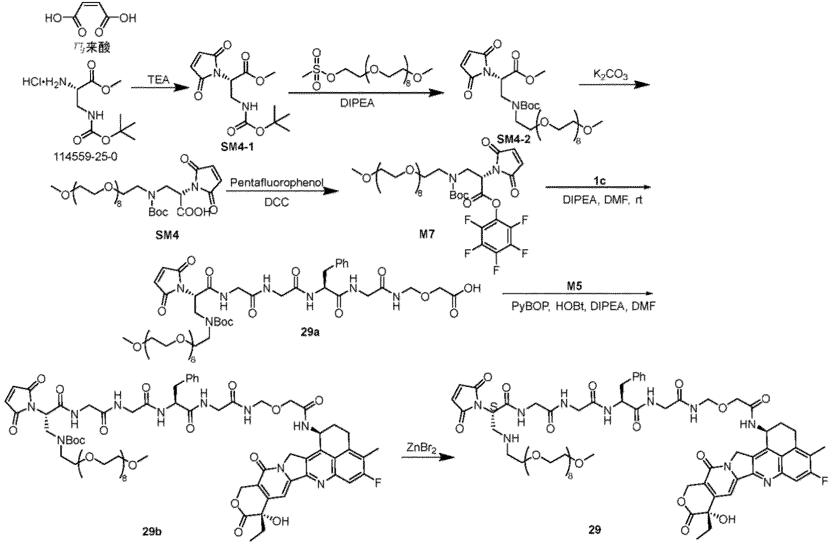

### First step: Compound SM4-1

In a 5000 mL single-neck flask, maleic acid (50 g, 431 mmol, 1.0 eq), 114559-25-0 (110 g, 431 mmol, 1 eq), TEA (263 g, 2.16 mol, 5 eq) and toluene (2000 mL) were mixed and then heated to reflux for 5 h and monitored by TLC. The insoluble matter was filtered out. Rotatory evaporation under reduced pressure was directly performed on the reaction solution to remove the solvent, and the residue was subjected to silica gel column chromatography (PE/EA=50/1-20/1-1/1) to obtain **SM4-1** (64.7 g), with a yield of 50%; LC-MS: [M+H]⁺=299.2.

### Second step: Compound SM4-2

In a 2000 mL single-neck flask, **SM4-1** (64 g, 215 mmol) was completely dissolved in 1000 mL of DMF, then DIPEA (71 mL, 430 mmol) was added, followed by addition of nonane ethylene glycol monomethyl ether mesylate (111.5 g, 220 mmol), and the resulting solution was subjected to room temperature to react for 2 h. After the reaction was found to be completed from the monitoring of HPLC, the reaction solution was purified by silica gel column chromatography (PE/EA=50/1-20/1-1/1), to obtain 59.9 g of the product; LC-MS: [M+H]⁺=709.4.

### Third step: Compound SM4

In a 2000 mL single-neck flask, **SM4-2** (59 g, 83 mmol) was completely dissolved in 1000 mL of MeOH, K₂CO₃ (11.75 g, 85 mmol) was added and the reaction was allowed at room temperature for 4 h and monitored by HPLC. After the reaction was found to be completed, the insoluble matter was filtered out. The reaction solution was directly purified by preparation. The preparation solution was concentrated in a reduced-pressure (water pump) water bath at 35 °C to remove acetonitrile, and then lyophilized to obtain Compound **SM4** (27 g); LC-MS: [M-H]⁻=693.5.

### Fourth step: Compound M7

In a 500 mL single-neck flask, **SM4** (25 g, 36 mmol, 1.0 eq), pentafluorophenol (7.3 g, 40 mmol, 1.1 eq), DCC (8.2 g, 40 mmol, 1.1 eq) and THF (200 mL) were mixed and the reaction was allowed at room temperature for 1 h and monitored by TLC. The insoluble matter was filtered out. The reaction solution was directly purified by preparation. The preparation solution was concentrated in a reduced-pressure (water pump) water bath at 35 °C to remove acetonitrile, and then lyophilized to obtain Compound **M7** (23.3 g) with a yield of 93%; LC-MS: [M+H]⁺=695.8.

### Fifth step: Compound 29a

In a 25 mL single-neck flask, **1c** (1 g, 2.36 mmol) was completely dissolved in 25 mL of DMF, followed by addition of DIPEA (430 uL, 2.6 mmol) and then **M7** (1640 mg, 2.36 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain 609 mg of the product. LC-MS: [M-H]⁻=1098.5.

### Sixth step: Compound 29b

In a 100 mL single-neck flask, **29a** (500 mg, 0.45 mmol), ixotecan mesylate **M5** (240 mg, 0.45 mmol), PyBOP (215 mg, 0.54 mmol), HOBt (215 mg, 0.54 mmol) and 10 mL of DMF were mixed and then DIPEA (248 uL, 1.5 mmol) was added in condition of an ice-water bath. The reaction was allowed at room temperature for 2 h. After the reaction was found to be completed from the monitoring of the HPLC, the reaction solution was purified by high-performance liquid chromatography to obtain a preparation solution of Compound **29b.** The preparation solution was lyophilized to obtain 187 mg of the compound; LC-MS: [M+H]⁺=1517.6.

### Seventh step: Compound 29

In a 25 mL single-neck flask, Compound **29b** (150 mg, 0.988 mmol), zinc bromide (223 mg, 0.988 mmol) and 10 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 114 mg of a solid; LC-MS: [M+H]⁺=1517.9.

### Example 38

### Synthesis of Compound 30:

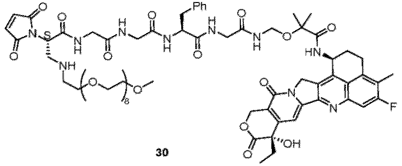

Taking Compounds **M7** and **3c** as starting raw materials, Compound **30** (125 mg) was obtained by referring to the synthesis route of example 37; LC-MS: [M+H]⁺=1445.6.

### Example 39

### Synthesis of Compounds 31 and 32:

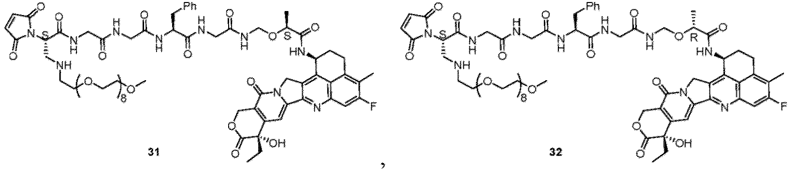

Taking Compounds **M7** and **5c** as starting raw materials, 61 mg of Compound **31** was obtained by referring to the synthesis route of example 37, LC-MS: [M+H]⁺=1431.7; 63 mg of Compound **32** was obtained, LC-MS: [M+H]⁺=1431.7.

### Example 40

### Synthesis of Compounds 33 and 34:

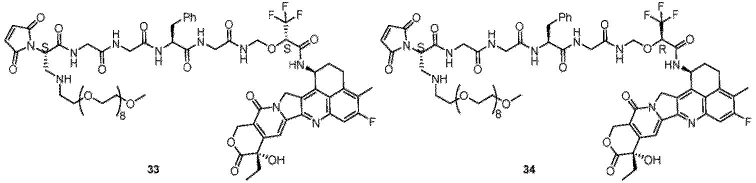

Taking Compounds **M7** and **7c** as starting raw materials, 60 mg of Compound **33** was obtained by referring to the synthesis route of example 37, LC-MS: [M+H]⁺=1485.6; 58 mg of Compound **34** was obtained, LC-MS: [M+H]⁺=1485.6.

### Example 41

### Synthesis of Compounds 35 and 36:

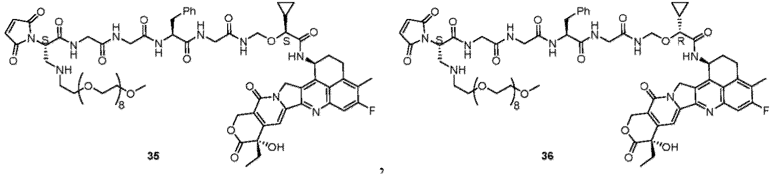

Taking Compounds **M7** and **19c** as starting raw materials, 102 mg of Compound **35** was obtained by referring to the synthesis route of example 37, LC-MS: [M+H]⁺=1457.8; 102 mg of Compound **36** was obtained, LC-MS: [M+H]⁺=1457.8.

### Example 42

### Synthesis of Compound 37:

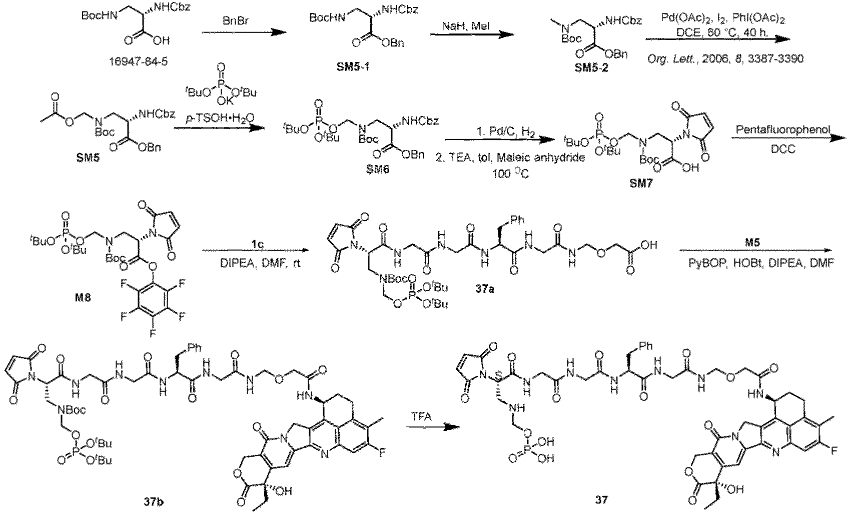

### First step: Compound SM5-1

In a 2000 mL single-neck flask, Compound 16947-84-5 (100 g, 295 mmol, 1.0 eq), DIPEA (50 mL, 300 mmol), Benzyl bromide (51.3 g, 300 mmol) and THF (1000 mL) were mixed, and the reaction was allowed at room temperature for 12 h and monitored by TLC. The insoluble matter was filled out. Rotatory evaporation under reduced pressure was directly performed on the reaction solution to remove the solvent, and the residue was subjected to silica gel column chromatography (PE/EA=50/1-20/1-2/1) to obtain **SM5-1** (110.1 g), with a yield of 87%; LC-MS: [M+H]⁺=429.2.

### Second step: Compound SM5-2

In a 2000 mL single-neck flask, Compound **SM5-1** (100 g, 233.4 mmol, 1.0 eq) and THF (1000 mL) were mixed and cooled in an ice water bath to 0 °C, NaH (37.4 g, 933.5 mmol) and MeI (132.5 g, 933.5 mmol) were then added step by step, and the reaction was allowed at 0 °C for 24 h and monitored by TLC. 500 mL of saturated NH₄Cl in water was added to quench the reaction. The reaction solution was extracted three times with 500 mL of ethyl acetate. The organic phase was dried with anhydrous Na₂SO₄ and filtered. Rotatory evaporation under reduced pressure was directly performed on the filtrate to remove the solvent and the residue was subjected to silica gel column chromatography (PE/EA=100/1-50/1-10/1) to obtain **SM5-2** (37.1 g); LC-MS: [M+H]⁺=443.3.

### Third step: Compound SM5 (see Org. Lett., 2006, 8, 3387-3390.)

In a 1000 mL single-neck flask, Compound **SM5-2** (35 g, 79 mmol, 1.0 eq) and DCE (500 mL) were mixed and then added in sequence with palladium diacetate, (180 mg, 0.8 mmol), I₂ (20 g, 79 mmol), and iodobenzene diacetate (40.8 g, 126.4 mmol), and the reaction was allowed at 60 °C for 40 h and monitored by TLC. 500 mL of saturated aqueous sodium thiosulfate solution in water was added to quench the reaction. The reaction solution was extracted three times with 500 mL of dichloromethane. The organic phase was dried with anhydrous Na₂SO₄ and filtered. Rotatory evaporation under reduced pressure was directly performed on the filtrate to remove the solvent and the residue was subjected to silica gel column chromatography (PE/EA=100/1-50/1-10/1) to obtain **SM5** (28 g); LC-MS: [M+H]⁺=501.3.

### Fourth step: Compound SM6

In a 500 mL single-neck flask, Compound **SM5** (25 g, 50 mmol, 1.0 eq), di-tert-butyl phosphate potassium salt (13.66 g, 55 mmol, 1.1 eq), p-toluenesulfonic acid monohydrate (951 mg, 5 mmol, 0.1 eq) and THF (200 mL) were mixed and the reaction was allowed at room temperature for 1 h and monitored by TLC. The insoluble matter was filtered out. The reaction solution was directly purified by preparation. The preparation solution was concentrated in a reduced-pressure (water pump) water bath at 35 °C to remove acetonitrile, and then lyophilized to obtain Compound **SM6** (15.1 g), with a yield of 46%; LC-MS: [M+H]⁺=651.4.

### Fifth step: Compound SM7

In a 250 mL single-neck flask, **SM6** (15 g, 23 mmol) was completely dissolved in 100 mL of DMF, and then added with 15 g of 5% Pd/C in an ice water bath. The atmosphere in the system was replaced with hydrogen three times. The reaction was allowed at room temperature for 12 h. Pd/C was removed, and reduced-pressure (oil pump) rotary evaporation was performed to remove the solvent. The resulting crude product was ready for later use.

In another 250 mL single-neck flask, the crude product and 100 mL of toluene, triethylamine (6.4 mL, 46 mmol), and maleic anhydride (2.4 g, 24 mmol) were mixed and dissolved completely. The reaction was allowed at 100 °C for 2 h and monitored by HPLC. After the reaction was found to be completed, the reaction solution was purified through liquid chromatography to obtain a preparation solution. The preparation solution was extracted with dichloromethane, then washed with saturated NaCl solution, dried with anhydrous Na₂SO₄ and filtered. The filtrate was concentrated to obtain 4.2 g of a solid product with a yield of 36%; LCMS: [M+H]⁺=507.3.

### Sixth step: Compound M8

In a 100 mL single-neck flask, Compound **SM7** (4 g, 7.9 mmol, 1.0 eq), pentafluorophenol (1.6 g, 8.7 mmol, 1.1 eq), DCC (1.8 g, 8.7 mmol, 1.1 eq) and THF (60 mL) were mixed and the reaction was allowed at room temperature for 1 h and monitored by TLC. The insoluble matter was filtered out. The reaction solution was directly purified by preparation. The preparation solution was concentrated in a reduced-pressure (water pump) water bath at 35 °C to remove acetonitrile, and then lyophilized to obtain Compound **M8** (3.7 g) with a yield of 70%; LC-MS: [M+H]⁺=673.2.

### Seventh step: Compound 37a

In a 25 mL single-neck flask, Compound **1c** (1 g, 2.36 mmol) was completely dissolved in 25 mL of DMF, followed by addition of DIPEA (430 uL, 2.6 mmol), and then Compound **M8** (1.2 g, 2.36 mmol). The reaction was allowed at room temperature for 1 h. After the reaction was found to be completed from the monitoring of the HPLC, the reaction solution was purified by high-performance liquid chromatography to obtain a preparation solution. The preparation solution was lyophilized to obtain 488 mg of the product; LC-MS: [M-H]⁻=911.0.

### Eighth step: Compound 37b

In a 100 mL single-neck flask, Compound **37a** (400 mg, 0.44 mmol), ixotecan mesylate **M5** (235 mg, 0.44 mmol), PyBOP (199 mg, 0.5 mmol), HOBt (69 mg, 0.5 mmol) and 10 mL of DMF were mixed and then DIPEA (218 uL, 1.32 mmol) was added in condition of an ice-water bath. The reaction was allowed at room temperature for 2 h. After the reaction was found to be completed from the monitoring of the HPLC, the reaction solution was purified by high-performance liquid chromatography to obtain a preparation solution of Compound **37b.** The preparation solution was lyophilized to obtain 201 mg of the Compound; LC-MS: [M+H]⁺=1329.6.

### Ninth step: Compound 37

In a 25 mL single-neck flask, Compound **37b** (130 mg, 0.098 mmol), zinc bromide (221 mg, 0.98 mmol) and 10 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 96 mg of a solid; LC-MS: [M+H]⁺=1117.4.

### Example 43

### Synthesis of Compound 38:

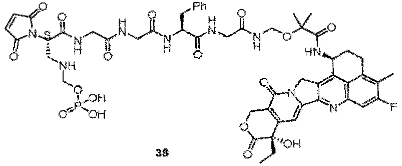

Taking Compounds **M8** and **3c** as starting raw materials, Compound **38** (51 mg) was obtained by referring to the synthesis route of example 42; LC-MS: [M+H]⁺=1145.6.

### Example 44

### Synthesis of Compounds 39 and 40:

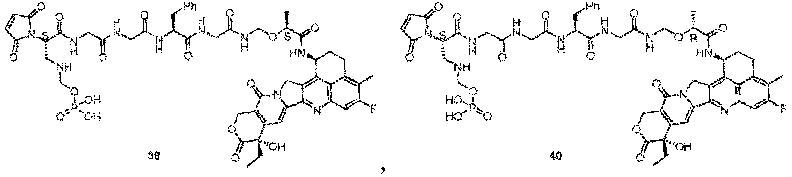

Taking Compounds **M8** and **5c** as starting raw materials, 57 mg of Compound **39** was obtained by referring to the synthesis route of example 42, LC-MS: [M+H]⁺=1131.4; 60 mg of Compound **40** was obtained, LC-MS: [M+H]⁺=1131.4.

### Example 45

### Synthesis of Compounds 41 and 42:

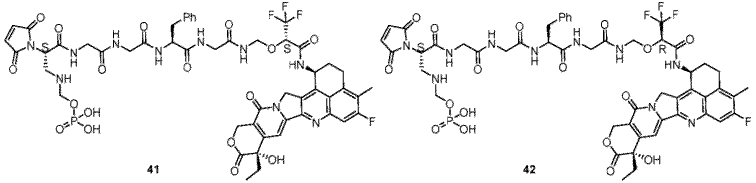

Taking Compounds **M7** and **7c** as starting raw materials, 44 mg of Compound **41** was obtained by referring to the synthesis route of example 42, LC-MS: [M+H]⁺=1185.3; 44 mg of Compound **42** was obtained, LC-MS: [M+H]⁺=1185.3.

### Example 46

### Synthesis of Compounds 43 and 44:

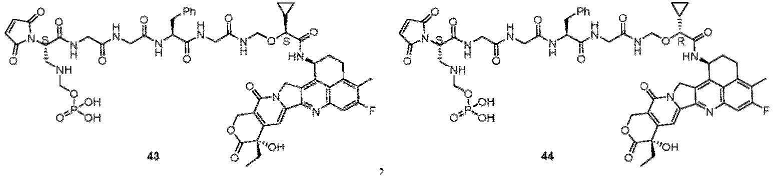

Taking Compounds **M8** and **19c** as starting raw materials, 62 mg of Compound **43** was obtained by referring to the synthesis route of example 42, LC-MS: [M+H]⁺=1157.4; 59 mg of Compound **44** was obtained, LC-MS: [M+H]⁺=1157.4.

### Example 47 (Comparative Example)

### Synthesis of Compound 45:

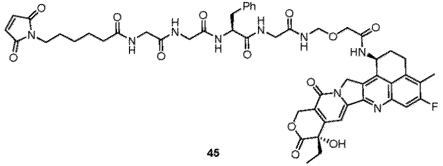

Compound **45** was obtained by referring to the synthesis route of Example 58 in patent application CN104755494A.

### Example 48

### Synthesis of Compound 46:

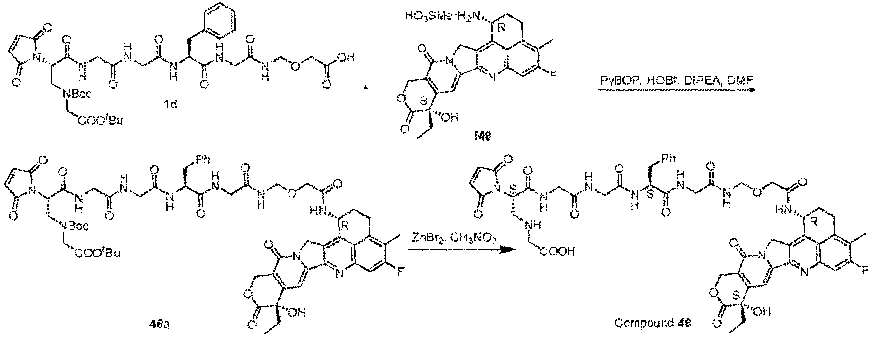

### First step: Compound 46a

In a 50 mL single-neck flask, **1d** (500 mg, 0.62 mmol), **M9** (310 mg, 0.62 mmol), PyBOP (448 mg, 0.86 mmol), HOBt (116 mg, 0.86 mmol) and 15 mL of DMF were mixed and then DIPEA (378 uL, 2.29 mmol) was added in condition of an ice-water bath. The reaction was allowed at room temperature for 2 h. After the reaction was found to be completed from the monitoring of the HPLC, the reaction solution was purified by high-performance liquid chromatography to obtain a preparation solution. The preparation solution was lyophilized to obtain **46a** (210 mg); LC-MS: [M+H]⁺=1221.6.

### Second step: Compound 46

In a 25 mL single-neck flask, **46a** (200 mg, 0.162 mmol), zinc bromide (736 mg, 3.26 mmol) and 10 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **46** (120 mg); LC-MS: [M+H]⁺=1065.3.

### Example 49

### Synthesis of Compound 47:

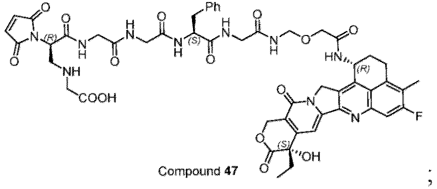

Compound **47** (81 mg) was obtained by referring to the synthesis route of example 48; LC-MS: [M+H]⁺=1065.3.

### Example 50

### Synthesis of Compound 48A:

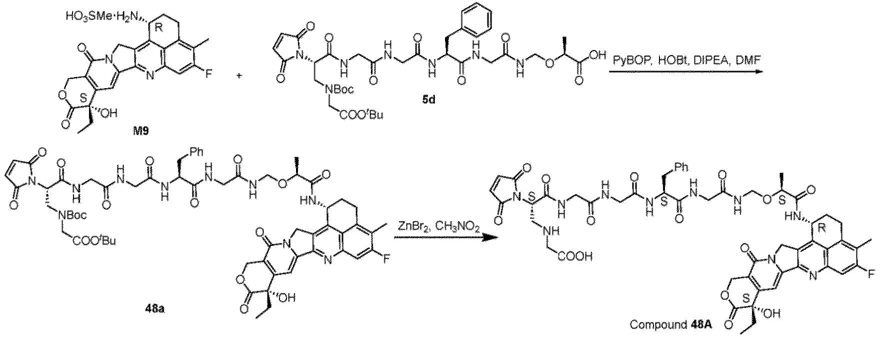

### First step: Compound 48a

In a 100 mL single-neck flask, **5d** (1.66 g, 2.02 mmol, 1.0 eq), **M9** (1.08 g, 2.02 mmol, 1.0 eq), PyBOP (1.58 g, 3.03 mmol, 1.5 eq), HOBt (0.41 g, 3.03 mmol, 1.5 eq) and DMF (40 mL) were mixed and then DIPEA (0.84 mL, 1.5 eq) was added in an ice water bath, and the reaction was allowed at room temperature for 2 h and monitored by HPLC. The reaction solution was directly purified by preparation. The preparation solution was concentrated in a reduced-pressure (water pump) water bath at 35 °C to remove acetonitrile, and then lyophilized to obtain Compound **48a** (1.54 g) with a yield of 61%; LC-MS: [M+H]⁺=1235.4.

### Sixth step: Compound 48A

In a 100 mL single-neck flask, Compound **48a** (1.0 g, 0.8 mmol, 1.0 eq) and 35 mL of nitromethane were mixed and dissolved, followed by addition of zinc bromide (3.64 g, 16 mmol, 20.0 eq). Reaction was allowed in an oil bath at 40 °C (the reaction system was preheated and stabilized in advance) for 30 min. The reaction solution was concentrated in a reduced-pressure (water pump) water bath at 45 °C to remove nitromethane to obtain a yellow solid residue (under the monitoring of HPLC). The preparation solution was obtained by acid preparation. The preparation solution was concentrated in a reduced-pressure (water pump) water bath at 35 °C to remove acetonitrile, and then lyophilized to obtain Compound **48A** (786 mg) with a yield of 90%.

### Example 51

### Synthesis of Compound 48B:

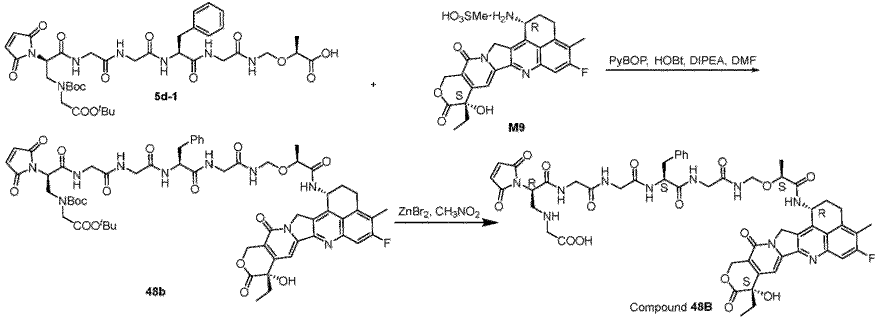

### Second step: Compound 48b

In a 25 mL single-neck flask, Compound **Sd-1** (200 mg, 0.24 mmol, 1.0 eq), **M9** (127 mg, 0.24 mmol, 1.0 eq), PyBOP (187 mg, 0.36 mmol, 1.2eq), HOBt (48 mg, 0.36 mmol, 1.2 eq) and DMF (6 mL) were mixed and then cooled to 0-5°C in an ice water bath, followed by addition of DIPEA (62 mg, 0.48 mmol, 2.0 eq). The reaction was allowed at 20±5 °C for 2 h. After the reaction was found to be completed from the monitoring of the HPLC. The reaction solution was directly purified by HPLC preparation. The product preparation solution was collected and then lyophilized to obtain Compound **48b** (150.2 mg); LC-MS: [M+H]⁺=1235.4.

### Second step: Compound 48B

In a 25 mL single-neck flask, Compound **48b** (100 mg, 0.081 mmol, 1.0 eq), ZnBr₂ (364 mg, 1.62 mmol, 20.0 eq) and CH₃NO₂ (10 mL) were added in sequence. The reaction was allowed at 40 °C for 0.5 h and then terminated. Reduced-pressure rotary evaporation was performed at 45 °C to obtain a yellow solid, then sampled, and monitored by HPLC. The solid subjected to rotary evaporation was directly purified by HPLC preparation. The product preparation solution was collected and then lyophilized to obtain Compound **48B** (70.0 mg); LC-MS: [M+H]⁺=1079.4.

### Example 52

### Synthesis of Compound 49A:

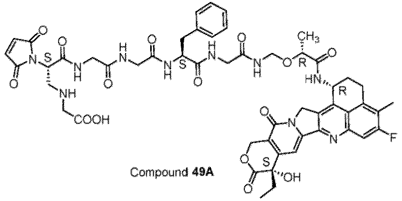

Compound **49A** (71 mg) was obtained by referring to the route of example 50; LC-MS:[M+H]⁺=1079.4.

### Example 53

### Synthesis of Compound 49B

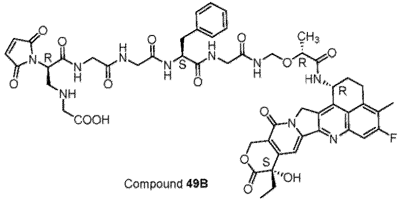

Compound **49B** (65 mg) was obtained by referring to the synthesis route of example 51; LC-MS: [M+H]⁺= 1079.4.

### Example 54

### Synthesis of Compounds 50A and 50B:

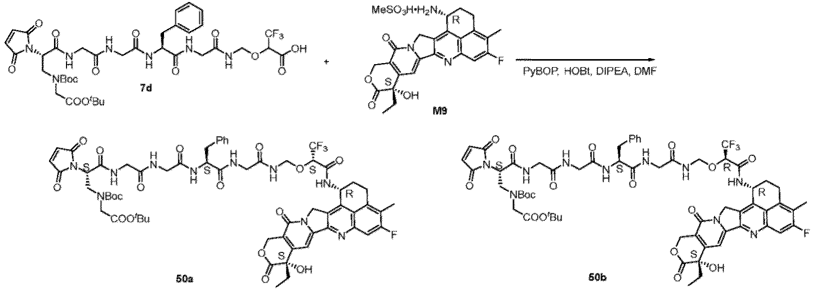

### First step: Compounds 50a and 50b

In a 50 mL single-neck flask, **7d** (500 mg, 0.57 mmol), **M9** (305 mg, 0.57 mmol), PyBOP (448 mg, 0.86mmol), HOBt (116 mg, 0.86 mmol) and 15 mL of DMF were mixed and then DIPEA (378 uL, 2.29 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compound **50a** and Compound **50b.** The preparation solutions were lyophilized separately to obtain 170 mg of Compound **50a,** LC-MS: [M+H]⁺=1289.46; 202 mg of Compound **50b,** LC-MS: [M+H]⁺=1289.46.

### Second step: Compound 50A

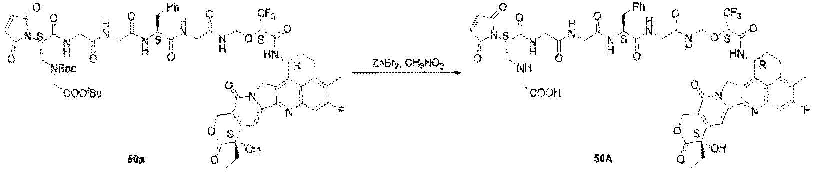

In a 25 mL single-neck flask, **50a** (100 mg, 0.077 mmol), zinc bromide (349 mg, 1.55 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 44 mg of a solid.

### Third step: Compound 50B

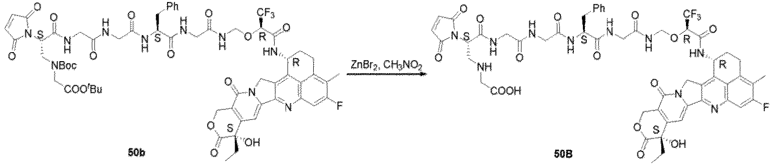

In a 25 mL single-neck flask, **50b** (100 mg, 0.077 mmol), zinc bromide (349 mg, 1.55 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 45 mg of a solid.

### Example 55

### Synthesis of Compounds 51A and 51B:

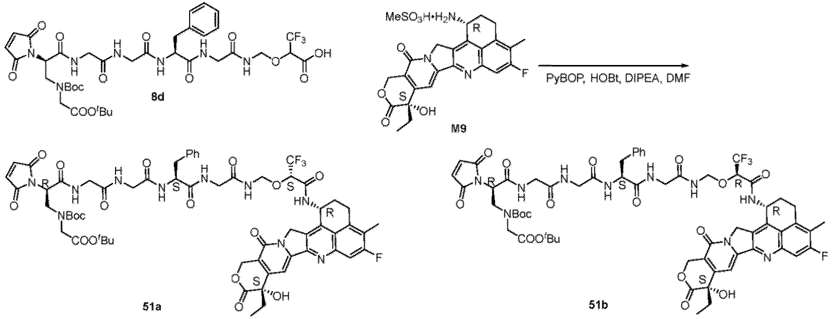

### First step: Compounds 51a and 51b

In a 50 mL single-neck flask, **8d** (500 mg, 0.57 mmol), **M9** (305 mg, 0.57 mmol), PyBOP (448 mg, 0.86 mmol), HOBt (116 mg, 0.86 mmol) and 15 mL of DMF were mixed and then DIPEA (378 uL, 2.29 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compound **51a** and Compound **51b.** The preparation solutions were separately lyophilized to obtain 190 mg of Compound **51a** and 186 mg of Compound **51b.** LC-MS of Compound **51a:** [M+H]⁺=1289.47; LC-MS of Compound **51b:** [M+H]⁺=1289.47.

### Second step: Compound 51A

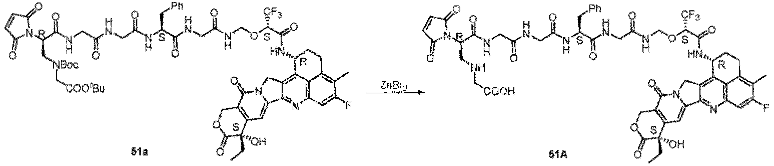

In a 25 mL single-neck flask, Compound **51a** (100 mg, 0.077 mmol), zinc bromide (349 mg, 1.55 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 39 mg of a solid;

### Third step: Compound 51B

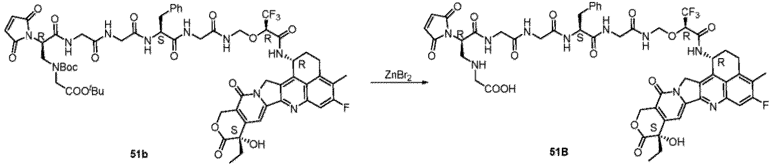

In a 25 mL single-neck flask, Compound **51b** (100 mg, 0.077 mmol), zinc bromide (349 mg, 1.55 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 60 mg of a solid.

### Example 56

### Synthesis of Compound 52A:

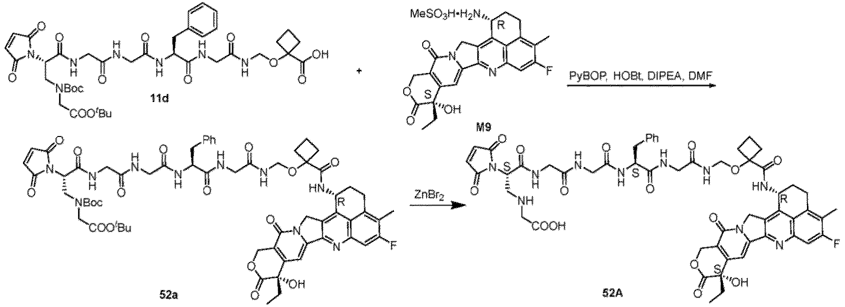

### First step: Compound 52a

In a 50 mL single-neck flask, **11d** (800 mg, 0.96 mmol), **M9** (480 mg, 0.96 mmol), PyBOP (500 mg, 0.96 mmol), HOBt (208 mg, 0.96 mmol) and 30 mL of DMF were mixed and then DIPEA (660 uL, 4.0 mmol) was added in condition of an ice-water bath. The reaction was allowed at room temperature for 4 h. After the reaction was found to be completed from the monitoring of the HPLC, the reaction solution was purified by high-performance liquid chromatography to obtain a preparation solution of Compound **52a.** The preparation solution was lyophilized to obtain **52a** (388 mg); LC-MS: [M+H]⁺=1261.4.

### Second step: Compound 52A

In a 25 mL single-neck flask, Compound **52a** (150 mg, 0.12 mmol), zinc bromide (532 mg, 2.4 mmol) and 10 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **52A** (79 mg); LC-MS: [M+H]⁺=1105.4.

### Example 57

### Synthesis of Compound 52B:

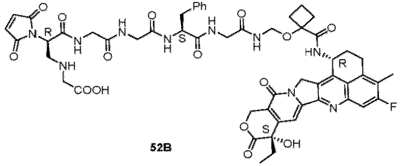

Compound **52B** (50 mg) was obtained by referring to the synthesis route of example 56. LC-MS: [M+H]⁺ 1105.4.

### Example 58

### Synthesis of Compound 53A:

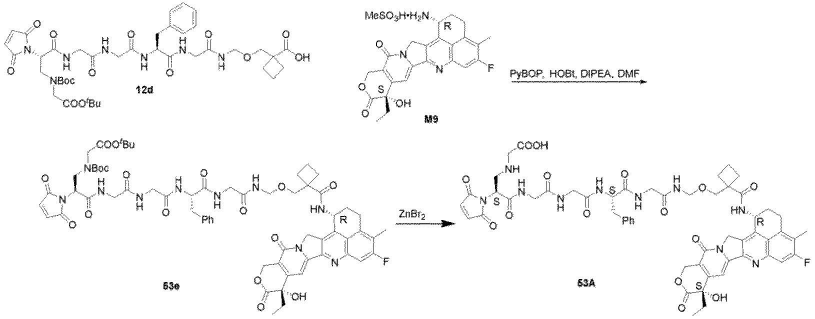

### First step: Compound 53a

In a 50 mL single-neck flask, **12d** (400 mg, 0.47 mmol), **M9** (240 mg, 0.47 mmol), PyBOP (250 mg, 0.47 mmol), HOBt (101 mg, 0.47 mmol) and 15 mL of DMF were mixed and then DIPEA (330 uL, 2.0 mmol) was added in condition of an ice-water bath. The reaction was allowed at room temperature for 3 h. After the reaction was found to be completed from the monitoring of the HPLC, the reaction solution was purified by high-performance liquid chromatography to obtain a preparation solution of Compound **53a.** The preparation solution was lyophilized to obtain **53a** (200 mg); LC-MS: [M+H]⁺=1275.4.

### Second step: Compound 53A

In a 25 mL single-neck flask, **53a** (100 mg, 0.08 mmol), zinc bromide (360 mg, 1.6 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **53A** (51 mg); LC-MS: [M+H]⁺=1119.4.

### Example 59

### Synthesis of Compound 53B:

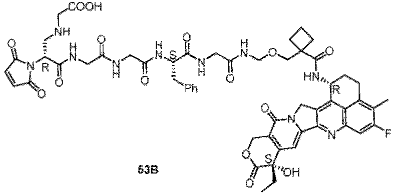

Compound **53B** (50 mg) was obtained by referring to the synthesis route of example 58; LC-MS: [M+H]⁺=1119.4.

### Example 60

### Synthesis of Compounds 54A and 54B:

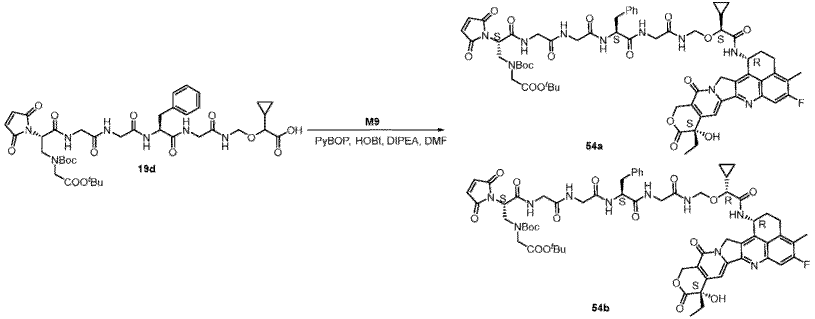

### First step: Compounds 54a and 54b

In a 50 mL single-neck flask, **19d** (500 mg, 0.59 mmol), **M9** (317 mg, 0.59 mmol), PyBOP (339 mg, 0.65 mmol), HOBt (88 mg, 0.86 mmol) and 10 mL of DMF were mixed and then DIPEA (292 uL, 1.77 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compounds **54a** and **54b.** The preparation solutions were separately lyophilized to obtain 103 mg of Compound **54a;** LC-MS: [M+H]⁺=1261.5; 111 mg of Compound **54b,** LC-MS: [M+H]⁺=1261.5.

### Second step: Compound 54A

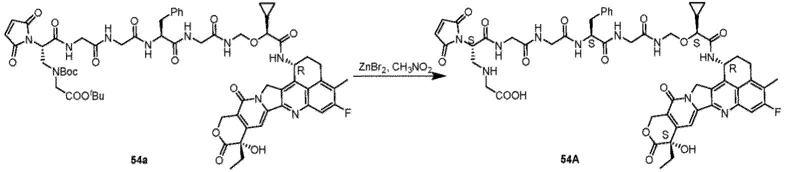

In a 25 mL single-neck flask, **54a** (100 mg, 0.079 mmol), zinc bromide (357 mg, 1.59 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 61 mg of a solid; LC-MS: [M+H]⁺=1105.4.

### Third step: Compound 54B

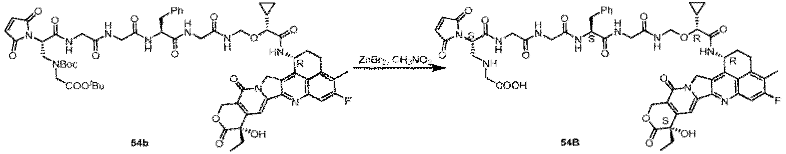

In a 25 mL single-neck flask, **54b** (100 mg, 0.079 mmol), zinc bromide (357 mg, 1.59 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 57 mg of a solid; LC-MS: [M+H]⁺=1105.4.

### Example 61

### Synthesis of Compounds 55A and 55B:

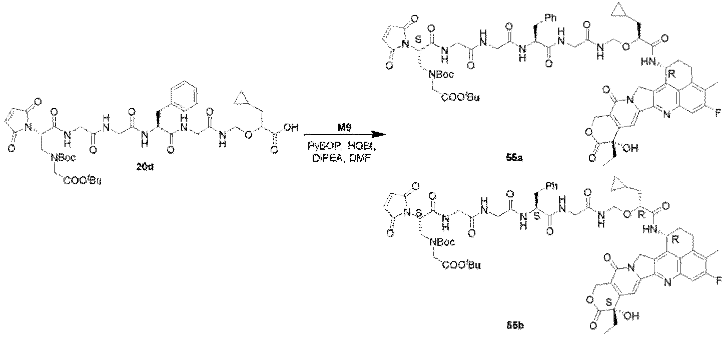

### First step: Compounds 55a and 55b

In a 50 mL single-neck flask, **20d** (400 mg, 0.47 mmol), **M9** (250 mg, 0.47 mmol), PyBOP (223 mg, 0.56 mmol), HOBt (83 mg, 0.56 mmol) and 10 mL of DMF were mixed and then DIPEA (248 uL, 1.5 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compounds **55a** and **55b**. The preparation solutions were separately lyophilized to obtain 100 mg of Compound **55a**, LC-MS: [M+H]⁺=1275.5; 101 mg Compound **55b**, LC-MS: [M+H]⁺=1275.5.

### Second step: Compound 55A

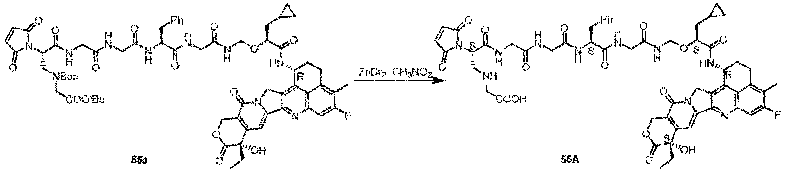

In a 25 mL single-neck flask, **55a** (100 mg, 0.078 mmol), zinc bromide (352 mg, 1.57 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 42 mg of a solid; LC-MS: [M+H]⁺=1119.4.

### Third step: Compound 55B

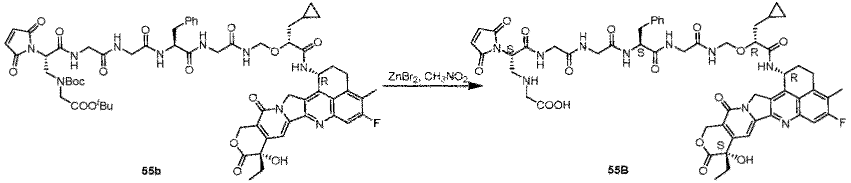

In a 25 mL single-neck flask, **55b** (100 mg, 0.079 mmol), zinc bromide (357 mg, 1.59 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 45 mg of a solid; LC-MS: [M+H]⁺=1119.4.

### Example 62

### Synthesis of Compound 56:

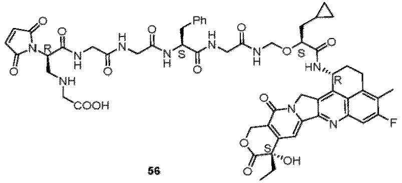

Compound **56** (50 mg) was obtained by referring to the synthesis route of example 60; LC-MS: [M+H]⁺=1119.3.

### Example 63

### Synthesis of Compound 57:

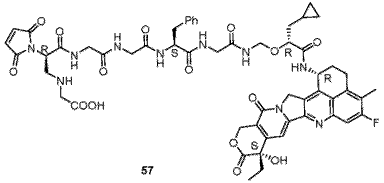

Compound **57** (50 mg) was obtained by referring to the synthesis route of example 61; LC-MS: [M+H]⁺=1119.4.

### Example 64

### Synthesis of Compound 58:

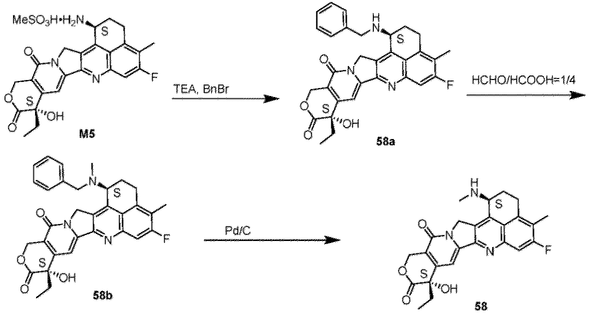

### First step: Synthesis of Compound 58a

400 mL of DMF was added to ixotecan mesylate **M5** (15 g, 28 mol, obtained by referring to the synthesis route disclosed by patent application EP0737683A1). The resulting solution was then cooled to 0 °C in an ice water bath, triethylamine was added dropwise to adjust the pH to 7-8, benzyl bromide (9.6 g, 56 mmol) was then added dropwise in an ice water bath, and the reaction was allowed at room temperature (25 °C) for 1 h. After the reaction was found to be completed from the monitoring of the TLC, the reaction solution was concentrated under reduced pressure. The crude product obtained was purified by preparative-grade high-performance liquid chromatography (acetonitrile/pure water system) and the target peak was collected. Acetonitrile was removed under reduced pressure and then lyophilization was performed to about 11 g of yellow solid Compound **58a**, with a yield of 74%, MS m/z: [M+H]⁺ 526.3.

### Second step: Synthesis of Compound 58b

In a 250 mL single-neck flask, Compound **58a** (11 g, 21 mol) and 120 mL of formic acid were added in sequence and dissolved at room temperature. 30 mL of formaldehyde (40% in water) was added to the resulting bright yellow solution. Reaction was allowed at 50 °C for 1 h. After the reaction was found to be completed from the monitoring of the TLC, the reaction solution was cooled to room temperature and then purified by preparative-grade high-performance liquid chromatography (acetonitrile/pure water system) and the target peak was collected. Acetonitrile was removed under reduced pressure and then lyophilization was performed to about 4.5 g of Compound 58b as yellow solid powder, with a yield of 40%, MS m/z : [M+H]⁺ 540.6. Third step: Synthesis of Compound **58**:
In a 250 mL single-neck flask, Compound **58b** (2.3 g, 4.3 mol) and 100 mL of DMF were added and dissolved at room temperature. 2.3 g of 5% Pd/C was added to the resulting bright yellow solution and the atmosphere in the system was replaced by a hydrogen balloon. Reaction was allowed at room temperature for 1.5 h. After the reaction was found to be completed from the monitoring of the HPLC, Pd/C was filtered out, the resulting reaction solution was concentrated and then purified by preparative-grade high-performance liquid chromatography (acetonitrile/pure water system) and the target peak was collected. Acetonitrile was removed under reduced pressure and then lyophilization was performed to about 1.0 g of Compound 58 as yellow solid powder, with a yield of 52%, MS m/z: [M+H]⁺ 450.5.

### Example 65

### Synthesis of Compound 59:

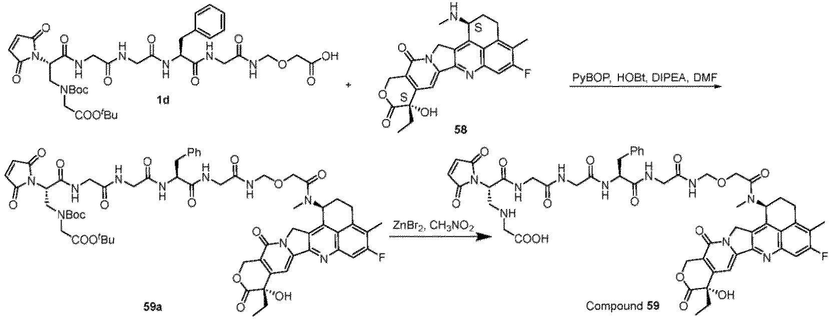

### First step: Compound 59a

In a 50 mL single-neck flask, **1d** (500 mg, 0.62 mmol), **58** (279 mg, 0.62 mmol), PyBOP (448 mg, 0.86 mmol), HOBt (116 mg, 0.86 mmol) and 15 mL of DMF were mixed and then DIPEA (378 uL, 2.29 mmol) was added in condition of an ice-water bath. The reaction was allowed at room temperature for 2 h. After the reaction was found to be completed from the monitoring of the HPLC, the reaction solution was purified by high-performance liquid chromatography to obtain a preparation solution. The preparation solution was lyophilized to obtain **59a** (166 mg); LC-MS: [M+H]⁺=1235.6.

### Second step: Compound 59

In a 25 mL single-neck flask, **59a** (100 mg, 0.081 mmol), zinc bromide (368 mg, 1.63 mmol) and 10 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **59** (43 mg); LC-MS: [M+H]⁺=1079.3.

### Example 66

### Synthesis of Compound 60:

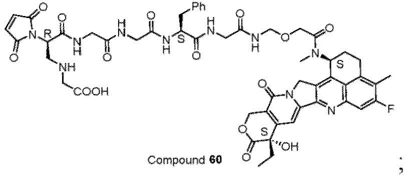

Compound **60** (40 mg) was obtained by referring to the synthesis route of example 65; LC-MS: [M+H]⁺=1079.3.

### Example 67

### Synthesis of Compound 61:

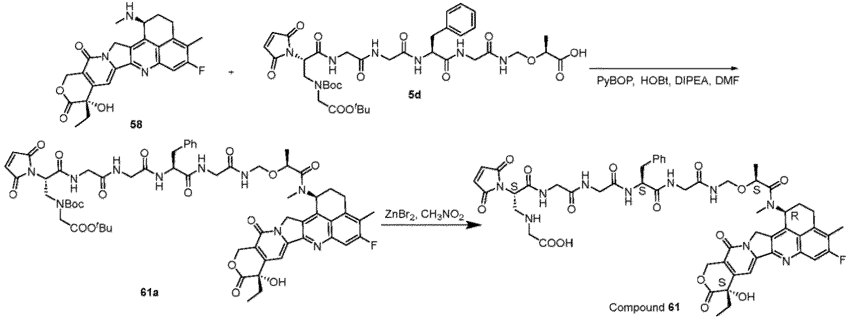

### First step: Compound 61a

In a 100 mL single-neck flask, **5d** (1.66 g, 2.02 mmol, 1.0 eq), **58** (0.91 g,2.02 mmol, 1.0 eq), PyBOP (1.58 g, 3.03 mmol, 1.5 eq), HOBt (0.41 g, 3.03 mmol, 1.5 eq) and DMF (40 mL) were mixed and then DIPEA (0.84 mL, 1.5 eq) was added in condition of an ice-water bath. The reaction was allowed at room temperature for 2 h (monitored by HPLC).
The reaction solution was directly purified by preparation. The preparation solution was concentrated in a reduced-pressure (water pump) water bath at 35 °C to remove acetonitrile, and then lyophilized to obtain Compound **61a** (1.21 g); LC-MS: [M+H]⁺=1249.4.

### Second step: Compound 61

In a 100 mL single-neck flask, Compound **61a** (1.0 g, 0.8 mmol, 1.0 eq) and 35 mL of nitromethane were mixed and dissolved, followed by addition of zinc bromide (3.64 g, 16 mmol, 20.0 eq). Reaction was allowed in an oil bath at 40 °C (the reaction system was preheated and stabilized in advance) for 30 min. The reaction solution was concentrated in a reduced-pressure (water pump) water bath at 45 °C to remove nitromethane to obtain a yellow solid residue (under the monitoring of HPLC). The preparation solution was obtained by acid preparation. The preparation solution was concentrated in a reduced-pressure (water pump) water bath at 35 °C to remove acetonitrile, and then lyophilized to obtain Compound **61** (786 mg), LC-MS: [M+H]⁺=1093.6.

### Example 68

### Synthesis of Compound 62:

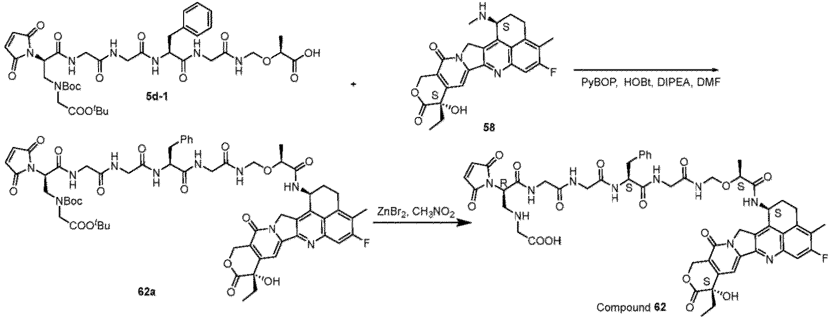

### First step: Compound 62a

In a 25 mL single-neck flask, Compound **5d-1** (200 mg, 0.24 mmol, 1.0 eq), **58** (110.3 mg, 0.24 mmol, 1.0 eq), PyBOP (187 mg, 0.36 mmol, 1.2eq), HOBt (48 mg, 0.36 mmol, 1.2 eq) and DMF (6 mL) were mixed and then cooled to 0-5°C in an ice water bath, followed by addition of DIPEA (62 mg, 0.48 mmol, 2.0 eq). The reaction was allowed at 20±5 °C for 2 h. After the reaction was found to be completed from the monitoring of the HPLC. The reaction solution was directly purified by HPLC preparation. The product preparation solution was collected and then lyophilized to obtain Compound **62a** (120.9 mg); LC-MS: [M+H]⁺=1249.4.

### Second step: Compound 62

In a 25 mL single-neck flask, Compound **62a** (100 mg, 0.081 mmol, 1.0 eq), ZnBr₂ (364 mg, 1.62 mmol, 20.0 eq) and CH₃NO₂ (10 mL) were added in sequence. The reaction was allowed at 40 °C for 0.5 h and then terminated. Reduced-pressure rotary evaporation was performed at 45 °C to obtain a yellow solid, then sampled, and monitored by HPLC. The solid subjected to rotary evaporation was directly purified by HPLC preparation. The product preparation solution was collected and then lyophilized to obtain Compound **62** (61 mg); LC-MS: [M+H]⁺=1093.4.

### Example 69

### Synthesis of Compound 63:

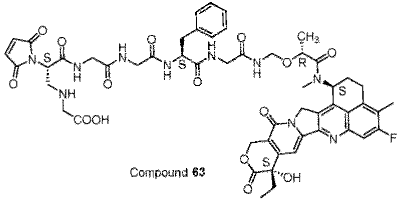

Compound **63** (60 mg) was obtained by referring to the route of example 67; LC-MS:[M+H]⁺=1093.4.

### Example 70

### Synthesis of Compound 64:

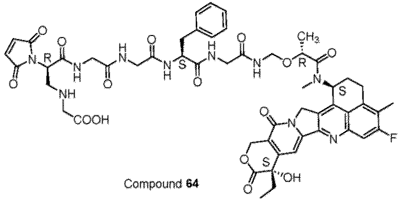

Compound **64** (65 mg) was obtained by referring to the synthesis route of example 68; LC-MS: [M+H]⁺=1093.4.

### Example 71

### Synthesis of Compounds 65A and 65B:

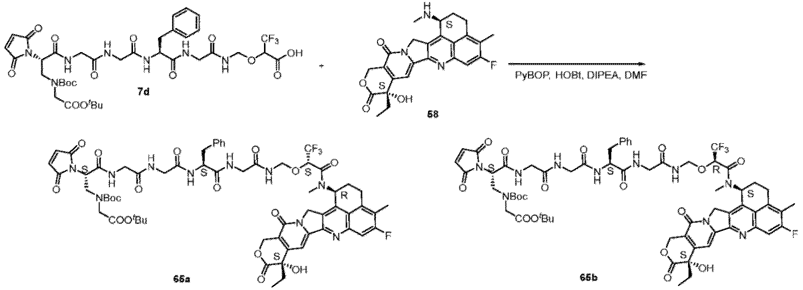

### First step: Compounds 65a and 65b

In a 50 mL single-neck flask, **7d** (500 mg, 0.57 mmol), **58** (256.8 mg, 0.57 mmol), PyBOP (448 mg, 0.86mmol), HOBt (116 mg, 0.86 mmol) and 15 mL of DMF were mixed and then DIPEA (378 uL, 2.29 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compound **65a** and Compound **65b**. The preparation solutions were lyophilized separately to obtain 155 mg of Compound **65a**, LC-MS: [M+H]⁺=1303.4; 158 mg of Compound **65b**, LC-MS: [M+H]⁺=1303.6.

### Second step: Compound 65A

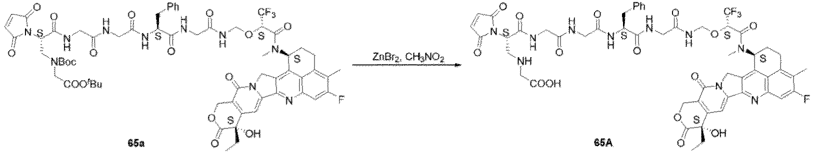

In a 25 mL single-neck flask, **50a** (100 mg, 0.077 mmol), zinc bromide (349 mg, 1.55 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the preparation solution. The preparation solution was lyophilized to obtain 49 mg of a solid.

### Third step: Compound 65B

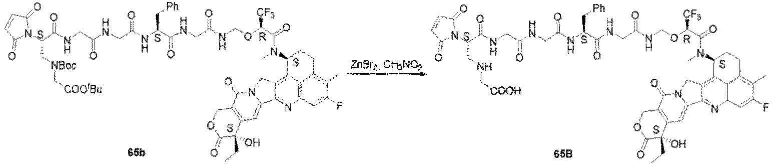

In a 25 mL single-neck flask, **65b** (100 mg, 0.077 mmol), zinc bromide (349 mg, 1.55 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the preparation solution. The preparation solution was lyophilized to obtain 47 mg of a solid.

### Example 72

### Synthesis of Compounds 66A and 66B:

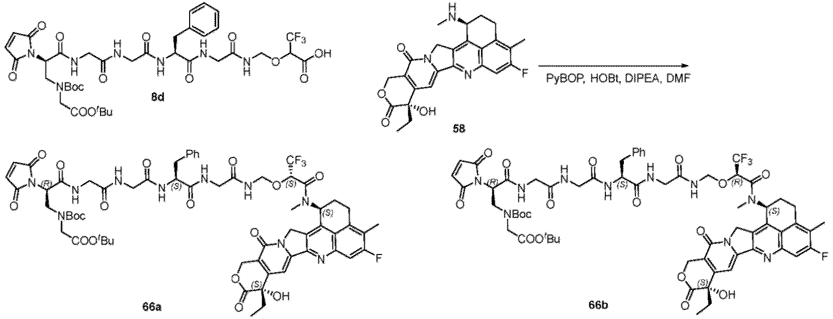

### First step: Compounds 66a and 66b

In a 50 mL single-neck flask, **8d** (500 mg, 0.57 mmol), **58** (256.8 mg, 0.57 mmol), PyBOP (448 mg, 0.86 mmol), HOBt (116 mg, 0.86 mmol) and 15 mL of DMF were mixed and then DIPEA (378 uL, 2.29 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compound **66a** and Compound **66b**. The preparation solutions were lyophilized separately to obtain 160 mg of Compound **66a** and 160 mg Compound **66b**. LC-MS of Compound **66a**: [M+H]⁺=1303.7; LC-MS of Compound **66b**: [M+H]⁺=1303.6.

### Second step: Compound 66A

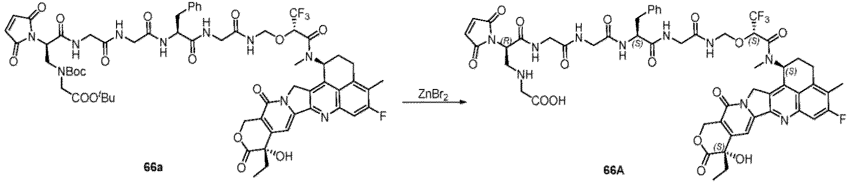

In a 25 mL single-neck flask, Compound **66a** (100 mg, 0.077 mmol), zinc bromide (349 mg, 1.55 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 57 mg of a solid, LC-MS: [M+H]⁺= 1147.5.

### Third step: Compound 66B

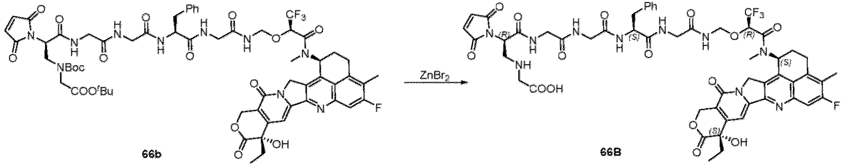

In a 25 mL single-neck flask, Compound **66b** (100 mg, 0.077 mmol), zinc bromide (349 mg, 1.55 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 57 mg of a solid, LC-MS: [M+H]⁺=1147.5.

### Example 73

### Synthesis of Compound 67A:

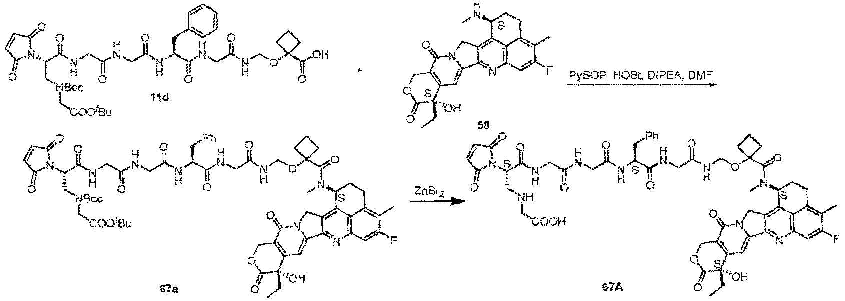

### First step: Compound 67a

In a 50 mL single-neck flask, **11d** (800 mg, 0.96 mmol), **58** (432.5 mg, 0.96 mmol), PyBOP (500 mg, 0.96 mmol), HOBt (208 mg, 0.96 mmol) and 30 mL of DMF were mixed and then DIPEA (660 uL, 4.0 mmol) was added in condition of an ice-water bath. The reaction was allowed at room temperature for 4 h. After the reaction was found to be completed from the monitoring of the HPLC, the reaction solution was purified by high-performance liquid chromatography to obtain a preparation solution of Compound **67a**. The preparation solution was lyophilized to obtain **67a** (402 mg); LC-MS: [M+H]⁺=1275.4.

### Second step: Compound 67A

In a 25 mL single-neck flask, **67a** (100 mg, 0.78 mmol), zinc bromide (356 mg, 1.57 mmol) and 10 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **67A** (47 mg); LC-MS: [M+H]⁺=1119.5.

### Example 74

### Synthesis of Compound 67B:

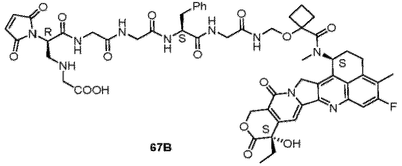

Compound **67B** (50 mg) was obtained by referring to the synthesis route of example 73. LC-MS: [M+H]⁺ 1119.4.

### Example 75

### Synthesis of Compound 68A:

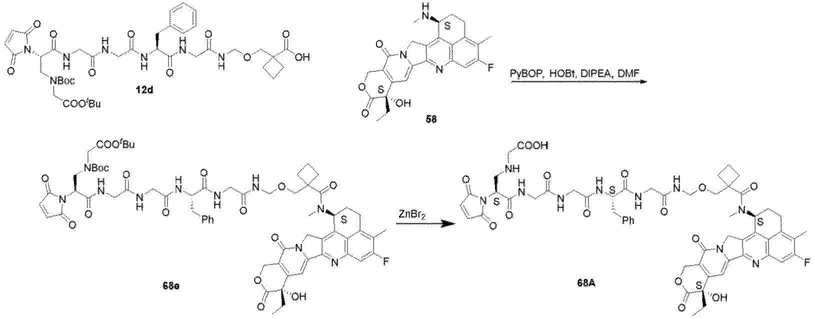

### First step: Compound 68a

In a 50 mL single-neck flask, Compound **12d** (400 mg, 0.47 mmol), **58** (211.7 mg, 0.47 mmol), PyBOP (250 mg, 0.47 mmol), HOBt (101 mg, 0.47 mmol) and 15 mL of DMF were mixed and then DIPEA (330 uL, 2.0 mmol) was added in condition of an ice-water bath. The reaction was allowed at room temperature for 3 h. After the reaction was found to be completed from the monitoring of the HPLC, the reaction solution was purified by high-performance liquid chromatography to obtain a preparation solution of Compound **68a**. The preparation solution was lyophilized to obtain **68a** (177 mg); LC-MS: [M+H]⁺=1289.4.

### Second step: Compound 68A

In a 25 mL single-neck flask, **68a** (100 mg, 0.08 mmol), zinc bromide (360 mg, 1.6 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **68A** (45 mg); LC-MS: [M+H]⁺=1133.4.

### Example 76

### Synthesis of Compound 68B:

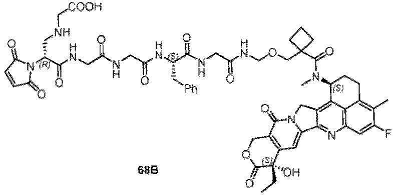

Compound **68B** (50 mg) was obtained by referring to the synthesis route of example 75; LC-MS: [M+H]⁺=1133.4.

### Example 77

### Synthesis of Compounds 69A and 68B:

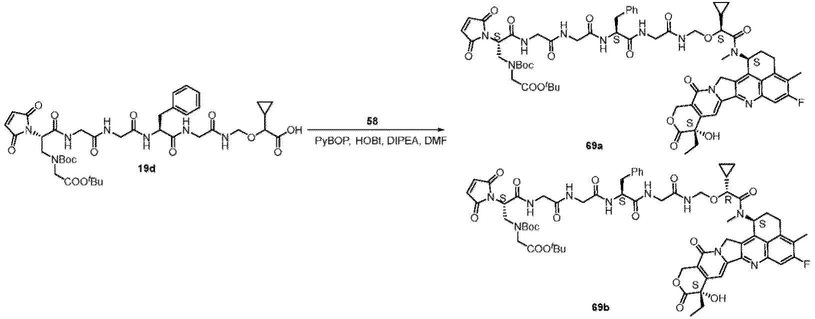

### First step: Compounds 69a and 69b

In a 50 mL single-neck flask, **19d** (500 mg, 0.59 mmol), **58** (266 mg, 0.59 mmol), PyBOP (339 mg, 0.65 mmol), HOBt (88 mg, 0.86 mmol) and 10 mL of DMF were mixed and then DIPEA (292 uL, 1.77 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compound **69a** and Compound **69b**. The preparation solutions were lyophilized separately to obtain 109 mg of Compound **69a**, LC-MS: [M+H]⁺=1275.5; 111 mg of Compound **69b**, LC-MS: [M+H]⁺=1275.7.

### Second step: Compound 69A

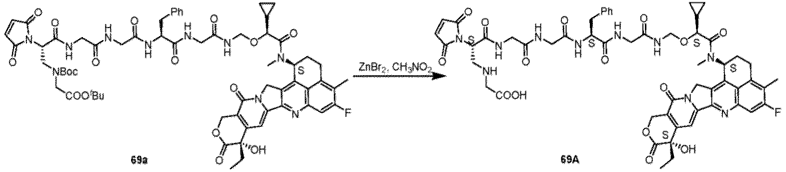

In a 25 mL single-neck flask, **69a** (100 mg, 0.078 mmol), zinc bromide (352 mg, 1.56 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 53 mg of a solid; LC-MS: [M+H]⁺=1119.4.

### Third step: Compound 69B

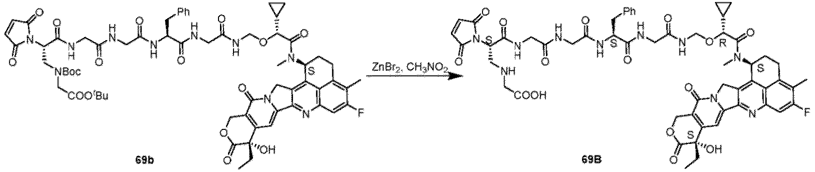

In a 25 mL single-neck flask, **69b** (100 mg, 0.078 mmol), zinc bromide (352 mg, 1.56 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 54 mg of a solid; LC-MS: [M+H]⁺=1119.4.

### Example 78

### Synthesis of Compounds 70A and 70B:

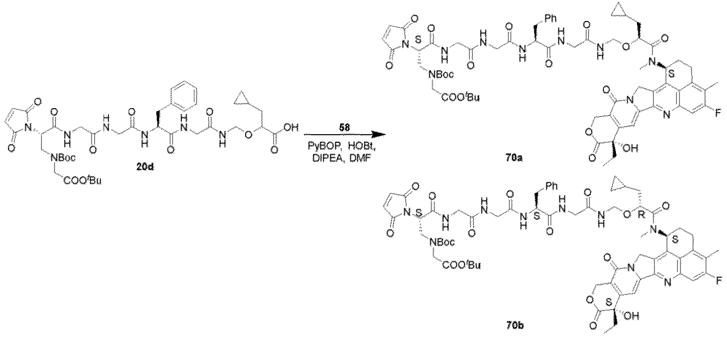

### First step: Compounds 70a and 70b

In a 50 mL single-neck flask, **20d** (400 mg, 0.47 mmol), **58** (211.7 mg, 0.47 mmol), PyBOP (223 mg, 0.56 mmol), HOBt (83 mg, 0.56 mmol) and 10 mL of DMF were mixed and then DIPEA (248 uL, 1.5 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of Compound **70a** and **70b**, and the preparation solutions were lyophilized to obtain 106 mg of Compound **70a**, LC-MS: [M+H]⁺=1289.5, and 101 mg of Compound **70b**, LC-MS: [M+H]⁺=1289.4.

### Second step: Compound 70A

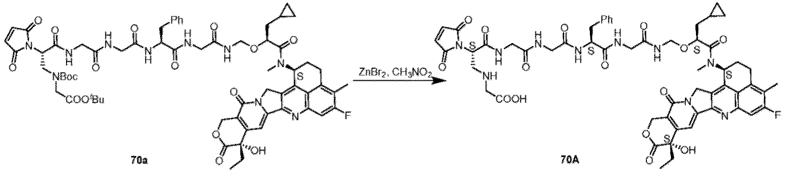

In a 25 mL single-neck flask, **70a** (100 mg, 0.078 mmol), zinc bromide (352 mg, 1.57 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 39 mg of a solid; LC-MS: [M+H]⁺=1133.4.

### Third step: Compound 70B

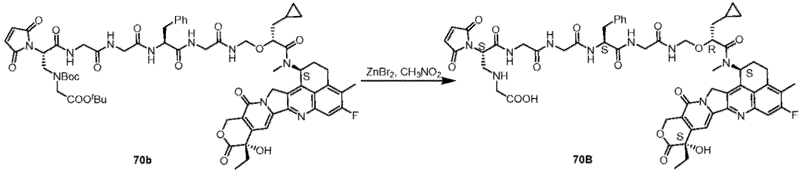

In a 25 mL single-neck flask, **70b** (100 mg, 0.078 mmol), zinc bromide (352 mg, 1.56 mmol) and 5 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain 35 mg of a solid; LC-MS: [M+H]⁺=1133.4.

### Example 79

### Synthesis of Compound 71:

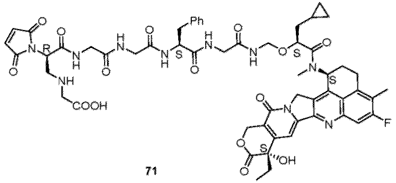

Compound **71** (30 mg) was obtained by referring to the synthesis route of example 78; LC-MS: [M+H]⁺=1133.3.

### Example 80

### Synthesis of Compound 72:

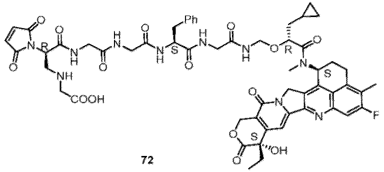

Compound **72** (33 mg) was obtained by referring to the synthesis route of example 78; LC-MS: [M+H]⁺=1133.4.

### Example 81

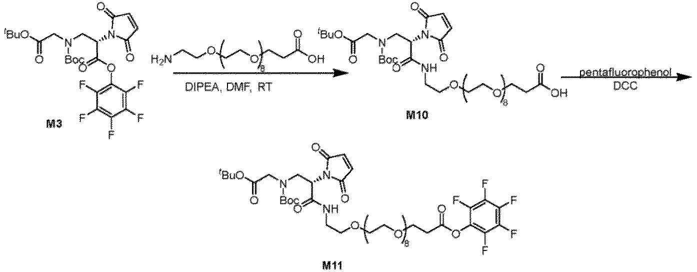

### Synthesis of Compound M11:

In a 100 mL single-neck flask, Compound **M3** (11.0 g, 19.5 mmol, 1.0 eq), DIPEA (2.8 g, 21.4 mmol, 1.1 eq), 27-amino-4, 7, 10, 13, 16, 19, 22, 25-octaoxaheptacosanoic acid (9.7 g, 20.5 mmol, 1.05 eq) and DMF (60 mL) were mixed and the reaction was allowed at room temperature for 20 min and monitored by TLC. The reaction solution was directly purified by preparation. The preparation solution was concentrated in a reduced-pressure (water pump) water bath at 35 °C to remove acetonitrile, and then lyophilized to obtain Compound **M10** (13.2 g) with a yield of 78%; LC-MS: [M+H]⁺=866.5.

In a 100 mL single-neck flask, **M10** (13.0 g, 15 mmol, 1.0 eq), pentafluorophenol (3 g, 16.5 mmol, 1.1 eq), DCC (3.4 g, 16.5 mmol, 1.1 eq) and THF (30 mL) were mixed and the reaction was allowed at room temperature for 30 min and monitored by TLC. The insoluble matter was filtered out. The reaction solution was directly purified by preparation. The preparation solution was concentrated in a reduced-pressure (water pump) water bath at 35 °C to remove acetonitrile, and then lyophilized to obtain Compound **M11** (14.2g) with a yield of 92%; LC-MS: [M+H]⁺=1032.5.

### Example 82

### Synthesis of Compound 73:

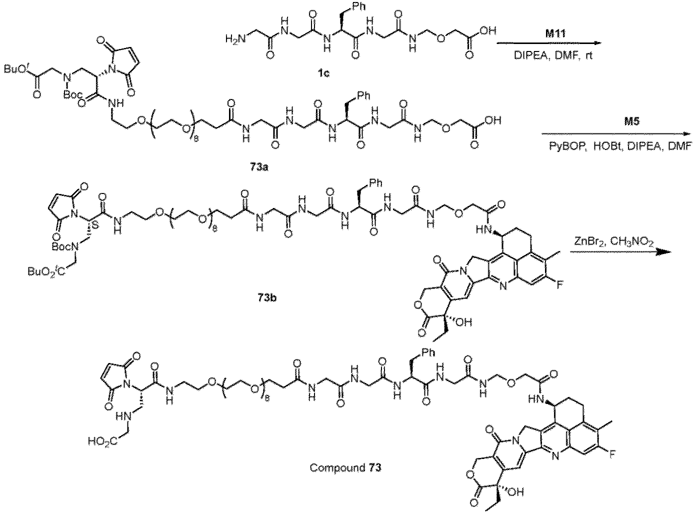

### First step: Synthesis of Compound 73a

10 mL of DMF was added to **M11** (1 g, 0.79 mol). The resulting solution was then cooled to 0 °C in an ice water bath, followed by addition of Compound **1c** (334 mg, 0.79 mol) and DIPEA (154 mg, 1.19 mol). Reaction was allowed under the same condition for 1 h. After the reaction was found to be completed from the monitoring of the TLC, the reaction solution was purified by preparative-grade high-performance liquid chromatography (acetonitrile/pure water system) and the target peak was collected. Acetonitrile was removed under reduced pressure and then lyophilization was performed to about 1.2 g of Compound **73a**, MS m/z: [M+H]⁺ =1271.9.

### Second step: Synthesis of Compound 73b

In a 25 mL single-neck flask, **73a** (1.2 g, 0.94 mmol), **M5** (500 mg, 0.94 mmol), PyBOP (625 mg, 1.2 mmol), HOBt (162 mg, 1.2 mmol), and 15 mL of DMF were mixed and then DIPEA (310 mg, 2.4 mmol) was added in condition of an ice-water bath. The reaction was allowed at room temperature for 2 h. After the reaction was found to be completed from the monitoring of the HPLC, the reaction solution was purified by high-performance liquid chromatography to obtain a preparation solution. The preparation solution was lyophilized to obtain **73b** (709 mg); LC-MS: [M+H]⁺=1720.8.

### Third step: Synthesis of Compound 73:

In a 25 mL single-neck flask, **73b** (200 mg, 0.116 mmol), zinc bromide (523 mg, 2.32 mmol) and 10 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **73** (88 mg); LC-MS: [M+H]⁺=1532.6.

### Example 83

### Synthesis of Compound 74:

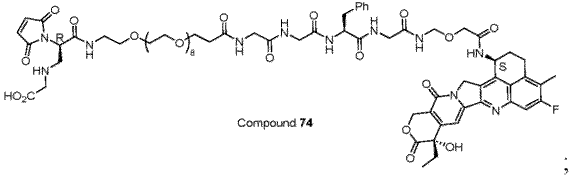

Compound **74** (90 mg) was obtained by referring to the synthesis route of example 82; LC-MS: [M+H]⁺=1532.6.

### Example 84

### Synthesis of Compound 75:

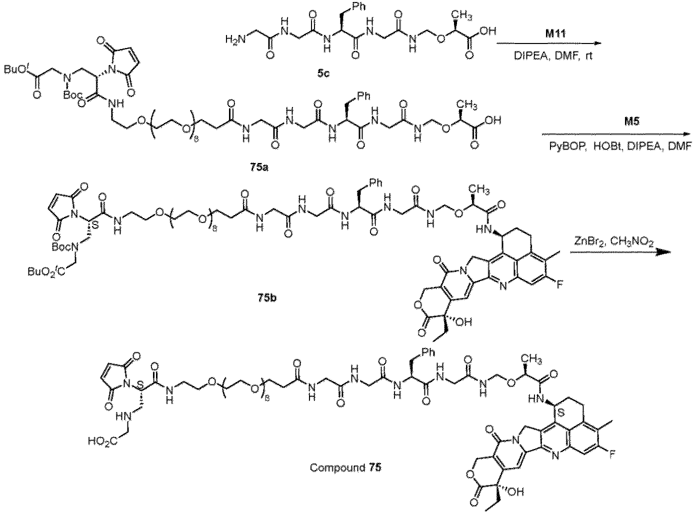

### First step: Synthesis of Compound 75a

10 mL of DMF was added to **M11** (1 g, 0.79 mol). The resulting solution was then cooled to 0 °C in an ice water bath, followed by addition of Compound **5c** (345 mg, 0.79 mol) and DIPEA (154 mg, 1.19 mol). Reaction was allowed under the same condition for 1 h. After the reaction was found to be completed from the monitoring of the TLC, the reaction solution was purified by preparative-grade high-performance liquid chromatography (acetonitrile/pure water system) and the target peak was collected. Acetonitrile was removed under reduced pressure and then lyophilization was performed to obtain 0.9 g of Compound **75a**, MS m/z : [M+H]⁺ =1285.6.

### Second step: Synthesis of Compound 75b

In a 25 mL single-neck flask, **75a** (700 mg, 0.54 mmol), **M5** (289 mg, 0.54 mmol), PyBOP (313 mg, 0.6 mmol), HOBt (81 mg, 0.6 mmol) and 10 mL of DMF were mixed and then DIPEA (155 mg, 1.2 mmol) was added in condition of an ice-water bath. The reaction was allowed at room temperature for 2 h. After the reaction was found to be completed from the monitoring of the HPLC, the reaction solution was purified by high-performance liquid chromatography to obtain a preparation solution of Compound **75b** (304 mg); LC-MS: [M+H]⁺=1734.8.

### Third step: Synthesis of Compound 75:

In a 25 mL single-neck flask, **75b** (200 mg, 0.116 mmol), zinc bromide (523 mg, 2.32 mmol) and 10 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **75** (96 mg); LC-MS: [M+H]⁺=1546.6.

### Example 85

### Synthesis of Compound 76:

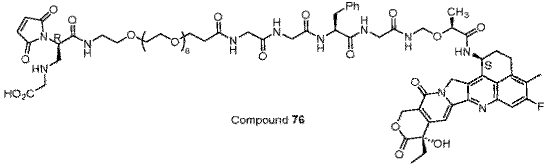

Compound **76** (92 mg) was obtained by referring to the synthesis route of example 84; LC-MS: [M+H]⁺=1546.5.

### Example 86

### Synthesis of Compound 77:

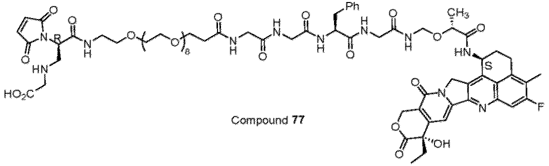

Compound **77** (87 mg) was obtained by referring to the synthesis route of example 84; LC-MS: [M+H]⁺=1546.5.

### Example 87

### Synthesis of Compound 78:

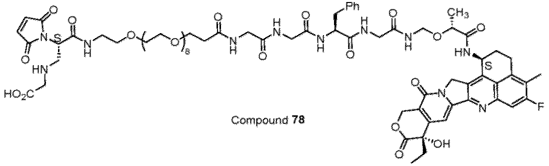

Compound **78** (94 mg) was obtained by referring to the synthesis route of example 84; LC-MS: [M+H]⁺=1546.7.

### Example 88

### Synthesis of Compounds 79 and 80:

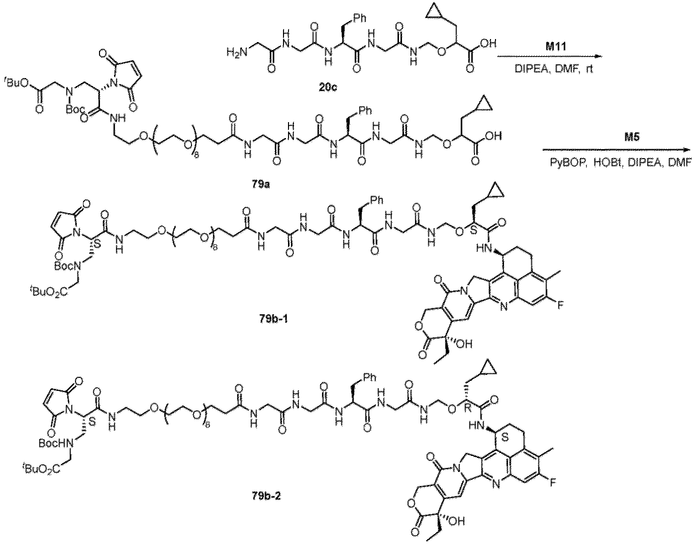

### First step: Synthesis of Compound 79a

10 mL of DMF was added to **M11** (1 g, 0.79 mol). The resulting solution was then cooled to 0 °C in an ice water bath, followed by addition of Compound **20c** (377 mg, 0.79 mol) and DIPEA (154 mg, 1.19 mol). Reaction was allowed under the same condition for 1 h. After the reaction was found to be completed from the monitoring of the TLC, the reaction solution was purified by preparative-grade high-performance liquid chromatography (acetonitrile/pure water system) and the target peak was collected. Acetonitrile was removed under reduced pressure and then lyophilization was performed to obtain 783 mg of Compound **79a**, MS m/z : [M+H]⁺ =1325.8.

### Second step: Synthesis of Compounds 79b-1 and 79b-2

In a 25 mL single-neck flask, **79a** (600 mg, 0.45 mmol), **M5** (240 mg, 0.45 mmol), PyBOP (261 mg, 0.5 mmol), HOBt (68 mg, 0.5 mmol) and 10 mL of DMF were mixed and then DIPEA (130 mg, 1 mmol) was added in condition of an ice-water bath. The reaction was allowed at room temperature for 2 h. After the reaction was found to be completed from the monitoring of the HPLC, the reaction solution was purified by high-performance liquid chromatography to obtain preparation solutions of **79b-1** and **79b-2**. The preparation solutions were lyophilized separately to obtain **79b-1** (124 mg); LC-MS: [M+H]⁺=1743.0; **79b-1** (122 mg); LC-MS: [M+H]⁺=1743.0.

### Third step: Synthesis of Compound 79

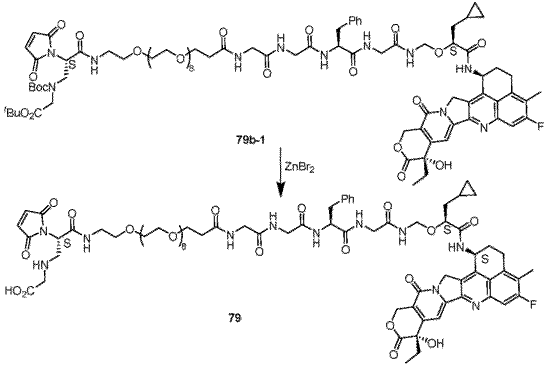

In a 25 mL single-neck flask, **79b-1** (100 mg, 0.057 mmol), zinc bromide (258 mg, 1.15 mmol) and 10 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **79** (30 mg); LC-MS: [M+H]⁺=1586.9.

### Fourth step: Synthesis of Compound 80

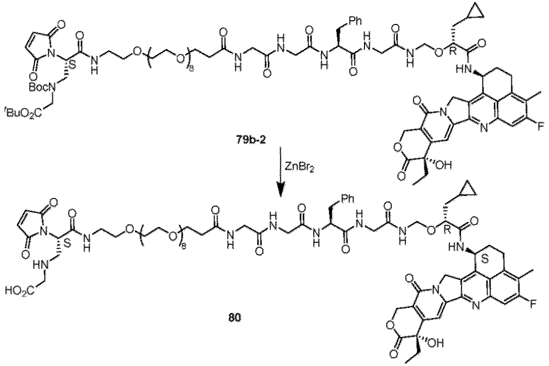

In a 25 mL single-neck flask, **79b-2** (100 mg, 0.057 mmol), zinc bromide (258 mg, 1.15 mmol) and 10 mL of nitromethane were mixed, and reaction was allowed at 40 °C for 1 h. After the reaction was found to be completed from the monitoring of the HPLC, the reaction solution was concentrated under reduced pressure to remove the solvent to obtain a crude product. The crude product was purified by high-performance liquid chromatography to obtain the product preparation solution. The preparation solution was lyophilized to obtain a solid Compound **80** (33 mg); LC-MS: [M+H]⁺=1587.0.

### Example 89

### Synthesis of Compound 81:

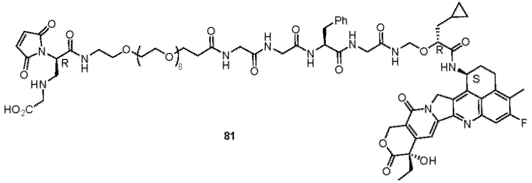

Compound **81** (24 mg) was obtained by referring to the synthesis route of example 88; LC-MS: [M+H]⁺=1586.9.

### Example 90

### Synthesis of Compound 82:

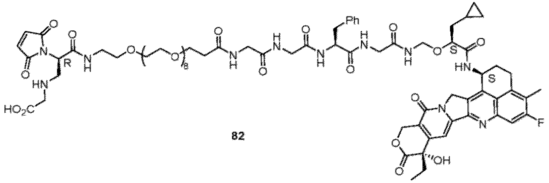

Compound **82** (29 mg) was obtained by referring to the synthesis route of example 88; LC-MS: [M+H]⁺=1586.9.

### Example 91

### 1) Expression and purification of antibody hu4D3:

Cells were suspended with Expi293 (Shanghai OPM Biosciences Co., Ltd.) to express the antibody hu4D3. One day before transfection, the cells were inoculated into a 1 L shake flask containing 300 mL of OPM-293 CD05 Medium (81075-001, Shanghai OPM Biosciences Co., Ltd.) at a density of 0.9×10⁶ cells/mL and cultured overnight on a cell culture shaker at 37 °C, 5% CO₂, and 120 rpm. The next day, PEI-MAX was used to transfect the antibody expression plasmid, in which the mass ratio of plasmid:PEI-MAX was 1:3. OPM-293 ProFeed was added at 5% (v/v) on day 1 after transfection, OPM-293 ProFeed was added again at 5% (v/v) on day 3 after transfection, and the supernatant was collected by centrifugation on day 6 after transfection.

The collected cell expression supernatant was loaded on a Protein A affinity chromatography column (UniMab 50, Suzhou NanoMicro Technology Co., Ltd), and eluted with 0.05 M sodium acetate (pH3.6). The captured antibody was adjusted to pH 7.0 with 1 M Tris-HCl (pH8.8) at a ratio of 0.7/10 (v/v), and then by using a gel filtration chromatography column SEC (Superdex 200, GE Company), impurities such as polymers were removed, and at the same time, the antibody Buffer was replaced with 20 mM PB (pH6.5).

Antibody hu4D3:
Nucleic acid coding sequence of the light chain
   SEQ ID NO: 38 
GATATCCAGATGACCCAGTCTCCATCTAGCCTGTCCGCTTCTGTGGGCGATAGAGTGAC
CATCACATGCAGAGCTTCTCAGGATATCAATAAGTATCTGGCTTGGTATCAGCAGAAGC
CTGGAAAGGTGCCTAAGCTGCTGATCTACTCTACATCTACCCTGCAGTCTGGAGTGCCT
TCTAGATTTTCTGGATCTGGCTCTGGCACCGATTTTACACTGACAATCTCTTCTCTGCAG
CCTGAGGATGTGGCTACATATTATTGTCTGCAGTATGATGATCTGTTCACCTTTGGCCAG
GGCACCAAGCTGGAGATCAAGCGTACGGTGGCTGCACCATCTGTCTTCATCTTCCCGC
CATCTGATGAGCAGTTGAAATCTGGAACTGCCTCTGTTGTGTGCCTGCTGAATAACTTC
TATCCCAGAGAGGCCAAAGTACAGTGGAAGGTGGATAACGCCCTCCAATCGGGTAACT
CCCAGGAGAGTGTCACAGAGCAGGACAGCAAGGACAGCACCTACAGCCTCAGCAGC
ACCCTGACGCTGAGCAAAGCAGACTACGAGAAACACAAAGTCTACGCCTGCGAAGTC
ACCCATCAGGGCCTGAGCTCGCCCGTCACAAAGAGCTTCAACAGGGGAGAGTGTTAG
Amino acid sequence of the light chain
   SEQ ID NO: 18 
DIQMTQSPSSLSASVGDRVTITCRASQDINKYLAWYQQKPGKVPKLLIYSTSTLQSGVPSR

FSGSGSGTDFTLTISSLQPEDVATYYCLQYDDLFTFGQGTKLEIKRTVAAPSVFIFPPSDEQL

KSGTASVVCLLNNFYPREAKVQWKVDNALQSGNSQESVTEQDSKDSTYSLSSTLTLSKAD

YEKHKVYACEVTHQGLSSPVTKSFNRGEC
where the viable region is shown as:
   SEQ ID NO: 14 
DIQMTQSPSSLSASVGDRVTITCRASQDINKYLAWYQQKPGKVPKLLIYSTSTLQSGVPSR
FSGSGSGTDFTLTISSLQPEDVATYYCLQYDDLFTFGQGTKLEIK
where, from left to right, the underlined parts are CDR1 (SEQ ID NO: 7), CDR2 (SEQ ID NO: 8), and CDR3 (SEQ ID NO: 9).

The nucleic acid coding sequence of the variable region of the light chain described above is SEQ ID NO: 34

GATATCCAGATGACCCAGTCTCCATCTAGCCTGTCCGCTTCTGTGGGCGATAGAGTGAC
CATCACATGCAGAGCTTCTCAGGATATCAATAAGTATCTGGCTTGGTATCAGCAGAAGC
CTGGAAAGGTGCCTAAGCTGCTGATCTACTCTACATCTACCCTGCAGTCTGGAGTGCCT
TCTAGATTTTCTGGATCTGGCTCTGGCACCGATTTTACACTGACAATCTCTTCTCTGCAG
CCTGAGGATGTGGCTACATATTATTGTCTGCAGTATGATGATCTGTTCACCTTTGGCCAG
GGCACCAAGCTGGAGATCAAGC

In the variable region of the light chain described above, the nucleotide coding sequences of CDR1 (SEQ ID NO: 7), CDR2 (SEQ ID NO: 8), and CDR3 (SEQ ID NO: 9) are as follows:
SEQ ID No.27
   CAAGACATTAATAAGTAT
SEQ ID No.28
   TCCACATCT
SEQ ID No.29
   CTGCAGTATGATGATCTATTCACG
Nucleic acid coding sequence of the heavy chain
   SEQ ID NO: 37 
ATGGGCTGGTCCTGGGTGTTCCTGTTCCTGCTGAGCGTGACCGCCGGCGTGCACTCCC
AGGTGCAGCTGGTGCAGTCCGGCGCCGAGGTGAAGAAGCCCGGCGCCTCCGTGAAGC
TGAGCTGTAAGGCCTCCGGCTACACCTTCACCTCCTTCGACATTAACTGGGTGCGGCA
GGCCCCCGAGCAGCGCCTGGAGTGGATGGGCTGGATCTTCCCCGGCGACGGCAACAC
CAAGTACTCCCAGAAGTTCCAGGGAAGAGCTACCATCACCAGAGATACATCCGCTTCT
ACAGCTTACATGGAGCTGTCTAGCCTGAGATCTGAGGATACAGCTGTGTATTACTGTGT
GAGAGGAGAGGCTCTGTACTATTTTGATTATTGGGGCCAGGGCACCCTGGTGACAGTG
TCTTCTGCTAGCACCAAGGGCCCATCGGTCTTCCCCCTGGCACCCTCCTCCAAGAGCA
CCTCTGGGGGCACAGCGGCCCTGGGCTGCCTGGTCAAGGACTACTTCCCCGAACCGGT
GACGGTGTCGTGGAACTCAGGCGCCCTGACCAGCGGCGTGCACACCTTCCCGGCTGT
CCTACAGTCCTCAGGACTCTACTCCCTCAGCAGCGTGGTGACCGTGCCCTCCAGCAGC
TTGGGCACCCAGACCTACATCTGCAACGTGAATCACAAGCCCAGCAACACCAAGGTG
GACAAGAGAGTTGAGCCCAAATCTTGTGACAAAACTCACACATGCCCACCGTGCCCA
GCACCTGAACTCCTGGGGGGACCGTCAGTCTTCCTCTTCCCCCCAAAACCCAAGGACA
CCCTCATGATCTCCCGGACCCCTGAGGTCACATGCGTGGTGGTGGACGTGAGCCACGA
AGACCCTGAGGTCAAGTTCAACTGGTACGTGGACGGCGTGGAGGTGCATAATGCCAA
GACAAAGCCGCGGGAGGAGCAGTACAACAGCACGTACCGTGTGGTCAGCGTCCTCAC
CGTCCTGCACCAGGACTGGCTGAATGGCAAGGAGTACAAGTGCAAGGTCTCCAACAA
AGCCCTCCCAGCCCCCATCGAGAAAACCATCTCCAAAGCCAAAGGGCAGCCCCGAGA
ACCACAGGTGTACACCCTGCCCCCATCCCGGGATGAGCTGACCAAGAACCAGGTCAG
CCTGACCTGCCTGGTCAAAGGCTTCTATCCCAGCGACATCGCCGTGGAGTGGGAGAGC
AATGGGCAGCCGGAGAACAACTACAAGACCACGCCTCCCGTGCTGGACTCCGACGGC
TCCTTCTTCCTCTATAGCAAGCTCACCGTGGACAAGAGCAGGTGGCAGCAGGGGAACG
TCTTCTCATGCTCCGTGATGCATGAGGCTCTGCACAACCACTACACGCAGAAGAGCCT
CTCCCTGTCTCCGGGTTAA
Amino acid sequence of the heavy chain
   SEQ ID NO: 17 QVQLVQSGAEVKKPGASVKLSCKASGYTFTSFDINWVRQAPEQRLEWMGWIFPGDGNTK 
YSQKFQGRATITRDTSASTAYMELSSLRSEDTAVYYCVRGEALYYFDYWGQGTLVTVSSA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPSV
FLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRWQQ
GNVFSCSVMHEALHNHYTQKSLSLSPG
where the viable region of the heavy chain is shown as:
   SEQ ID NO: 13 
QVQLVQSGAEVKKPGASVKLSCKASGYTFTSFDINWVRQAPEQRLEWMGWIFPGDGNTK
YSQKFQGRATITRDTSASTAYMELSSLRSEDTAVYYCVRGEALYYFDYWGQGTLVTVSS
where, from left to right, the underlined parts are CDR1 (SEQ ID NO: 1), CDR2 (SEQ ID NO: 2), and CDR3 (SEQ ID NO: 3),

The nucleic acid coding sequence of the variable region of the heavy chain described above is SEQ ID NO: 33

CAGGTGCAGCTGGTGCAGTCCGGCGCCGAGGTGAAGAAGCCCGGCGCCTCCGTGAAG
CTGAGCTGTAAGGCCTCCGGCTACACCTTCACCTCCTTCGACATTAACTGGGTGCGGC
AGGCCCCCGAGCAGCGCCTGGAGTGGATGGGCTGGATCTTCCCCGGCGACGGCAACA
CCAAGTACTCCCAGAAGTTCCAGGGAAGAGCTACCATCACCAGAGATACATCCGCTTC
TACAGCTTACATGGAGCTGTCTAGCCTGAGATCTGAGGATACAGCTGTGTATTACTGTG
TGAGAGGAGAGGCTCTGTACTATTTTGATTATTGGGGCCAGGGCACCCTGGTGACAGT
GTCTTCTG

In the variable region of the heavy chain described above, the nucleotide coding sequences of CDR1 (SEQ ID NO: 1), CDR2 (SEQ ID NO: 2), and CDR3 (SEQ ID NO: 3) are as follows:
SEQ ID No.21
   GGCTACACCTTCACAAGCTTTGAT
SEQ ID No.22
   ATTTTTCCTGGAGATGGTAAT
SEQ ID No.23
   GTAAGAGGGGAGGCCCTGTATTACTTTGACTAC

2) Expression and purification of antibody hu7F11: The antibody hu7F11 was expressed and purified in a similar way.

Antibody hu7F11:
Nucleic acid coding sequence of the light chain
   SEQ ID NO: 40 
GATATCGTGATGACACAGTCTCCTGATTCTCTGGCCGTGTCTCTGGGCGAAAGAGCTAC
AATCAACTGTAAGGCTTCTCAGTCTGTGTCTAATGATGTGGTGTGGTACCAGCAGAAGC
CTGGGCAGCCCCCCAAGCTGCTGATCTACTACGCCTCCAACAGGTACACCGGCGTGCC
CGACAGGTTCTCCGGCTCCGGCTACGGCACCGACTTCACCCTGACCATCTCCTCCCTG
CAGGCCGAGGACGTGGCCGTGTACTACTGCCAGCAGGACTACTCCTCCCCCTGGACCT
TCGGCGGCGGCACCAAGGTGGAGATCAAGCGTACGGTGGCTGCACCATCTGTCTTCAT
CTTCCCGCCATCTGATGAGCAGTTGAAATCTGGAACTGCCTCTGTTGTGTGCCTGCTGA
ATAACTTCTATCCCAGAGAGGCCAAAGTACAGTGGAAGGTGGATAACGCCCTCCAATC
GGGTAACTCCCAGGAGAGTGTCACAGAGCAGGACAGCAAGGACAGCACCTACAGCCT
CAGCAGCACCCTGACGCTGAGCAAAGCAGACTACGAGAAACACAAAGTCTACGCCTG
CGAAGTCACCCATCAGGGCCTGAGCTCGCCCGTCACAAAGAGCTTCAACAGGGGAGA
GTGTTAG
Amino acid sequence of the light chain
   SEQ ID NO: 20 
DIVMTQSPDSLAVSLGERATINCKASQSVSNDVVWYQQKPGQPPKLLIYYASNRYTGVPD

RFSGSGYGTDFTLTISSLQAEDVAVYYCQQDYSSPWTFGGGTKVEIKRTVAAPSVFIFPPSD

EQLKSGTASVVCLLNNFYPREAKVQWKVDNALQSGNSQESVTEQDSKDSTYSLSSTLTLS

KADYEKHKVYACEVTHQGLSSPVTKSFNRGEC
where the viable region of the light chain is shown as:
   SEQ ID NO: 16 
DIVMTQSPDSLAVSLGERATINCKASQSVSNDVVWYQQKPGQPPKLLIYYASNRYTGVPD

RFSGSGYGTDFTLTISSLQAEDVAVYYCQQDYSSPWTFGGGTKVEIK
where, from left to right, the underlined parts are CDR1 (SEQ ID NO: 10), CDR2 (SEQ ID NO: 11), and CDR3 (SEQ ID NO: 12).

The nucleic acid coding sequence of the variable region of the light chain described above is SEQ ID NO: 36

GATATCGTGATGACACAGTCTCCTGATTCTCTGGCCGTGTCTCTGGGCGAAAGAGCTAC
AATCAACTGTAAGGCTTCTCAGTCTGTGTCTAATGATGTGGTGTGGTACCAGCAGAAGC
CTGGGCAGCCCCCCAAGCTGCTGATCTACTACGCCTCCAACAGGTACACCGGCGTGCC
CGACAGGTTCTCCGGCTCCGGCTACGGCACCGACTTCACCCTGACCATCTCCTCCCTG
CAGGCCGAGGACGTGGCCGTGTACTACTGCCAGCAGGACTACTCCTCCCCCTGGACCT
TCGGCGGCGGCACCAAGGTGGAGATCAAGC

In the variable region of the light chain described above, the nucleotide coding sequences of CDR1 (SEQ ID NO: 10), CDR2 (SEQ ID NO: 11), and CDR3 (SEQ ID NO: 12) are as follows:
SEQ ID No. 30
   CAGAGTGTGAGTAATGAT
SEQ ID No. 31
   TATGCATCC
SEQ ID No.32
   CAGCAGGATTATTCCTCTCCGTGGACG
Nucleic acid coding sequence of the heavy chain
   SEQ ID NO: 39 
CAGGTGCAGCTGGTGCAGTCTGGGGCCGAGGTGAAAAAGCCAGGCGCTTCTGTGAAG
GTGTCTTGCAAGGCCTCCGGCTACACCTTCACCGACCACGTGATCTCCTGGGTGCGCC
AGGCCACCGGCCAGGGCCTGGAGTGGATGGGCCAGATCTACCCCGGCTCCGACAACT
CCTACTACGCCCAGAAGTTCCAGGGCAGGGTGACTCTGACCGCCGACAAGTCCATCAA
CACCGCCTACATGGAGCTGTCCTCCCTGAGGTCCGAGGACACCGCCGTGTACTACTGC
GCCAGGGAGGGCTACGGCTACGGCAAGAACGGCGTGGGCTACGCCATGGATTATTGGG
GCCAGGGCACCCTGGTGACAGTGTCTTCTGCTAGCACCAAGGGCCCATCGGTCTTCCC
 
CCTGGCACCCTCCTCCAAGAGCACCTCTGGGGGCACAGCGGCCCTGGGCTGCCTGGTC
AAGGACTACTTCCCCGAACCGGTGACGGTGTCGTGGAACTCAGGCGCCCTGACCAGC
GGCGTGCACACCTTCCCGGCTGTCCTACAGTCCTCAGGACTCTACTCCCTCAGCAGCG
TGGTGACCGTGCCCTCCAGCAGCTTGGGCACCCAGACCTACATCTGCAACGTGAATCA
CAAGCCCAGCAACACCAAGGTGGACAAGAGAGTTGAGCCCAAATCTTGTGACAAAAC
TCACACATGCCCACCGTGCCCAGCACCTGAACTCCTGGGGGGACCGTCAGTCTTCCTC
TTCCCCCCAAAACCCAAGGACACCCTCATGATCTCCCGGACCCCTGAGGTCACATGCG
TGGTGGTGGACGTGAGCCACGAAGACCCTGAGGTCAAGTTCAACTGGTACGTGGACG
GCGTGGAGGTGCATAATGCCAAGACAAAGCCGCGGGAGGAGCAGTACAACAGCACGT
ACCGTGTGGTCAGCGTCCTCACCGTCCTGCACCAGGACTGGCTGAATGGCAAGGAGTA
CAAGTGCAAGGTCTCCAACAAAGCCCTCCCAGCCCCCATCGAGAAAACCATCTCCAA
AGCCAAAGGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCCCAGCGAC
ATCGCCGTGGAGTGGGAGAGCAATGGGCAGCCGGAGAACAACTACAAGACCACGCCT
CCCGTGCTGGACTCCGACGGCTCCTTCTTCCTCTATAGCAAGCTCACCGTGGACAAGA
GCAGGTGGCAGCAGGGGAACGTCTTCTCATGCTCCGTGATGCATGAGGCTCTGCACAA
CCACTACACGCAGAAGAGCCTCTCCCTGTCTCCGGGTTAA
Amino acid sequence of the heavy chain
   SEQ ID NO: 19 
QVQLVQSGAEVKKPGASVKVSCKASGYTFTDHVISWVRQATGQGLEWMGQIYPGSDNS
YYAQKFQGRVTLTADKSINTAYMELSSLRSEDTAVYYCAREGYGYGKNGVGYAMDYWG
QGTLVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKRVEPKSCDKTHTCPPCPA
PELLGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVYTL
PPSRDELTKNQVSLTCLVKGFYPSDIAVEWESNGQPENNYKTTPPVLDSDGSFFLYSKLTVD
KSRWQQGNVFSCSVMHEALHNHYTQKSLSLSPG
where the viable region of the heavy chain is shown as:
   SEQ ID NO: 15 
QVQLVQSGAEVKKPGASVKVSCKASGYTFTDHVISWVRQATGQGLEWMGQIYPGSDNS
YYAQKFQGRVTLTADKSINTAYMELSSLRSEDTAVYYCAREGYGYGKNGVGYAMDYWG
QGTLVTVSS
where, from left to right, the underlined parts are CDR1 (SEQ ID NO: 4), CDR2 (SEQ ID NO: 5), and CDR3 (SEQ ID NO: 6),

The nucleic acid coding sequence of the variable region of the heavy chain described above is SEQ ID NO: 35

CAGGTGCAGCTGGTGCAGTCTGGGGCCGAGGTGAAAAAGCCAGGCGCTTCTGTGAAG
GTGTCTTGCAAGGCCTCCGGCTACACCTTCACCGACCACGTGATCTCCTGGGTGCGCC
AGGCCACCGGCCAGGGCCTGGAGTGGATGGGCCAGATCTACCCCGGCTCCGACAACT
CCTACTACGCCCAGAAGTTCCAGGGCAGGGTGACTCTGACCGCCGACAAGTCCATCAA
CACCGCCTACATGGAGCTGTCCTCCCTGAGGTCCGAGGACACCGCCGTGTACTACTGC
GCCAGGGAGGGCTACGGCTACGGCAAGAACGGCGTGGGCTACGCCATGGATTATTGGG
GCCAGGGCACCCTGGTGACAGTGTCTTCTG

In the variable region of the heavy chain described above, the nucleotide coding sequences of CDR1 (SEQ ID NO: 4), CDR2 (SEQ ID NO: 5), and CDR3 (SEQ ID NO: 6) are as follows:
SEQ ID No.24
   GGATACACATTCACTGACCATGTC
SEQ ID No.25
   ATTTATCCTGGAAGTGATAATAGT
SEQ ID No.26

GCAAGAGAGGGCTATGGTTATGGAAAAAACGGAGTTGGCTATGCTATGGAC
TAC

3) Expression and purification of antibody hTINA1: The antibody hTINA1 was expressed and purified in a similar way. For details, please see item "hTINA1-H1L1" disclosed in Table 2, patent application CN105849126A, description, pages 67/91.

Antibody hTINA1:
Nucleic acid coding sequence of the light chain: SEQ ID No. 43 
ATGGTGCTGCAGACCCAGGTGTTCATCTCCCTGCTGCTGTGGATCTCCGGCGCGTACGG
CGACATCCAGATGACCCAGAGCCCTAGCAGCCTGAGCGCCAGCGTGGGCGACAGAGT
GACCATCACATGCAAGGCCAGCCAGGACGTGTCCACAGCCGTGGCCTGGTATCAGCAG
AAGCCTGGCAAGGCCCCCAAGCTGCTGATCTACAGCGCCAGCTACCGGTACACCGGCG
TGCCCAGCAGATTTTCTGGCAGCGGCTCCGGCACCGACTTCACCCTGACAATCAGCAG
CCTGCAGCCCGAGGACTTCGCCGTGTACTACTGCCAGCAGCACTACATCACCCCCCTG
ACCTTTGGCCAGGGCACCAAGCTGGAAATCAAGCGTACGGTGGCCGCCCCCTCCGTGT
TCATCTTCCCCCCCTCCGACGAGCAGCTGAAGTCCGGCACCGCCTCCGTGGTGTGCCT
GCTGAATAACTTCTACCCCAGAGAGGCCAAGGTGCAGTGGAAGGTGGACAACGCCCT
GCAGTCCGGGAACTCCCAGGAGAGCGTGACCGAGCAGGACAGCAAGGACAGCACCT
ACAGCCTGAGCAGCACCCTGACCCTGAGCAAAGCCGACTACGAGAAGCACAAGGTGT
ACGCCTGCGAGGTGACCCACCAGGGCCTGAGCTCCCCCGTCACCAAGAGCTTCAACA
GGGGGGAGTGT
Amino acid sequence of the light chain: SEQ ID No. 44 
MVLQTQVFISLLLWISGAYGDIQMTQSPSSLSASVGDRVTITCKASQDVSTAVAWYQQKPG
KAPKLLIYSASYRYTGVPSRFSGSGSGTDFTLTISSLQPEDFAVYYCQQHYITPLTFGQGTKL
EIKRTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
 where the viable region is shown as: 
DIQMTQSPSSLSASVGDRVTITCKASQDVSTAVAWYQQKPGKAPKLLIYSASYRYTGVPSR
FSGSGSGTDFTLTISSLQPEDFAVYYCQQHYITPLTFGQGTKLEIK
Nucleic acid coding sequence of the heavy chain: SEQ ID No. 41 
ATGAAACACCTGTGGTTCTTCCTCCTGCTGGTGGCAGCTCCCAGATGGGTGCTGAGCC
AGGTGCAGCTGGTGCAGTCTGGCGCCGAAGTGAAGAAACCAGGCGCCAGCGTGAAG
GTGTCCTGCAAGGCCAGCGGCTACACCTTTACCACCGCCGGCATGCAGTGGGTGCGCC
AGGCTCCTGGACAGGGCCTGGAATGGATGGGCTGGATCAACACCCACAGCGGCGTGC
CCAAATACGCCGAGGACTTCAAGGGCAGAGTGACCATCAGCGCCGACACCAGCACCT
CCACAGCCTACCTGCAGCTGAGCAGCCTGAAGTCCGAGGACACCGCCGTGTACTACTG
CGCCAGAAGCGGCTTCGGCAGCAGCTACTGGTACTTCGACGTGTGGGGCCAGGGCAC
CCTCGTGACCGTCAGCTCAGCCTCCACCAAGGGCCCAAGCGTCTTCCCCCTGGCACCC
 
TCCTCCAAGAGCACCTCTGGCGGCACAGCCGCCCTGGGCTGCCTGGTCAAGGACTACT
TCCCCGAACCCGTGACCGTGAGCTGGAACTCAGGCGCCCTGACCAGCGGCGTGCACA
CCTTCCCCGCTGTCCTGCAGTCCTCAGGACTCTACTCCCTCAGCAGCGTGGTGACCGT
GCCCTCCAGCAGCTTGGGCACCCAGACCTACATCTGCAACGTGAATCACAAGCCCAGC
AACACCAAGGTGGACAAGAGAGTTGAGCCCAAATCTTGTGACAAAACTCACACATGC
CCACCCTGCCCAGCACCTGAACTCCTGGGGGGACCCTCAGTCTTCCTCTTCCCCCCAA
AACCCAAGGACACCCTCATGATCTCCCGGACCCCTGAGGTCACATGCGTGGTGGTGGA
CGTGAGCCACGAAGACCCTGAGGTCAAGTTCAACTGGTACGTGGACGGCGTGGAGGT
GCATAATGCCAAGACAAAGCCCCGGGAGGAGCAGTACAACAGCACGTACCGGGTGGT
CAGCGTCCTCACCGTCCTGCACCAGGACTGGCTGAATGGCAAGGAGTACAAGTGCAA
GGTCTCCAACAAAGCCCTCCCAGCCCCCATCGAGAAAACCATCTCCAAAGCCAAAGG
CCAGCCCCGGGAACCACAGGTGTACACCCTGCCCCCATCCCGGGAGGAGATGACCAA
GAACCAGGTCAGCCTGACCTGCCTGGTCAAAGGCTTCTATCCCAGCGACATCGCCGTG
GAGTGGGAGAGCAATGGCCAGCCCGAGAACAACTACAAGACCACCCCTCCCGTGCTG
GACTCCGACGGCTCCTTCTTCCTCTACAGCAAGCTCACCGTGGACAAGAGCAGGTGGC
AGCAGGGCAACGTCTTCTCATGCTCCGTGATGCATGAGGCTCTGCACAACCACTACAC
CCAGAAGAGCCTCTCCCTGTCTCCCGGCAAA
Amino acid sequence of the heavy chain: SEQ ID No. 42 
MKHLWFFLLLVAAPRWVLSQVQLVQSGAEVKKPGASVKVSCKASGYTFTTAGMQWVRQ
APGQGLEWMGWINTHSGVPKYAEDFKGRVTISADTSTSTAYLQLSSLKSEDTAVYYCARS
GFGSSYWYFDVWGQGTLVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTKVDKRVE
PKSCDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK
where the viable region of the heavy chain is shown as:

QVQLVQSGAEVKKPGASVKVSCKASGYTFTTAGMQWVRQAPGQGLEWMGWINTH
SGVPKYAEDFKGRVTISADTSTSTAYLQLSSLKSEDTAVYYCARSGFGSSYWYFDVWGQG
TLVTVSS

### Example 92

1) Synthesis of an antibody hu4D3-drug conjugate sample by coupling antibody hu4D3 and payload:
   After being expressed in cells and purified by Protein A affinity chromatography and molecular sieve chromatography, the anti-human Trop2 antibody was replaced in 20 mM NaAc-HAc, pH 6.0 buffer and then concentrated or diluted until the anti-human Trop2 antibody reached a protein concentration of 5 mg/mL. The linker-drug was a white powder and dissolved in DMA to the concentration of 10 mg/mL for later use. In order to open the inter-chain disulfide bond of the anti-human Trop2 antibody, 15-fold TCEP was first added according to the molecular ratio and reaction was allowed at room temperature for 2 h; then, 16-fold linker-drug solution was added according to the molecular ratio and reaction was allowed at room temperature for 2 h. After the reaction was found to be completed, the reaction solution was ultrafiltrated using a 30 KDa ultrafiltration centrifuge tube to remove the linker-drug not coupled to the anti-human Trop2 antibody, and then the anti-human Trop2 antibody-drug conjugate sample was obtained.

The resulting human Trop2 antibody-drug conjugate sample will be tested for the percentage of monomer by SEC-HPLC and for the drug loading by RP-HPLC or HIC-HPLC.
2) Synthesis of an antibody hu7F11-drug conjugate sample by coupling antibody hu7F11 and payload in a similar way.

### Example 93

### Synthesis of an antibody TINA1-drug conjugate sample by coupling antibody DS1062a and payload

After being expressed in cells and purified by Protein A affinity chromatography and molecular sieve chromatography, the anti-human Trop2 antibody was replaced in 20 mM NaAc-HAc, pH 6.0 buffer and then concentrated or diluted until the anti-human Trop2 antibody reached a protein concentration of 5 mg/mL. The linker-drug was a white powder and dissolved in DMA to the concentration of 10 mg/mL for later use. In order to open the inter-chain disulfide bond of the anti-human Trop2 antibody, 8-12-fold TCEP was first added according to the molecular ratio and reaction was allowed at room temperature for 1-2 h; then, 5-8-fold linker-drug solution was added according to the molecular ratio and reaction was allowed at room temperature for 1-2 h. After the reaction was found to be completed, the reaction solution was ultrafiltrated using a 30 KDa ultrafiltration centrifuge tube to remove the linker-drug not coupled to the anti-human Trop2 antibody, and then the anti-human Trop2 antibody-drug conjugate sample was obtained.

The resulting human Trop2 antibody-drug conjugate sample will be tested for the percentage of monomer by SEC-HPLC and for the drug loading by RP-HPLC or HIC-HPLC.

### Example 94

ADC-1 was synthesized according to the general coupling method of Example 92,

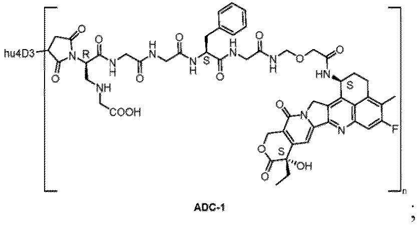

### Example 95

ADC-2 was synthesized according to the general coupling method of Example 92,

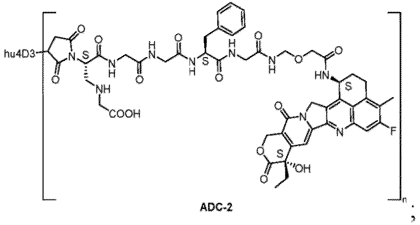

### Example 96

ADC-3 was synthesized according to the general coupling method of Example 92,

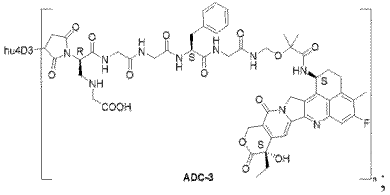

### Example 97

ADC-4 was synthesized according to the general coupling method of Example 92,

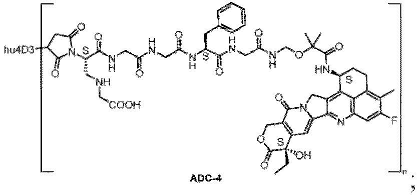

### Example 98

ADC-5 was synthesized according to the general coupling method of Example 92,

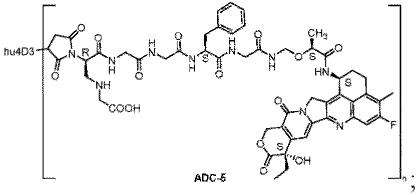

### Example 99

ADC-6 was synthesized according to the general coupling method of Example 92,

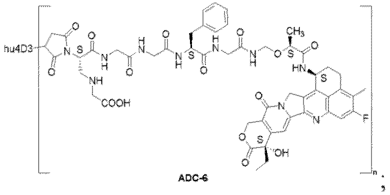

### Example 100

ADC-7 was synthesized according to the general coupling method of Example 92,

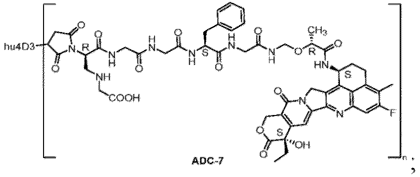

### Example 101

ADC-8 was synthesized according to the general coupling method of Example 92,

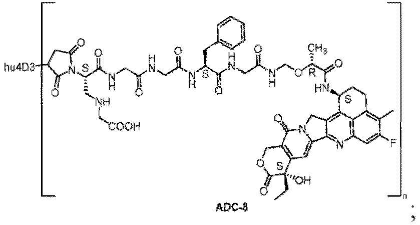

### Example 102

ADC-9 was synthesized according to the general coupling method of Example 92,

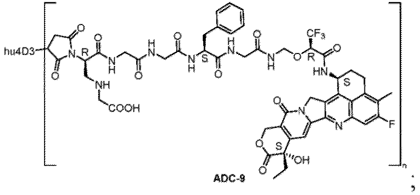

### Example 103

ADC-10 was synthesized according to the general coupling method of Example 92,

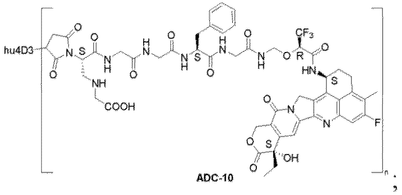

### Example 104

ADC-11 was synthesized according to the general coupling method of Example 92,

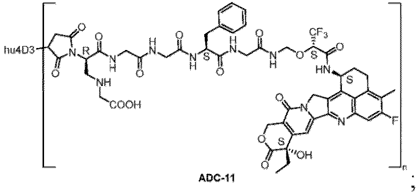

### Example 105

ADC-12 was synthesized according to the general coupling method of Example 92,

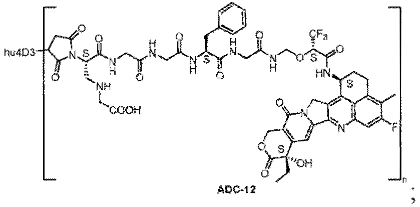

### Example 106

ADC-13 was synthesized according to the general coupling method of Example 92,

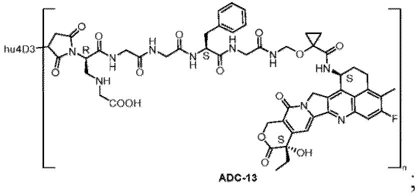

### Example 107

ADC-14 was synthesized according to the general coupling method of Example 92,

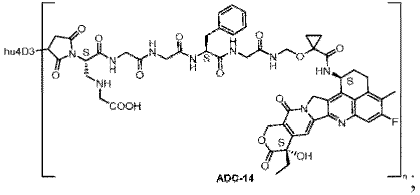

### Example 108

ADC-15 was synthesized according to the general coupling method of Example 92,

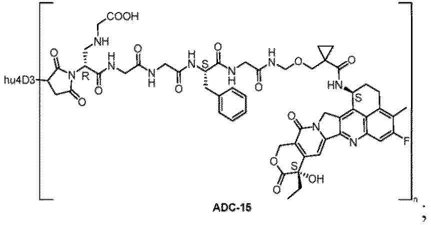

### Example 109

ADC-16 was synthesized according to the general coupling method of Example 92,

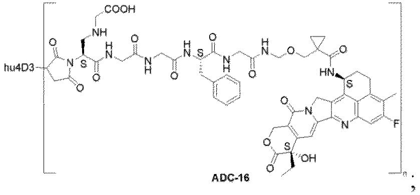

### Example 110

ADC-17 was synthesized according to the general coupling method of Example 92,

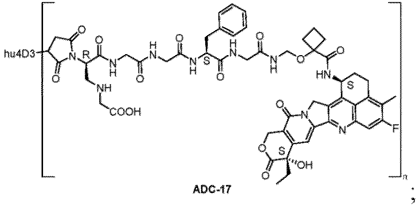

### Example 111

ADC-18 was synthesized according to the general coupling method of Example 92,

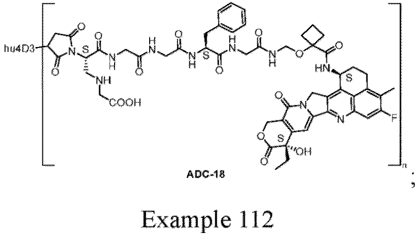

ADC-19 was synthesized according to the general coupling method of Example 92,

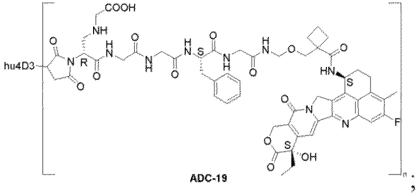

### Example 113

ADC-20 was synthesized according to the general coupling method of Example 92,

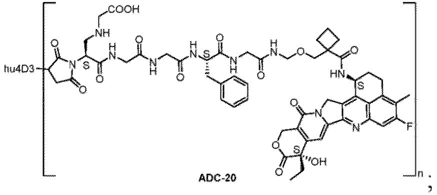

### Example 114

ADC-21 was synthesized according to the general coupling method of Example 92,

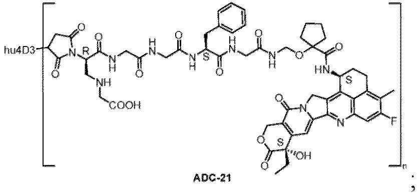

### Example 115

ADC-22 was synthesized according to the general coupling method of Example 92,

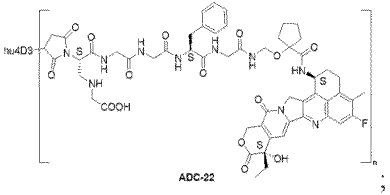

### Example 116

ADC-23 was synthesized according to the general coupling method of Example 92,

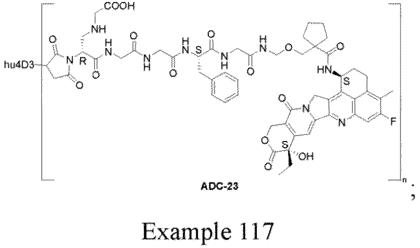

ADC-24 was synthesized according to the general coupling method of Example 92,

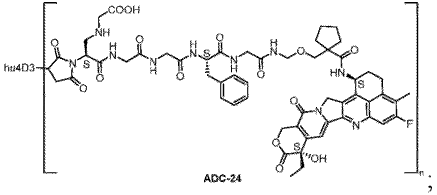

### Example 118

ADC-25 was synthesized according to the general coupling method of Example 92,

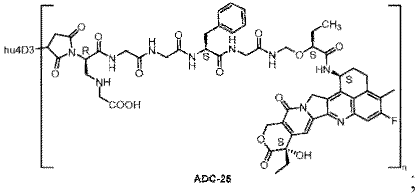

### Example 119

ADC-26 was synthesized according to the general coupling method of Example 92,

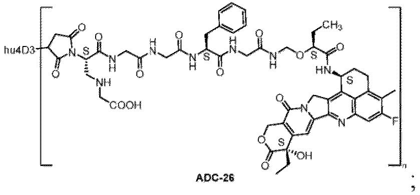

### Example 120

ADC-27 was synthesized according to the general coupling method of Example 92,

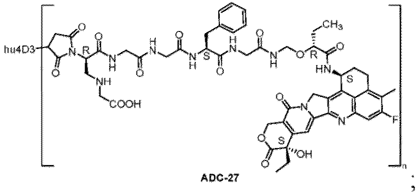

### Example 121

ADC-28 was synthesized according to the general coupling method of Example 92,

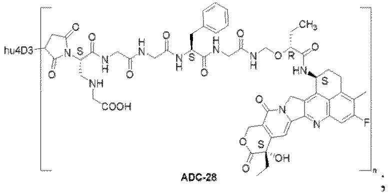

### Example 122

ADC-29 was synthesized according to the general coupling method of Example 92,

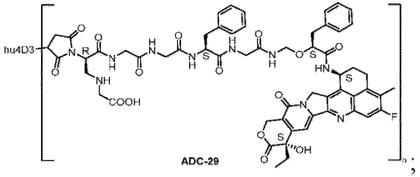

### Example 123

ADC-30 was synthesized according to the general coupling method of Example 92,

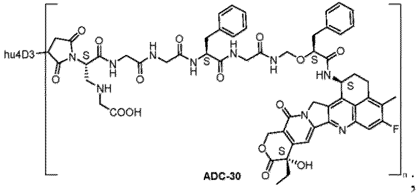

### Example 124

ADC-31 was synthesized according to the general coupling method of Example 92,

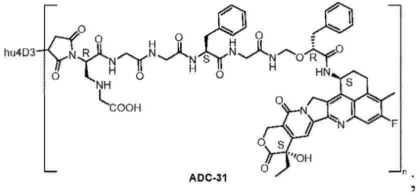

### Example 125

ADC-32 was synthesized according to the general coupling method of Example 92,

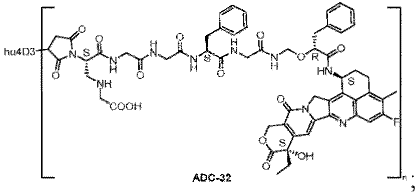

### Example 126

ADC-33 was synthesized according to the general coupling method of Example 92,

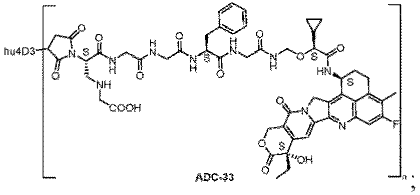

### Example 127

ADC-34 was synthesized according to the general coupling method of Example 92,

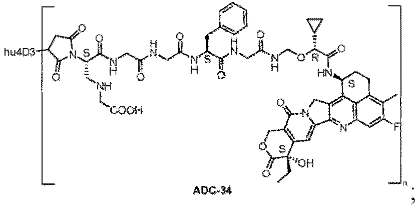

### Example 128

ADC-35 was synthesized according to the general coupling method of Example 92,

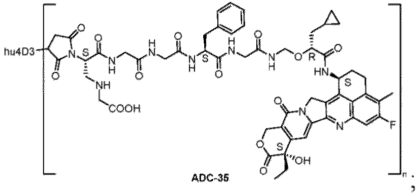

### Example 129

ADC-36 was synthesized according to the general coupling method of Example 92,

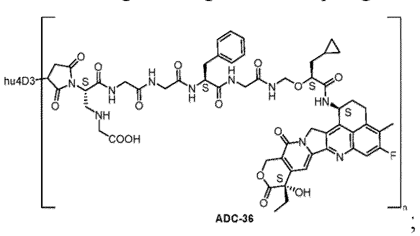

### Example 130

ADC-37 was synthesized according to the general coupling method of Example 92,

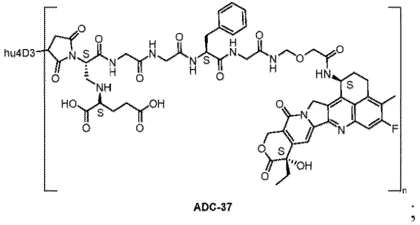

### Example 131

ADC-38 was synthesized according to the general coupling method of Example 92,

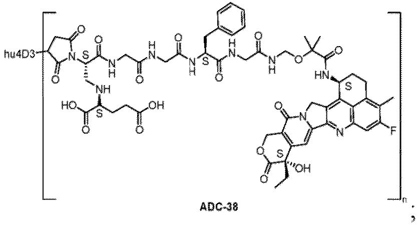

### Example 132

ADC-39 was synthesized according to the general coupling method of Example 92,

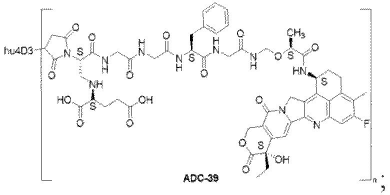

### Example 133

ADC-40 was synthesized according to the general coupling method of Example 92,

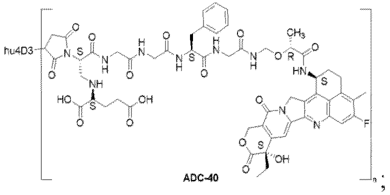

### Example 134

ADC-41 was synthesized according to the general coupling method of Example 92,

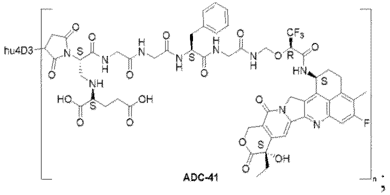

### Example 135

ADC-42 was synthesized according to the general coupling method of Example 92,

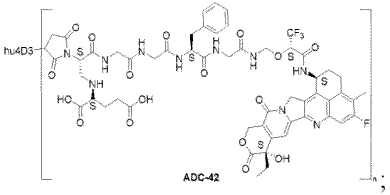

### Example 136

ADC-43 was synthesized according to the general coupling method of Example 92,

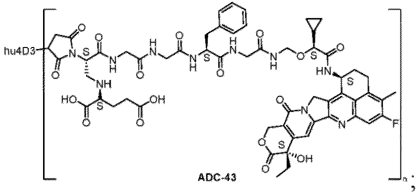

### Example 137

ADC-44 was synthesized according to the general coupling method of Example 92,

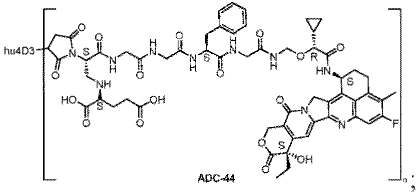

### Example 138

ADC-45 was synthesized according to the general coupling method of Example 92,

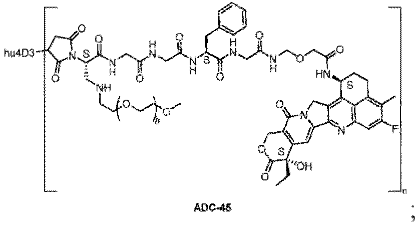

### Example 139

ADC-46 was synthesized according to the general coupling method of Example 92,

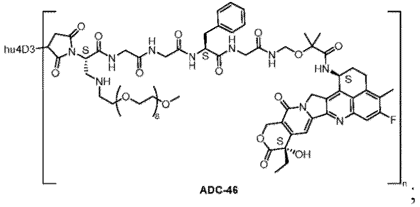

### Example 140

ADC-47 was synthesized according to the general coupling method of Example 92,

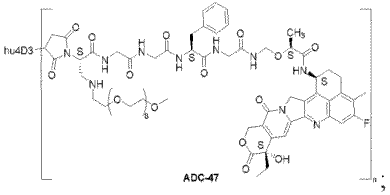

### Example 141

ADC-48 was synthesized according to the general coupling method of Example 92,

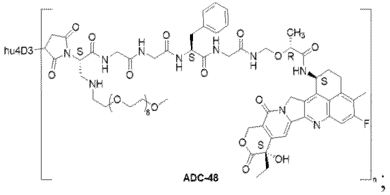

### Example 142

ADC-49 was synthesized according to the general coupling method of Example 92,

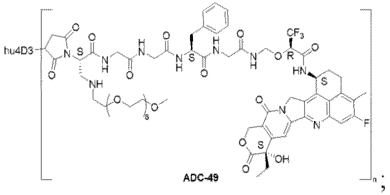

### Example 143

ADC-50 was synthesized according to the general coupling method of Example 92,

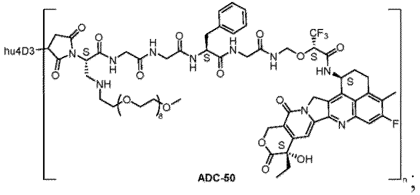

### Example 144

ADC-51 was synthesized according to the general coupling method of Example 92,

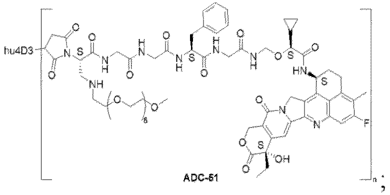

### Example 145

ADC-52 was synthesized according to the general coupling method of Example 92,

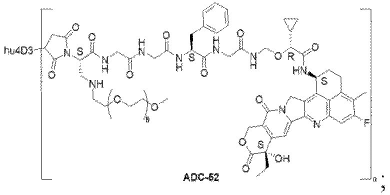

### Example 146

ADC-53 was synthesized according to the general coupling method of Example 92,

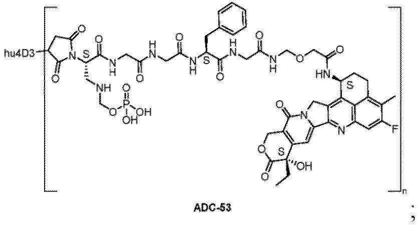

### Example 147

ADC-54 was synthesized according to the general coupling method of Example 92,

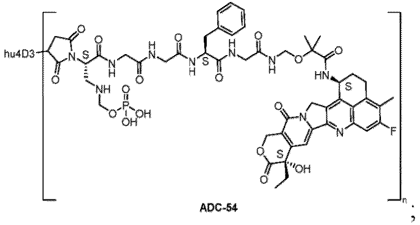

### Example 148

ADC-55 was synthesized according to the general coupling method of Example 92,

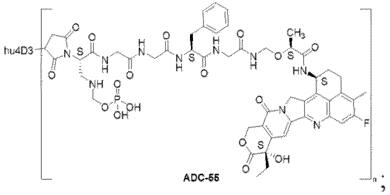

### Example 149

ADC-56 was synthesized according to the general coupling method of Example 92,

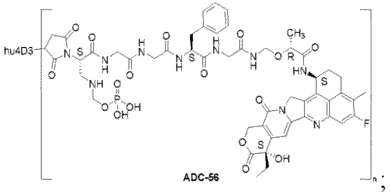

### Example 150

ADC-57 was synthesized according to the general coupling method of Example 92,

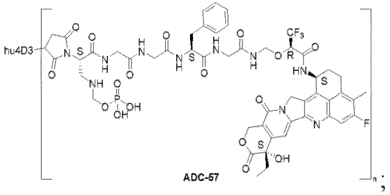

### Example 151

ADC-58 was synthesized according to the general coupling method of Example 92,

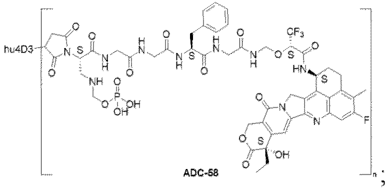

### Example 152

ADC-59 was synthesized according to the general coupling method of Example 92,

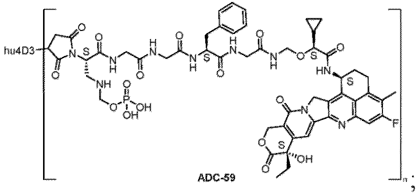

### Example 153

ADC-60 was synthesized according to the general coupling method of Example 92,

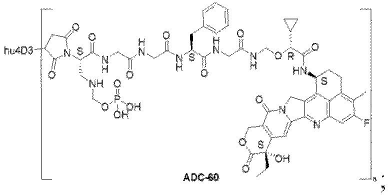

### Example 154

ADC-61 was synthesized according to the general coupling method of Example 92,

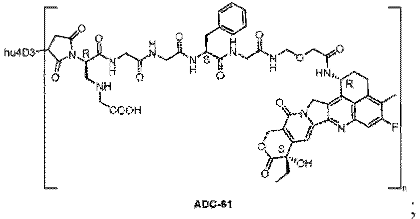

### Example 155

ADC-62 was synthesized according to the general coupling method of Example 92,

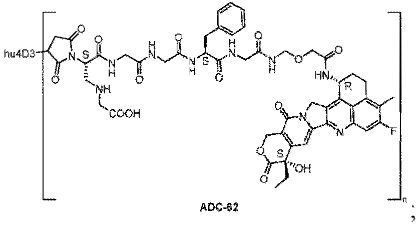

### Example 156

ADC-63 was synthesized according to the general coupling method of Example 92,

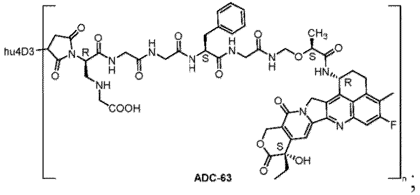

### Example 157

ADC-64 was synthesized according to the general coupling method of Example 92,

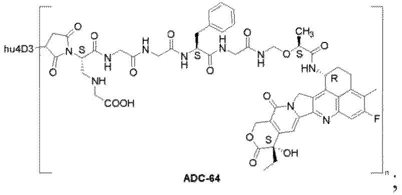

### Example 158

ADC-65 was synthesized according to the general coupling method of Example 92,

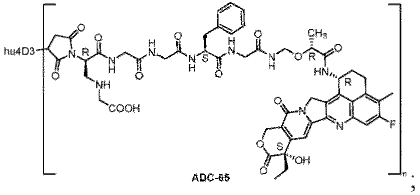

### Example 159

ADC-66 was synthesized according to the general coupling method of Example 92,

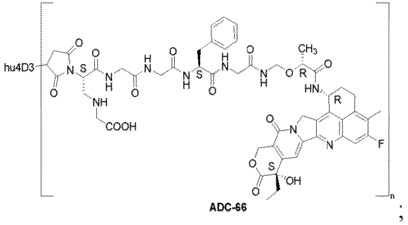

### Example 160

ADC-67 was synthesized according to the general coupling method of Example 92,

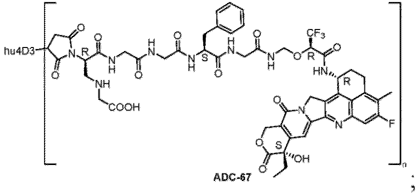

### Example 161

ADC-68 was synthesized according to the general coupling method of Example 92,

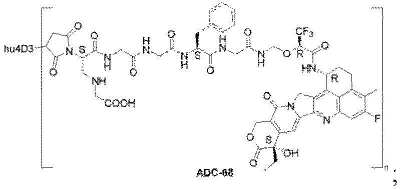

### Example 162

ADC-69 was synthesized according to the general coupling method of Example 92,

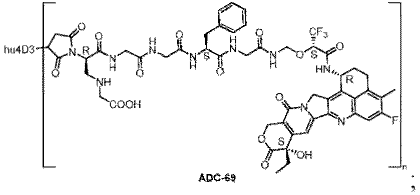

### Example 163

ADC-70 was synthesized according to the general coupling method of Example 92,

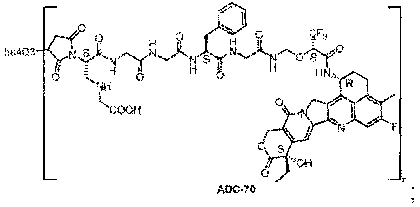

### Example 164

ADC-71 was synthesized according to the general coupling method of Example 92,

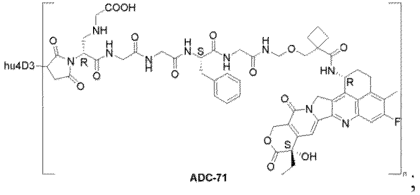

### Example 165

ADC-72 was synthesized according to the general coupling method of Example 92,

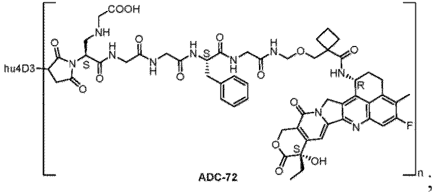

### Example 166

ADC-73 was synthesized according to the general coupling method of Example 92,

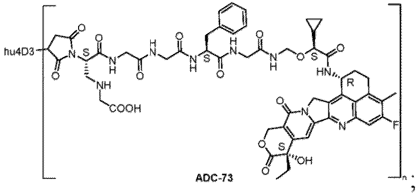

### Example 167

ADC-74 was synthesized according to the general coupling method of Example 92,

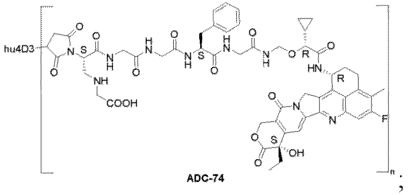

### Example 168

ADC-75 was synthesized according to the general coupling method of Example 92,

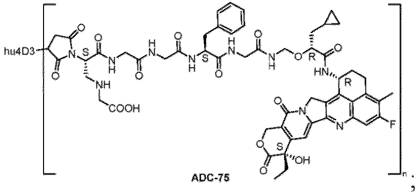

### Example 169

ADC-76 was synthesized according to the general coupling method of Example 92,

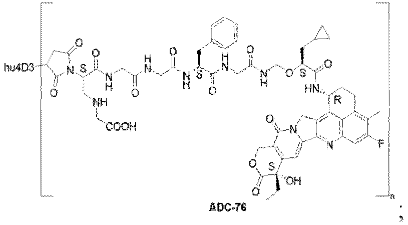

### Example 170

ADC-77 was synthesized according to the general coupling method of Example 92,

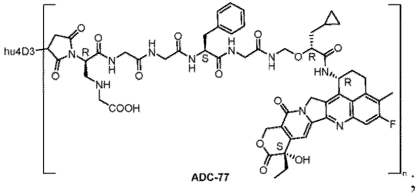

### Example 171

ADC-78 was synthesized according to the general coupling method of Example 92,

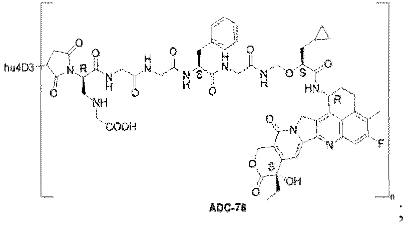

### Example 172

ADC-79 was synthesized according to the general coupling method of Example 92,

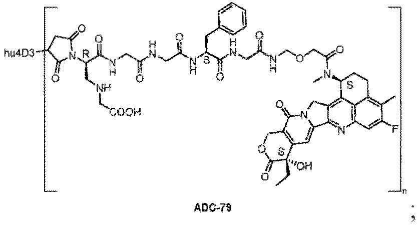

### Example 173

ADC-80 was synthesized according to the general coupling method of Example 92,

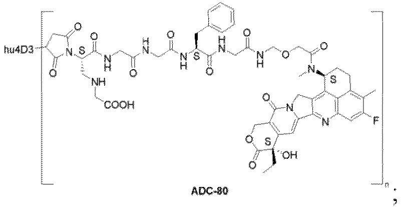

### Example 174

ADC-81 was synthesized according to the general coupling method of Example 92,

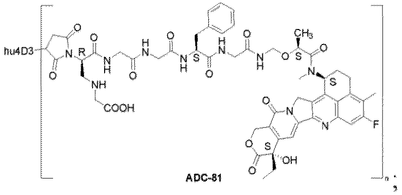

### Example 175

ADC-82 was synthesized according to the general coupling method of Example 92,

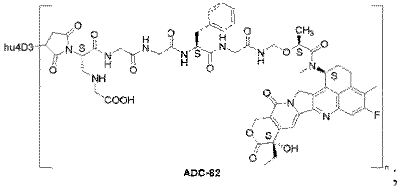

### Example 176

ADC-83 was synthesized according to the general coupling method of Example 92,

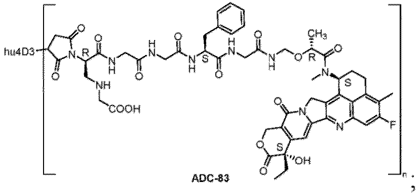

### Example 177

ADC-84 was synthesized according to the general coupling method of Example 92,

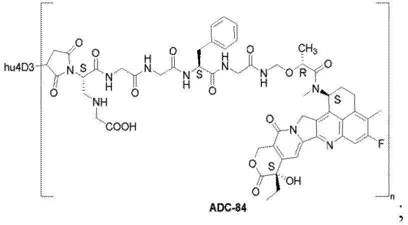

### Example 178

ADC-85 was synthesized according to the general coupling method of Example 92,

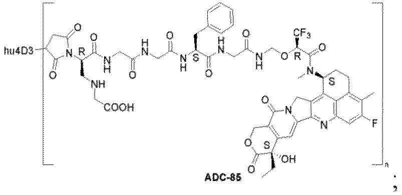

### Example 179

ADC-86 was synthesized according to the general coupling method of Example 92,

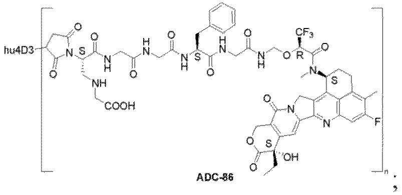

### Example 180

ADC-87 was synthesized according to the general coupling method of Example 92,

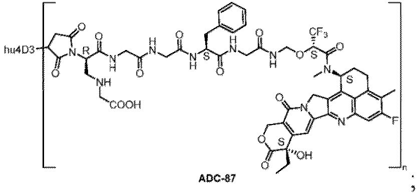

### Example 181

ADC-88 was synthesized according to the general coupling method of Example 92,

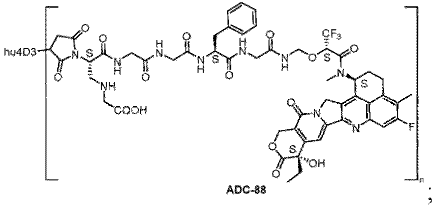

### Example 182

ADC-89 was synthesized according to the general coupling method of Example 92,

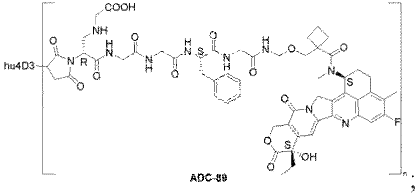

### Example 183

ADC-90 was synthesized according to the general coupling method of Example 92,

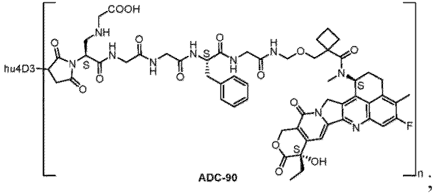

### Example 184

ADC-91 was synthesized according to the general coupling method of Example 92,

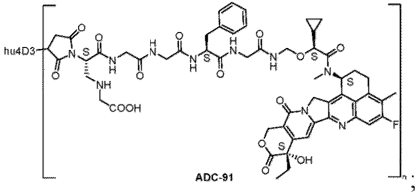

### Example 185

ADC-92 was synthesized according to the general coupling method of Example 92,

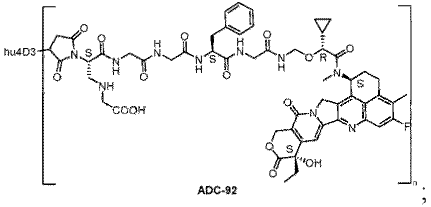

### Example 186

ADC-93 was synthesized according to the general coupling method of Example 92,

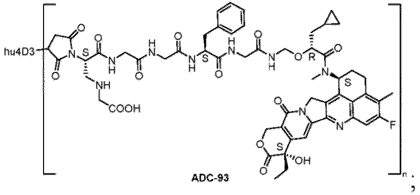

### Example 187

ADC-94 was synthesized according to the general coupling method of Example 92,

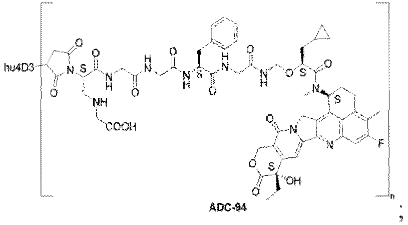

### Example 188

ADC-95 was synthesized according to the general coupling method of Example 92,

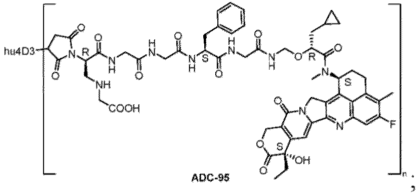

### Example 189

ADC-96 was synthesized according to the general coupling method of Example 92,

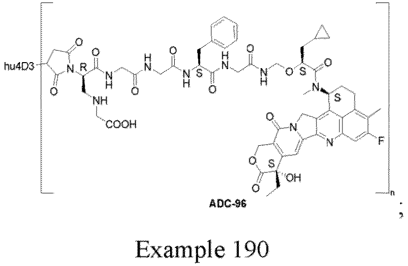

ADC-97 was synthesized according to the general coupling method of Example 92,

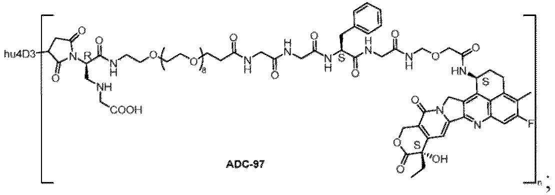

### Example 191

ADC-98 was synthesized according to the general coupling method of Example 92,

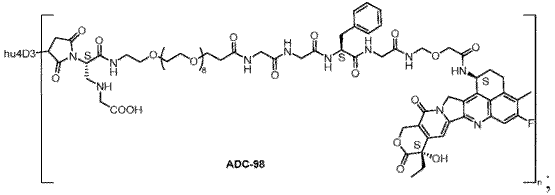

### Example 192

ADC-99 was synthesized according to the general coupling method of Example 92,

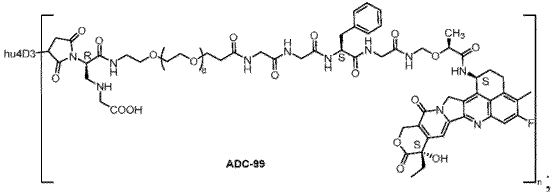

### Example 193

ADC-100 was synthesized according to the general coupling method of Example 92,

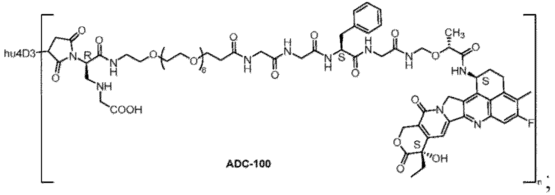

### Example 194

ADC-101 was synthesized according to the general coupling method of Example 92,

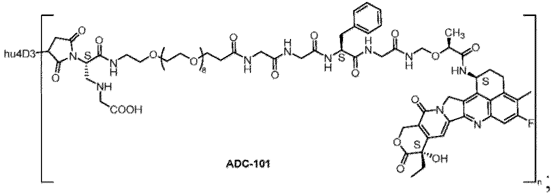

### Example 195

ADC-102 was synthesized according to the general coupling method of Example 92,

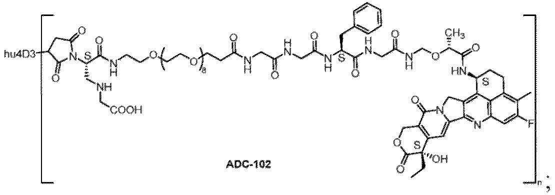

### Example 196

ADC-103 was synthesized according to the general coupling method of Example 92,

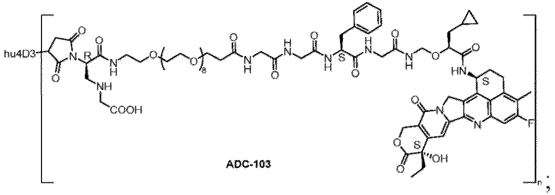

### Example 197

ADC-104 was synthesized according to the general coupling method of Example 92,

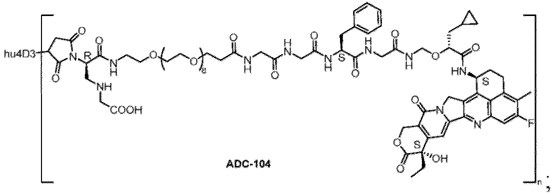

### Example 198

ADC-105 was synthesized according to the general coupling method of Example 92,

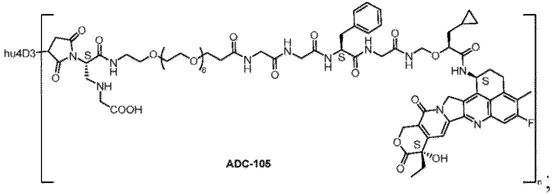

### Example 199

ADC-106 was synthesized according to the general coupling method of Example 92,

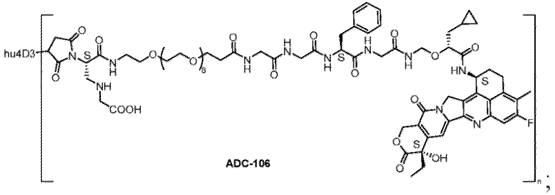

### Example 200

ADC-DS was synthesized from Compound 45 according to the general coupling method of Example 92,

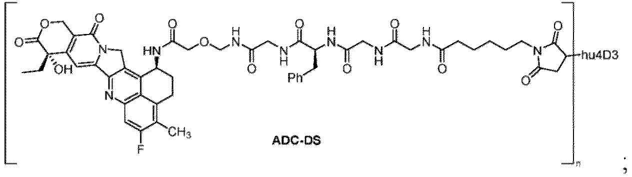

### Example 201

ADC-107 was synthesized according to the general coupling method of Example 92,

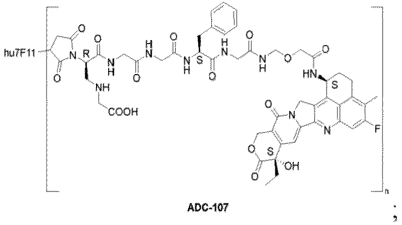

### Example 202

ADC-108 was synthesized according to the general coupling method of Example 92,

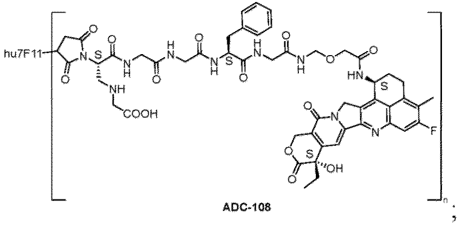

### Example 203

ADC-109 was synthesized according to the general coupling method of Example 92,

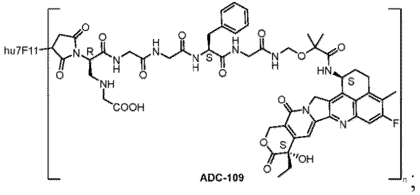

### Example 204

ADC-110 was synthesized according to the general coupling method of Example 92,

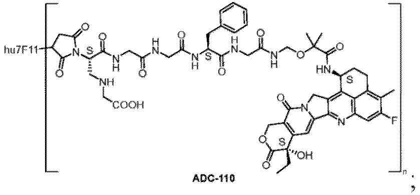

### Example 205

ADC-111 was synthesized according to the general coupling method of Example 92,

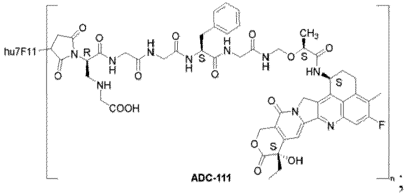

### Example 206

ADC-112 was synthesized according to the general coupling method of Example 92,

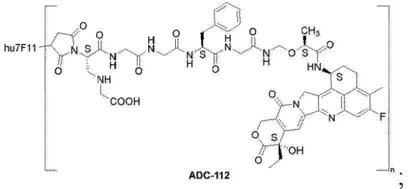

### Example 207

ADC-113 was synthesized according to the general coupling method of Example 92,

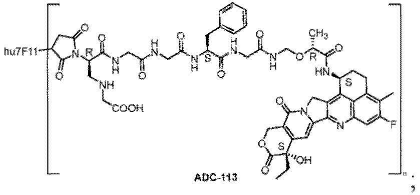

### Example 208

ADC-114 was synthesized according to the general coupling method of Example 92,

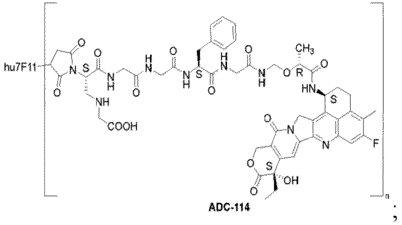

### Example 209

ADC-115 was synthesized according to the general coupling method of Example 92,

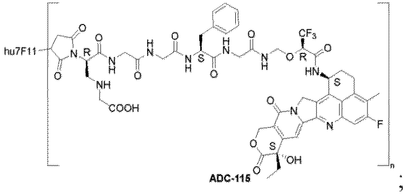

### Example 210

ADC-116 was synthesized according to the general coupling method of Example 92,

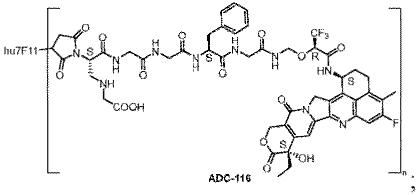

### Example 211

ADC-117 was synthesized according to the general coupling method of Example 92,

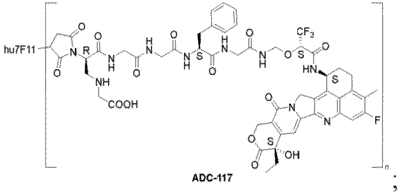

### Example 212

ADC-118 was synthesized according to the general coupling method of Example 92,

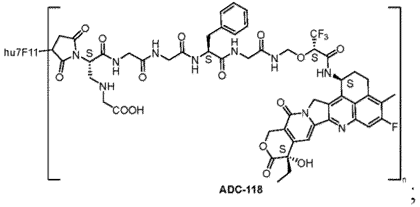

### Example 213

ADC-119 was synthesized according to the general coupling method of Example 92,

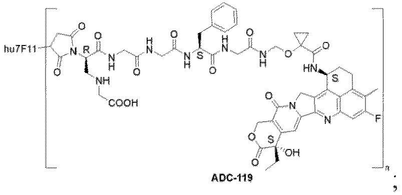

### Example 214

ADC-120 was synthesized according to the general coupling method of Example 92,

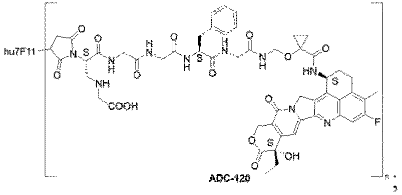

### Example 215

ADC-121 was synthesized according to the general coupling method of Example 92,

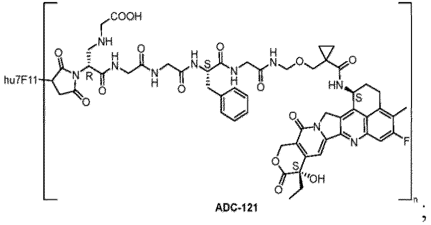

### Example 216

ADC-122 was synthesized according to the general coupling method of Example 92,

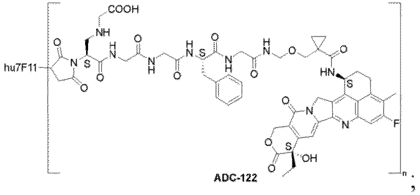

### Example 217

ADC-123 was synthesized according to the general coupling method of Example 92,

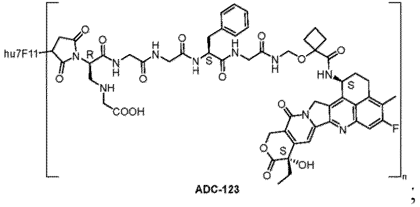

### Example 218

ADC-124 was synthesized according to the general coupling method of Example 92,

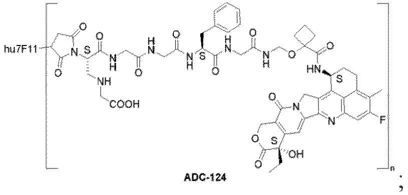

### Example 219

ADC-125 was synthesized according to the general coupling method of Example 92,

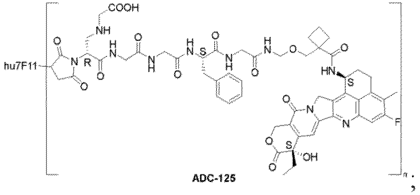

### Example 220

ADC-126 was synthesized according to the general coupling method of Example 92,

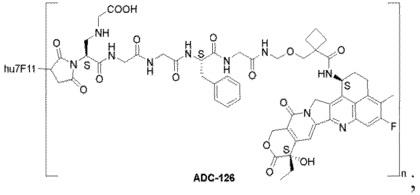

### Example 221

ADC-127 was synthesized according to the general coupling method of Example 92,

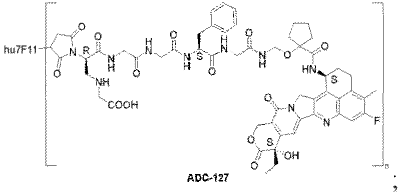

### Example 222

ADC-128 was synthesized according to the general coupling method of Example 92,

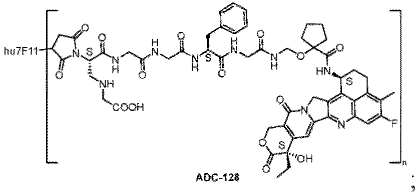

### Example 223

ADC-129 was synthesized according to the general coupling method of Example 92,

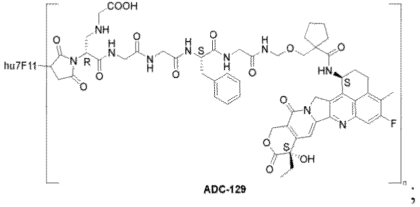

### Example 224

ADC-130 was synthesized according to the general coupling method of Example 92,

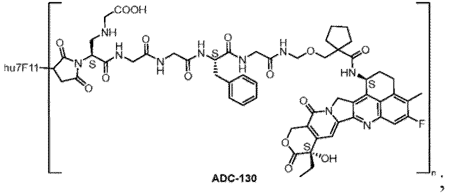

### Example 225

ADC-131 was synthesized according to the general coupling method of Example 92,

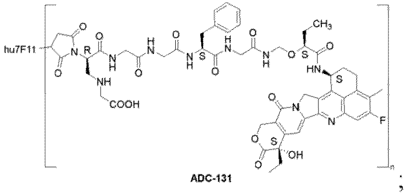

### Example 226

ADC-132 was synthesized according to the general coupling method of Example 92,

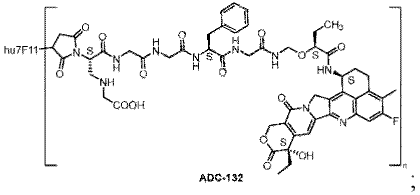

### Example 227

ADC-133 was synthesized according to the general coupling method of Example 92,

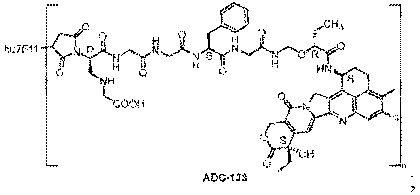

### Example 228

ADC-134 was synthesized according to the general coupling method of Example 92,

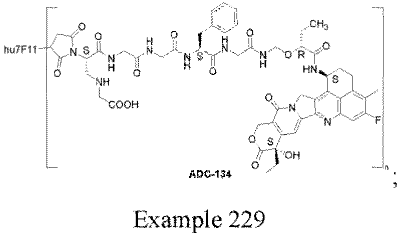

ADC-135 was synthesized according to the general coupling method of Example 92,

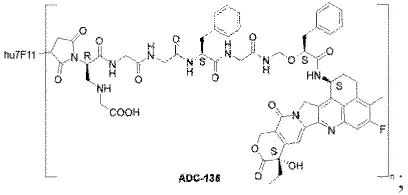

### Example 230

ADC-136 was synthesized according to the general coupling method of Example 92,

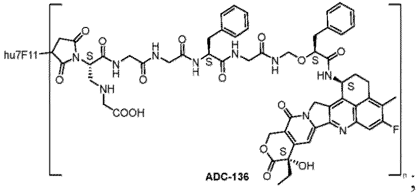

### Example 231

ADC-137 was synthesized according to the general coupling method of Example 92,

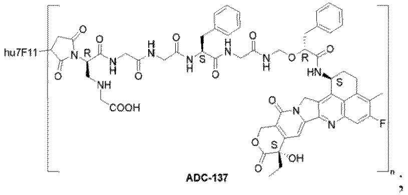

### Example 232

ADC-138 was synthesized according to the general coupling method of Example 92,

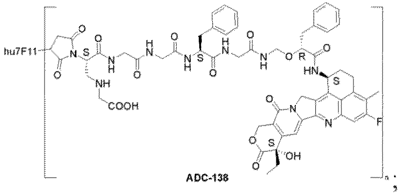

### Example 233

ADC-139 was synthesized according to the general coupling method of Example 92,

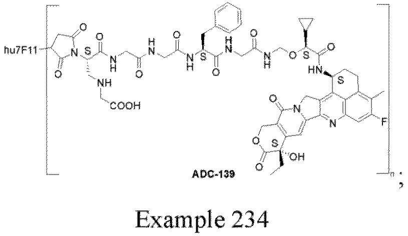

ADC-140 was synthesized according to the general coupling method of Example 92,

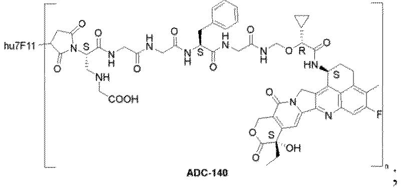

### Example 235

ADC-141 was synthesized according to the general coupling method of Example 92,

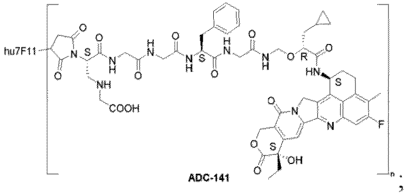

### Example 236

ADC-142 was synthesized according to the general coupling method of Example 92,

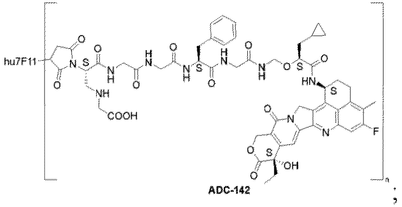

### Example 237

ADC-143 was synthesized according to the general coupling method of Example 92,

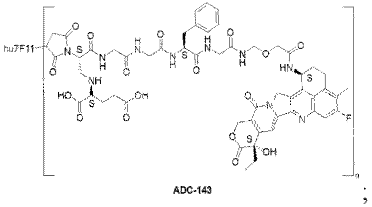

### Example 238

ADC-144 was synthesized according to the general coupling method of Example 92,

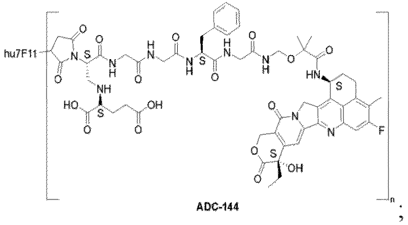

### Example 239

ADC-145 was synthesized according to the general coupling method of Example 92,

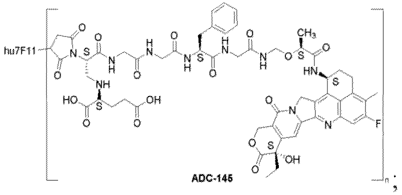

### Example 240

ADC-146 was synthesized according to the general coupling method of Example 92,

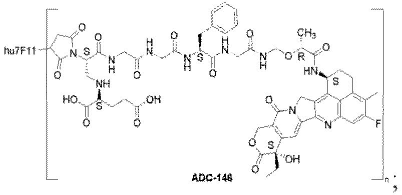

### Example 241

ADC-147 was synthesized according to the general coupling method of Example 92,

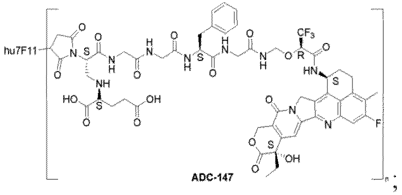

### Example 242

ADC-148 was synthesized according to the general coupling method of Example 92,

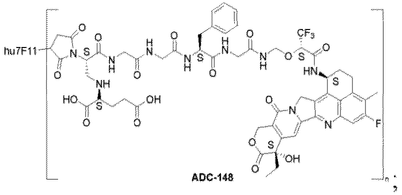

### Example 243

ADC-149 was synthesized according to the general coupling method of Example 92,

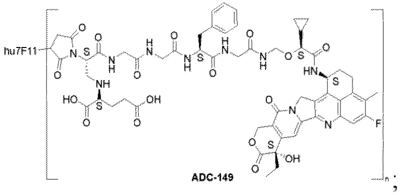

### Example 244

ADC-150 was synthesized according to the general coupling method of Example 92,

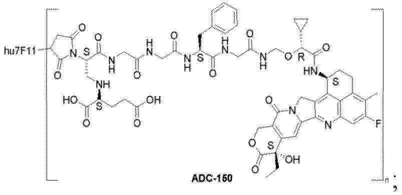

### Example 245

ADC-151 was synthesized according to the general coupling method of Example 92,

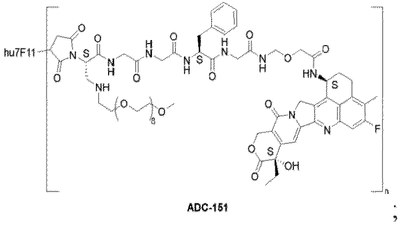

### Example 246

ADC-152 was synthesized according to the general coupling method of Example 92,

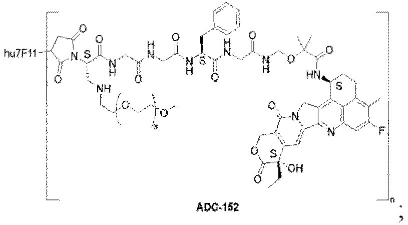

### Example 247

ADC-153 was synthesized according to the general coupling method of Example 92,

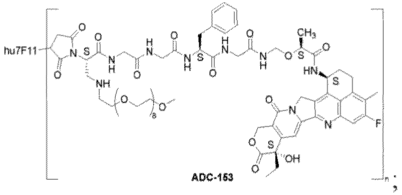

### Example 248

ADC-154 was synthesized according to the general coupling method of Example 92,

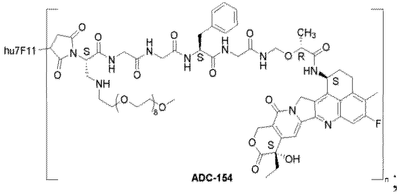

### Example 249

ADC-155 was synthesized according to the general coupling method of Example 92,

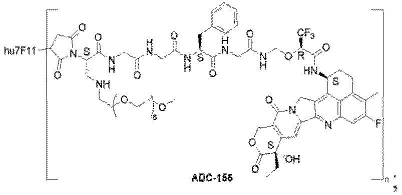

### Example 250

ADC-156 was synthesized according to the general coupling method of Example 92,

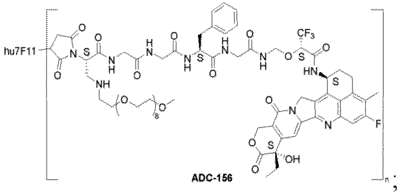

### Example 251

ADC-157 was synthesized according to the general coupling method of Example 92,

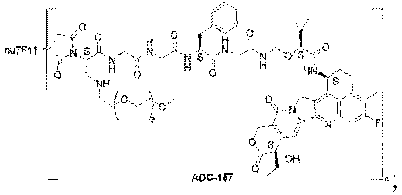

### Example 252

ADC-158 was synthesized according to the general coupling method of Example 92,

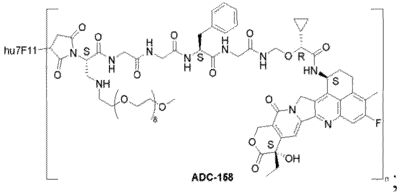

### Example 253

ADC-159 was synthesized according to the general coupling method of Example 92,

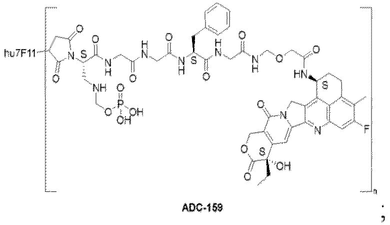

### Example 254

ADC-160 was synthesized according to the general coupling method of Example 92,

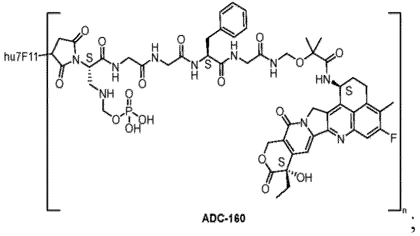

### Example 255

ADC-161 was synthesized according to the general coupling method of Example 92,

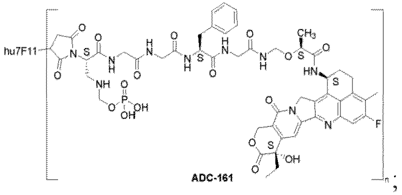

### Example 256

ADC-162 was synthesized according to the general coupling method of Example 92,

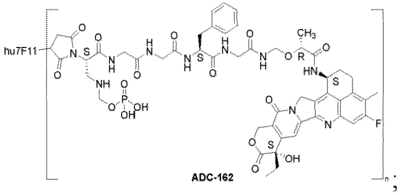

### Example 257

ADC-163 was synthesized according to the general coupling method of Example 92,

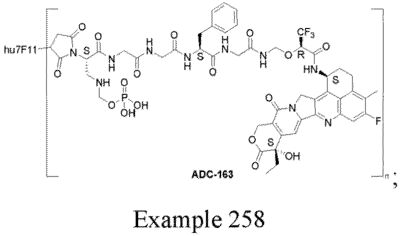

ADC-164 was synthesized according to the general coupling method of Example 92,

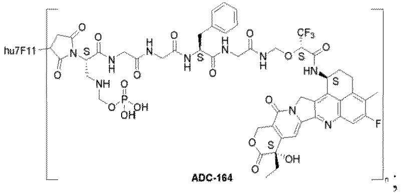

### Example 259

ADC-165 was synthesized according to the general coupling method of Example 92,

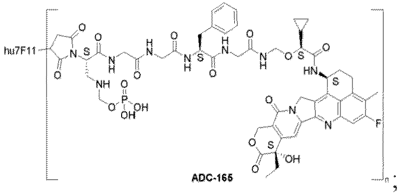

### Example 260

ADC-166 was synthesized according to the general coupling method of Example 92,

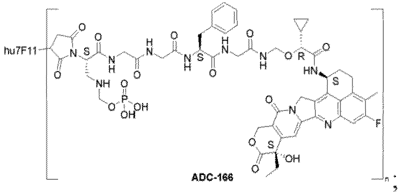

### Example 261

ADC-167 was synthesized according to the general coupling method of Example 92,

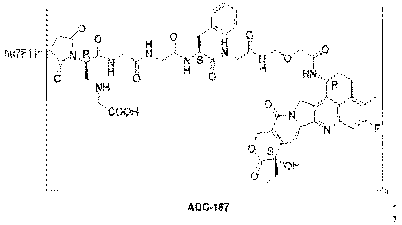

### Example 262

ADC-168 was synthesized according to the general coupling method of Example 92,

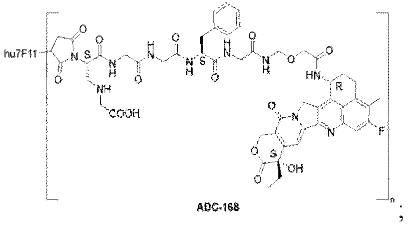

### Example 263

ADC-169 was synthesized according to the general coupling method of Example 92,

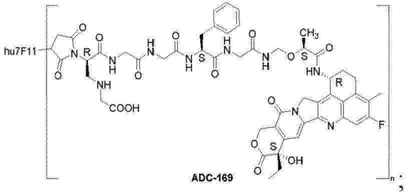

### Example 264

ADC-170 was synthesized according to the general coupling method of Example 92,

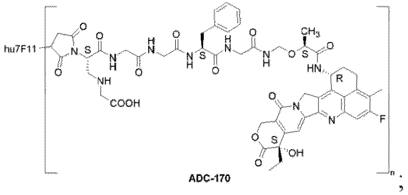

### Example 265

ADC-171 was synthesized according to the general coupling method of Example 92,

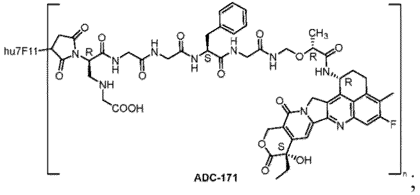

### Example 266

ADC-172 was synthesized according to the general coupling method of Example 92,

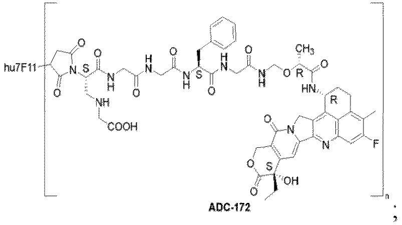

### Example 267

ADC-173 was synthesized according to the general coupling method of Example 92,

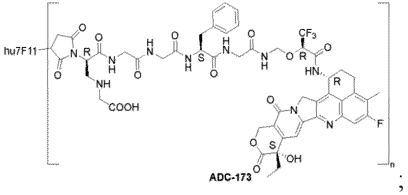

### Example 268

ADC-174 was synthesized according to the general coupling method of Example 92,

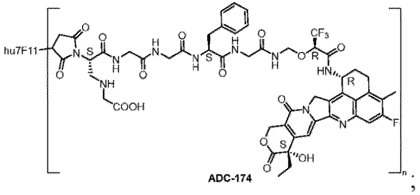

### Example 269

ADC-175 was synthesized according to the general coupling method of Example 92,

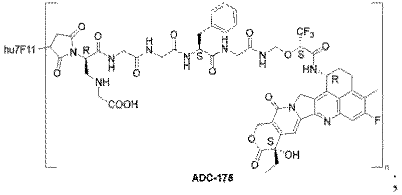

### Example 270

ADC-176 was synthesized according to the general coupling method of Example 92,

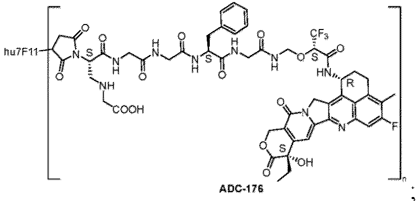

### Example 271

ADC-177 was synthesized according to the general coupling method of Example 92,

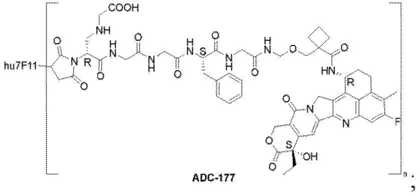

### Example 272

ADC-178 was synthesized according to the general coupling method of Example 92,

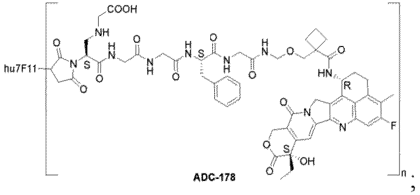

### Example 273

ADC-179 was synthesized according to the general coupling method of Example 92,

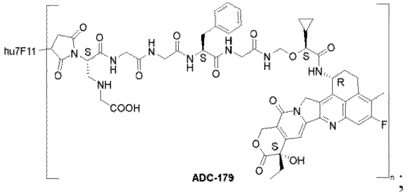

### Example 274

ADC-180 was synthesized according to the general coupling method of Example 92,

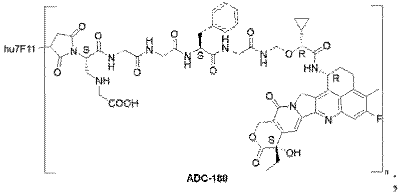

### Example 275

ADC-181 was synthesized according to the general coupling method of Example 92,

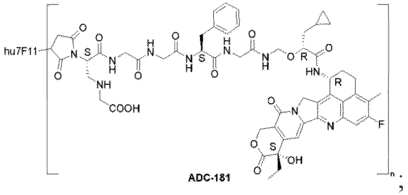

### Example 276

ADC-182 was synthesized according to the general coupling method of Example 92,

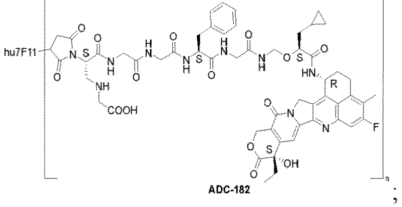

### Example 277

ADC-183 was synthesized according to the general coupling method of Example 92,

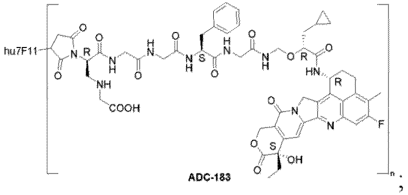

### Example 278

ADC-184 was synthesized according to the general coupling method of Example 92,

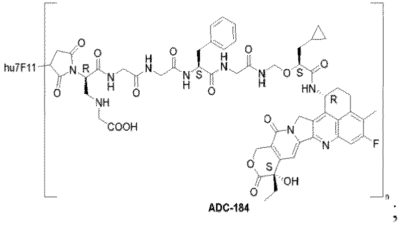

### Example 279

ADC-185 was synthesized according to the general coupling method of Example 92,

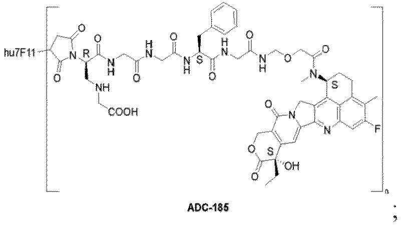

### Example 280

ADC-186 was synthesized according to the general coupling method of Example 92,

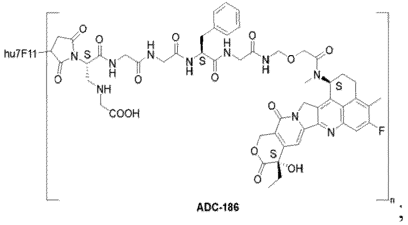

### Example 281

ADC-187 was synthesized according to the general coupling method of Example 92,

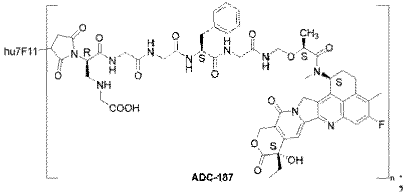

### Example 282

ADC-188 was synthesized according to the general coupling method of Example 92,

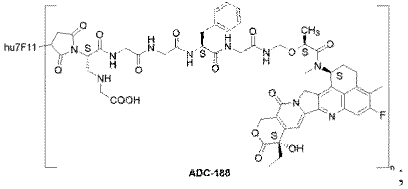

### Example 283

ADC-189 was synthesized according to the general coupling method of Example 92,

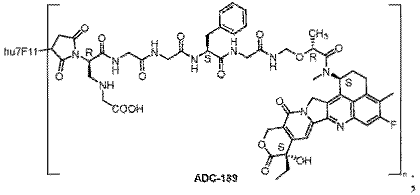

### Example 284

ADC-190 was synthesized according to the general coupling method of Example 92,

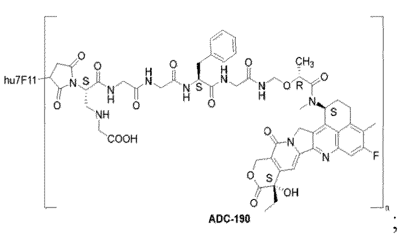

### Example 285

ADC-191 was synthesized according to the general coupling method of Example 92,

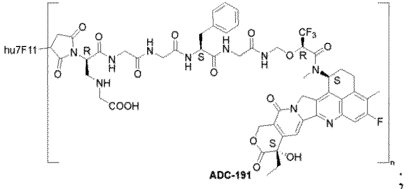

### Example 286

ADC-192 was synthesized according to the general coupling method of Example 92,

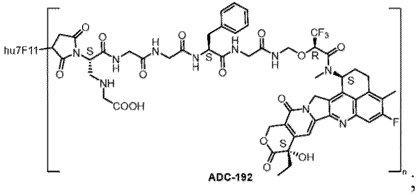

### Example 287

ADC-193 was synthesized according to the general coupling method of Example 92,

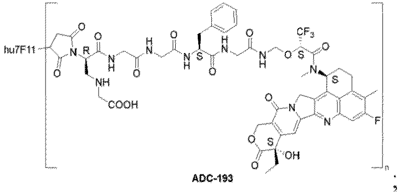

### Example 288

ADC-194 was synthesized according to the general coupling method of Example 92,

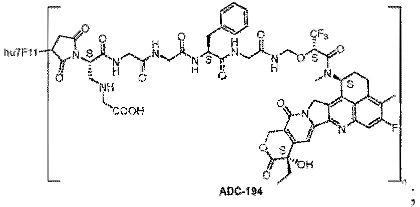

### Example 289

ADC-195 was synthesized according to the general coupling method of Example 92,

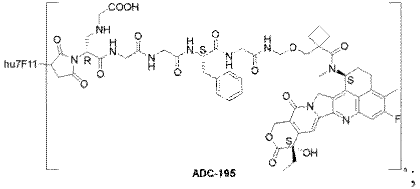

### Example 290

ADC-196 was synthesized according to the general coupling method of Example 92,

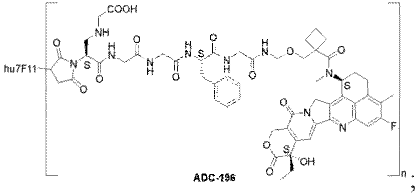

### Example 291

ADC-197 was synthesized according to the general coupling method of Example 92,

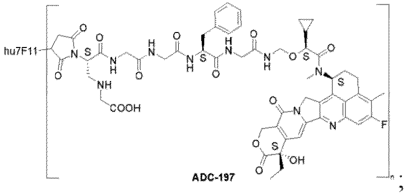

### Example 292

ADC-198 was synthesized according to the general coupling method of Example 92,

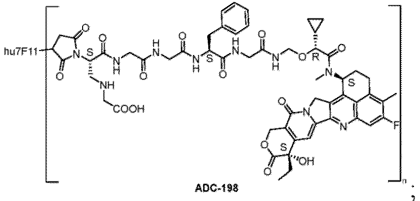

### Example 293

ADC-199 was synthesized according to the general coupling method of Example 92,

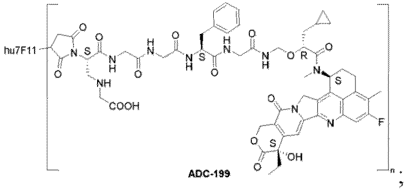

### Example 294

ADC-200 was synthesized according to the general coupling method of Example 92,

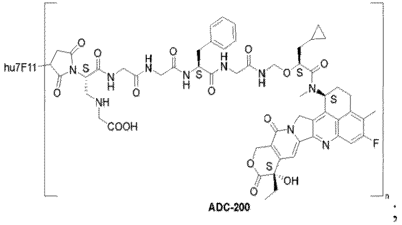

### Example 295

ADC-201 was synthesized according to the general coupling method of Example 92,

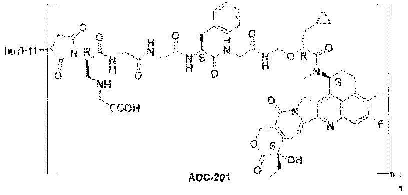

### Example 296

ADC-202 was synthesized according to the general coupling method of Example 92,

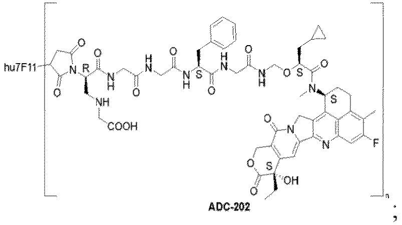

### Example 297

ADC-203 was synthesized according to the general coupling method of Example 92,

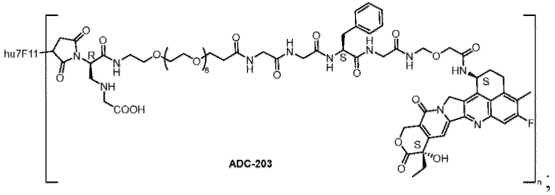

### Example 298

ADC-204 was synthesized according to the general coupling method of Example 92,

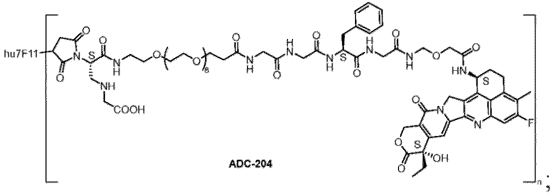

### Example 299

ADC-205 was synthesized according to the general coupling method of Example 92,

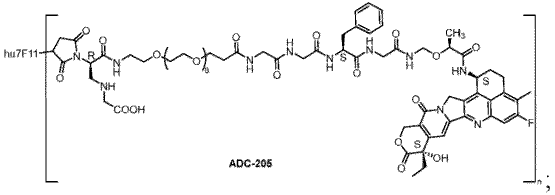

### Example 300

ADC-206 was synthesized according to the general coupling method of Example 92,

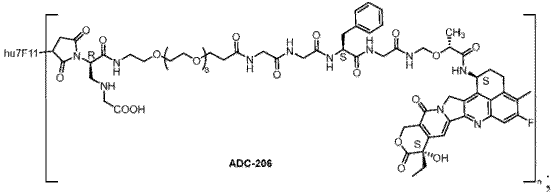

### Example 301

ADC-207 was synthesized according to the general coupling method of Example 92,

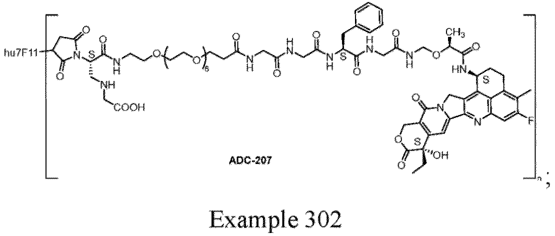

ADC-208 was synthesized according to the general coupling method of Example 92,

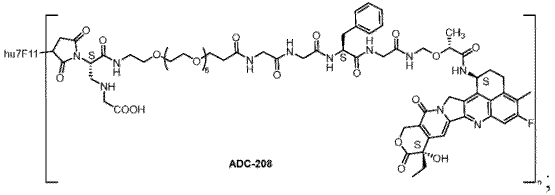

### Example 303

ADC-209 was synthesized according to the general coupling method of Example 92,

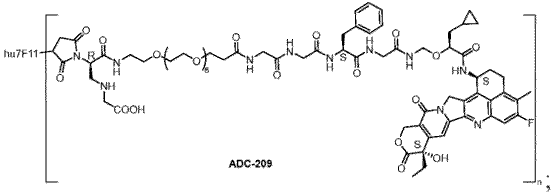

### Example 304

ADC-210 was synthesized according to the general coupling method of Example 92,

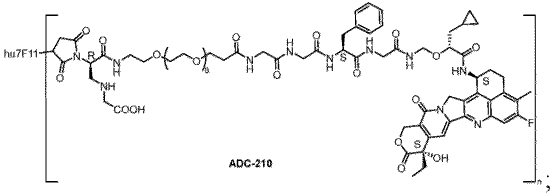

### Example 305

ADC-211 was synthesized according to the general coupling method of Example 92,

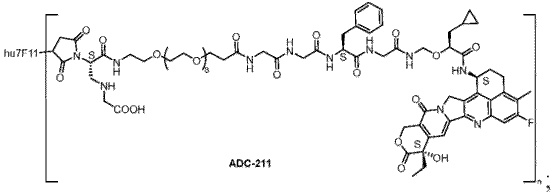

### Example 306

ADC-212 was synthesized according to the general coupling method of Example 92,

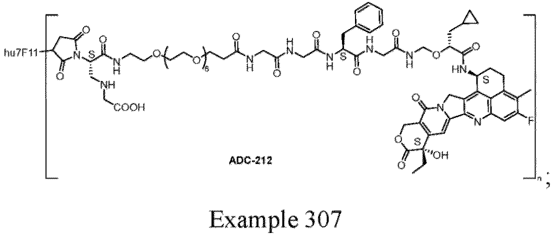

ADC-213 was synthesized from Compound 45 according to the general coupling method of Example 92,

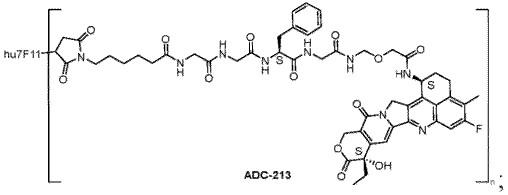

### Example 308

DS 1062a was synthesized from Compound 5A according to the general coupling method of Example 92, 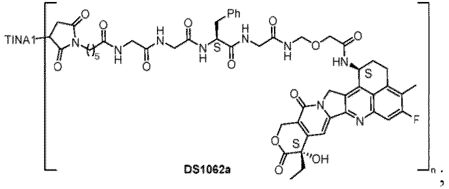 where the antibody TINA1 is an antibody targeting human Trop2, and n is 4 or 8.

### Example 309

Procedure 1 for determining the percentage of monomer by SEC-HPLC:
Chromatography column: Biocore SEC-300 5 µm, 4.6×300 mm;
Manufacturer: NanoChrom, Product code: B213-050030-04630S;
Mobile phase: 50 mM PB+300 mM NaCl+200 mM Arg+5% IPA, pH=6.5;

**Table 1: Process parameters**

| Parameter | Settings |
|---|---|
| Flow rate | 0.3 mL/min |
| Wavelength | 214 nm and 280 nm |
| Column temperature | 30 °C |
| Tray temperature | Room temperature |
| Injection volume: | 20 ug |
| Max. pressure | 150 bar/15 MPa/2175 PSI |
| Gradient | Isometric |
| Operating time | 20 min |

Procedure 2 for determining the percentage of monomer by SEC-HPLC:
Chromatography column: ACQUITY UPLC Protein SEC 4.6x150mm 1.7µm
Manufacturer: waters Product code:186005225
Mobile phase: 50 mM PB+300 mM NaCl+200 mM Arg+5% IPA, pH=6.5

**Table 2: Process parameters**

| | |
|---|---|
| Flow rate | 0.8 mL/min |
| Wavelength | 214 nm and 280 nm |
| Column temperature | 30 °C |
| Gradient | Isometric |
| Operating time | 12 min |
| Confirmation | Process parameters were confirmed. |

**Table 3 Data on the percentage of monomer of ADCs**

| Molecule | Degradation % | Aggregate % | Monomer % |
|---|---|---|---|
| DS1062a (DAR=4) | 0.108 | 3.431 | 96.461 |
| ADC-1 | 0 | 2.956 | 97.044 |
| ADC-2 | 0 | 1.276 | 98.724 |
| ADC-6 | 0 | 1.236 | 98.764 |
| ADC-10 | 0 | 2.530 | 97.470 |

Conclusion: The ADCs disclosed herein have the characteristics of low degradation and low aggregation and have the excellent property of high percentage of monomer.

### Example 310

Determination of DAR by RP-HPLC:
Chromatography column: Proteomix RP-1000 4.6×100 mm 5 µm 1000A; Manufacturer: Sepax;

**Table 4: Process parameters**

| Parameter | Settings | | | |
|---|---|---|---|---|
| Mobile phase | A: 0.1%TFA in water; B: 0.1%TFA in acetonitrile | | | |
| Flow rate | 0.5 mL/min | | | |
| Wavelength | 214 nm and 280 nm | | | |
| Column temperature | 65 °C | | | |
| Tray temperature | Room temperature | | | |
| Injection volume | 25 ug | | | |
| Max. pressure | 100bar/10MPa/1450PSI | | | |

| | Time (min) | Flow rate (mL/min) | Mobile phase A (%) | Mobile phase B (%) |
|---|---|---|---|---|
| Gradient | 0.0 | 0.5 | 75 | 25 |
| | 3 | 0.5 | 75 | 25 |
| | 28 | 0.5 | 50 | 50 |
| | 30 | 0.5 | 5 | 95 |
| | 32 | 0.5 | 5 | 95 |
| | 33 | 0.5 | 75 | 25 |
| | 40 | 0.5 | 75 | 25 |

**Table 5: Details on DAR of ADCs**

| Sample | RP-DAR |
|---|---|
| DS1062a | 4.13 |
| ADC-1 | 7.62 |
| ADC-2 | 7.51 |
| ADC-12 | 7.54 |
| ADC-6 | 7.38 |
| ADC-10 | 7.62 |
| ADC-70 | 7.52 |
| ADC-72 | 7.39 |
| ADC-73 | 6.95 |
| ADC-176 | 7.54 |
| ADC-178 | 7.37 |
| ADC-179 | 6.75 |

Conclusion: The ADCs disclosed herein have the excellent property of high DAR, and can significantly increase the drug concentration at the target location under the same dosage of ADC.

### Example 311

### Data on plasma stability of ADCs

The mixture of an ADC and IgG-depleted plasma were formulated with the final concentration of ADC being 0.6 mg/mL. The plasma mixture was incubated in a water bath box in a 37 °C incubator. The incubation time was set to 0 days, 3 days, and 7 days. At the same time, the unincubated plasma control was set. The incubated sample was purified and extracted and then tested for the DAR to reflect the stability of the ADC in plasma.

**Table 6 Plasma stability of ADCs**

| Description | DAR | | | |
|---|---|---|---|---|
| | Uninc ubated | Incubated in plasma for 0 days | Incubated in plasma for 3 days | Incubated in plasma for 7 days |
| ADC-6 | 7.38 | 7.30 | 7.25 | 7.26 |
| DS-1062a (in house, DAR=4) | 4.13 | 4.21 | 3.75 | 3.30 |
| DS-1062a (in house, DAR=8) | 7.62 | 7.49 | 6.59 | 5.81 |

Conclusion: The ADCs disclosed herein have good plasma stability, and no major change in DAR occurs during incubation in plasma. However, the control DS-1062a has a significant decrease in DAR during incubation in plasma, indicating poor plasma stability.

### Example 312

The ADCs maintain the original affinity of the corresponding anti-Trop2 antibodies hu4D3 and hu7F11 to TROP2:
Comparison of relative affinity to TROP2 between hu4D3 and ADC-6/ADC10/ADC12 and between TINA1 and ADC-1/DS1062a was performed using double-antigen sandwich ELISA.

Specific procedures are as follows.

The recombinant TROP2-His*6 antigen was coated on the plate and then blocked with 1% bovine serum albumin; hu4D3 and the corresponding ADC and TINA1 and the corresponding ADC were diluted respectively, and then serially diluted 3 folds at a starting concentration of 2000 ng/mL respectively, for a total of 11 concentrations. After the sample was incubated on the coated elisa plate for a period of time, the secondary antibody labeled by goat anti-human Fc-HRP was added for incubation. Finally, TMB color development was performed and terminated by sulfuric acid solution, and the absorption value at 450 nm was detected on an elisa plate. The results are plotted on concentration at OD450 nm.

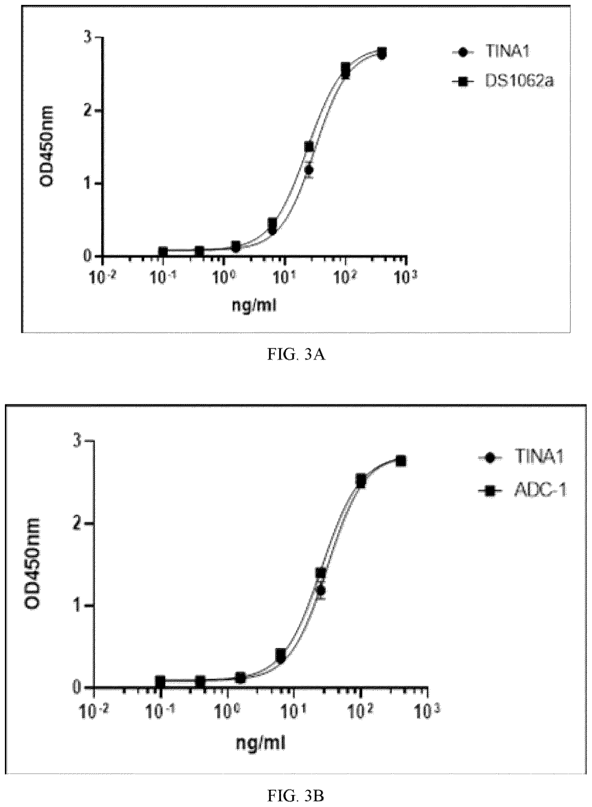

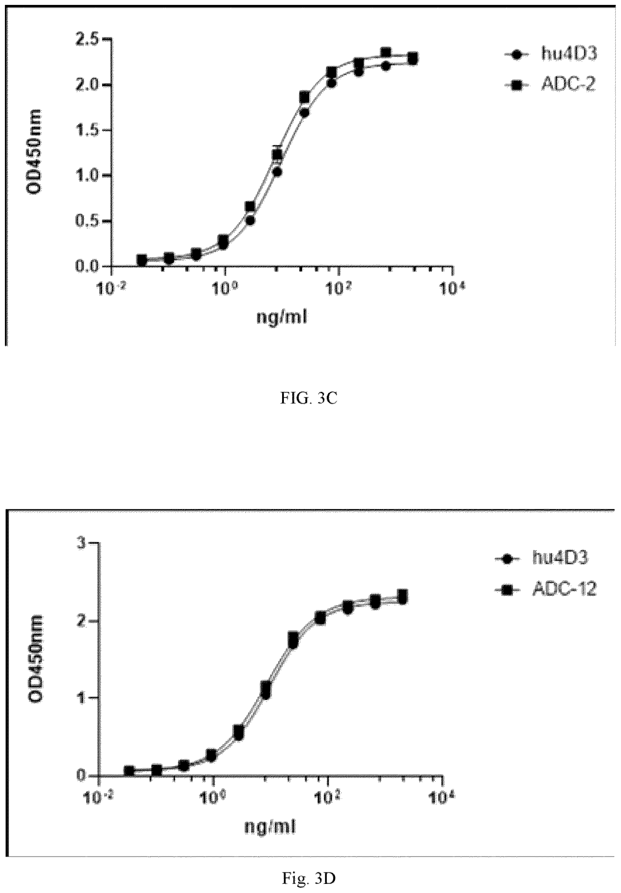

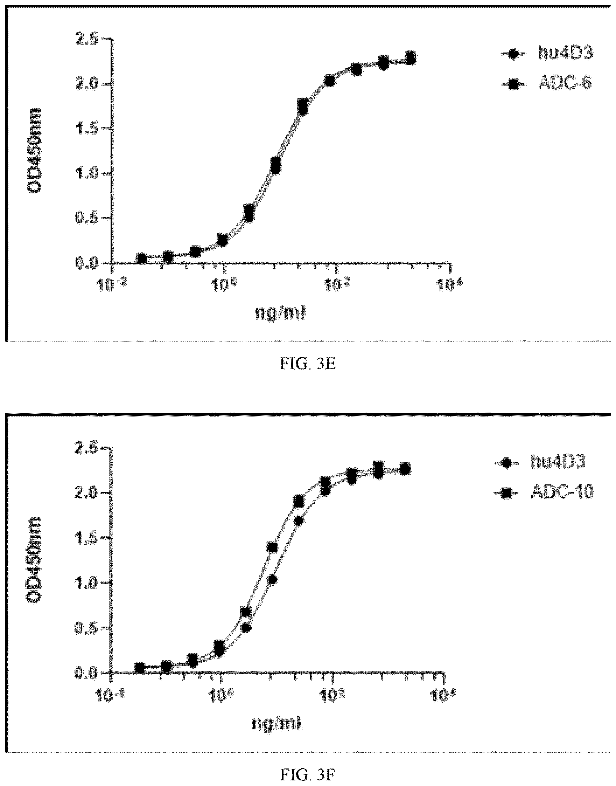

Conclusion: As shown in FIGS. 3A-3F, compared with the corresponding antibodies, the ADCs after conjugation maintain similar affinity and have no significant difference in EC50, indicating that conjugating hu4D3 to the toxin does not affect its affinity to the antigen.

### Example 313

### Test on in vitro drug efficacy:

In the present invention, a human tumor cell line (BxPC-3) was used as an experimental model to evaluate the in vitro efficacy of ADCs. A certain number of tumor cells were inoculated into a 96-well plate, and gradient dilutions of the test antibody and the corresponding ADC were added to the cells, the cells were treated for 5 days, cell viability was detected by using MTS, and IC50 was calculated to evaluate the in vitro inhibitory effect of the test antibody and ADCs on the tumor cell line. The cells were treated for 5 days with the antibody drugs having initial concentration of 500 nM, diluted 7 folds, with a total of 8 concentration points. Finally, the cell survival was calculated as: survival rate =(experimental group-blank group)/(control group-blank group)×100%. Then, by using Graph Pad Prism, the curve was fitted and the half inhibitory concentration (IC50) was calculated.

**Table 7: In vitro efficacy results of the naked antibody Hu4D3 and ADC-2, ADC-12, ADC-6, and ADC-10, as well as the naked antibody TINA1 and DS1062a in the human tumor cell line BxPC-3:**

| **Group** | **Description** | **IC50 (nM)** |
|---|---|---|
| BxPC-3 | hu4D3 | >500 |
| | TINA 1 | >500 |
| | ADC-2 | 84.87 |
| | ADC-12 | 2.13 |
| | ADC-6 | 3.31 |
| | ADC-10 | 35.92 |
| | DS1062a | 307.60 |

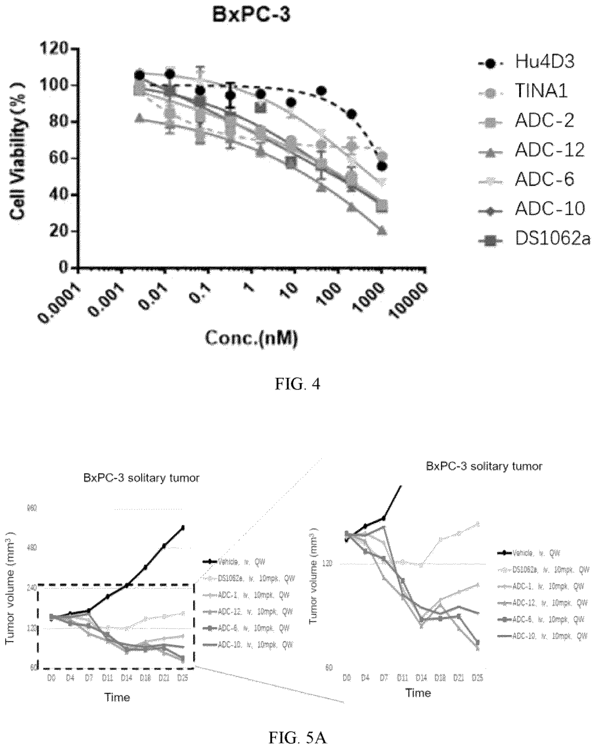

Conclusion: As shown in Table 7 and FIG. 4, in BxPC-3 cells, naked antibodies TINA1 and hu4D3 do not show obvious cytotoxicity against tumor cells; the in vitro cytotoxicity of ADC-2, ADC-6, ADC-10 and ADC-12 of the present invention was significantly greater than that of DS1062a.

### Example 314

### Test on in vivo drug efficacy:

Various human tumor cell lines (BXPC3, A431+SW620) were inoculated subcutaneously into BALB/c nude mice as experimental models to evaluate the in vivo efficacy of ADCs. In BALB/c nude mice inoculated with a certain number of tumor cell lines, when the tumor volume grew to 150-300 mm³, the antibodies or the corresponding ADCs were injected into the tail vein once a week, four times in total, and continuously observed. The tumors were measured twice a week to evaluate the inhibitory effects of test antibodies and ADCs on tumor cell lines.

### Conclusion:

In the efficacy experiment on tumor-bearing mice inoculated with the cell line BXPC3 having medium-high expression of TROP2, the in vivo efficacy experimental results of ADC-6, ADC-10 and ADC-12 were superior to those of DS1062a.

In the A431+SW620 heterogeneous tumor, the in vivo efficacy experimental results of ADC-6 and ADC-12 were superior to those of DS1062a, and ADC-6 and ADC-12 have a good "bystander effect".

### Example 315

In vivo drug efficacy of Linker-drug Compound **5A:**

### 1) Experimental materials:

Cells: The cells used for testing were from the Cell Bank of the Chinese Academy of Sciences;
Cell culture medium DMEM: Gibco;
FBS: BIOWEST.

### 2) Formulation of culture mediums:

Growth medium (with 10% FBS, Penicillin/streptomycin (100 U/mL);
Test medium (with 1% FBS, Penicillin/streptomycin (100 U/mL).

### 3) Operation:

The UV light of the biosafety cabinet was turned on 30 min in advance, and then the ventilation system was enabled for 3 min. The growth medium, the detection medium, D-PBS and trypsin were preheated in a 37 °C constant-temperature water bath, and then had surface disinfected with alcohol and then placed in the biosafety cabinet. The cells with a confluence rate of about 80% were placed in the biosafety cabinet, the old culture medium was removed, the cells were rinsed with D-PBS, and the D-PBS was then discarded. The cells were digested with trypsin for 2-3 min, then neutralized with the growth medium and centrifuged at 1200 rpm for 3 min, and the supernatant was removed. The cell solution was mixed with 4 mL of test medium, and 100 uL of cell solution was taken for counting (where 50 uL of cell solution was well mixed with 50 uL of Trypan Blue Stain and then counted). According to a pre-optimized cell plating density, the cells were plated in a 96-well plate at 80 ul/well. Only 80 uL of test medium was added to wells E11 and F11, and 150 uL of D-PBS was added to wells on the edges. After 24 hours of plating, diluted antibodies were added with 20 uL per well and control was set. Only 20 uL of test medium was added to cells in column 11. Two duplicate wells were set at each concentration. The cells were then well mixed on the cell vortex shaker at 550 rpm for 3 min.

Dilution of the solution: The test medium was used to formulate 300 uL of a test solution with a starting concentration of 5 uM in the first column of the V-shaped 96-well plate. 240 uL of test medium was added to the subsequent columns 2 to 10. 60 uL of the well mixed test solution was taken from the first column and added to the second column, and the cell solution was mixed up and down with a pipette for 10 times, and then the pipette tip was discarded. The same operation was performed for other seven concentrations one by one.

### 4) Test:

After 4 days, the MTS reagent was taken out, thawed at room temperature in the dark, and vortex mixed thoroughly. In the biosafety cabinet, 20 µL of CellTiter One Solution Reagen MTS reagent was added per 100 µL cell culture volume along the side wall of each well, and the plate surface was gently tapped to well mix the MTS solution. The cells were incubated for 2 h in the dark in an incubator. After the reaction, the 96-well plate was taken out for testing the OD490 nm absorption on an elisa plate, and the test data were recorded, organized, analyzed and stored.

### (5) Results:

Table 8 In vitro inhibitory effect of Compound 5A against Human non-small cell lung adenocarcinoma cell H1975, human non-small cell lung cancer cell HCC827, human epidermal cancer cell A431, human gastric cancer cell NCI-N87, human orthotopic pancreatic adenocarcinoma cell BXPC-3, human epidermal cancer cell A431 + human colon cancer cell SW620, human breast cancer cell ZR-75-1, human plasma cell leukemia cell H929, human multiple myeloma cell RPMI8226, human leukemia cell JJN-3, human breast cancer cell MDA-MB-361, and human breast cancer cell SK-BR-3

| **Group** | **IC50 (nM)** | **Efficacy %** |
|---|---|---|
| H1975 | 970.33 | 76.78 |
| HCC827 | 655.60 | 80.57 |
| A431 | 438.77 | 84.97 |
| NCI-N87 | 1023.92 | 63.93 |
| BxPC-3 | 320.09 | 64.4 |
| A431+SW620 | 604.87 | 68.26 |
| ZR-75-1 | 557.95 | 54.79 |
| H929 | 23.94 | 99.03 |
| RPMI8226 | 147.00 | 93.61 |
| JJN-3 | 36.11 | 80.59 |
| MDA-MB-361 | 572.13 | 62.34 |
| SK-BR-3 | 732.43 | 70.9 |

From Table 8, it shows that Compound **5A** (Linker-drug) has good inhibitory effects on the above solid tumor cells and hematological tumor cells.

### Example 316

In vitro efficacy experiments of naked antibodies and Compound **d₃, das, d₃₉** and **d44:**

### 1) Experimental materials:

Cells: N87, SW620, SW620+A431, Fadu, and HCC827 were sourced from the Cell Bank of the Chinese Academy of Sciences;
Cell culture medium DMEM: Gibco;
FBS: BIOWEST.

### 2) Formulation of culture mediums:

Growth medium (with 10% FBS, Penicillin/streptomycin (100 U/mL);
Test medium (with 1% FBS, Penicillin/streptomycin (100 U/mL).

### 3) Operation

In a 96-well plate, cells were plated at an appropriate cell density. After 24 h, the drugs were added. After 24 h, the drugs were diluted with the test medium (1 uM starting concentration, 5-fold dilution, 9 concentrations, test medium was added in the tenth column as blank control). The diluted drugs were added to the corresponding cell wells and then shaken for 3 min with a microplate shaker (model: MX100-4A) at a shaking speed of 550 rpm/min. After shaking, the cells were incubated in a carbon dioxide incubator for 3 days.

### 4) Test

After 4 days, the MTS reagent was taken out, thawed at room temperature in the dark, and vortex mixed thoroughly. In the biosafety cabinet, 20 µL of CellTiter One Solution Reagen MTS reagent per 100 µL cell culture volume was added along the side wall of each well, and the plate surface was gently tapped to well mix the MTS solution. The cells were incubated for 2 h in the dark in an incubator. After the reaction, the 96-well plate was taken out for testing the OD490 nm absorption on an elisa plate, and the test data were recorded, organized, analyzed and stored.

### 5) Results

Table 9 In vitro efficacy experiments of naked antibodies and Compound **d₃, das, d₃₉** and **d₄₄**

| IC₅₀ (nM) | | | | | |
|---|---|---|---|---|---|
| Group | N87 | SW620 | SW620+A431 | Fadu | HCC827 |
| hu4D3 | >500 | >500 | >500 | >500 | >500 |
| hu7F11 | >500 | >500 | >500 | >500 | >500 |
| d₃ | 1.613 | 0.543 | 2.809 | 0.531 | 0.778 |
| d₃₈ | 0.058 | <0.005 | 0.079 | <0.005 | 0.001 |
| d₃₉ | 0.466 | <0.005 | 0.468 | 0.110 | 0.092 |
| d₄₄ | 0.197 | 0.014 | 0.182 | 0.029 | 0.038 |

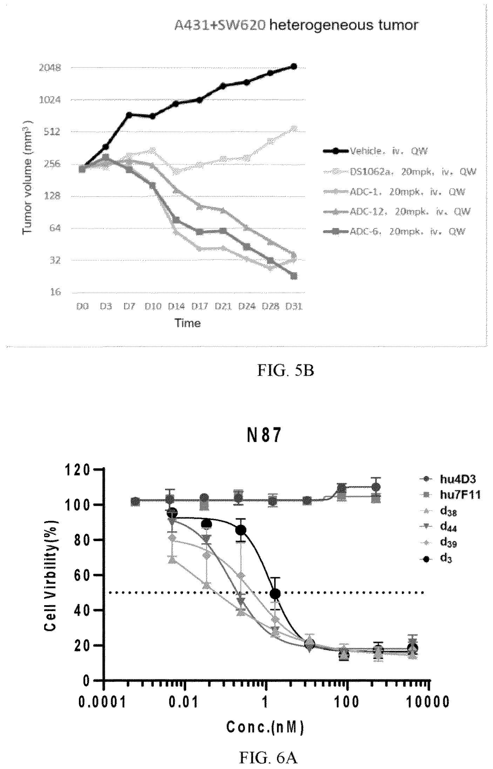

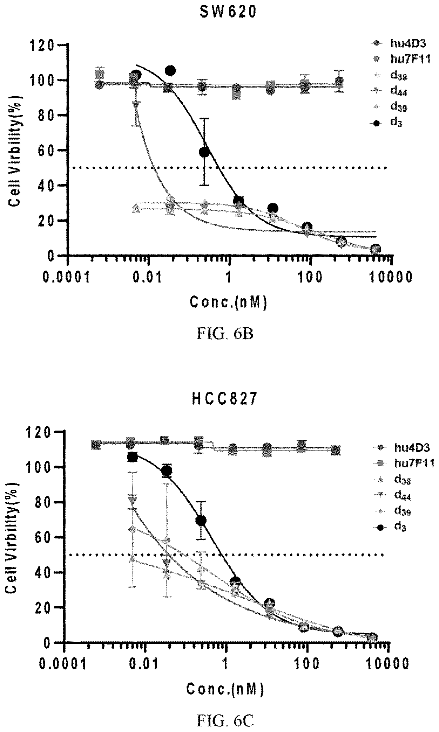

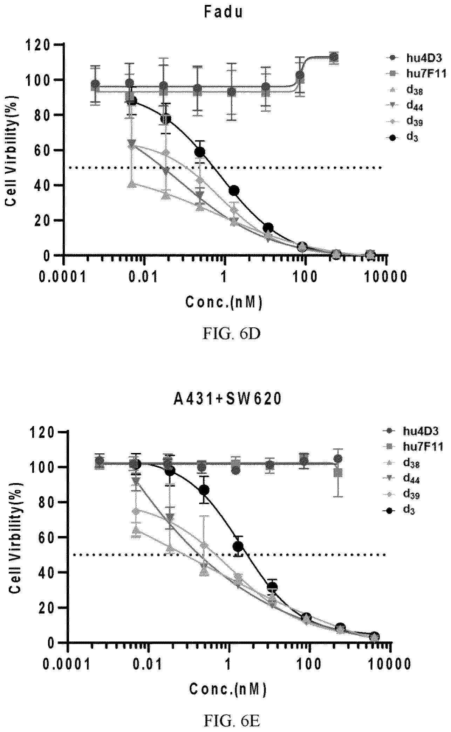

Conclusion: As shown in Table 9 and FIGS. 6A-6E, the small molecule drugs of the present invention exhibit excellent in vitro tumor inhibitory activity in experimental models of various human tumor cell lines (N87, SW620, Fadu, and HCC827) and SW620+A431 heterogeneous tumor.

### Example 317

In vitro drug efficacy test of ADCs:
The in vitro efficacy of ADCs was evaluated by using experimental models of various human tumor cell lines (N87, SW620, Fadu, and HCC827) and SW620+A431 heterogeneous tumor.

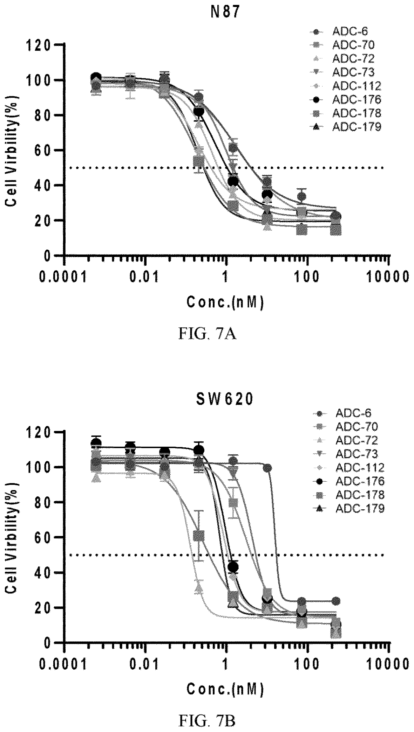

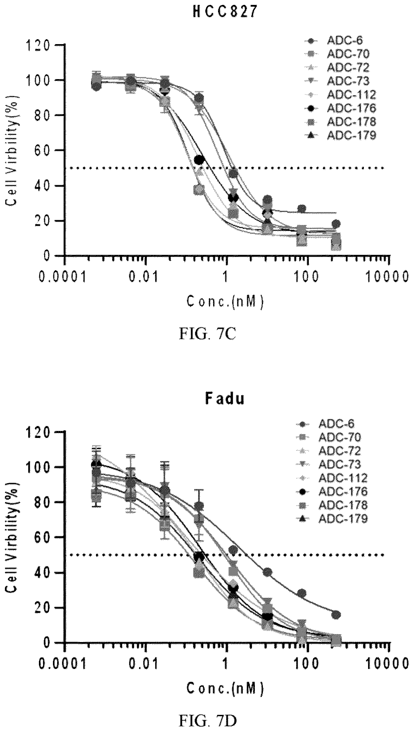

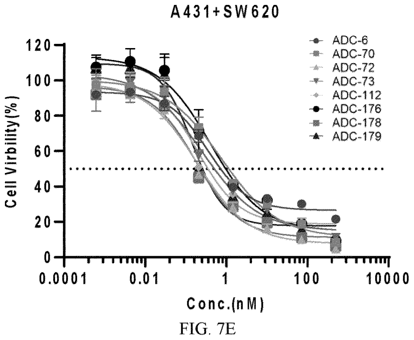

A certain number of tumor cells were inoculated into a 96-well plate, and gradient dilutions of the test antibody and the corresponding ADC were added to the cells, the cells were treated for 5 days, cell viability was detected by using Alamar Blue or MTS, and IC50 was calculated to evaluate the inhibitory effect of the test ADCs on the tumor cell line. The cells were treated for 5 days with the antibody drugs having initial concentration of 500 nM, diluted at 7 times, with a total of 8 concentration points. Finally, the cell survival was calculated as: survival rate =(experimental group-blank group)/(control group-blank group)×100%. Then, by using Graph Pad Prism, the curve was fitted and the half inhibitory concentration (IC50) was calculated. The results are shown in Table 10 and FIGS. 7A-7E.

**Table 10 In vitro drug efficacy test of ADCs:**

| IC₅₀ (nM) | | | | | |
|---|---|---|---|---|---|
| Group | N87 | SW620 | SW620+A431 | Fadu | HCC827 |
| ADC-6 | 4.053 | 17.093 | 0.719 | 3.160 | 1.334 |
| ADC-70 | 4.061 | 4.137 | 1.147 | 0.862 | 1.630 |
| ADC-72 | 1.478 | 5.311 | 0.506 | 0.994 | 0.860 |
| ADC-73 | 0.635 | 0.142 | 0.235 | 0.181 | 0.263 |
| ADC-112 | 0.417 | 1.073 | 0.353 | 0.244 | 0.151 |
| ADC-176 | 1.047 | 1.264 | 0.962 | 0.298 | 0.392 |
| ADC-178 | 0.276 | 0.795 | 0.259 | 0.176 | 0.147 |
| ADC-179 | 0.283 | 0.378 | 0.227 | 0.118 | 0.146 |

Conclusion The ADCs prepared by the present invention show excellent tumor inhibitory activity in the experimental models of various human tumor cell lines (N87, SW620, Fadu, and HCC827) and SW620+A431 heterogeneous tumor. Experimental data on SW620+A431 heterogeneous tumor show that the ADCs prepared by the present invention can well exert the bystander effect.

## Claims

1. A ligand-camptothecin derivative conjugate shown in general formula I, or a pharmaceutically acceptable salt or solvate thereof; 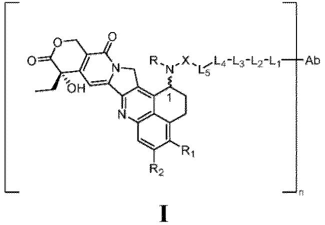 in the formula:
an Ab is an antibody targeting human Trop2 or an antigen-binding fragment thereof;
L₁ is selected, without limitation, from the group consisting of: 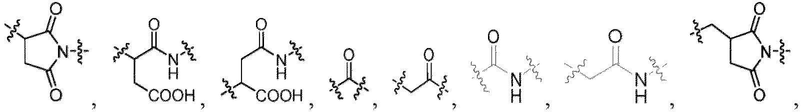 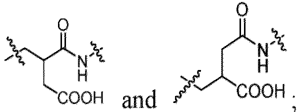
preferably, L₁ is 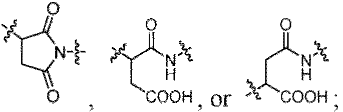
preferably, L₁ is 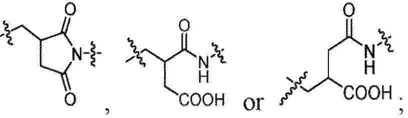
L₂ has a structure shown in formula A, 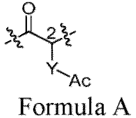
wherein Y is a scaffold, selected from the group consisting of C1-C6 alkyl, substituted C1-C6 alkyl and C3-C8 cycloalkyl; preferably Y is an C1-C6 alkyl; Ac is a hydrophilic structural unit; carbon No. 2 connected to the Y has an absolute chiral configuration R or S;
L₃ is present or absent, and when present, L₃ is selected from PEG hydrophilic units: 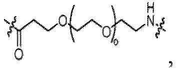 o is selected from integers between 1 and 10, preferably between 2 and 8;
L₄ is an enzyme-cleavable unit;
L₅ is a linking unit;
in formula I, chiral carbon atom No. 1 connected to N has an absolute chiral configuration R or S;
R is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, C6-C10 aryl, substituted C6-C10 aryl, 5- to 10-membered heteroaryl, and substituted 5- to 10-membered heteroaryl;
preferably, R is selected from the group of hydrogen atom and C1-C6 alkyl;
R₁ is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, carboxyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₁ is selected from the group of hydrogen atom or C1-C6 alkyl;
more preferably, R₁ is selected from C1-C6 alkyl;
R₂ is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, carboxyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₂ is selected from the group of hydrogen atom, halogen, and C1-C6 alkyl;
more preferably, R₂ is selected from halogen;
X is selected from the group consisting of -C(O)-CRₐR_{b}-(CR₃R₄)ₘ-O-, -C(O)-CRₐR_{b}-(CR₃R₄)ₘ-NH- and -C(O)-CRₐR_{b}-(CR₃R₄)ₘ-S-;
preferably, X is selected from -C(O)-CRₐR_{b}-(CR₃R₄)ₘ-O-;
Rₐ and R_{b} are each independently selected from the group consisting of hydrogen atom, deuterium atom, halogen, C1-C6 alkyl, deuterated C1-C6 alkyl, halogenated C1-C6 alkyl, C3-C8 cycloalkyl, C3-C8 cycloalkyl C1-C6 alkyl, C6-C10 aryl C1-C6 alkyl, C1-C6 alkoxy C1-C6 alkyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, Rₐ and R_{b} are each independently selected from the group consisting of hydrogen atom, C1-C6 alkyl, halogenated C1-C6 alkyl, C3-C8 cycloalkyl C1-C6 alkyl, and C6-C10 aryl C1-C6 alkyl;
alternatively, Rₐ, R_{b} and carbon atoms to which Rₐ and R_{b} are connected formC3-C8 cycloalkyl, C3-C8 cycloalkyl C1-C6 alkyl, 3- to 7-membered heterocyclyl, or substituted 3- to 7-membered heterocyclyl; preferably Rₐ, R_{b} and the carbon atoms to which Rₐ and R_{b} are connected form C3-C8 cycloalkyl;
R₃ and R₄ are the same or different, and are each independently hydrogen atom, deuterium atom, halogen, C1-C6 alkyl, halogenated C1-C6 alkyl, deuterated C1-C6 alkyl, C1-C6 alkoxy, hydroxyl, amino, cyano, nitro, hydroxyl C1-C6 alkyl, C3-C8 cycloalkyl, 3- to 7-membered heterocyclyl, or substituted 3- to 7-membered heterocyclyl;
preferably, R₃ and R₄ are each independently hydrogen atom or C1-C6 alkyl;
alternatively, R₃, R₄ and carbon atoms to which R₃ and R₄ are connected form C3-C8 cycloalkyl, C3-C8 cycloalkyl C1-C6 alkyl, 3- to 7-membered heterocyclyl, or substituted 3- to 7-membered heterocyclyl;
m is selected from integers between 0 and 4, and preferably is 0 or 1; n is selected from integers between 1 and 10.

2. The ligand-camptothecin derivative conjugate shown in general formula I according to claim 1, or a pharmaceutically acceptable salt or solvate thereof, wherein the Ab is an antibody targeting human Trop2 or an antigen-binding fragment thereof.

3. The ligand-camptothecin derivative conjugate shown in general formula I according to claim 1 or 2, or a pharmaceutically acceptable salt or solvate thereof, wherein the antibody of the Ab comprises: IgG1 heavy chains and κ light chains, the antibody comprising IgG1 heavy chains and κ light chains specifically recognize human Trop2 protein.

4. The ligand-camptothecin derivative conjugate shown in general formula I according to any one of claims 1-3, or a pharmaceutically acceptable salt or solvate thereof, wherein in the antibody of the Ab, the κ light chain comprises: CDRs as shown in SEQ ID NO: 7, SEQ ID NO: 8 and SEQ ID NO: 9, and the IgG1 heavy chain comprises CDRs as shown in SEQ ID NO: 1, SEQ ID NO: 2 and SEQ ID NO: 3.

5. The ligand-camptothecin derivative conjugate shown in general formula I according to any one of claims 1-4, or a pharmaceutically acceptable salt or solvate thereof, wherein the antibody of the Ab comprises a heavy chain and a light chain, the light chain comprises CDRL1, CDRL2 and CDRL3 having nucleic acid coding sequences as shown in SEQ ID NO: 27, SEQ ID NO: 28, and SEQ ID NO: 29, and the heavy chain comprises CDRH1, CDRH2 and CDRH3 having nucleic acid coding sequences as shown in SEQ ID NO: 21, SEQ ID NO: 22, and SEQ ID NO: 23, respectively.

6. The ligand-camptothecin derivative conjugate shown in general formula I according to any one of claims 1-5, or a pharmaceutically acceptable salt or solvate thereof, wherein the antibody of the Ab comprises a heavy chain and a light chain, the heavy chain comprises a VH having a sequence as shown in SEQ ID NO: 13 and the light chain comprises a VL having a sequence as shown in SEQ ID NO: 14.

7. The ligand-camptothecin derivative conjugate shown in general formula I according to any one of claims 1-6, or a pharmaceutically acceptable salt or solvate thereof, wherein the antibody of the Ab comprises a heavy chain and a light chain, the heavy chain comprises a VH having a nucleic acid coding sequence as shown in SEQ ID NO: 33, and the light chain comprises a VL having a nucleic acid coding sequence as shown in SEQ ID NO: 34.

8. The ligand-camptothecin derivative conjugate shown in general formula I according to any one of claims 1-7, or a pharmaceutically acceptable salt or solvate thereof, wherein in the antibody of the Ab, the heavy chain has an amino acid sequence as shown in SEQ ID NO: 17, and the light chain has an amino acid sequence as shown in SEQ ID NO: 18.

9. The ligand-camptothecin derivative conjugate shown in general formula I according to any one of claims 1-8, or a pharmaceutically acceptable salt or solvate thereof, wherein in the antibody of the Ab, the heavy chain has a nucleic acid coding sequence as shown in SEQ ID NO: 37, and the light chain has a nucleic acid coding sequence as shown in SEQ ID NO: 38.

10. The ligand-camptothecin derivative conjugate shown in general formula I according to any one of claims 1-3, or a pharmaceutically acceptable salt or solvate thereof, wherein in the antibody of the Ab, the κ light chain comprises CDRs as shown in SEQ ID NO: 10, SEQ ID NO: 11 and SEQ ID NO: 12, and the IgG1 heavy chain comprises CDRs as shown in SEQ ID NO: 4, SEQ ID NO: 5 and SEQ ID NO: 6.

11. The ligand-camptothecin derivative conjugate shown in general formula I according to any one of claims 1-3, or 10, or a pharmaceutically acceptable salt or solvate thereof, wherein in the antibody of the Ab, the κ light chain comprises CDRL1, CDRL2 and CDRL3 having nucleic acid coding sequences as shown in SEQ ID NO: 30, SEQ ID NO: 31, and SEQ ID NO: 32, and in the antibody of the Ab, the heavy chain comprises CDRH1, CDRH2 and CDRH3 having nucleic acid coding sequences as shown in SEQ ID NO: 24, SEQ ID NO: 25, and SEQ ID NO: 26, respectively.

12. The ligand-camptothecin derivative conjugate shown in general formula I according to any one of claims 1-3, 10-11, or a pharmaceutically acceptable salt or solvate thereof, wherein the antibody of the Ab comprises a heavy chain and a light chain, the heavy chain comprises a VH having a sequence as shown in SEQ ID NO: 15 and the light chain comprises a VL having a sequence as shown in SEQ ID NO: 16.

13. The ligand-camptothecin derivative conjugate shown in general formula I according to any one of claims 1-3, 10-12, or a pharmaceutically acceptable salt or solvate thereof, wherein the antibody of the Ab comprises a heavy chain and a light chain, the heavy chain comprises a VH having a nucleic acid coding sequence as shown in SEQ ID NO: 35, and the light chain comprises a VL having a nucleic acid coding sequence as shown in SEQ ID NO: 36.

14. The ligand-camptothecin derivative conjugate shown in general formula I according to any one of claims 1-3, 10-13, or a pharmaceutically acceptable salt or solvate thereof, wherein in the antibody of the Ab, the heavy chain has an amino acid sequence as shown in SEQ ID NO: 19, and the light chain has an amino acid sequence as shown in SEQ ID NO: 20.

15. The ligand-camptothecin derivative conjugate shown in general formula I according to any one of claims 1-3, 10-14, or a pharmaceutically acceptable salt or solvate thereof, wherein in the antibody of the Ab, the heavy chain has a nucleic acid coding sequence as shown in SEQ ID NO: 39, and the light chain has a nucleic acid coding sequence as shown in SEQ ID NO: 40.

16. The ligand-camptothecin derivative conjugate shown in general formula I according to any one of claims 1-15, or a pharmaceutically acceptable salt or solvate thereof, wherein the X is selected, without limitation, from the following structures or isomers thereof: 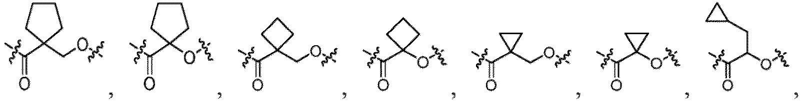 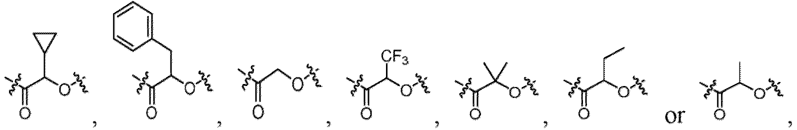 wherein a wavy line on the left is connected to a camptothecin derivative moiety, and a wavy line on the right is connected to L₅.

17. The ligand-camptothecin derivative conjugate shown in general formula I according to any one of claims 1-16, or a pharmaceutically acceptable salt or solvate thereof, wherein L₄ is selected, without limitation, from peptide residues comprising an amino acid,
wherein optionally, the amino acid is further substituted with one or more substituents selected from the group consisting of deuterium atom, halogen, hydroxyl, cyano, amino, nitro, carboxyl, C1-C6 alkyl, substituted C1-C6 alkyl, C1-C6 alkoxy, and C3-C8 cycloalkyl or substituted C3-C8 cycloalkyl.
preferably, the peptide residue is a peptide residue composed of one, two or more amino acids selected from the group consisting of phenylalanine (F), glycine (G), valine (V), lysine (K), citrulline (C), serine (S), glutamic acid (E) or aspartic acid (D);
more preferably, the peptide residue is a tetrapeptide residue consisting of glycine (G)-glycine (G)-phenylalanine (F)-glycine (G).

18. The ligand-camptothecin derivative conjugate shown in general formula I according to any one of claims 1-17, or a pharmaceutically acceptable salt or solvate thereof, wherein
L₅ is selected, without limitation, from -NR₅(CR₆R₇)_{q}- and a chemical bond, q is selected from integers between 0 and 6;
R₅, R₆ and R₇ are the same or different and each independently selected from the group consisting of hydrogen atom, deuterium atom, halogen, C1-C6 alkyl, substituted C1-C6 alkyl, deuterated C1-C6 alkyl, C3-C8 cycloalkyl, C3-C8 cycloalkyl C1-C6 alkyl, C1-C6 alkoxy C1-C6 alkyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₅, R₆ and R₇ are each independently selected from hydrogen atom and C1-C6 alkyl;
more preferably, R₅, R₆ and R₇ are each independently selected from hydrogen atom.

19. The ligand-camptothecin derivative conjugate shown in general formula I according to any one of claims 1-18, or a pharmaceutically acceptable salt or solvate thereof, wherein the linking unit -L₁-L₂-L₃-L₄-L₅- is selected, without limitation, from the following structures: 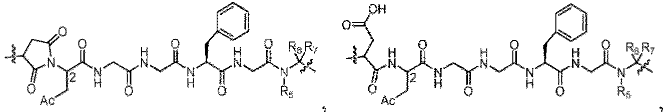 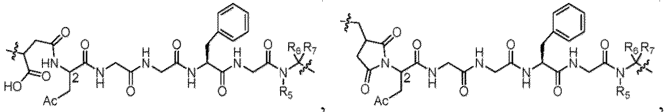 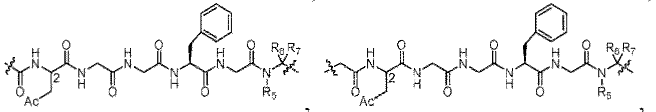 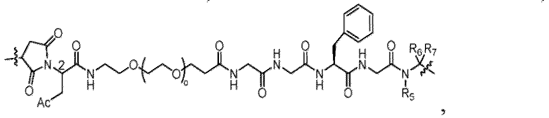 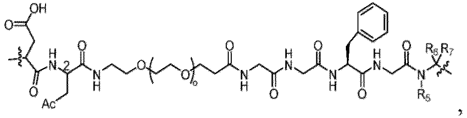 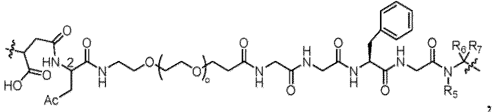 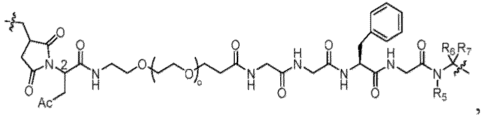 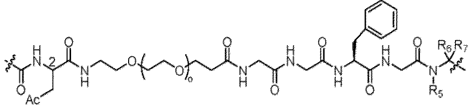 and 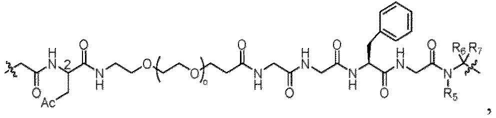 preferably, 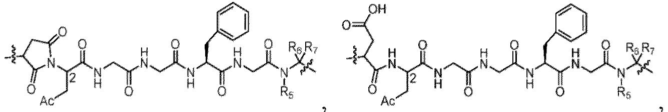 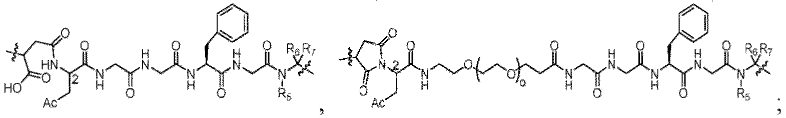 wherein
Ac is a hydrophilic structural unit;
R₅, R₆ and R₇ are the same or different and each independently selected from the group consisting of hydrogen atom, deuterium atom, halogen, C1-C6 alkyl, substituted C1-C6 alkyl, deuterated C1-C6 alkyl, C3-C8 cycloalkyl, C3-C8 cycloalkyl C1-C6 alkyl, C1-C6 alkoxy C1-C6 alkyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₅, R₆ and R₇ are each independently selected from hydrogen atom or C1-C6 alkyl;
more preferably, R₅, R₆ and R₇ are each independently selected from hydrogen atom;
carbon atom No. 2 connected to N has an absolute chiral configuration R or S;
a wavy line on the left is connected to an antibody or an antigen-binding fragment of the antibody, and a wavy line on the right is connected to X;
o is selected from integers between 1 and 10.

20. A ligand-camptothecin derivative conjugate shown in general formula II or a pharmaceutically acceptable salt or solvate thereof; 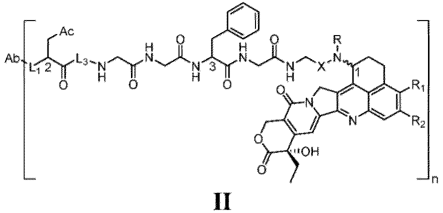 wherein
an Ab is an antibody targeting human Trop2 or an antigen-binding fragment thereof;
L₁ is a linking unit connected to the Ab and is selected, without limitation, from: 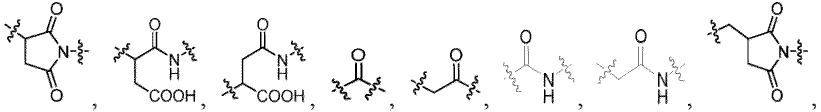 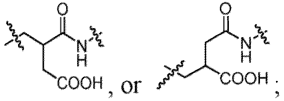
L₁ is preferably 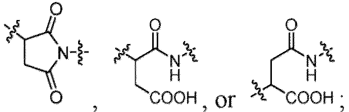
preferably L₁ is 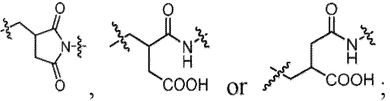
L₃ is present or absent, and when present, L₃ is selected from 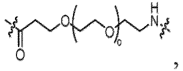 and o is selected from integers between 1 and 10, preferably between 2 and 8;
the Ac is a hydrophilic structural unit;
chiral carbon atoms at positions 1, 2 and 3 have an absolute chiral configuration R or S;
R is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, C6-C10 aryl, substituted C6-C10 aryl, 5- to 10-membered heteroaryl, and substituted 5- to 10-membered heteroaryl;
preferably, R is selected from the group of hydrogen atom and C1-C6 alkyl;
R₁ is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, carboxyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₁ is selected from the group of hydrogen atom and C1-C6 alkyl;
more preferably, R₁ is selected from C1-C6 alkyl;
R₂ is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, carboxyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₂ is selected from the group of hydrogen atom, halogen, and C1-C6 alkyl;
more preferably, R₂ is selected from halogen;
X is selected from the group consisting of -C(O)-CRₐR_{b}-(CR₃R₄)ₘ-O-, -C(O)-CRₐR_{b}-(CR₃R₄)ₘ-NH- or -C(O)-CRₐR_{b}-(CR₃R₄)ₘ-S-;
preferably, X is selected from -C(O)-CRₐR_{b}-(CR₃R₄)ₘ-O-;
Rₐ and R_{b} are each independently selected from the group consisting of hydrogen atom, deuterium atom, halogen, C1-C6 alkyl, deuterated C1-C6 alkyl, halogenated C1-C6 alkyl, C3-C8 cycloalkyl, C3-C8 cycloalkyl C1-C6 alkyl, C1-C6 alkoxy C1-C6 alkyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, Rₐ and R_{b} are each independently selected from the group consisting of hydrogen atom, C1-C6 alkyl, halogenated C1-C6 alkyl, C3-C8 cycloalkyl C1-C6 alkyl, or C6-C10 aryl C1-C6 alkyl;
alternatively, Rₐ, R_{b} and carbon atoms to which Rₐ and R_{b} are connected form C3-C8 cycloalkyl, C3-C8 cycloalkyl C1-C6 alkyl, 3- to 7-membered heterocyclyl, or substituted 3- to 7-membered heterocyclyl; preferably Rₐ, R_{b} and the carbon atoms to which Rₐ and R_{b} are connected form C3-C8 cycloalkyl;
R₃ and R₄ are the same or different, and are each independently hydrogen atom, deuterium atom, halogen, C1-C6 alkyl, halogenated C1-C6 alkyl, deuterated C1-C6 alkyl, C1-C6 alkoxy, hydroxyl, amino, cyano, nitro, hydroxyl C1-C6 alkyl, C3-C8 cycloalkyl, 3- to 7-membered heterocyclyl, or substituted 3- to 7-membered heterocyclyl;
preferably, R₃ and R₄ are each independently hydrogen atom or C1-C6 alkyl;
alternatively, R₃, R₄ and carbon atoms to which R₃ and R₄ are connected form C3-C8 cycloalkyl, C3-C8 cycloalkyl C1-C6 alkyl, or 3- to 7-membered heterocyclyl, substituted 3- to 7-membered heterocyclyl;
m is selected from integers between 0 and 4 and preferably is 0 or 1;
n is selected from integers between 1 and 10.

21. The ligand-camptothecin derivative conjugate according to any one of claims 1-20, or a pharmaceutically acceptable salt or solvate thereof, wherein the Ac has a structure shown in formula B, 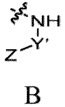 wherein
Z is selected, without limitation, from the group consisting of one or more of hydrophilic structural carboxyl, phosphoric acid, polyphosphoric acid, phosphorous acid, sulfonic acid, sulfinic acid and polyethylene glycol (PEG);
preferably, Z is selected from the group consisting of hydrophilic structure carboxyl, phosphoric acid and PEG;
Y' is an optional scaffold connecting -NH- and Z; preferably, Y' is a C1-C6 alkylene;
the Ac is connected to the labeled 2-position carbon in structural formula I through the scaffold Y.

22. The ligand-camptothecin derivative conjugate according to any one of claims 1-21, or a pharmaceutically acceptable salt or solvate thereof, wherein the Ac is selected, without limitation, from the group consisting of glycine, (*D*/*L*) alanine, (*D*/*L*) leucine, (*D*/*L*) isoleucine, (*D*/*L*) valine, (*D*/*L*) phenylalanine, (*D*/*L*) proline, (*D*/*L*) tryptophan, (*D*/*L*) serine, (*D*/*L*) tyrosine, (*D*/*L*) cysteine, (*D*/*L*) cystine, (*D*/*L*) arginine, (*D*/*L*) histidine, (*D*/*L*) methionine, (*D*/*L*) asparagine, (*D*/*L*) glutamine, (*D*/*L*) threonine, (*D*/*L*) aspartic acid, (*D*/*L*) glutamic acid, natural or unnatural amino acid derivatives or the following structures or isomers thereof, 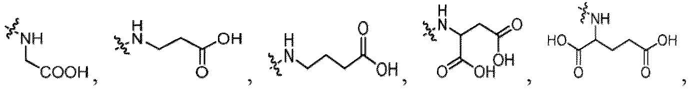 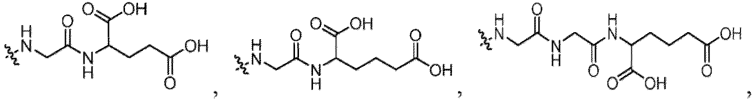 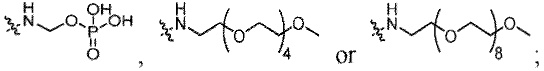 preferably

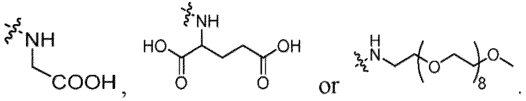

23. The ligand-camptothecin derivative conjugate according to any one of claims 1-22, or a pharmaceutically acceptable salt or solvate thereof, wherein the Ac is selected, without limitation, from the group consisting of glycine, phosphoric acid, (D/L) glutamic acid and polyethylene glycol hydrophilic structure.

24. The ligand-camptothecin derivative conjugate according to any one of claims 1-23, or a pharmaceutically acceptable salt or solvate thereof, wherein the 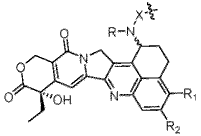 has a structure shown in formula d; 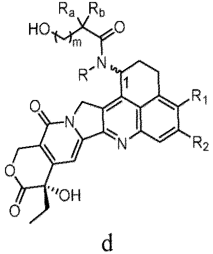 wherein
R is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, C6-C10 aryl, substituted C6-C10 aryl, 5- to 10-membered heteroaryl, and substituted 5- to 10-membered heteroaryl;
preferably, R is selected from the group of hydrogen atom and C1-C6 alkyl;
R₁ is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, carboxyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₁ is selected from the group of hydrogen atom and C1-C6 alkyl;
more preferably, R₁ is selected from C1-C6 alkyl;
R₂ is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, carboxyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, R₂ is selected from the group of hydrogen atom, halogen, and C1-C6 alkyl;
more preferably, R₂ is selected from halogen;
Rₐ and R_{b} are each independently selected from the group consisting of hydrogen atom, deuterium atom, halogen, C1-C6 alkyl, deuterated C1-C6 alkyl, halogenated C1-C6 alkyl, C3-C8 cycloalkyl, C3-C8 cycloalkyl C1-C6 alkyl, C1-C6 alkoxy C1-C6 alkyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
preferably, Rₐ and R_{b} are each independently selected from the group consisting of hydrogen atom, C1-C6 alkyl, halogenated C1-C6 alkyl, C3-C8 cycloalkyl C1-C6 alkyl, and C6-C10 aryl;
alternatively, Rₐ, R_{b} and carbon atoms to which Rₐ and R_{b} are connected formC3-C8 cycloalkyl, C3-C8 cycloalkyl C1-C6 alkyl, 3- to 7-membered heterocyclyl, or substituted 3- to 7-membered heterocyclyl; preferably Rₐ, R_{b} and the carbon atoms to which Rₐ and R_{b} are connected form C3-C8 cycloalkyl;
1-position chiral carbon atom has an absolute chiral configuration R or S;
m is 0 or 1.

25. The ligand-camptothecin derivative conjugate according to any one of claims 1-24, or a pharmaceutically acceptable salt or solvate thereof, wherein the structural formula d is selected, without limitation, from the following compounds:

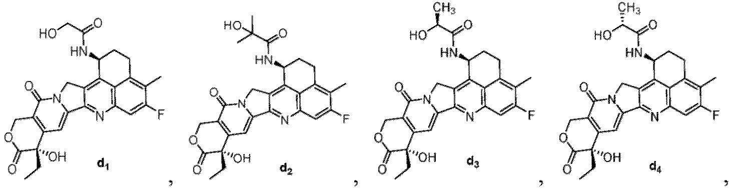

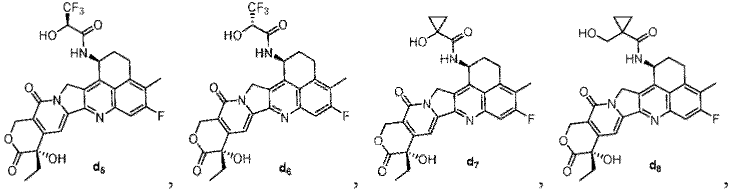

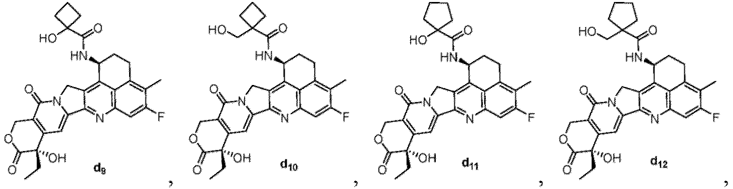

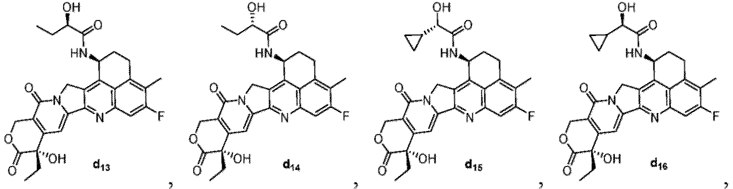

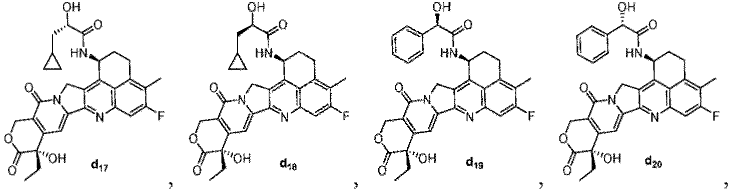

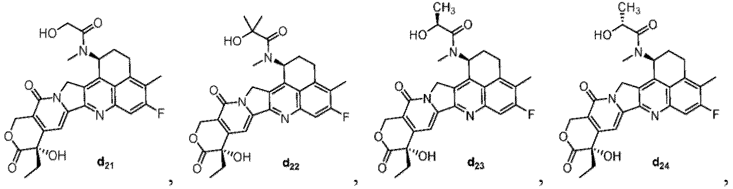

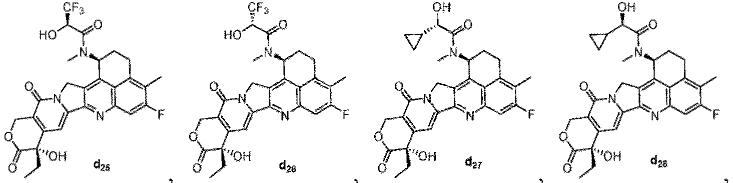

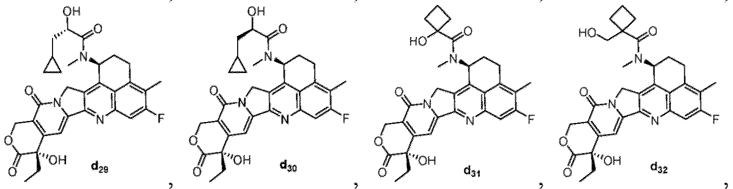

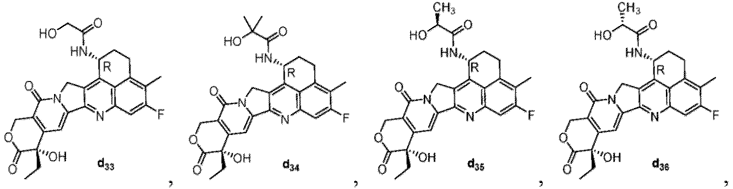

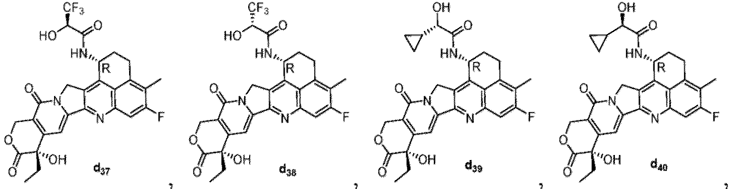

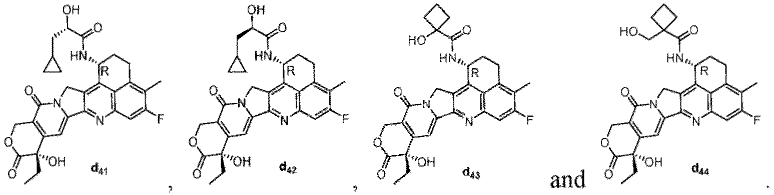

26. A linker-drug compound or a pharmaceutically acceptable salt or solvate thereof, having a structure shown in formula **III**, 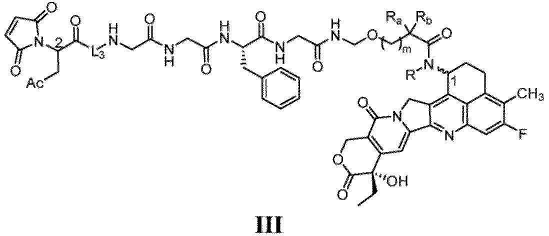 wherein
R is selected from the group consisting of hydrogen atom, deuterium atom, halogen, C1-C6 alkyl, substituted C1-C6 alkyl, deuterated C1-C6 alkyl, C3-C8 cycloalkyl C1-C6 alkyl, C1-C6 alkoxy C1-C6 alkyl, C6-C10 aryl, substituted C6-C10 aryl, 5- to 10-membered heteroaryl, and substituted 5- to 10-membered heteroaryl;
Rₐ is selected from the group consisting of hydrogen atom, deuterium atom, halogen, C1-C6 alkyl, deuterated C1-C6 alkyl, halogenated C1-C6 alkyl, C3-C8 cycloalkyl, C3-C8 cycloalkyl C1-C6 alkyl, C1-C6 alkoxy C1-C6 alkyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
R_{b} is selected from the group consisting of hydrogen atom, deuterium atom, halogen, C1-C6 alkyl, deuterated C1-C6 alkyl, halogenated C1-C6 alkyl, C3-C8 cycloalkyl, C3-C8 cycloalkyl C1-C6 alkyl, C1-C6 alkoxy C1-C6 alkyl, 3- to 7-membered heterocyclyl, substituted 3- to 7-membered heterocyclyl, C6-C10 aryl, substituted C6-C10 aryl, 5- to 10-membered heteroaryl, and substituted 5- to 10-membered heteroaryl;
alternatively, Rₐ, R_{b} and carbon atoms to which Rₐ and R_{b} are connected form C3-C8 cycloalkyl, C3-C8 cycloalkyl C1-C6 alkyl, 3- to 7-membered heterocyclyl, and substituted 3- to 7-membered heterocyclyl;
L₃ is present or absent, and when present, L₃ is selected from 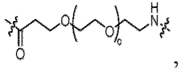 and o is selected from integers between 1 and 10;
1-position or 2-position chiral carbon atom has an absolute chiral configuration R or S;
the Ac is a hydrophilic structural unit;
m is 0 or 1;
preferably, the linker-drug compound or a pharmaceutically acceptable salt or solvate thereof is used for coupling with the ligand Ab to form the ligand-camptothecin derivative conjugate of Formula **I** or **II** according to any one of claims 1-25.

27. The linker-drug compound according to claim 26, or a pharmaceutically acceptable salt or solvate thereof, wherein the Ac has a structure shown in formula B, 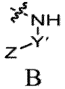 wherein
Z is composed of one or more selected from the group consisting of hydrophilic structural carboxyl, phosphoric acid, polyphosphoric acid, phosphorous acid, sulfonic acid, sulfinic acid, and polyethylene glycol (PEG);
Y' is an optional scaffold connecting -NH- and Z;
the Ac is connected to the labeled 2-position carbon in structural formula **I** through the scaffold Y.

28. The linker-drug compound according to claim 26 or 27, or a pharmaceutically acceptable salt or solvate thereof, wherein the Ac is selected, without limitation, from glycine, (D/L) alanine, (D/L) leucine, (D/L) isoleucine, (D/L) valine, (D/L) phenylalanine, (D/L) proline, (D/L) tryptophan, (D/L) serine, (D/L) tyrosine, (D/L) cysteine, (*D*/*L*) cystine, (D/L) arginine, (D/L) histidine, (D/L) methionine, (D/L) asparagine, (D/L) glutamine, (D/L) threonine, (D/L) aspartic acid, (D/L) glutamic acid, natural or unnatural amino acid derivatives or the following structures, 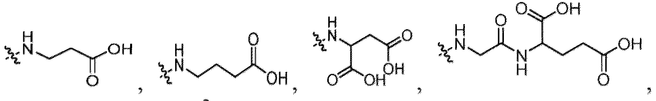 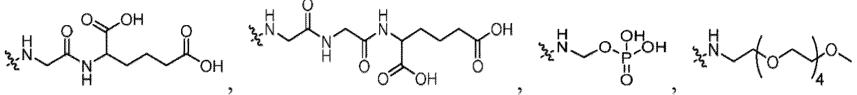 and

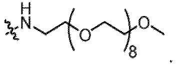

29. The linker-drug compound according to any one of claims 26-28, or a pharmaceutically acceptable salt or solvate thereof, wherein the Ac is selected, without limitation, from the group consisting of glycine, phosphoric acid, (D/L) glutamic acid and polyethylene glycol hydrophilic structure.

30. The linker-drug compound according to any one of claims 26-29, or a pharmaceutically acceptable salt or solvate thereof, wherein the linker-drug compound is selected, without limitation, from the following structures or isomers thereof, 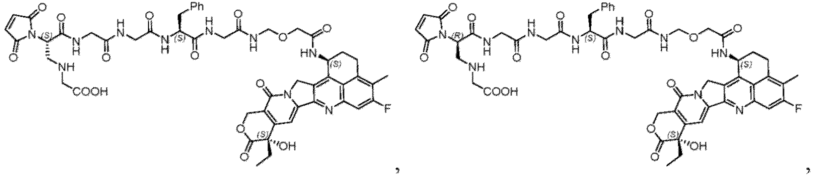 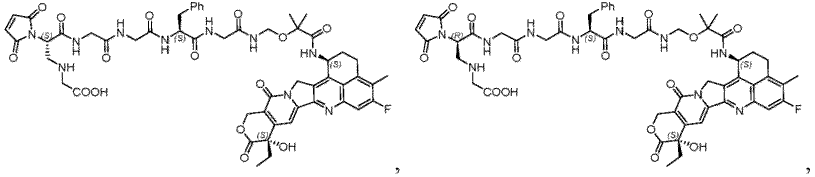 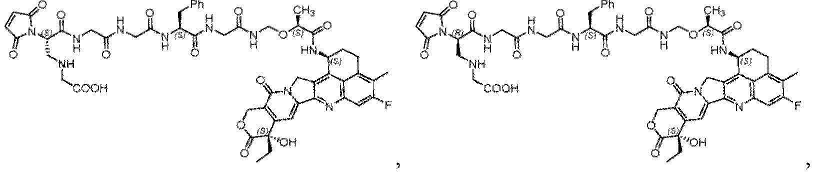 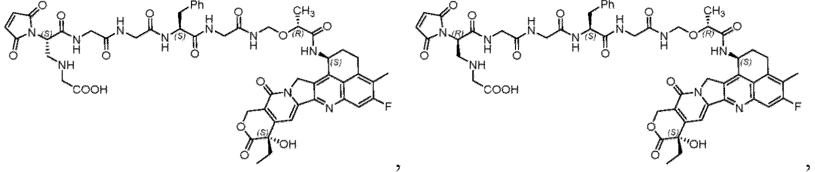 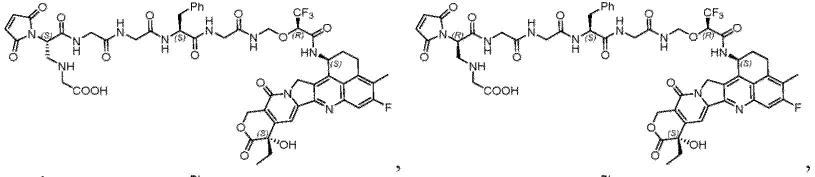 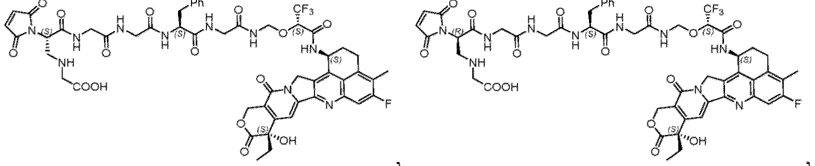 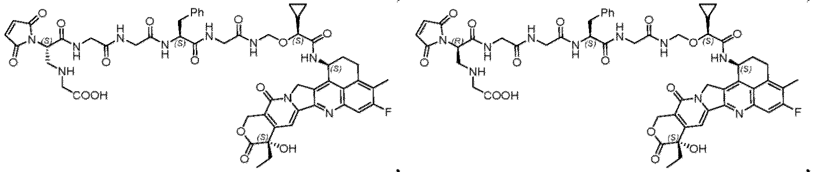 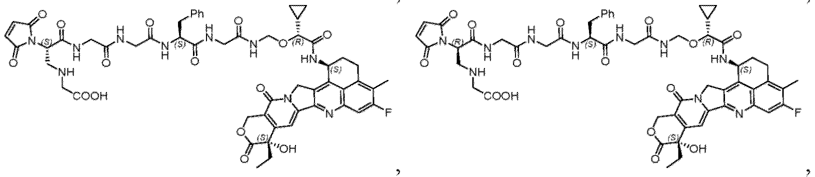 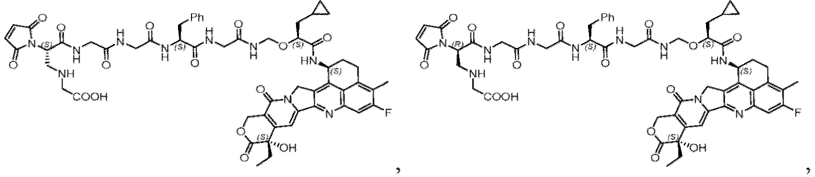 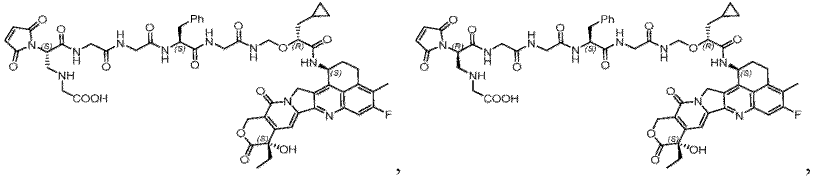 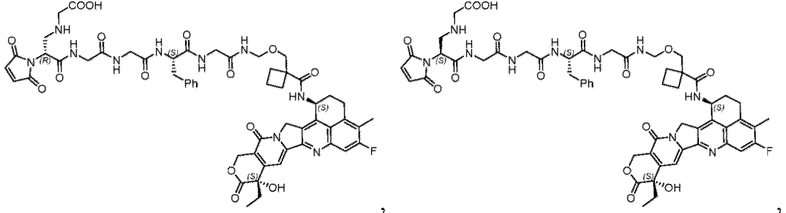 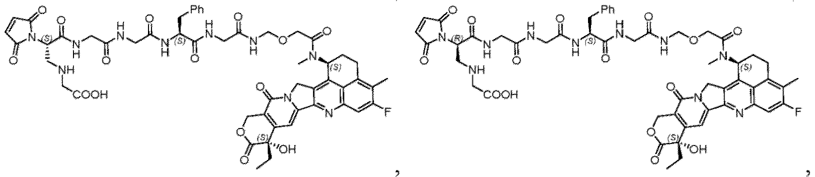 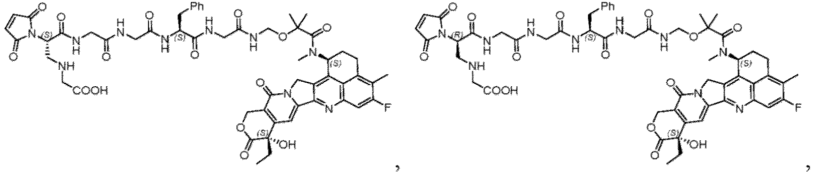 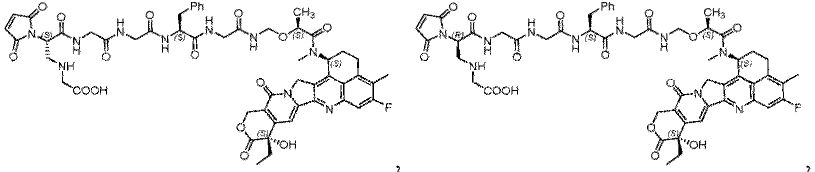 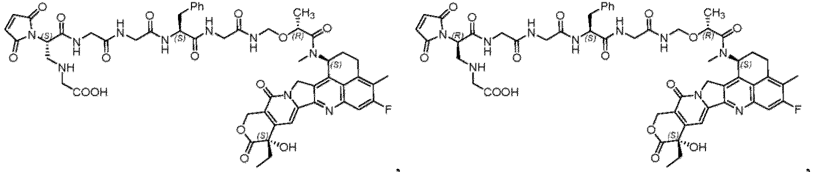 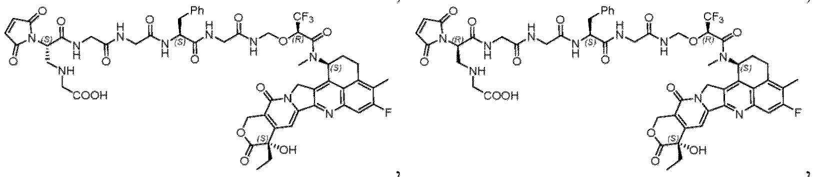 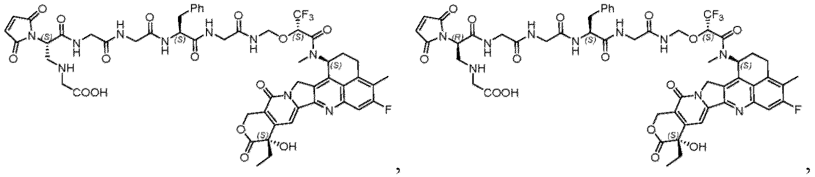 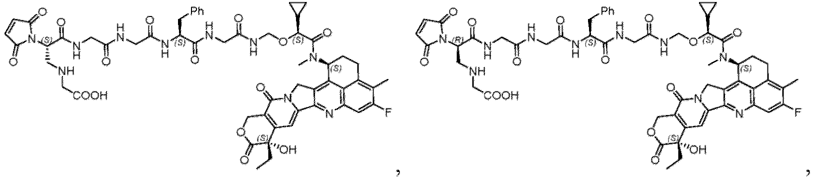 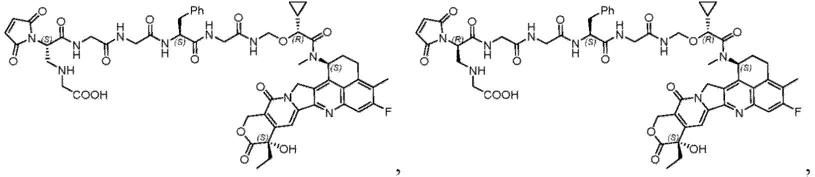 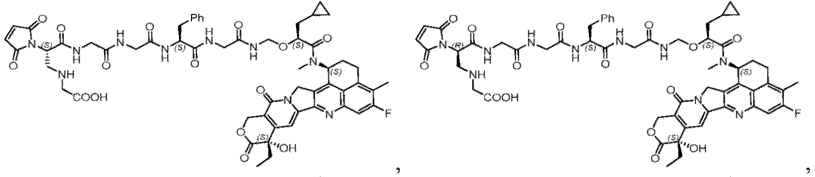 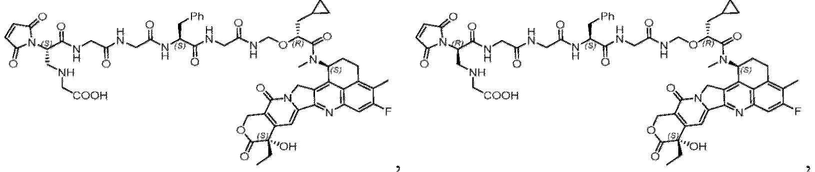 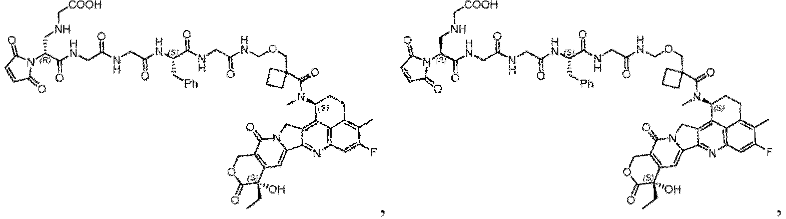 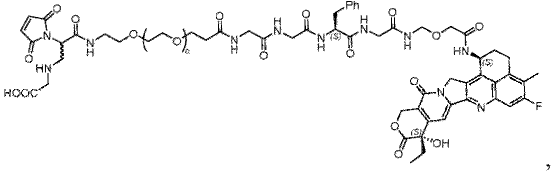 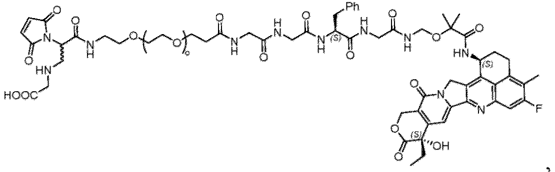 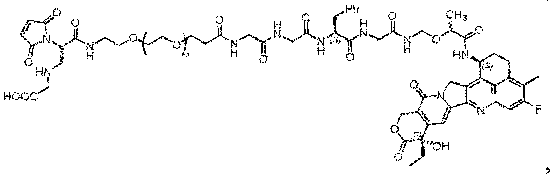 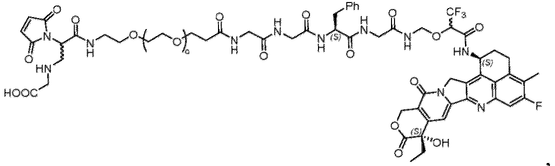 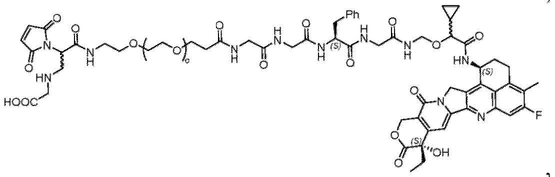 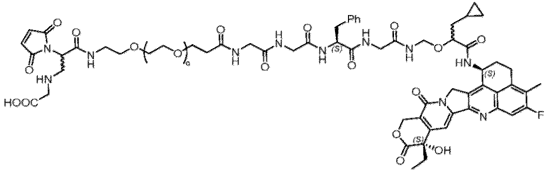 and 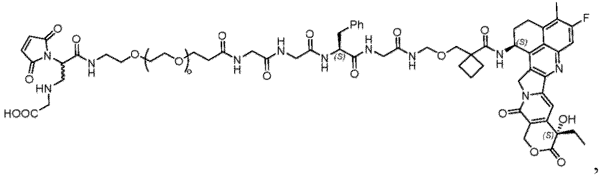 wherein o is selected from integers between 1 and 10.

31. A method for preparing the ligand-camptothecin derivative conjugate shown in the general formula **I** or in the general formula **II**, or a pharmaceutically acceptable salt or solvate, comprising the following steps, 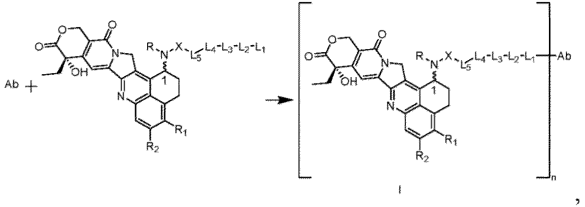 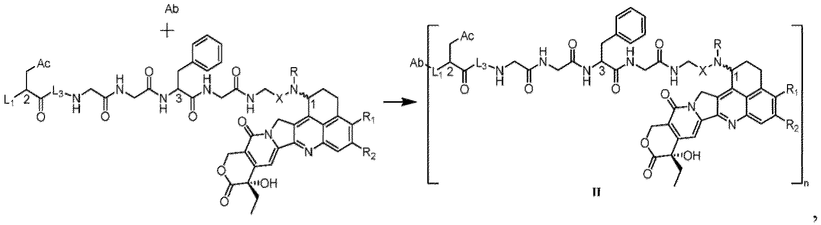
allowing a reduced antibody or an antigen-binding fragment thereof to have a coupling reaction with a linker-drug compound to obtain the ligand-camptothecin derivative conjugate shown in the general formula **I** or general formula **II**;
wherein 1-positionm 2-position or 3-position chiral carbon atom has an absolute chiral configuration R or S;
the Ab, L₁, L₂, L₃, L₄, L₅, X, R, R₁, R₂ and n are as defined in any one of claims 1-30.

32. The ligand-camptothecin derivative conjugate according to any one of claims 1-25, or a pharmaceutically acceptable salt or solvate thereof, or the method according to claim 31, wherein the ligand-camptothecin derivative conjugate or a pharmaceutically acceptable salt or solvate thereof is selected, without limitation, from the following structures, their derived structures in which rings in succinimide groups are in open form,and their isomers, 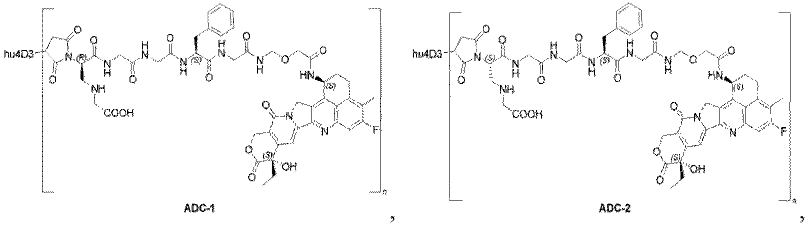 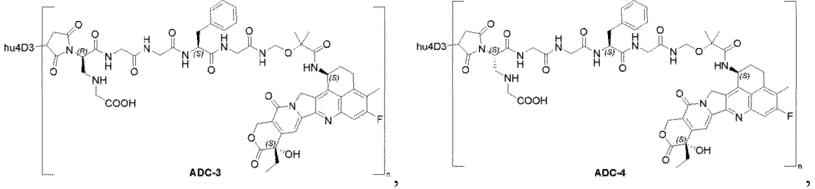 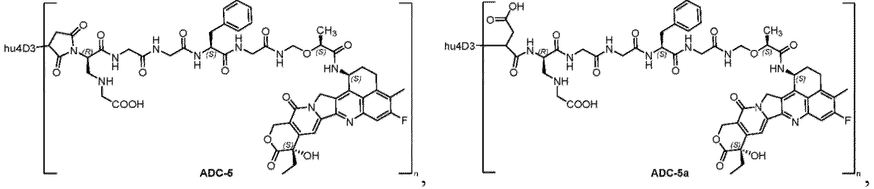 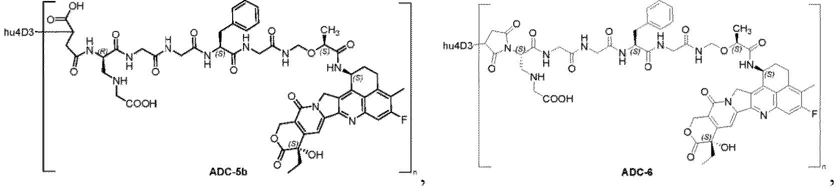 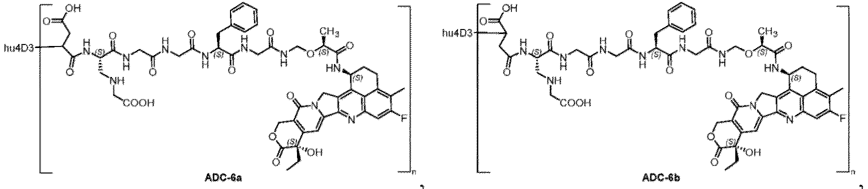 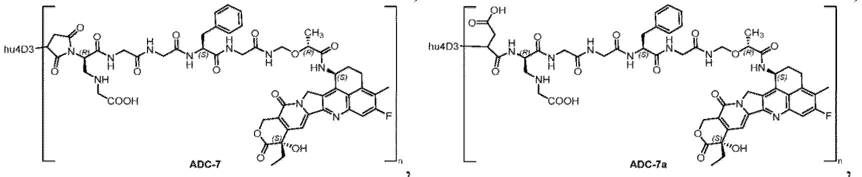 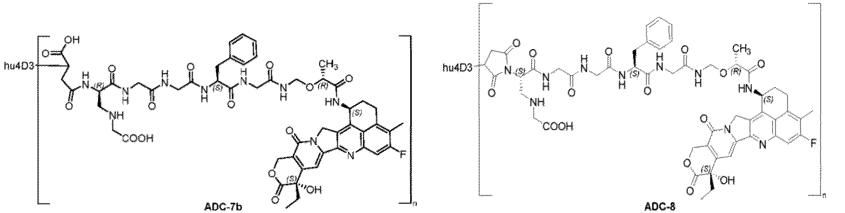 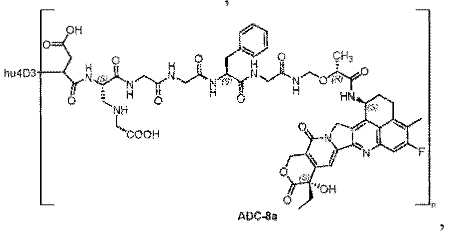 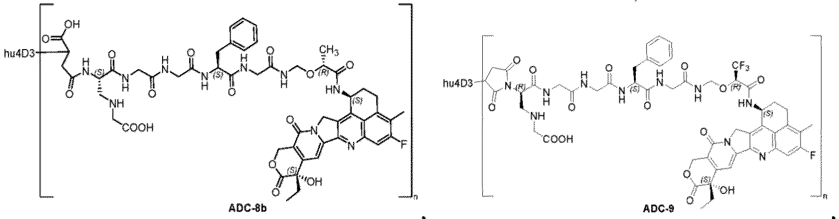 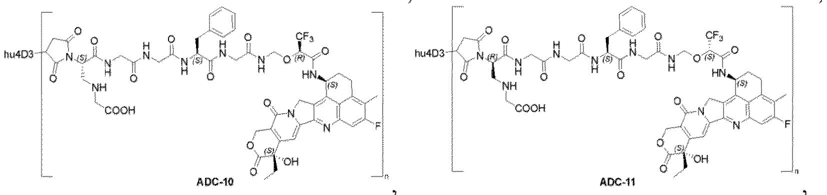 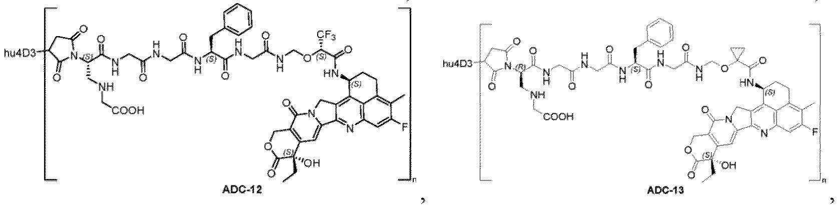 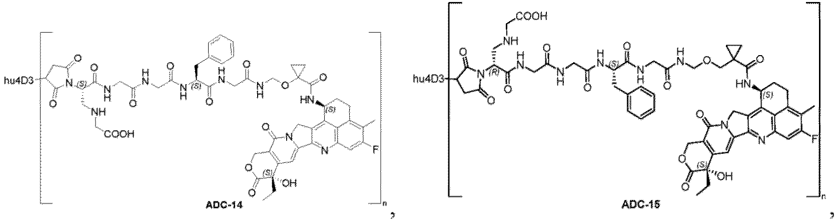 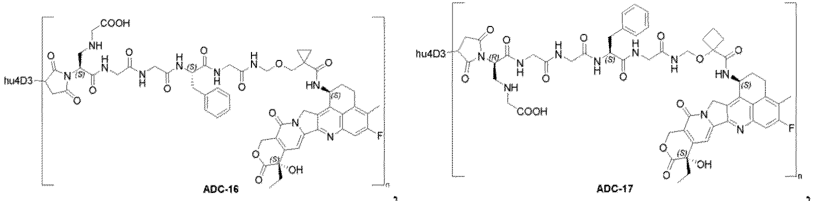 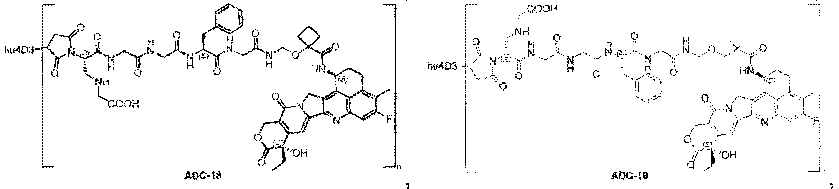 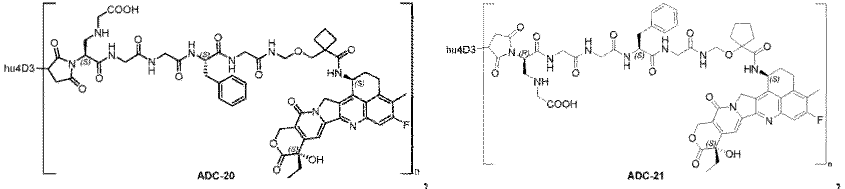 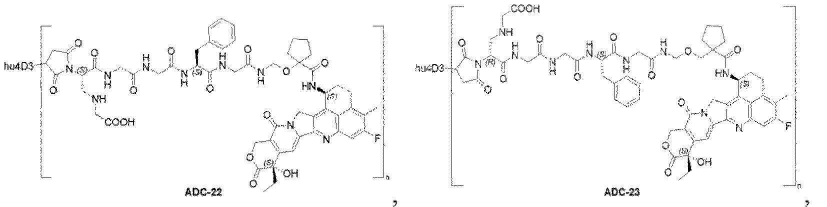 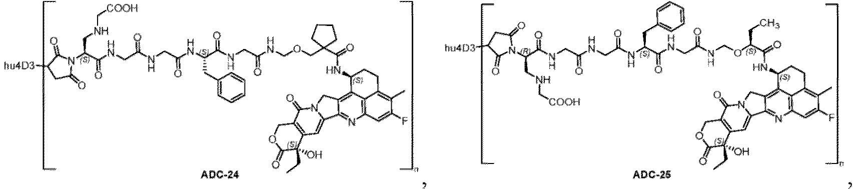 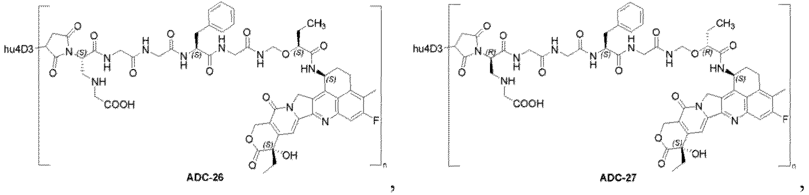 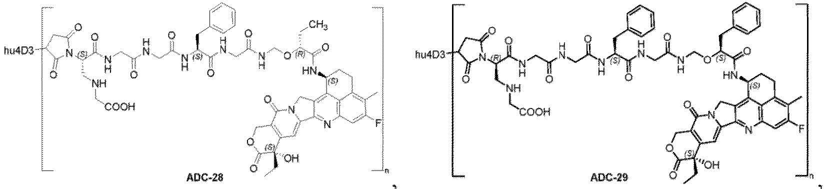 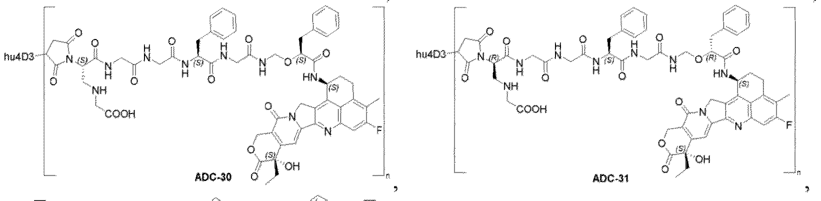 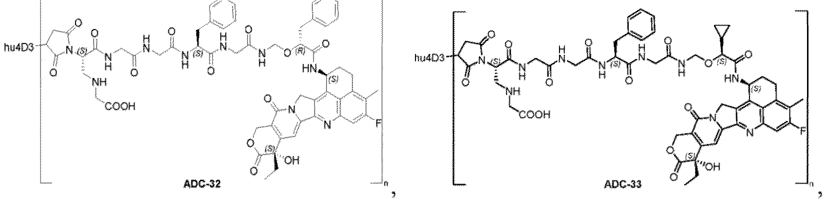 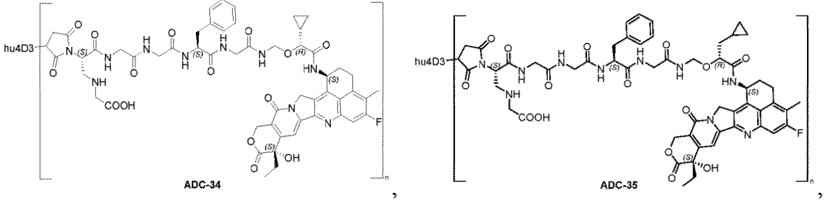 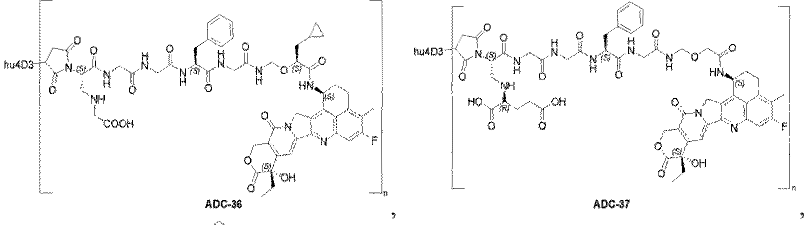 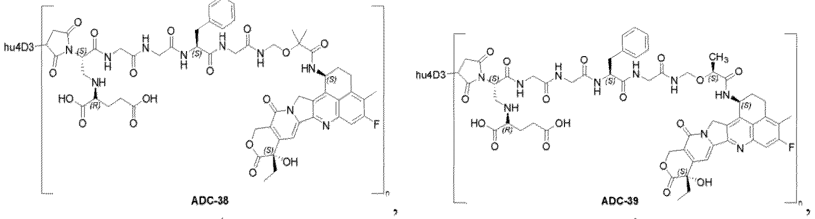 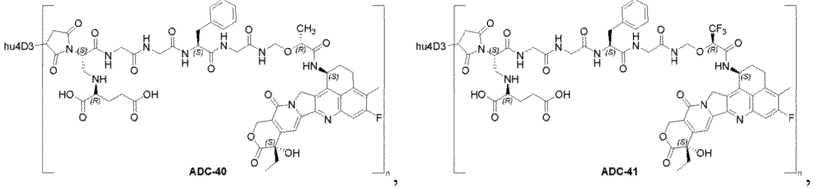 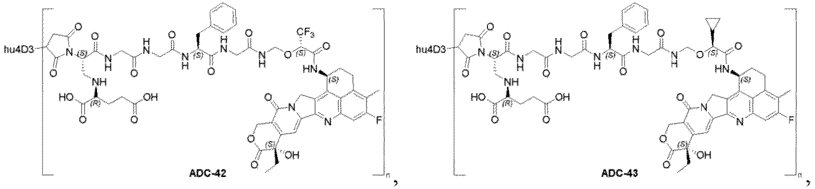 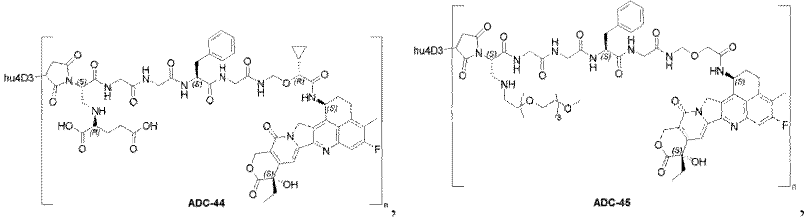 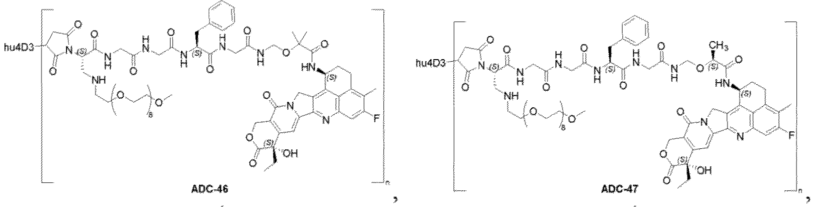 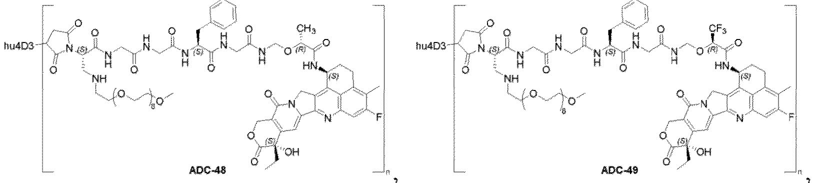 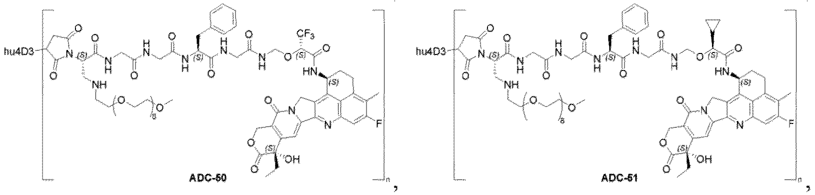 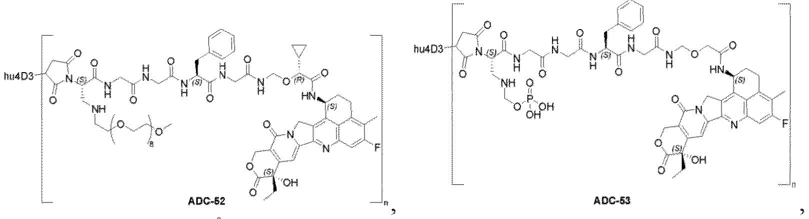 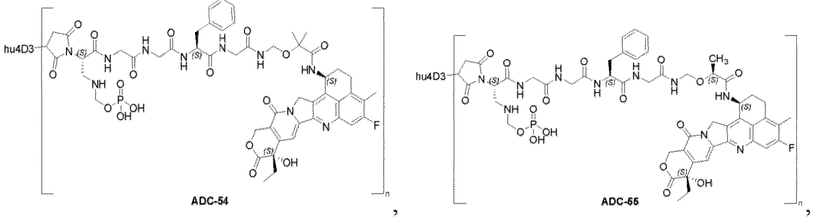 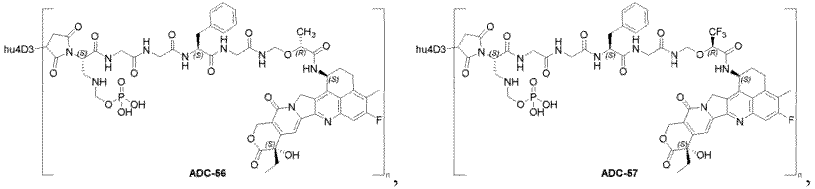 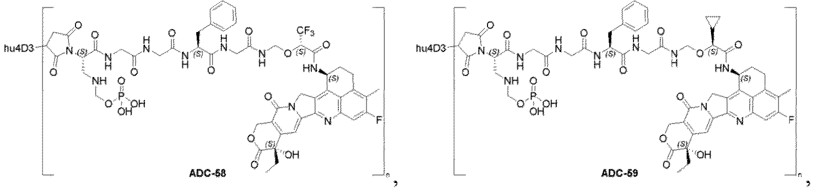 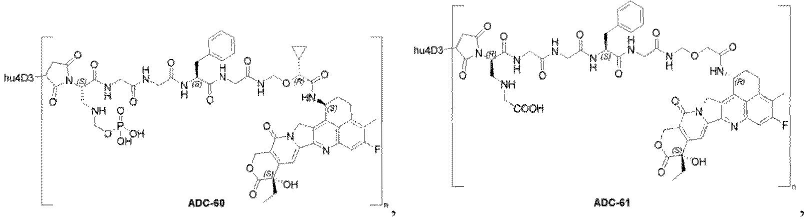 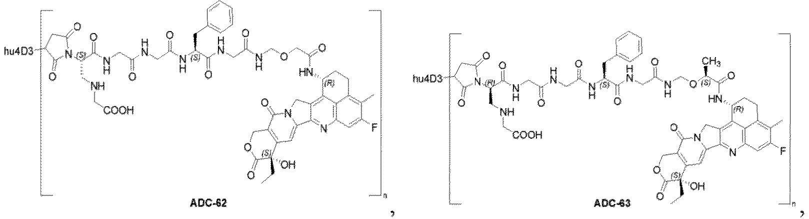 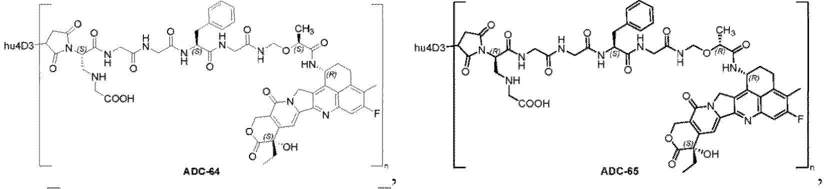 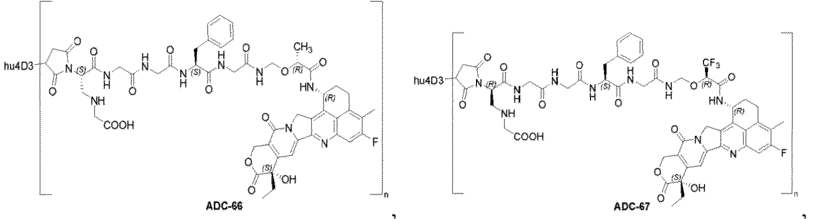 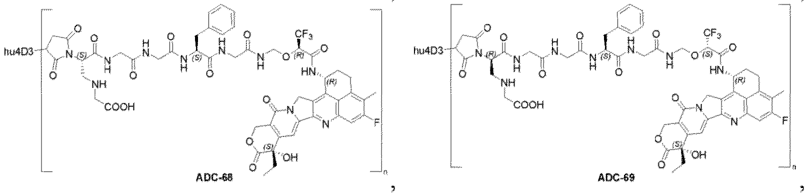 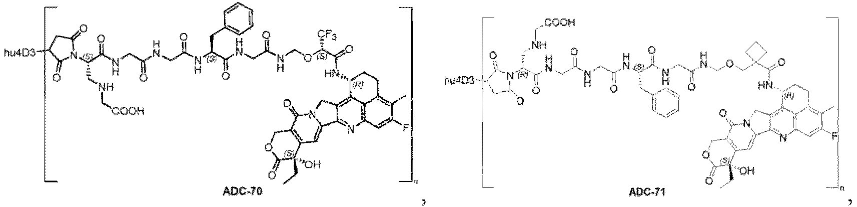 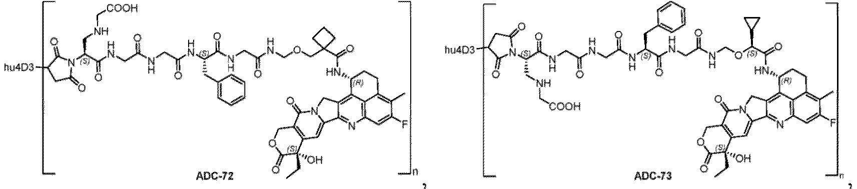 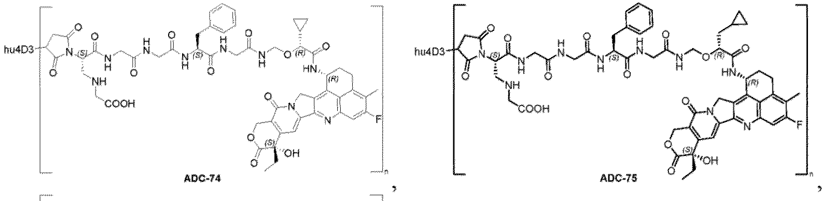 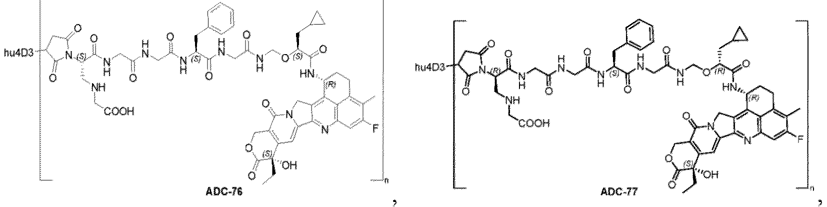 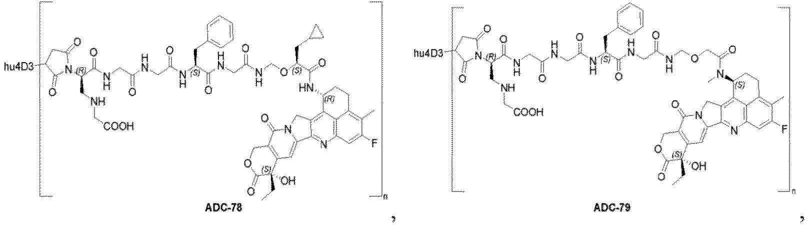 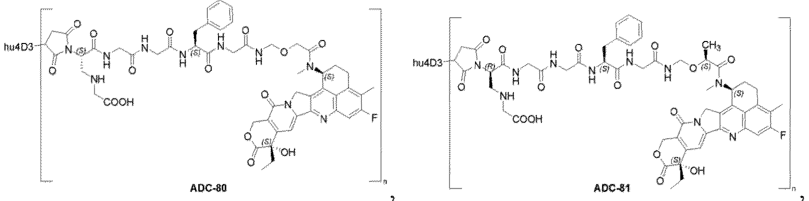 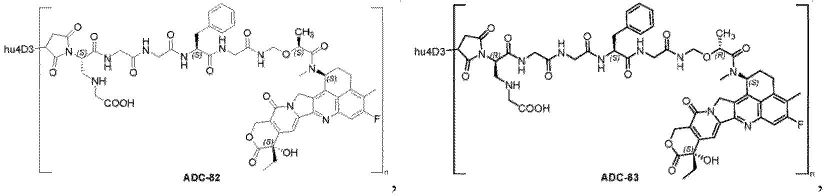 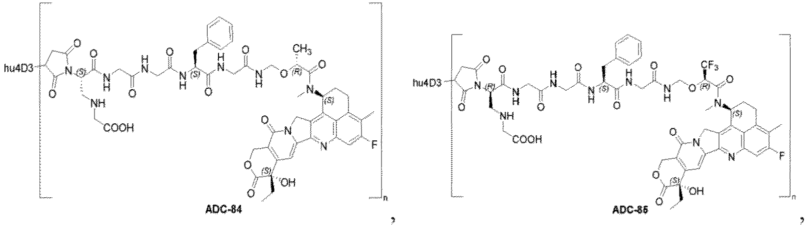 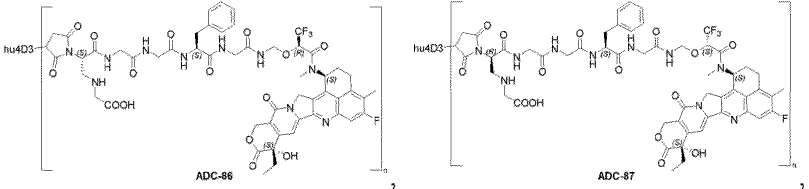 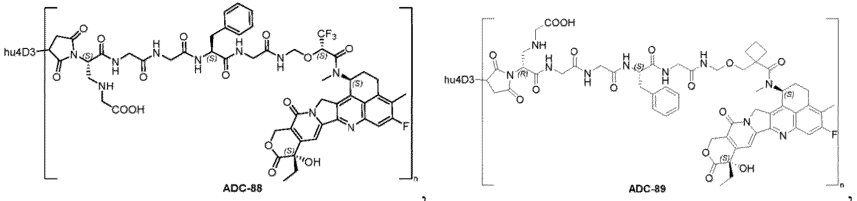 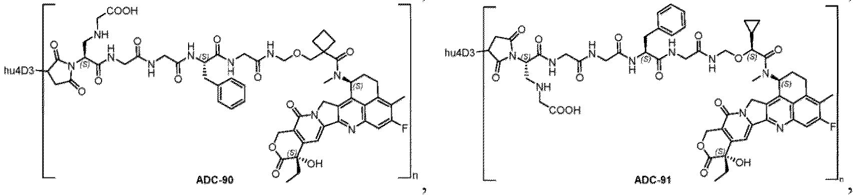 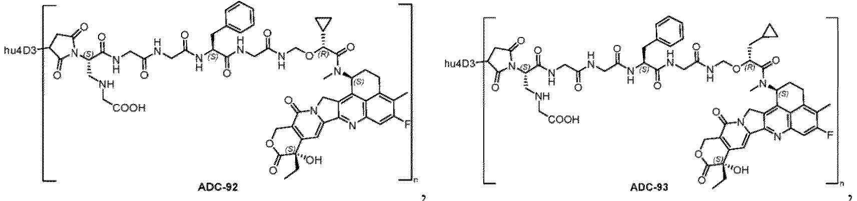 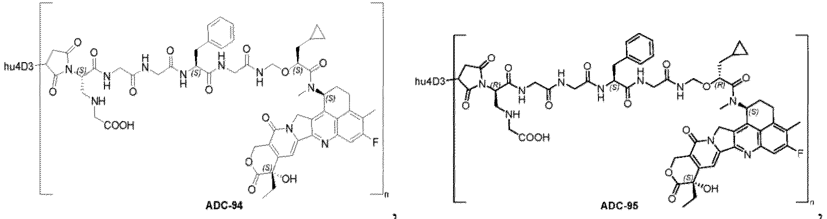 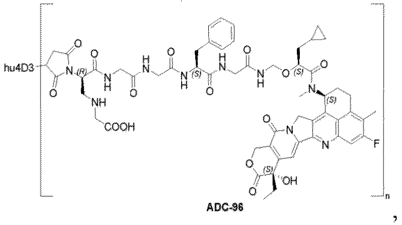 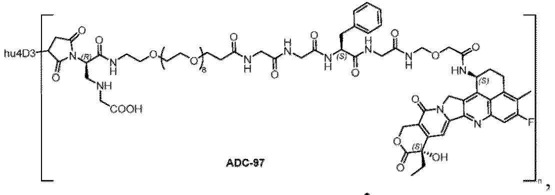 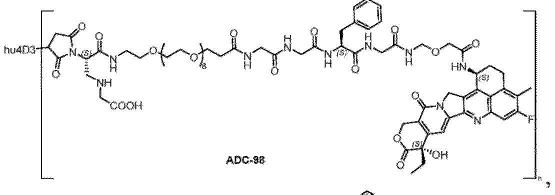 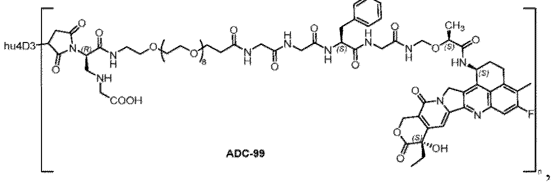 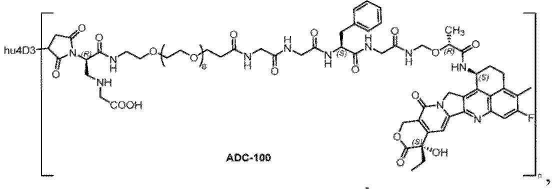 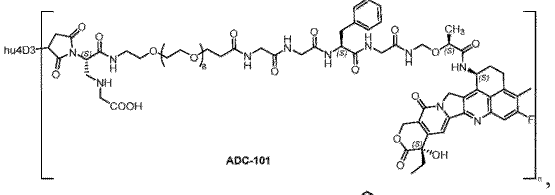 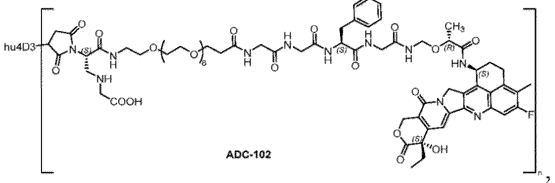 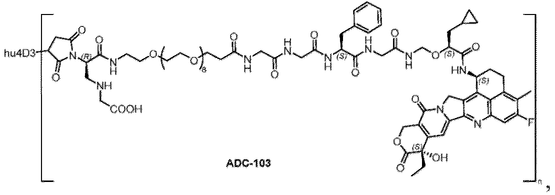 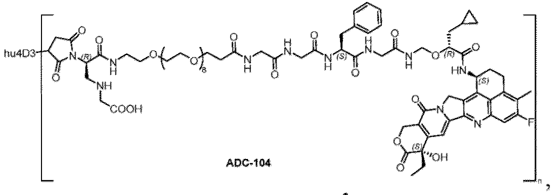 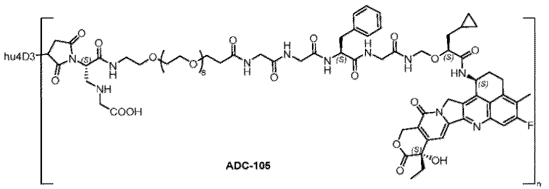 and 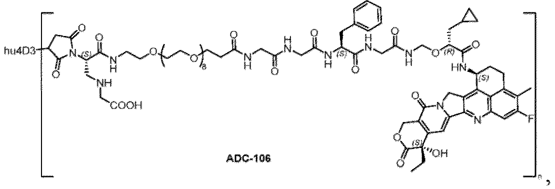 wherein
hu4D3 is an antibody targeting human Trop2 or an antigen-binding fragment thereof;
n is selected from integers between 1 and 10.

33. The ligand-camptothecin derivative conjugate according to any one of claims 1-25, or a pharmaceutically acceptable salt or solvate thereof or the method according to claim 31, wherein the ligand-camptothecin derivative conjugate or a pharmaceutically acceptable salt or solvate thereof is selected, without limitation, from the following structures, their derived structures in which rings in succinimide groups are in open form, and their isomers, 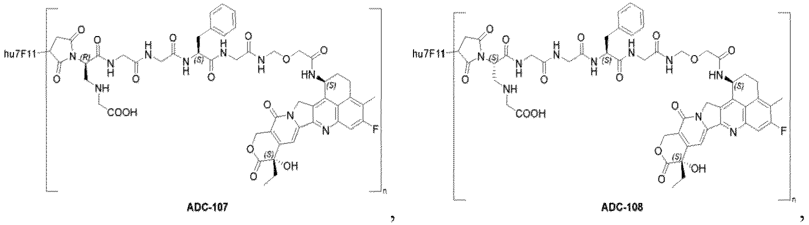 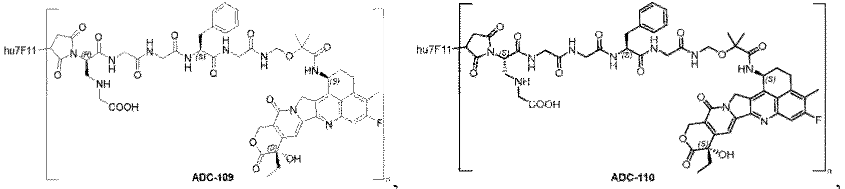 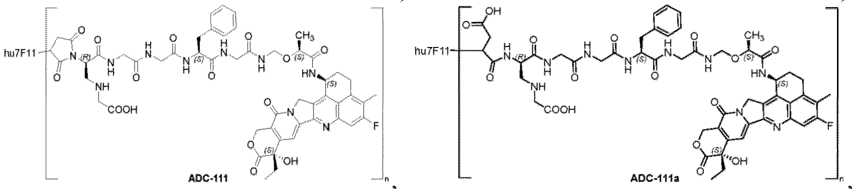 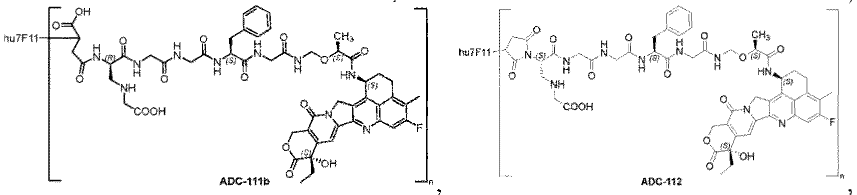 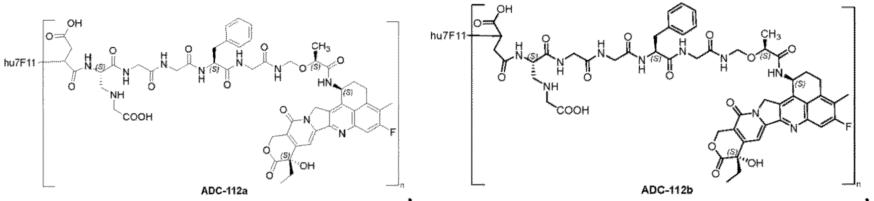 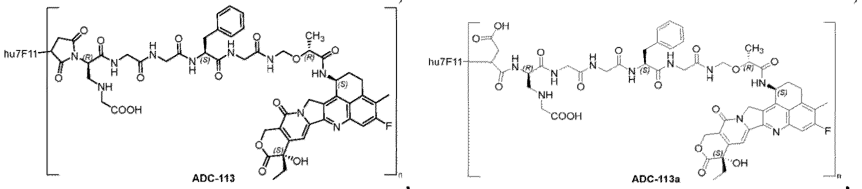 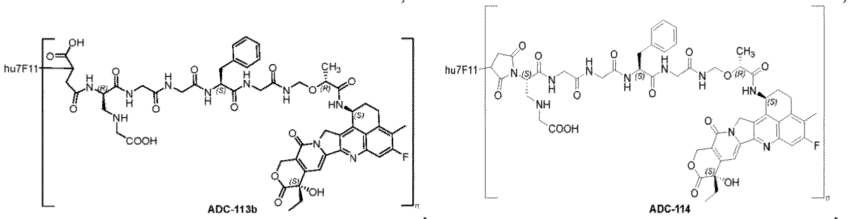 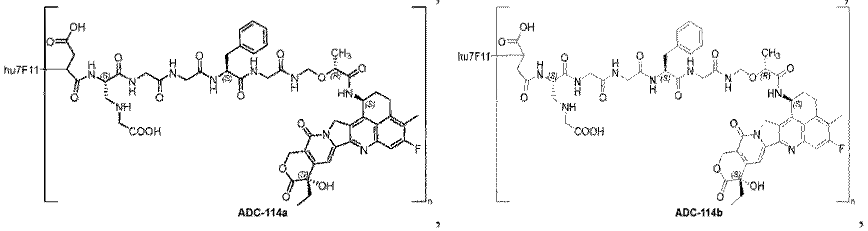 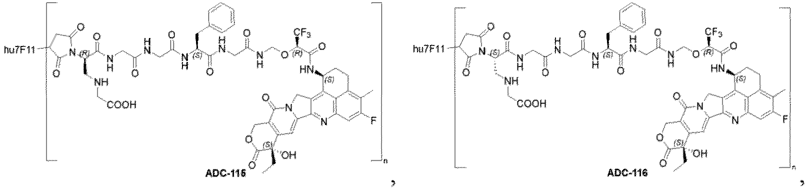 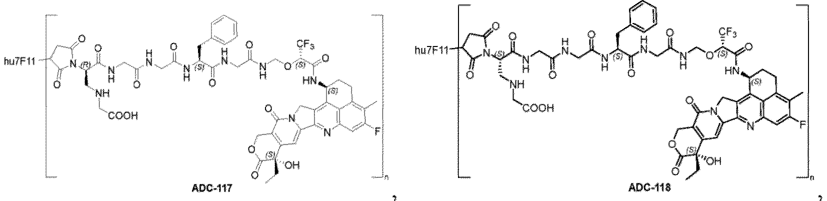 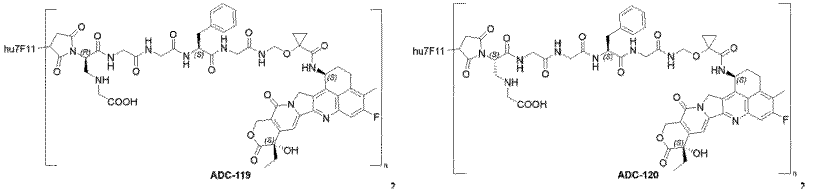 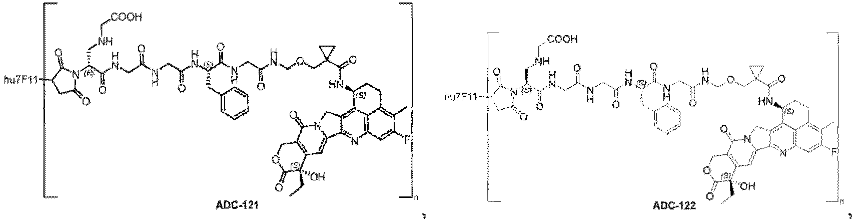 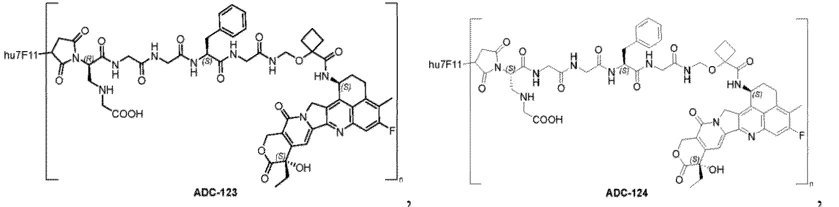 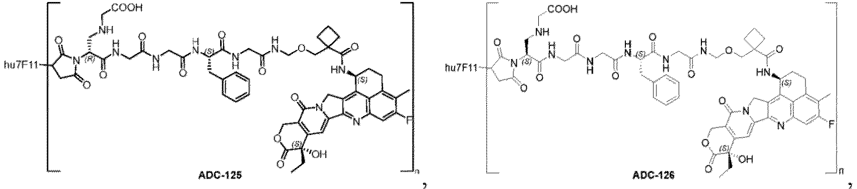 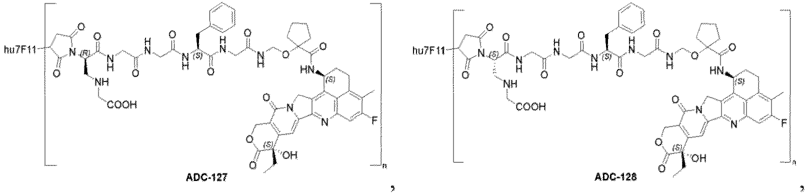 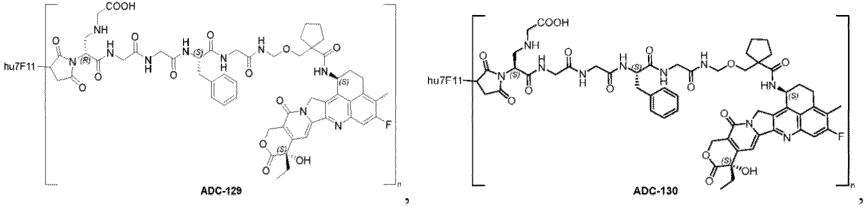 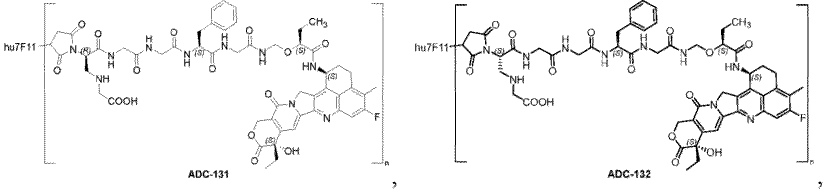 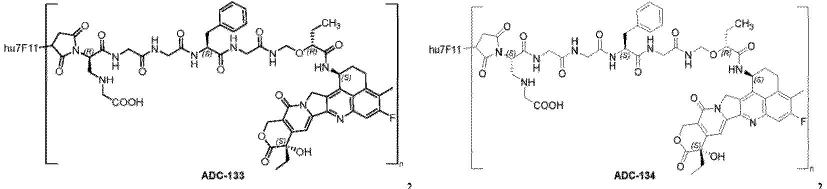 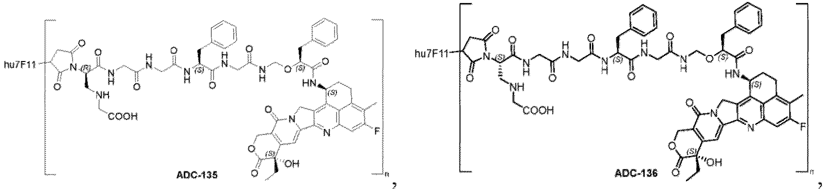 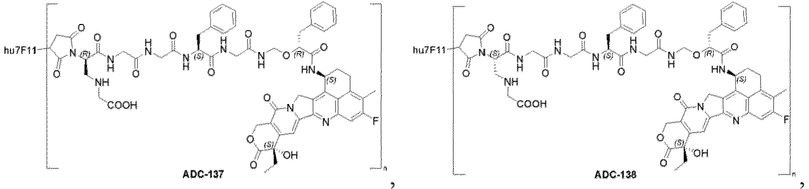 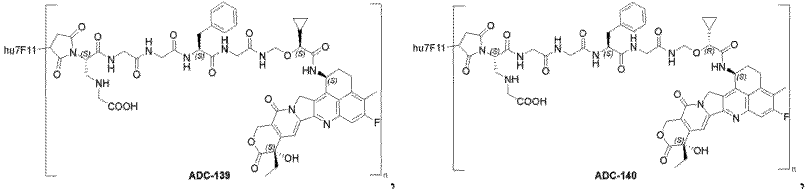 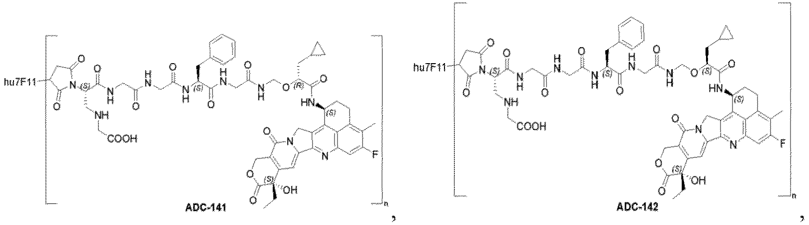 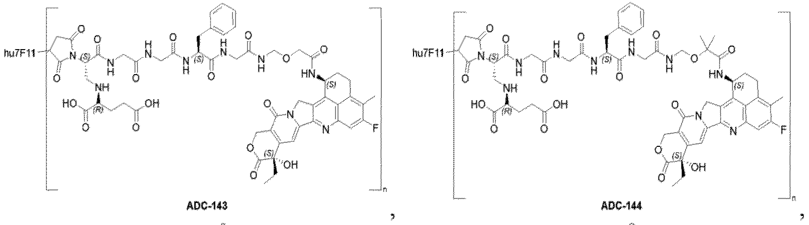 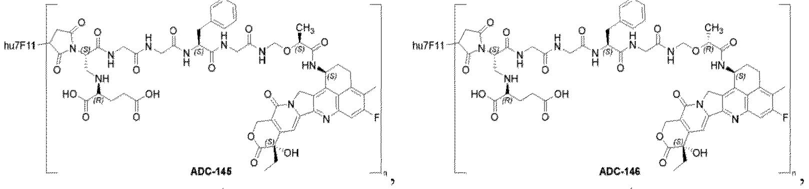 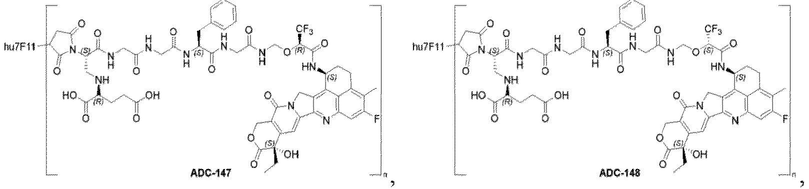 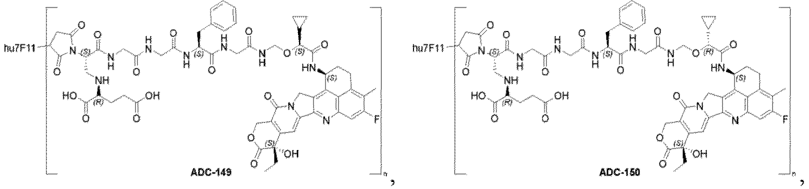 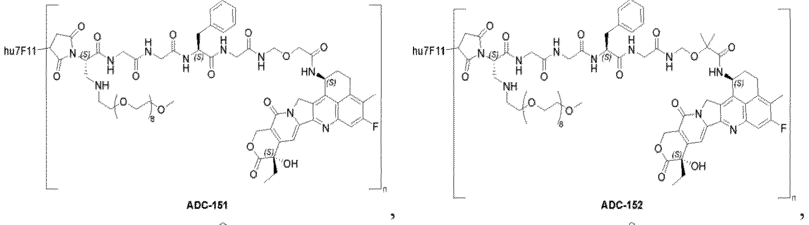 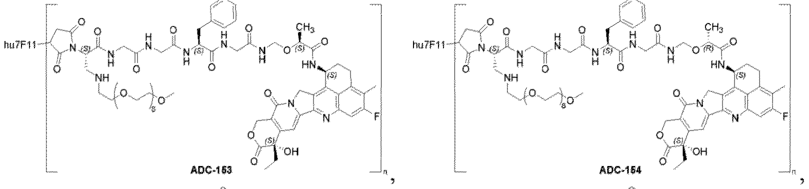 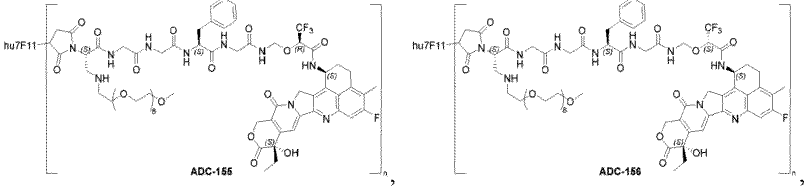 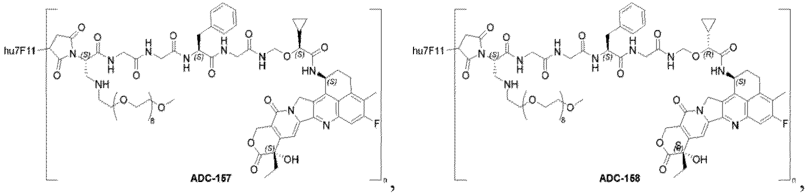 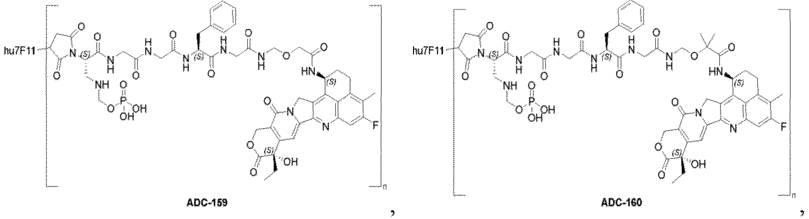 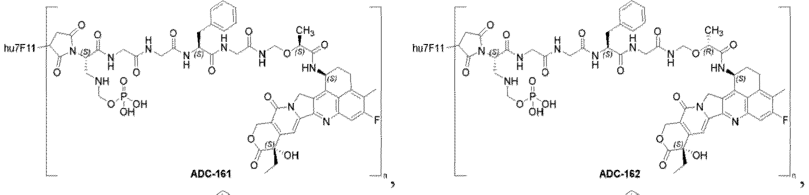 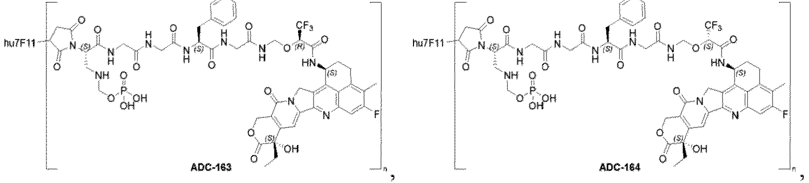 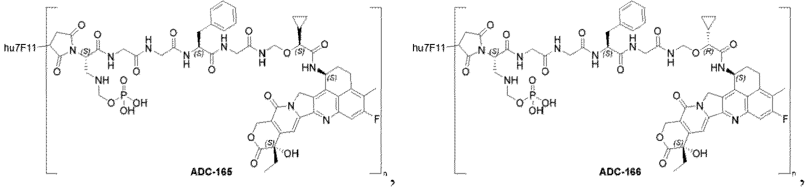 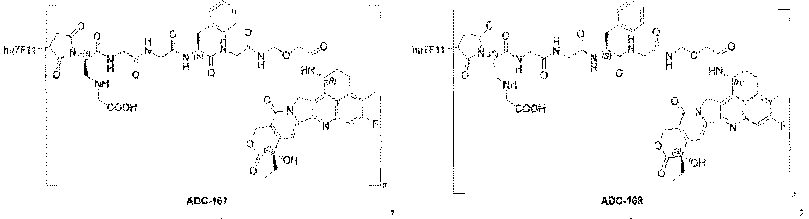 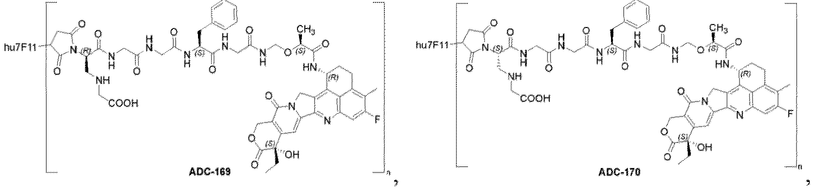 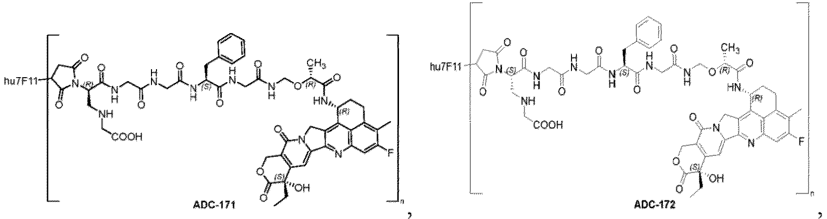 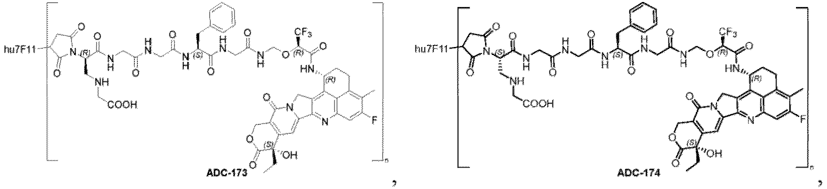 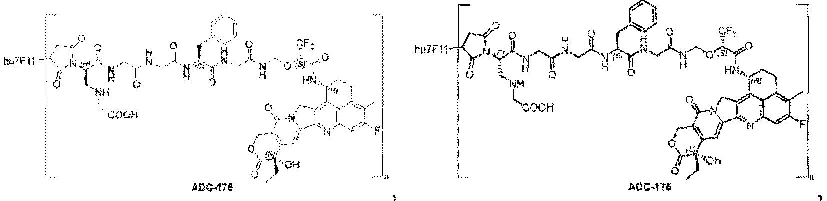 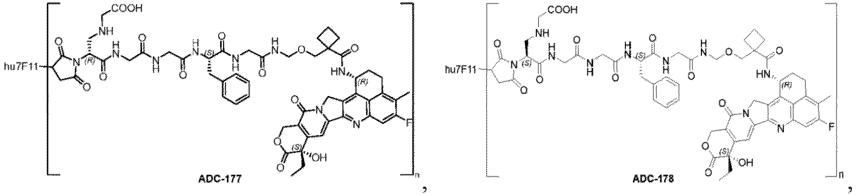 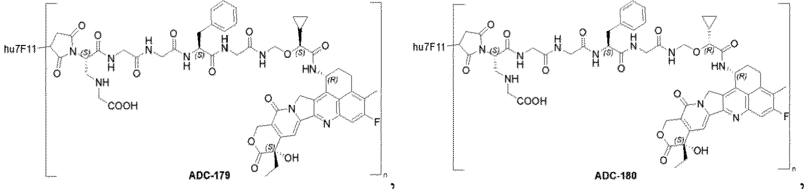 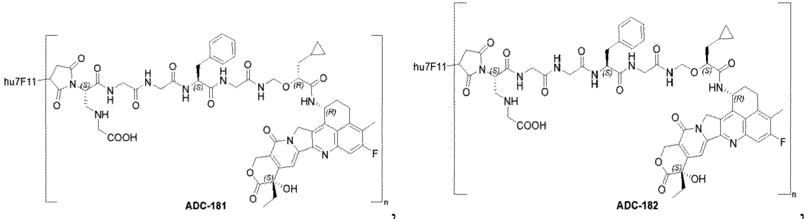 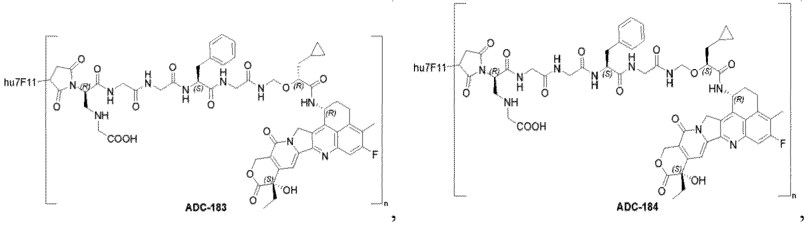 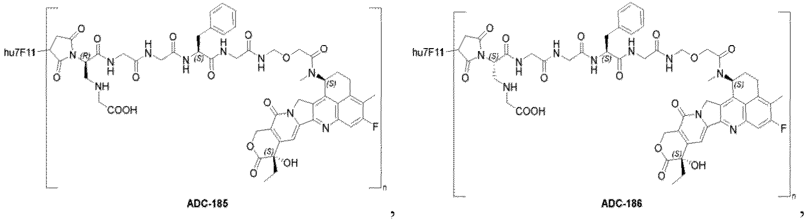 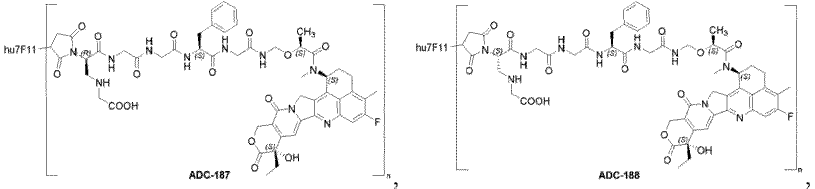 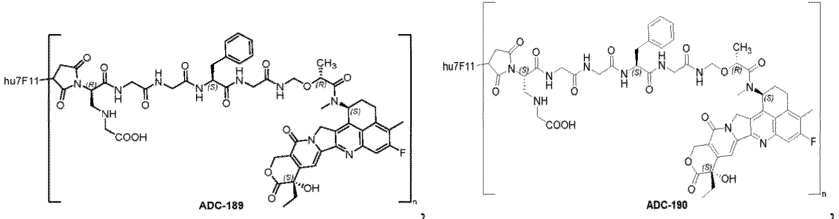 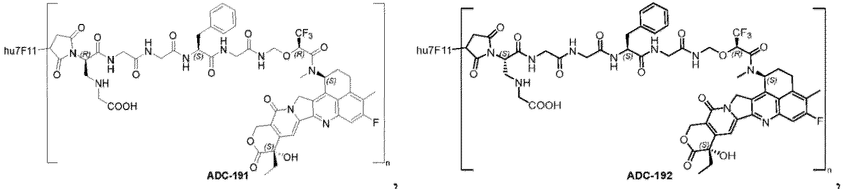 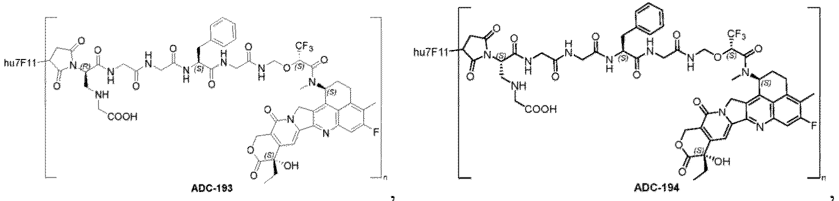 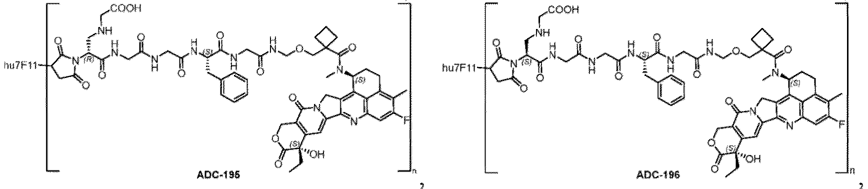 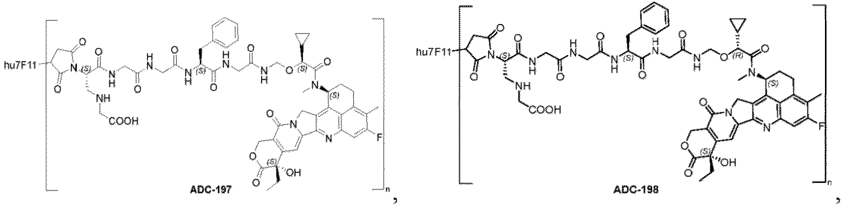 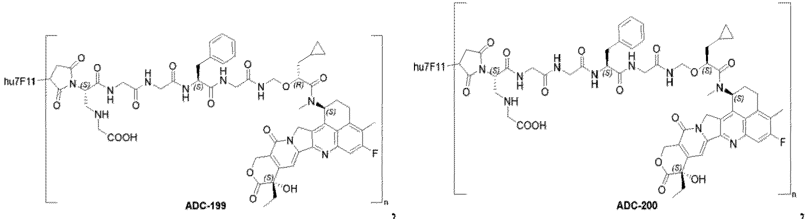 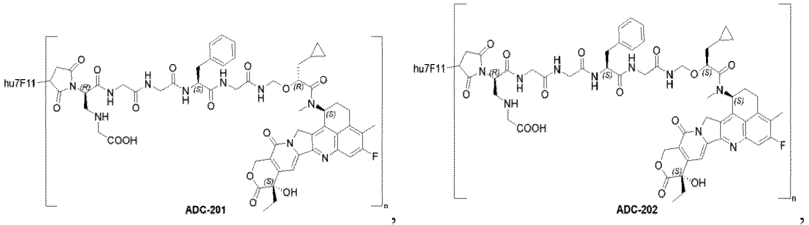 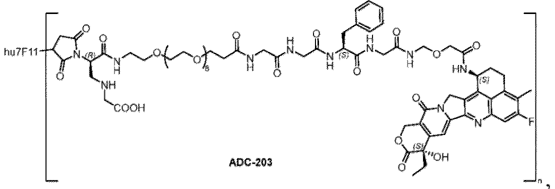 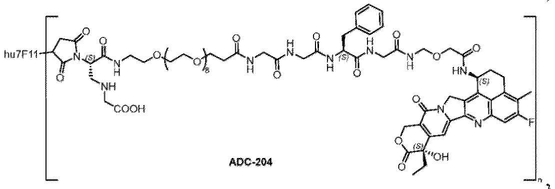 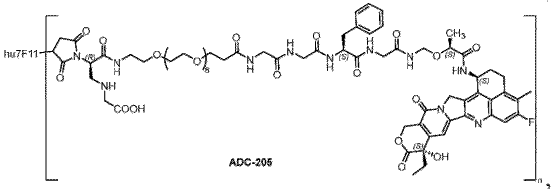 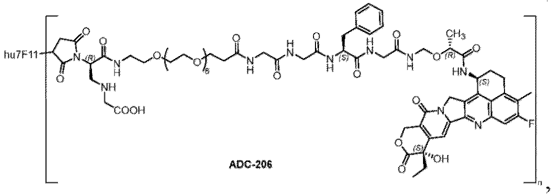 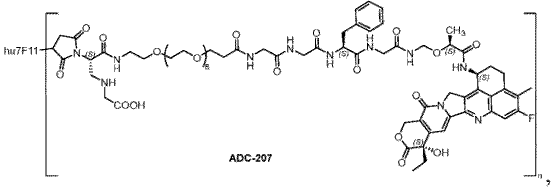 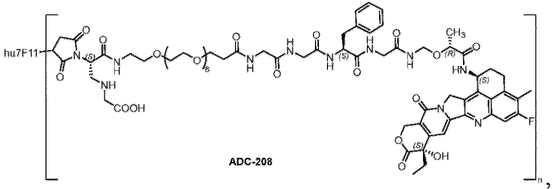 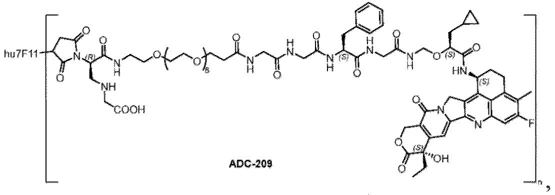 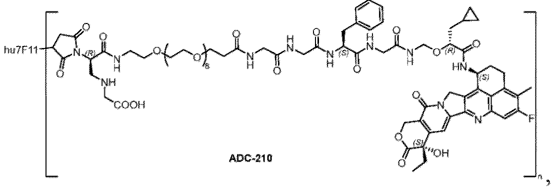 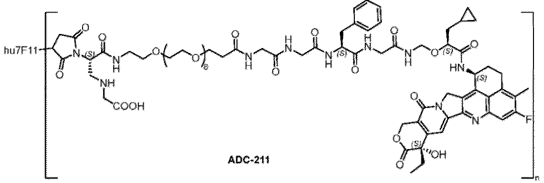 and 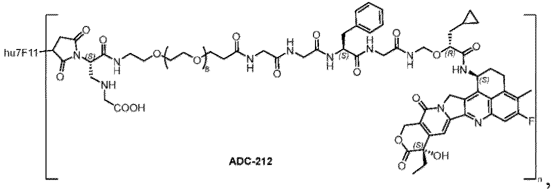 wherein
hu7F 11 is an antibody targeting human Trop2 or an antigen-binding fragment thereof;
n is selected from integers between 1 and 10.

34. The ligand-camptothecin derivative conjugate according to any one of claims 1-25, 32-33, or a pharmaceutically acceptable salt or solvate thereof or the linker-drug compound according to any one of claims 26-30, or a pharmaceutically acceptable salt or solvate thereof, wherein the pharmaceutically acceptable salt includes sodium salts, potassium salts, calcium salts or magnesium salts formed with acidic functional groups in the structural formula, and acetates, trifluoroacetates, citrates, oxalates, tartrates, malates, nitrates, chlorides, bromides, iodides, sulfates, bisulfates, phosphates, lactates, oleates, ascorbates, salicylates, formates, glutamates, methanesulfonates, ethanesulfonates, benzenesulfonates or p-toluenesulfonates formed with basic functional groups in the structure.

35. A pharmaceutical composition comprising the ligand-camptothecin derivative conjugate according to any one of claims 1-25 or 32-33, or a pharmaceutically acceptable salt or solvate thereof or the linker-drug compound according to any one of claims 26-30, or a pharmaceutically acceptable salt or solvate thereof, and optionally a pharmaceutically acceptable carrier.

36. A pharmaceutical formulation comprising the ligand-camptothecin derivative conjugate according to any one of claims 1-25 or 32-33, or a pharmaceutically acceptable salt or solvate thereof or the linker-drug compound according to any one of claims 26-30, or a pharmaceutically acceptable salt or solvate thereof.

37. Use of the ligand-camptothecin derivative conjugate according to any one of claims 1-25 or 32-33, or a pharmaceutically acceptable salt or solvate thereof, or the linker-drug compound according to any one of claims 26-32, or a pharmaceutically acceptable salt or solvate thereof, the pharmaceutical composition according to claim 35 and/or the pharmaceutical formulation according to claim 36 in preparation of a medicament for treating or preventing a cancer or tumor;
alternatively, the ligand-camptothecin derivative conjugate according to any one of claims 1-25 or 32-33, or a pharmaceutically acceptable salt or solvate thereof, or the linker-drug compound according to any one of claims 26-30, or a pharmaceutically acceptable salt or solvate thereof, the pharmaceutical composition according to claim 34 and/or the pharmaceutical formulation according to claim 36, for treating or preventing a cancer or tumor;
preferably, the cancer or tumor expresses TROP2;
more preferably, the cancer or tumor is selected from solid tumors or blood tumors, such as adenocarcinoma, ovarian cancer, cervical cancer, uterine cancer, prostate cancer, kidney cancer, urethra cancer, bladder cancer, liver cancer, gastric cancer, endometrial cancer, salivary gland cancer, esophageal cancer, lung cancer, colon cancer, triple negative breast cancer, rectal cancer, colorectal cancer, bone cancer, skin cancer, thyroid cancer, pancreatic cancer, melanoma, glioma, neuroblastoma, glioblastoma multiforme, sarcoma, lymphoma and leukemia.

38. A method for treating or preventing a cancer or tumor, including: administrating to a subj ect in need thereof a prophylactically or therapeutically effective amount of the ligand-camptothecin derivative conjugate according to any one of claims 1-25 or 32-33, or a pharmaceutically acceptable salt or solvate thereof, or the linker-drug compound according to any one of claims 26-30, or a pharmaceutically acceptable salt or solvate thereof, or the pharmaceutical composition according to claim 35 and/or the pharmaceutical formulation according to claim 36;
preferably, the cancer or tumor expresses TROP2;
more preferably, the cancer or tumor is selected from solid tumors or blood tumors, such as adenocarcinoma, ovarian cancer, cervical cancer, uterine cancer, prostate cancer, kidney cancer, urethra cancer, bladder cancer, liver cancer, gastric cancer, endometrial cancer, salivary gland cancer, esophageal cancer, lung cancer, colon cancer, triple negative breast cancer, rectal cancer, colorectal cancer, bone cancer, skin cancer, thyroid cancer, pancreatic cancer, melanoma, glioma, neuroblastoma, glioblastoma multiforme, sarcoma, lymphoma and leukemia.
